# EUROPEAN PATENT APPLICATION

(11) **EP 4 198 163 A1**
(43) Date of publication of application: **21.06.2023**
(21) Application number: 21855986.2
(22) Date of filing: 11.08.2021
(51) Int. Cl.: C23C 14/12, C07C 211/56, C23C 14/04, H01G 9/20, H01L 51/50, H05B 33/26

(54) **METAL PATTERNING MATERIAL, AMINE COMPOUND, ELECTRONIC DEVICE, AND METHOD FOR FORMING METAL PATTERN**

(30) Priority: 12.08.2020 JP 2020136094; 15.02.2021 JP 2021021921; 06.07.2021 JP 2021112394
(71) Applicant: Tosoh Corporation, Yamaguchi 746-8501 (JP)
(72) Inventor: MATSUMOTO, Naoki, Ayase-Shi, Kanagawa 252-1123 (JP); KAWASHIMA, Hiroyuki, Ayase-Shi, Kanagawa 252-1123 (JP); NOMURA, Shintaro, Ayase-Shi, Kanagawa 252-1123 (JP)
(74) Representative: TBK
(86) International application number: PCT/JP2021/029689
(87) International publication number: WO 2022/034907

(57) **Abstract**

The present invention provides: a metal patterning material which exhibits excellent heat resistance and is suppressed in the formation of a metal thin film on a film surface; an amine compound; a method for forming a metal pattern using these; and an electronic device. The present invention uses a metal patterning material that contains a compound which comprises, in each molecule, an aromatic ring and/or a heteroaromatic ring, a fluorine atom and at least one tertiary amine,
wherein:
the aromatic ring is composed of at least one ring selected from the group consisting of a monocyclic aromatic ring, a linked aromatic ring and a fussed aromatic ring having from 6 to 15 carbon atoms;
a fused aromatic ring having 16 or more carbon atoms is not contained;
the proportion of carbon atoms directly bonded to a fluorine atom among the carbon atoms that form the aromatic ring and the carbon atoms that form the heteroaromatic ring is 10% or more;
the molecular weight is from 500 to 3,000; and
the glass transition temperature is 60°C or higher.

## Description

### TECHNICAL FIELD

The present disclosure relates to a metal patterning material, amine compound, electronic device, and a method for forming metal patterns.

### BACKGROUND ART

In recent years, organic electronic devices such as organic electroluminescence (EL) elements, organic thin film solar cell, organic transistors, organic sensors have widely developed. With organic electronic devices use metal thin films as the electrodes; however, it is necessary to pattern the metal thin film into the desired shapes.

As the patterning method of metal electrodes, a method has been known which patterning forms the metal patterning material for which attachment of metal is suppressed as the base layer, and deposits the metal on this base layer. With this method, since the metal film is selectively formed on a portion on which the metal patterning material is not formed, it is possible to form a metal electrode patterned into the desired shape.

As the metal patterning material, Patent Document 1 discloses 1,2-diarylethene derivatives. According to Patent Document 1, the method using 1,2-diarylethene derivatives can form metal patterns in free shapes and high precision. In addition, as the metal patterning material, Non-Patent Document 1 discloses 1H,1H,2H,2H-perfluorooctyltricholorosilane (hereinafter abbreviated as FTS) .
Patent Document 1: Japanese Unexamined Patent Application, Publication No.2007-188854
Non-Patent Document 1: Materials Horizons, 2020, Vol. 7, p. 143

### DISCLOSURE OF THE INVENTION

### Problems to be Solved by the Invention

However, in the case of the 1,2-diarylethene derivative according to Patent Document 1, the adhesive property of metal to 1,2-diarylethene derivative depends on the glass transition temperature of the material. Therefore, in order to suppress metal adhesion using 1,2-diarylethene derivative, it is necessary to use a material of low glass transition temperature. However, a material having low glass transition temperature cannot be used in applications in which heat resistance is demanded due to problems of liquefaction or crystallization of the organic material thin film. In addition, FTS according to Non-Patent Document 1 has a boiling point in the 80°C range, and low glass transition temperature.

Therefore, an aspect of the present disclosure is directed at providing a metal patterning material having excellent heat resistance and suppressing the formation of a metal thin film on a film surface, an amine compound, a metal patterning thin film using these materials, an organic electroluminescence element, a method for forming a metal pattern, and an electronic device.

### Means for Solving the Problems

An aspect of the present disclosure provide a metal patterning material containing a compound having, in a molecule,
an aromatic ring and/or heteroaromatic ring, a fluorine atom, and at least one tertiary amine,
in which the aromatic ring is at least one selected from the group consisting of a monocyclic aromatic ring, a linked aromatic ring, and a condensed aromatic ring having 6 or more and 15 or less carbon atoms,
and is free of condensed aromatic rings having 16 or more carbon atoms,
a proportion of carbon atom number directly binding with the fluorine atom among a carbon atom number forming the aromatic ring and a carbon atom number forming the heteroaromatic ring is at least 10%,
molecular weight is at least 500 and no more than 3,000, and a glass transition temperature is 60°C or higher.

Another aspect of the present disclosure provides an amine compound represented by Formula (7) or (8), in which

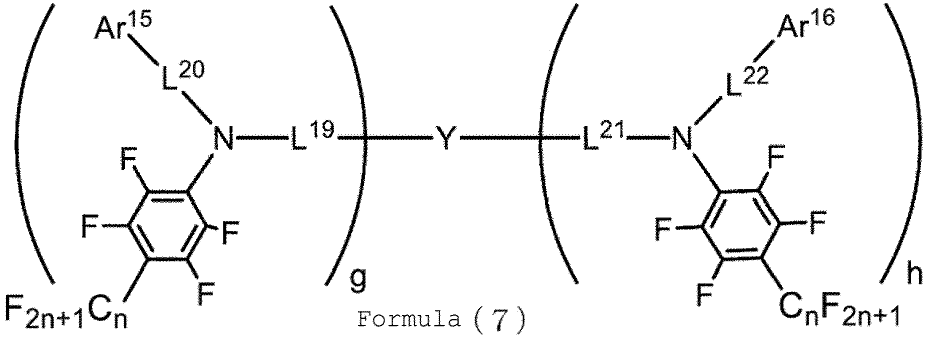

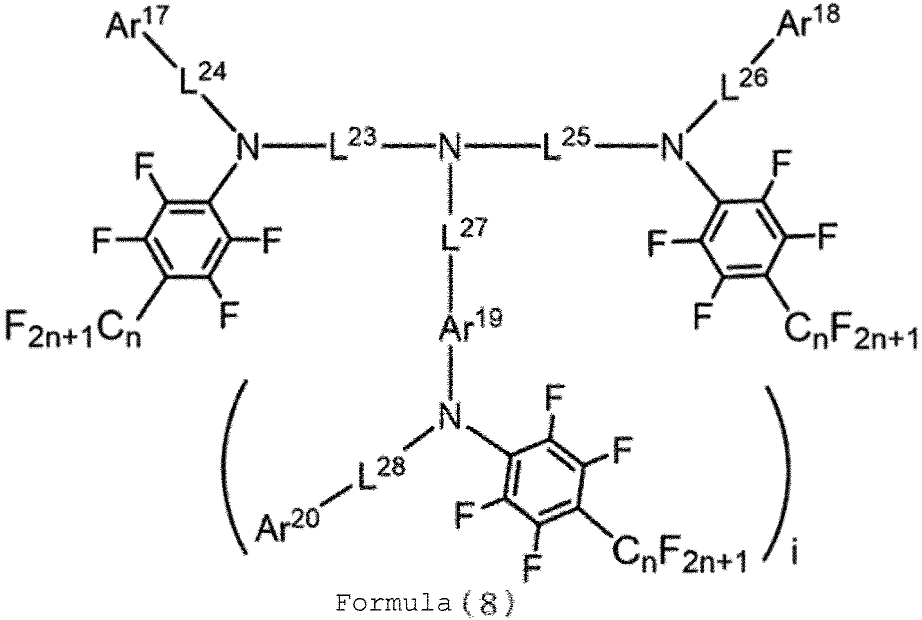

Ar¹⁵ to Ar²⁰ each independently represent
a monocyclic, linked or condensed aromatic hydrocarbon group having 6 to 25 carbon atoms which may be substituted, or
a monocyclic, linked or condensed heteroaromatic group having 3 to 25 carbon atoms which may be substituted;
L¹⁹ to L²⁸ each independently represent
a monocyclic, linked or condensed divalent aromatic hydrocarbon group having 6 to 25 carbon atoms which may be substituted,
a monocyclic, linked or condensed divalent heteroaromatic group having 3 to 25 carbon atoms which may be substituted, or
a single bond;
Y represents
a monocyclic, linked or condensed divalent to tetravalent aromatic hydrocarbon group having 6 to 25 carbon atoms which may be substituted,
a monocyclic, linked or condensed divalent to tetravalent heteroaromatic group having 3 to 25 carbon atoms which may be substituted,
a linear, branched or cyclic divalent to tetravalent alkyl group having 1 to 10 carbon atoms which may be substituted, or
a divalent to tetravalent silicon atom which may be substituted;
n represents an integer of 1 to 18;
g and h each independently represents an integer of 1 or 2; and
i represents an integer of 0 or 1.

Another aspect of the present disclosure provides an amine compound represented by Formula (9) or (10), in which 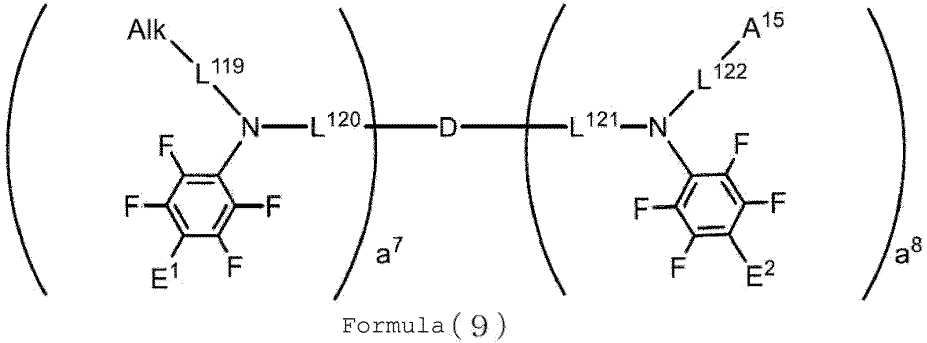 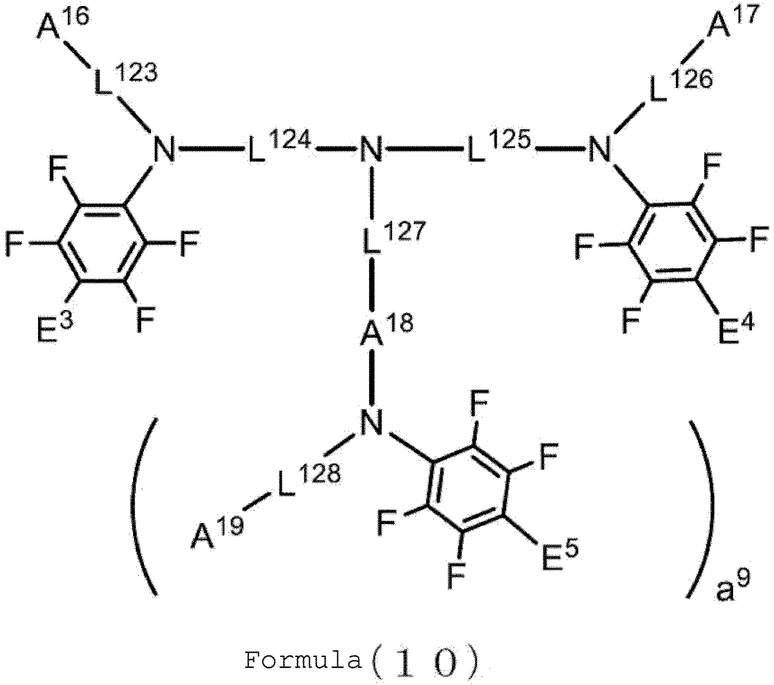 a⁷ represents an integer of 1 or 2;
a⁸ represents an integer of 0 to 2;
a⁹ represents an integer of 0 or 1;
Alk represents a cyclic alkyl group having 3 to 25 carbon atoms which may be substituted;
A¹⁵ to A¹⁹ each independently represent
a monocyclic, linked or condensed aromatic hydrocarbon group having 6 to 25 carbon atoms which may be substituted,
a monocyclic, linked or condensed heteroaromatic group having 3 to 25 carbon atoms which may be substituted, or
a cyclic alkyl group having 3 to 25 carbon atoms which may be substituted;
provided that
when a⁹ is 0, at least one of A¹⁶ to A¹⁸ are each independently a cyclic alkyl group having 3 to 25 carbon atoms which may be substituted, and
when a⁹ is 1, at least one of A¹⁶ to A¹⁹ are each independently a cyclic alkyl group having 3 to 25 carbon atoms which may be substituted;
L¹¹⁹ to L¹²⁸ each independently represent:
   a monocyclic, linked or condensed divalent aromatic hydrocarbon group having 6 to 25 carbon atoms which may be substituted,
   a monocyclic, linked or condensed divalent heteroaromatic group having 3 to 25 carbon atoms which may be substituted, or
   a single bond;
   D represents
   a monocyclic, linked or condensed monovalent to tetravalent aromatic hydrocarbon group having 6 to 25 carbon atoms which may be substituted,
   a monocyclic, linked or condensed monovalent to tetravalent aromatic hydrocarbon group having 3 to 25 carbon atoms which may be substituted,
   a monocyclic, linked or condensed monovalent to alkyl group having 1 to 10 carbon atoms which may be substituted, or
   a monovalent to tetravalent silicon atom which may be substituted;
   E¹ to E⁵ each independently represent
   a fluorine atom, or
   a linear, branched or cyclic alkyl group having 1 to 18 carbon atoms which may be substituted by a fluorine atom.

Another aspect of the present disclosure provides a metal patterning material containing a compound having:
an aromatic ring and/or heteroaromatic ring, and a fluorine atom, in which
a proportion of carbon atom number directly binding with the fluorine atom among carbon atom number forming the aromatic ring and carbon atom number forming the heteroaromatic ring is at least 10%,
a proportion of fluorine atoms relative to carbon atoms in the molecule is at least 50%,
molecular weight is at least 500 and no more than 3000, and
glass transition temperature is at least 60°C.

Another aspect of the present disclosure provides a thin film for metal patterning containing a metal patterning material, and capable of patterning a metal film or a metal laminated film, in which
the metal patterning material has
an aromatic ring substituted by at least one fluorine atom, and/or heteroaromatic ring,
the molecular weight being at least 500 and no more than 3000, the glass transition temperature is at least 60°C,
the metal film or metal laminated film contains at least one selected from the group consisting of ytterbium, magnesium, silver, aluminum and an alloy of magnesium and silver.

Another aspect of the present disclosure provides an organic electroluminescence element including a negative electrode, in which
the negative electrode contains
at least one selected from the group consisting of ytterbium, magnesium, silver, aluminum, and an alloy or magnesium and silver, and
being patterned by a metal patterning material,
in which the metal patterning material has, in a molecule,
an aromatic ring substituted by at least one fluorine atom, and/or heteroaromatic ring,
the molecular weight being at least 500 and no more than 3000, and
the glass transition temperature is at least 60°C.

Another aspect of the present disclosure provides a method for forming a metal pattern, including:
forming an organic material pattern including the metal patterning material, or the amine compound; and
applying a metal material on a formation region of the organic material pattern and a non-formation region of the organic material pattern to form a metal pattern on the non-formation region.

Another aspect of the present disclosure provides an electronic device containing the metal patterning material, or the amine compound.

### Effects of the Invention

According to one aspect of the present disclosure, it is possible to provide a metal patterning material having excellent heat resistance and suppressing the formation of a metal thin film on a film surface, an amine compound, a metal patterning thin film using these materials, an organic electroluminescence element, a method for forming a metal pattern, and an electronic device.

### BRIEF DESCRIPTION OF THE DRAWINGS

FIG. 1 is a view showing the result of transmittance measurement prior to metal vapor deposition for compound (A177);
FIG. 1B is a view showing the result of transmittance measurement after metal vapor deposition for compound (A177);
FIG. 2A is a view showing the result of transmittance measurement prior to metal vapor deposition for compound (A433);
FIG. 2B is a view showing the result of transmittance measurement after metal vapor deposition for compound (A433);
FIG. 3A is a view showing the result of transmittance measurement prior to metal vapor deposition for compound (X1);
FIG. 3B is a view showing the result of transmittance measurement after metal vapor deposition for compound (X1);
FIG. 4A is a view showing the result of transmittance measurement prior to metal vapor deposition for compound (X4); and
FIG. 4B is a view showing the result of transmittance measurement after metal vapor deposition for compound (X4).

### PREFERRED MODE FOR CARRYING OUT THE INVENTION

Hereinafter, a metal patterning material, amine compound and electronic device, as well as metal patterning thin film, organic electro-luminescence element and method for forming a metal pattern using these will be explained in detail.

### (First Embodiment: Metal patterning material having amine substituent)

The metal patterning material according to an aspect of the present disclosure contains a compound having, in the molecule, an aromatic ring and/or heteroaromatic ring, a fluorine atom, and at least one tertiary amine,
in which the aromatic ring is at least one selected from the group consisting of a monocyclic aromatic ring, a linked aromatic ring, and a condensed aromatic ring having from 6 to 15 carbon atoms,
and is free of condensed aromatic rings having 16 or more carbon atoms,
in which a proportion of carbon atom number directly binding with the fluorine atom among a carbon atom number forming the aromatic ring and a carbon atom number forming the heteroaromatic ring is at least 10%,
molecular weight is at least 500 and no more than 3,000, and a glass transition temperature is 60°C or higher. More specifically, the above-mentioned compound can be used as a metal patterning material.

### (Aromatic Ring)

The aromatic ring is at least one type selected from the group consisting of a monocyclic aromatic ring, and condensed aromatic ring having 6 to 15 carbons. The carbon number of the monocyclic aromatic ring and linked aromatic ring is preferably 6 to 25. The carbon number of the condensed aromatic ring is preferably 6 to 14, and more preferably 6 to 13. The metal patterning material has an aromatic group derived from the above-mentioned aromatic ring. As the above-mentioned aromatic group derived from the aromatic ring, for example, a phenyl group, a biphenylyl group, a terphenylyl group, a naphthyl group, a fluorenyl group, a spirobiflurenyl group, a benzofluorenyl group, a dibenzofluorenyl group, a phenanthryl group, a fluoranthenyl group, an anthoryl group, and a group in which one or more selected from the group consisting of benzene, naphthalene, and phenanthrene are condensed in these groups can be exemplified.

### (Heteroaromatic Ring)

The heteroaromatic ring is preferably a monocyclic heteroaromatic ring, a linked heteroaromatic ring, or a condensed heteroaromatic ring having 3 to 25 carbon atoms. The metal patterning material has a heteroaromatic group derived from the heteroaromatic ring. As the heteroaromatic group derived from the heteroaromatic ring, for example, a pyrrolyl group, a thienyl group, a furyl group, an imidazolyl group, a thiazolyl group, an isothiazolyl group, an oxazolyl group, an isoxazolyl group, a pyridyl group, a pyrazyl group, a pyrazyl group, an indolyl group, a benzothienyl group, a benzofuranyl group, a benzoimidazolyl group, an indazolyl group, a benzothiazolyl group, a benzoisothiazolyl group, a 2,1,3-benzothiadiazolyl group, a benzoxazolyl group, a benzoisoxazolyl group, a 2,1,3-benzoxadiazolyl group, a quinolyl group, an isoquinolyl group, a carbazolyl group, a dibenzothienyl group, a dibenzofuranyl group, a phenoxazinyl group, a phenothiazinyl group, a phenazinyl group, and a thianthrenyl group, and a group in which one or more selected from the group consisting of benzene, naphthalene, and phenanthrene are condensed in these groups can be exemplified.

The metal patterning material lacks a condensed aromatic ring having 16 or more carbon atoms. In the case of the metal patterning material having such a condensed aromatic ring, it is not possible to effectively suppress the formation of a metal film on the film surface. As the group derived from the condensed aromatic ring having 16 or more carbon atoms, for example, a triphenylenyl group, a pyrenyl group, a tetracenyl group, a crisenyl group, a perylenyl group, and a pentacenyl group can be exemplified.

### (Tertiary Amines)

As the substituent bonded to the nitrogen atom forming the tertiary amine, it may be any of an aliphatic hydrocarbon group, an aromatic group, or a heteroaromatic group. Among these substituents, an aromatic group and a heteroaromatic group are preferable from the point of being able to effectively suppress the formation of a metal film on the surface of the film. As the tertiary amine, an aromatic tertiary amine in which any one of the above aromatic groups and heteroaromatic groups is bonded to three bonds of a nitrogen atom is more preferable. The aromatic group and the heteroaromatic group have the same meaning as the aromatic group and the heteroaromatic group shown in the above (aromatic ring) and (heteroaromatic ring).

### (Content of Fluorine Atom)

In the metal patterning material, due to being able to suppress the formation of metal film on the film surface, among the number of carbon atoms forming the aromatic ring and number of carbon atoms forming the heteroaromatic ring, the proportion of the number of carbon atoms directly bonding with a fluorine atom is at least 10%. This proportion is more preferable in the order of at least 15%, at least 20%, at least 25%, at least 30%, at least 35% and at least 40%.

### (Glass Transition Temperature)

Due to being able to maintain a stable thin film even under high-temperature conditions, the metal patterning material has a glass transition temperature of at least 60°C. The glass transition temperature is more preferably at least 65°C, at least 70°C, at least 75°C, at least 80°C, at least 85°C, at least 90°C, at least 95°C and at least 100°C.

### (Molecular Weight)

Due to being able to lower the heating temperature during deposition, and being able to suppress thermal decomposition of material in the deposition process forming the thin film thereof, the metal patterning has a molecular weight no more than 3000. The molecular weight is more preferably no more than 2800, no more than 2500, no more than 2300, no more than 2100, no more than 2000, no more than 1900, no more than 1800 and no more than 1700. In addition, the metal patterning material has a molecular weight of at least 500. The molecular weight is preferably at least 500 and no more than 2000.

The metal patterning material is preferably a compound represented by Formula (1), (2) or (3).

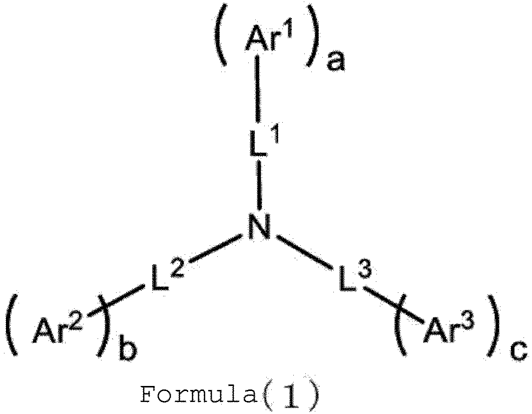

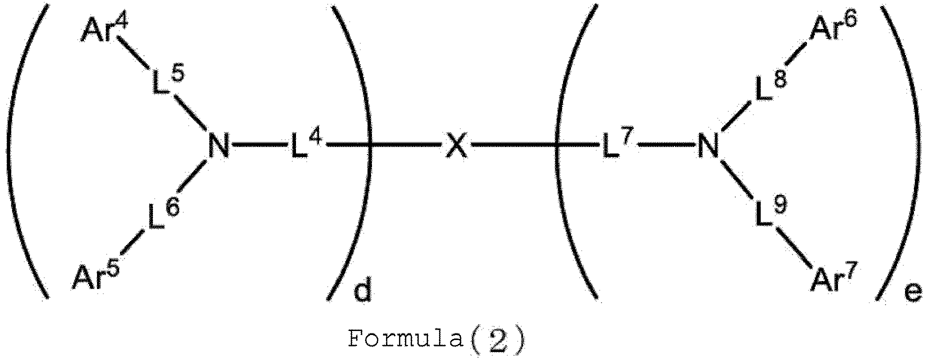

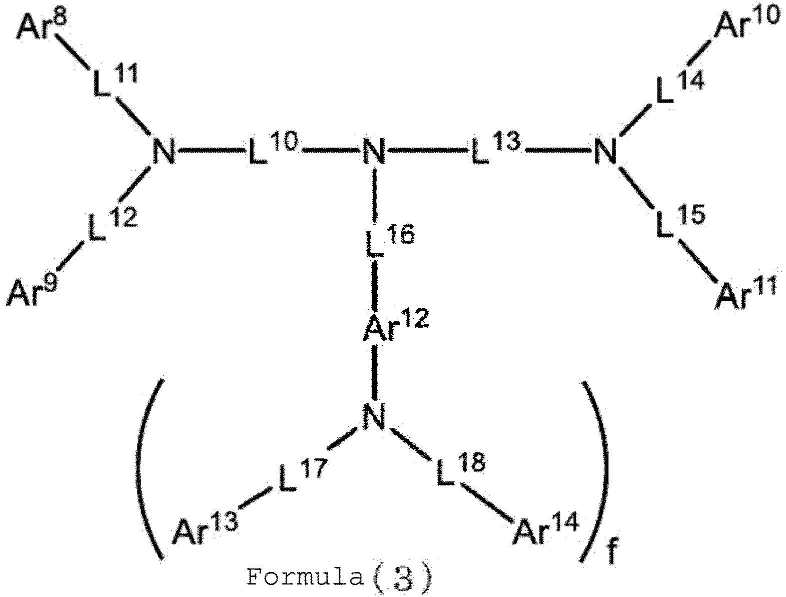

In the formula,
Ar¹ to Ar¹⁴ each independently represent
an optionally substituted monocyclic, linked or condensed aromatic hydrocarbon group having 6 to 25 carbon atoms, or an optionally substituted monocyclic, linked, or condensed heteroaromatic groups of 3 to 25 carbon atoms;
L¹ to L¹⁸ each independently represent
an optionally substituted monocyclic, linked, or condensed monocyclic divalent aromatic hydrocarbon group having 6 to 25 carbon atoms,
an optionally substituted monocyclic, linked or condensed ring divalent heteroaromatic group having of 3 to 25 carbon atoms, or
a single bond;
X represents
an optionally substituted monocyclic, linked or condensed ring di- to tetravalent aromatic hydrocarbon group having 6 to 25 carbon atoms,
an optionally substituted monocyclic, linked or condensed ring di- to tetravalent heteroaromatic group having of 3 to 25 carbon atoms,
an optionally substituted linear, branched or cyclic di- to tetravalent alkyl group having 1 to 10 carbon atoms, or an optionally substituted di- to tetravalent silicon atom;
a, b and c each independently represent an integer of 1 to 3; d and e each independently represent an integer of 1 or 2; and f represents an integer of 0 or 1.

Due to being able to suppress formation of metal film on the film surface, the carbon number of the condensed aromatic hydrocarbon group in Formulas (1) to (3) may be at least 6 to no more than 15. More specifically, it may from 6 to 15, may be from 6 to 14, or may be from 6 to 13. Herein, when Ar¹ to Ar¹⁴ are substituted aromatic hydrocarbon groups or substituted heteroaromatic groups, these groups are each independently preferably substituted by at least one group selected from the group consisting of:
a linear, branched, or cyclic alkyl group having 1 to 18 carbon atoms which may be substituted with a fluorine atom;
a linear, branched, or cyclic alkoxy group having 1 to 18 carbon atoms which may be substituted with a fluorine atom;
an aromatic hydrocarbon group having 6 to 20 carbon atoms which may be substituted with a fluorine atom;
a heteroaromatic group having 3 to 20 carbon atoms which may be substituted with a fluorine atom;
a cyano group;
a fluorine atom; and
a deuterium atom;
when L¹ to L¹⁸ are substituted divalent aromatic hydrocarbon groups or substituted divalent heteroaromatic groups, these groups are each independently preferably substituted by at least one group selected from the group consisting of:
   a linear, branched, or cyclic alkyl group having 1 to 18 carbon atoms which may be substituted with a fluorine atom;
   a linear, branched, or cyclic alkoxy group having 1 to 18 carbon atoms which may be substituted with a fluorine atom;
   an aromatic hydrocarbon group having 6 to 20 carbon atoms which may be substituted with a fluorine atom;
   a heteroaromatic group having 3 to 20 carbon atoms which may be substituted with a fluorine atom;
   a cyano group,
   a fluorine atom; and
   a deuterium atom;
   when X is a substituted divalent to tetravalent aromatic hydrocarbon group, substituted divalent to tetravalent heteroaromatic group, substituted divalent to tetravalent alkyl group or substituted divalent to tetravalent silicon atom, these groups are each independently preferably substituted by at least one group selected from the group consisting of:
      a linear, branched, or cyclic alkyl group having 1 to 18 carbon atoms which may be substituted with a fluorine atom;
      a linear, branched, or cyclic alkoxy group having 1 to 18 carbon atoms which may be substituted with a fluorine atom;
      an aromatic hydrocarbon group having 6 to 20 carbon atoms which may be substituted with a fluorine atom;
      a heteroaromatic group having 3 to 20 carbon atoms which may be substituted with a fluorine atom;
      cyano group,
      fluorine atom, and
      a deuterium atom.

As the monocyclic, linked, or condensed aromatic hydrocarbon group having 6 to 25 carbon atoms, a phenyl group, a biphenylyl group, a terphenylyl group, a naphthyl group, a fluorenyl group, a spirobifluorenyl group, a benzofluorenyl group, a dibenzofluorenyl group, a phenanthryl group, a fluoranthenyl group, a triphenylenyl group, a pyrenyl group, an an anthoryl group, a tetracenyl group, a cisenyl group, a perylenyl group, and a pentacenyl group, as well as a group in which at least one selected from the group consisting of benzene, naphthalene and phenanthrene are condensed in these groups can be exemplified.

As the monocyclic, linked, or condensed heteroaromatic group having 3 to 25 carbon atoms, a pyrrolidyl group, a thienyl group, a furyl group, an imidazolyl group, a thiazolyl group, an isothiazolyl group, an oxazolyl group, an isoxazolyl group, a pyridyl group, a pyrazyl group, an indolyl group, a benzothienyl group, a benzofuranyl group, a benzoimidazolyl group, a benzoimidazolyl group, an indazolyl group, a benzothiazolyl group, a benzoisothiazolyl group, a 2,1,3-benzothiadiazolyl group. Examples thereof include a benzoxazolyl group, a benzoxazolyl group, a 2,1,3-benzoxadiazolyl group, a quinolyl group, an isoquinolyl group, a carbazolyl group, a dibenzothienyl group, a dibenzofuranyl group, a phenoxazinyl group, a phenothiazinyl group, a phenazinyl group, and a thianthrenyl group, as well as a group in which at least one selected from the group consisting of benzene, naphthalene and phenanthrene are condensed in these groups can be exemplified.

As the monocyclic, linked, or condensed divalent aromatic hydrocarbon group having 6 to 25 carbon atoms, for example, a phenylene group, a biphenylylene group, a terphenylylene group, a naphthylene group, a fluorenylene group, a phenanthrenediyl group, a triphenylenediyl group, an anthracenediyl group, and a pyrenediyl group can be exemplified.

As the monocyclic, linked, or condensed divalent heteroaromatic group having 3 to 25 carbon atom, for example, a thiophenediyl group, a furanyl group, a dibenzothiophenediyl group, a dibenzofuranyl group, a pyridinediyl group, a pyrimidinediyl group, a triazinediyl group, a quinolinediyl group, a carbazolediyl group, a phenoxazinediyl group, a phenothiazinediyl group, and a xanthenediyl group can be exemplified.

As the linear, branched, or cyclic divalent alkyl group having 1 to 10 carbon atoms, for example, a methylene group, an ethylene group, a propanediyl group, a butanediyl group, a pentanediyl group, a hexanediyl group, a cyclohexanediyl group, and an adamantanediyl group can be exemplified.

As the monocyclic, linked, or condensed trivalent aromatic hydrocarbon group having 6 to 25 carbon atoms, a phenyltriyl group, a biphenyltriyl group, a terphenyltriyl group, a naphthylylyl group, a fluorenetriyl group, a spirobifluorenetriyl group, a benzofluorenetriyl group, a dibenzofluorenetriyl group, a phenanthrenetriyl group, a fluoranthenetriyl group, a triphenylenetriyl group, a pyrenetriyl group, an an anthracenetriyl group. Examples thereof include a tetracenetriyl group, a crycenetriyl group, a perylenetriyl group, a pentacenetriyl group, as well as a group in which at least one selected from the group consisting of benzene, naphthalene and phenanthrene are condensed in these groups can be exemplified.

As the monocyclic, linked, or condensed trivalent heteroaromatic group having 3 to 25 carbon atoms, for example, a pyrroletriyl group, thiophenetriyl group, furantriyl group, imidazoletriyl group, thiazoletriyl group, isothiazoletriyl group, oxazoletriyl group, isoxazoletriyl group, pyridinetriyl group, pyrazinetriyl group, indoletriyl group, benzothiophenetriyl group, benzofurantriyl group, benzoimidazole triyl group, indazole triyl group, benzothiazole triyl group, benzoisothiazole triyl group, 2,1,3-benzothiadiazole triyl group, benzooxazole triyl group, benzoisoxazole triyl group, 2,1,3-benzooxadiazole triyl group, quinoline triyl group, isoquinoline triyl group, carbazole triyl group, dibenzothiophene triyl group, dibenzofuran triyl group a phenoxazine triyl group, a phenothiazine triyl group, a phenazine triyl group, and a thianthrene triyl group, as well as a group in which at least one selected from the group consisting of benzene, naphthalene and phenanthrene are condensed in these groups can be exemplified.

As the linear, branched, or cyclic trivalent alkyl group having 1 to 10 carbon atoms, for example, a methyltriyl group, an ethyltriyl group, a propanetriyl group, a butanetriyl group, a pentanetriyl group, a hexanetriyl group, a cyclohexanetriyl group, and an adamantanetriyl group can be exemplified.

As the monocyclic, linked, or condensed tetravalent aromatic hydrocarbon group having 6 to 25 carbon atoms, for example, a phenyl tetrayl group, a biphenyl tetrayl group, a terphenyl tetrayl group, a naphthyl tetrayl group, a fluorene tetrayl group, a spirobifluorene tetrayl group, a benzofluorene tetrayl group, a dibenzofluorene tetrayl group, a phenanthrene tetrayl group, a fluoranthene tetrayl group, a triphenylene tetrayl group, a pyrene tetrayl group, an anthracene tetrayl group, a tetracene tetrayl group, a chrysene tetrayl group, a perylene tetrayl group, and a pentacene tetrayl group, as well as a group in which at least one selected from the group consisting of benzene, naphthalene and phenanthrene are condensed in these groups can be exemplified.

As the monocyclic, linked, or condensed tetravalent heteroaromatic group having 3 to 25 carbon atoms, for example, a pyrrole tetrayl group, thiophene tetrayl group, furan tetrayl group, imidazole tetrayl group, thiazole tetrayl group, isothiazole tetrayl group, oxazole tetrayl group, isoxazole tetrayl group, pyridine tetrayl group, pyrazine tetrayl group, indole tetrayl group, benzothiophene tetrayl group, benzofuran tetrayl group, benzimidazole tetrayl group, indazole tetrayl group, benzothiazole tetrayl group, benzoisothiazole tetrayl group, 2,1,3-benzothiadiazole tetrayl group, benzooxazole tetrayl group, benzoisoxazole tetrayl group, 2,1,3-benzooxadiazole tetrayl group, quinoline tetrayl group, isoquinoline tetrayl group, carbazole tetrayl group, a dibenzothiophene tetrayl group, a dibenzofuran tetrayl group, a phenoxazine tetrayl group, a phenothiazine tetrayl group, a phenazine tetrayl group, and a thianthrene tetrayl group, as well as a group in which at least one selected from the group consisting of benzene, naphthalene and phenanthrene are condensed in these groups can be exemplified.

As the linear, branched, or cyclic tetravalent alkyl group having 1 to 10 carbon atoms, for example, a methyl tetrayl group, an ethyl tetrayl group, a propane tetrayl group, a butane tetrayl group, a pentane tetrayl group, a hexane tetrayl group, a cyclohexane tetrayl group, and an adamantane tetrayl group can be exemplified.

It should be noted that, as described above, the monocyclic, linked, or condensed aromatic hydrocarbon group having 6 to 25 carbon atoms; monocyclic, linked, or condensed heteroaromatic group having 3 to 25 carbon atoms; di- to tetravalent aromatic hydrocarbon group having 6 to 25 carbon atoms and having a monocyclic, linked, or condensed ring; dito tetravalent heteroaromatic group of a monocyclic, linked, or condensed ring having 3 to 25 carbon atoms; and linear, branched, or cyclic be- to tetravalent alkyl group having 1 to 10 carbon atoms may have a substituent. In the case of these having a substituent, each independently is substituted by at least one group selected from the group consisting of: a linear, branched, or cyclic alkyl group having 1 to 18 carbon atoms which may be substituted with a fluorine atom, a linear, branched, or cyclic alkoxy group having 1 to 18 carbon atoms which may be substituted with a fluorine atom, an aromatic hydrocarbon group having 6 to 20 carbon atoms which may be substituted with a fluorine atom, a heteroaromatic group having 3 to 20 carbon atoms which may be substituted with a fluorine atom, a cyano group, a fluorine atom, or a deuterium atom. In this case, the number of substituents is not particularly limited.

As the linear, branched, or cyclic alkyl group having 1 to 18 carbon atoms which may be substituted with a fluorine atom, for example, a methyl group, a propyl group, an isopropyl group, a butyl group, a sec-butyl group, a tert-butyl group, a pentyl group, a hexyl group, a heptyl group, an octyl group, a stearyl group, a cyclopentyl group, a cyclohexyl group, a difluoromethyl group, a trifluoromethyl group, a 2,2,2-trifluoroethyl group, a pentafluoroethyl group, a heptafluoropropyl group, a heptafluoroisopropyl group, a nonafluorobutyl group, a perfluoropentyl group, a perfluorohexyl group, a perfluoroheptyl group, a perfluorooctyl group, and a perfluorocyclohexyl group can be exemplified.

As the linear, branched, or cyclic alkoxy group having 1 to 18 carbon atoms which may be substituted with a fluorine atom, for example, a propoxy group, an isopropoxy group, an n-butoxy group, a sec-butoxy group, a tert-butoxy group, a pentyloxy group, a hexyloxy group, a stearyloxy group, a difluoromethoxy group, a trifluoromethoxy group, a 2,2,2-trifluoroethoxy group, a pentafluoroethoxy group, a heptafluoropropoxy group, a nonafluorobutoxy group, a perfluoropentyloxy group, a perfluorohexyloxy group, a perfluoroheptyloxy group, and a perfluorooctyloxy group can be exemplified.

As the aromatic hydrocarbon group having 6 to 20 carbon atoms which may be substituted with a fluorine atom, for example, a phenyl group, monofluorophenyl group, difluorophenyl group, trifluorophenyl group, tetrafluorophenyl group, perfluorophenyl group, perfluorotrile group, perfluorodimethylphenyl group, perfluorotrimethylphenyl group, perfluoroisopropylphenyl group, perfluorotert-butylphenyl group, biphenylyl group, monofluorobiphenylyl group, difluorobiphenylyl group, trifluorobiphenylyl group, tetrafluorobiphenylyl group, pentafluorobiphenylyl group, hexafluorobiphenylyl group, heptafluorobiphenylyl group, octafluorobiphenylyl group, perfluorobiphenylyl group, terphenylyl group, monofluoroterfenyl group, difluoroterfenyl group, trifluoroterfenyl groups, tetrafluoroterfenyl group, pentafluoroterfenilyl group, hexafluoroterfenilyl group, heptafluoroterfenilyl group, octafluoroterfenilyl group, nonafluoroterfenilyl group, decafluoroterfenilyl group, undecafluoroterfenilyl group, dodecafluoroterfenilyl group, perfluoroterfenilyl group, naphthyl group, monofluoronaphthyl group, difluoronaphthyl group, trifluoronaphthyl group, tetrafluoronaphthyl group, pentafluoronaphthyl group, hexafluoronaphthyl group, heptafluoronaphthyl group, fluorenyl group, monofluorofluorenyl group, difluorofluorenyl group, trifluorofluorenyl group, tetrafluorofluorenyl group, pentafluorofluorenyl group, hexafluorofluorenyl group, heptafluorofluorenyl group, spirobiflurenyl group, benzofluorenyl group, dibenzofluorenyl group, phenanthryl group, perfluorophenanthryl group, triphenylenyl group, perfluorotriphenylenyl group, pyrenyl group, perfluoropyrenyl group, anthryl group, and perfluoroanthryl group can be exemplified.

As the heteroaromatic group having 3 to 20 carbon atoms which may be substituted with a fluorine atom, for example, a pyrrolidyl group, thienyl group, perfluorothienyl group, furyl group, perfluorofuryl group, imidazolyl group, thiazolyl group, isothiazolyl group, oxazolyl group, isoxazolyl group, isoxazolyl group, pyridyl group, monofluoropyridyl group, difluoropyridyl group, trifluoropyridyl group, perfluoropyridyl group, pyrazyl group, indolyl group, benzothienyl group, benzofuranyl group, benzimidazolyl group, indazolyl group, benzothiazolyl group, enzoisothiazolyl group, 2,1,3-benzothiadiazolyl group, benzooxazolyl group, benzoisoxazolyl group, 2,1,3-benzooxadiazolyl group, quinolyl group, isoquinolyl group, carbazolyl group, dibenzothienyl group, dibenzofuranyl group, phenoxazinyl group, phenothiazinyl group, phenazinyl group, phenazinyl group, and thianthrenyl group can be exemplified.

Specific examples of Ar¹ to Ar¹⁴ include a 4-methylphenyl group, a 3-methylphenyl group, 2-methylphenyl group, 2,4-dimethylphenyl group, 2,5-dimethylphenyl group, 3,4-dimethylphenyl group, 3,5-dimethylphenyl group, 2,6-dimethylphenyl group, 2,3,5-trimethylphenyl group, 2,3,6-trimethylphenyl group, 3,4,5-trimethylphenyl group, a 4-biphenyl group, 3-biphenyl group, 2-biphenyl group, 2-methyl-1,1'-biphenyl-4-yl group, 3-methyl-1,1'-biphenyl -4-yl group, 2'-methyl-1,1'-biphenyl-4-yl group, 3'-methyl -1,1'-biphenyl-4-yl group, 4'-methyl-1,1'-biphenyl-4-yl group, 2,6-dimethyl-1,1'-biphenyl-4-yl group, 2,2'-dimethyl-1,1'-biphenyl-4-yl group, 2,3'-dimethyl-1,1'-biphenyl-4-yl group, 2,4'-dimethyl-1,1'-biphenyl-4-yl group, 3,2'-dimethyl-1,1'-biphenyl-4-yl group, 2',3'-dimethyl-1,1'-biphenyl-4-yl group, 2',4'-dimethyl-1,1'-biphenyl-4-yl group, 2',5'-dimethyl-1,1'-biphenyl-4-yl group, 2',6'-dimethyl-1,1'-biphenyl-4-yl group, p-terphenyl-2-yl group; P-terphenyl-3-yl group, p-terphenyl-4-yl group, p-terphenyl-2'-yl group, m-terphenyl-2-yl group, m-terphenyl-3-yl group, m-terpheny-4-yl group, m-terphenyl-2'-yl group, m-terphenyl-4'-yl group, m-terphenyl-5'-yl group, o-terphenyl-2-yl group, o-terphenyl-3-yl group, o-terphenyl-4-yl group, o-terphenyl-3-yl group, o-erphenyl-3'-yl group; o-terphenyl-4'-yl group, 1-naphthyl group, 2-naphthyl group, 2-methylnaphthalene-1-yl group, 4-methylnaphthalene-1-yl group, 6-methylnaphthalene-2-yl group, 4-(1-naphthyl)phenyl group, 4-(2-naphthyl)phenyl group, 3-(1-naphthyl)phenyl group, 3-(2-naphthyl)phenyl group, 3-methyl-4-(1-naphthyl)phenyl group, 3-methyl-4-(2-naphthyl)phenyl group; 4-(1-naphthyl)biphenyl group, 4-(2-naphthyl)biphenyl group, 3-(1-naphthyl)biphenyl group, 3-(2-naphthyl)biphenyl group, 4-(2-methylnaphthalene-1-yl)phenyl group, 3-(2-methylnaphthalene-1-yl)phenyl group, 4-phenylnaphthalene-1-yl group, 4-(2-methylphenyl)naphthalene-1-yl group; 4-(3-methylphenyl)naphthalene-1-yl group, 4-(4-methylphenyl)naphthalene-1-yl group, 6-phenylnaphthalene-2-yl group, 4-(2-methylphenyl)naphthalene-2-yl group, 4-(3-methylphenyl)naphthalene-2-yl group, 4-(4-methylphenyl)naphthalene-2-yl group, tetraphenylsilane-4-yl group, tetraphenylsilane-3-yl group, 2-fluorenyl group, 9,9-dimethyl-2-fluorenyl group, 9,9-diphenyl -2-fluorenyl group, 9,9-diphenyl-4-fluorenyl group, 9,9'-spirobifluorene-2-yl group, 9,9'-spirobifluorene-4-yl group, 4-(9,9'-spirobifluorene-4-yl)phenyl group, 3-(9,9'-spirobifluorene-4-yl)phenyl group, 4-(9,9'-spirobifluorene-4-yl)biphenyl group; 3-(9,9'-Spirobifluorene-4-yl)biphenyl group, 4-(9,9'-diphenylfluorene-4-yl)phenyl group, 3-(9,9'-diphenylfluorene-4-yl)phenyl group, 4-(9,9'-diphenylfluorene-4-yl)biphenyl group, 3-(9,9'-diphenylfluorene-4-yl)biphenyl group, 3-(1-triphenylenyl)biphenyl group, 9-phenanthryl group, 2-phenanthryl group, 4-(9-phenanthryl)phenyl group, 3-(9-phenanthryl)phenyl group, 4-(9-phenanthryl)biphenyl group, 3-(1-naphthyl)biphenyl group, 3-(9-phenanthryl)biphenyl group, 1-triphenylenyl group, 2-triphenylenyl group, 3-triphenylenyl group, 4-triphenylenyl group, 4-(1-triphenylenyl)phenyl group, 3-(1-triphenylenyl)phenyl group; 4-(1-triphenylrenyl)biphenyl group, 3-(1-triphenylrenyl)biphenyl group, 3-(1-triphenylrenyl)biphenyl group, 11,11'-dimethylbenzo [a ]fluorene-9-yl group, 11,11'-dimethylbenzo [a]fluorene-3-yl group, 11,11'-dimethylbenzo [b]fluorene-9-yl group, 11,11'-dimethylbenzo [b]fluorene-3-yl group, 11,11'-dimethylbenzo [c]fluorene-9-yl group, 11,11'-dimethylbenzo [c]fluorene-2-yl group, 3-fluoranthenyl group, 8-fluoranthenyl group, monofluorophenyl group, difluorophenyl group, trifluorophenyl group, tetrafluorophenyl group, pentafluorophenyl group, monofluorobiphenylyl group, difluorobiphenylyl group, trifluorobiphenylyl groups, tetrafluorobiphenylyl group, pentafluorobiphenylyl group, hexafluorobiphenylyl group, heptafluorobiphenylyl group, octafluorobiphenylyl group, nonafluorobiphenyl group, Monofluoroterfenylyl group, difluoroterfenylyl group, trifluoroterfenylyl group, tetrafluoroterfenylyl group, pentafluoroterfenylyl group, hexafluoroterfenyl group, heptafluoroterfenyl group, octafluoroterfenyl group, nonafluoroterfenyl group, decafluoroterfenyl group, undecafluoroterfenyl group, dodecafluoroterfenyl group, tridecafluoroterfenyl group, monofluoronaphthyl group, difluoronaphthyl group, trifluoronaphthyl group, tetrafluoronaphthyl group, pentafluoronaphthyl group, hexafluoronaphthyl group, heptafluoronaphthyl group, monofluorofluorenyl group, difluorofluorenyl group, trifluorofluorenyl group, tetrafluorofluorenyl group, pentafluorofluorenyl group, hexafluorofluorenyl group, heptafluorofluorenyl group, 1-imidazolyl group, 2-phenyl-1-imidazolyl group, 2-henyl-3,4-dimethyl-1-imidazolyl group, 2,3,4-triphenyl-1-imidazolyl group, 2-(2-naphthyl)-3,4-dimethyl-1-imidazolyl group, 2-(2-naphthyl)-3,4-diphenyl-1-imidazolyl group, 1-methyl-2-imidazolyl group, 1-ethyl-2-imidazolyl group, 1-phenyl-2-imidazolyl group; 1-methyl-4-phenyl-2-imidazolyl group, 1-methyl-4,5-dimethyl-2-imidazolyl group, 1-methyl-4,5-diphenyl-2-imidazolyl group, 1-phenyl-4,5-dimethyl-2-imidazolyl group, 1-phenyl-4,5-diphenyl-2-imidazolyl group, 1-phenyl-4,5-dibiphenylyl-2-imidazolyl group, 1-methyl-3-pyrazolyl group; 1-phenyl-3-pyrazolyl group, 1-methyl -4-pyrazolyl group, 1-phenyl-4-pyrazolyl group, 1-methyl-5-pyrazolyl group, 1-phenyl-5-pyrazolyl group, 2-thiazolyl group, 4-hiazolyl group, 5-thiazolyl group, 3-sothiazolyl group, 4-isothiazolyl group, 5-sothiazolyl group, 2-oxazolyl group, 4-xazolyl group, 5-oxazolyl group, 3-isoxazolyl group, 4-isoxazolyl group. 5-isoxazolyl group, 2-pyridyl group, 3-methyl -2-pyridyl group, 4-methyl-2-pyridyl group, 5-methyl-2-pyridyl group, 6-methyl-2-pyridyl group, 3-pyridyl group, 4-methyl-3-pyridyl group, 4-pyridyl group, 2-pyrimidyl group, 2,2'-bipyridine-3-yl group, 2,2'-bipyridine-4-yl group, 2,2'-bipyridine-5-yl group, 2,3'-bipyridine-3-yl group, 2,3'-bipyridine-4-yl group, 2,3'-bipyridine-5-yl group, 5-pyrimidyl group, pyrazyl group, 1,3,5-triazyl group, 4,6-diphenyl-1,3,5-triazine-2-yl group, 1-benzoimidazolyl group, 2-methyl-1-benzoimidazolyl group, 2-phenyl-1-benzoimidazolyl group, 1-methyl-2-benzoimidazolyl group, 1-phenyl-2-benzoimidazolyl group, 1-methyl-5-benzoimidazolyl group; 1,2-dimethyl-5-benzimidazolyl group, 1-methyl -2-phenyl-5-benzimidazolyl group, 1-phenyl-5-benzimidazolyl group, 1,2-diphenyl-5-benzimidazolyl group, 1-methyl-6-benzimidazolyl group, 1,2-dimethyl-6-benzimidazolyl group, 1-methyl-2-phenyl -6-benzimidazolyl group, 1-phenyl-6-benzimidazolyl group, 1,2-diphenyl-6-benzimidazolyl group, 1-methyl-3-indazolyl group, 1-phenyl-3-indazolyl group, 2-benzothiazolyl group, 4-benzothiazolyl group, 5-benzothiazolyl group; 6- benzothiazolyl group, 7-benzothiazolyl group, 3-benzoisothiazolyl group, 4-benzoisothiazolyl group, 5-benzoisothiazolyl group, 6-benzoisothiazolyl group, 7-benzoisothiazolyl group, 2,1,3-benzothiadiazole-4-yl group, 2,1,3-benzothiadiazole-5-yl group, 2-benzoxazolyl group, 4-benzoxazolyl group, 5-benzoxazolyl group, 6-benzoxazolyl group, 7-benzoxazolyl group, 3-benzoisoxazolyl group, 4-benzoisoxazolyl group, 5-benzoisoxazolyl group, 6-benzoisoxazolyl group, 7-benzoisoxazolyl group, 2,1,3-benzooxadiazolyl-4-yl group, 2,1,3-benzooxadiazolyl-5-yl group, 2-quinolyl group, 3-quinolyl group, 5-quinolyl group, 6-quinolyl group, 1-isoquinolyl group, 4-isoquinolyl group, 5-isoquinolyl group, 2-acridinyl, 9-acridinyl, 1,10-phenanthroline-3-yl, 1,10-phenanthroline-5-yl, 2-thienyl, 3-thienyl, 2-benzothienyl, 3-benzothienyl, 2-dibenzothienyl, 4-dibenzothienyl, 2-furanyl, 3-furanyl, 2-benzofuranyl, 3-benzofuranyl, 3-benzofuranyl, 3-benzofuranyl, 2-dibenzofuranyl, 4-dibenzofuranyl, carbazol-9-yl, 9-methylcarbazole-2-yl group, 9-methylcarbazole-3-yl group, 9-methylcarbazole-4-yl group, 9-phenylcarbazole-2-yl group, 9-phenylcarbazole-3-yl group, 9-phenylcarbazole-4-yl group, 9-biphenylcarbazole-2-yl group, 9-biphenylcarbazole-3-yl group, 9-biphenylcarbazole-4-yl group, 2-(9-carbazolyl)phenyl group; 3-(9-carbazolyl)phenyl group, 4-(9-carbazolyl)phenyl group, 2-(9-carbazolyl)biphenyl group, 3-(9-carbazolyl)biphenyl group, 4-(9-carbazolyl)biphenyl group; 2-(9-phenylcarbazol-3-yl)phenyl group, 3-(9-phenylcarbazol-3-yl)phenyl group, 4-(9-phenylcarbazol-3-yl)phenyl group, 2-thianthryl group, 10-phenylphenothiazine-3-yl group, 10-phenylphenothiazine-2-yl group, 10-phenylphenoxazine-3-yl group, 10-phenylphenoxazine-2-yl group, 1-methylindole-2-yl group; 1-phenylindole-2-yl group, 1-methylindole-2-yl group, 1-phenylindole-2-yl group, 4-(2-pyridyl)phenyl group, 4-(3-pyridyl)phenyl group, 4-(4-pyridyl)phenyl group, 3-(2-pyridyl)phenyl group, 3-(3-pyridyl)phenyl group, 3-(4-pyridyl)phenyl group, 4-(2-phenylimidazol-1-yl)phenyl group; 4-(1-phenylimidazol-2-yl)phenyl group, 4-(2,3,4-triphenylimidazol-1-yl)phenyl group, 4-(1-methyl-4,5-diphenylimidazol-2-yl)phenyl group, 4-(2-methylbenzoimidazol-1-yl)phenyl group, 4-(2-phenylbenzoimidazol-1-yl)phenyl group, 4-(1-methylbenzoimidazol-2-yl)phenyl group; 4-(2-phenylbenzimidazol-1-yl)phenyl group, 3-(2-methylbenzimidazol-1-yl)phenyl group, 3-(2-phenylbenzimidazol-1-yl)phenyl group, 3-(1-methylbenzimidazol-2-yl)phenyl group, 3-(2-phenylbenzimidazol-1-yl)phenyl group, 4-(3,5-diphenyltriazine-1-yl)phenyl group, 4-(2-thienyl)phenyl group; 4-(2-furanyl)phenyl group, 5-phenylthiophene-2-yl group, 5-phenylfuran-2-yl group, 4-(5-phenylthiophene-2-yl)phenyl group, 4-(5-phenylfuran-2-yl)phenyl group, 3-(5-phenylthiophene -2-yl)phenyl group; 3-(5-phenylfuran-2-yl)phenyl group, 4-(2-benzothienyl)phenyl group, 4-(3-benzothienyl)phenyl group, 3-(2-benzothienyl)phenyl group, 3-(3-benzothienyl)phenyl group, 4-(2-dibenzothienyl)phenyl group, 4-(4-dibenzothienyl)phenyl group, 3-(2-dibenzothienyl)phenyl group, 3-(4-dibenzothienyl)phenyl group, 4-(2-dibenzothienyl)phenyl group; 4-(4-Dibenzofuranyl)phenyl group, 3-(2-dibenzofuranyl)phenyl group, 3-(2-dibenzofuranyl)phenyl group, 3-(4-dibenzofuranyl)phenyl group, 4-(2-benzothienyl)phenyl group, 4-(3-benzothienyl)phenyl group, 3-(2-benzothienyl)biphenyl group, 3-(3-benzothienyl)biphenyl group, 4-(2-dibenzothienyl)biphenyl group, 4-(4-dibenzothienyl)biphenyl group, 3-(2-dibenzothienyl)biphenyl group, 3-(4-dibenzothienyl)biphenyl group, 4-(2-dibenzofuranyl)biphenyl group, 4-(4-dibenzofuranyl)biphenyl group, 3-(2-dibenzofuranyl)biphenyl group, 3-(4-dibenzofuranyl)biphenyl group, 5-phenylpyridine-2-yl group, 4-phenylpyridine-2-yl group, and 5-phenylpyridine - 3-yl group.

In Formulas (1) to (3), due to having a high glass transition temperature and being able to suppress formation of a metal film on the film surface, Ar¹ to Ar¹⁴ each independently preferably represent
(i) a phenyl group, a biphenylyl group, a terphenylyl group, a naphthyl group, a fluorenyl group, a spirobifluorenyl group, a benzofluorenyl group, a phenanthryl group, a fluoranthenyl group, a triphenylenyl group, an anthryl group, a pyrenyl group, a pyridyl group, a carbazolyl group, a benzofuranyl group, a benzothienyl group, a dibenzofuranyl group, or a dibenzothienyl group; or
(ii) a group in which the group represented by (i) is substituted with one or more groups selected from the group consisting of: a methyl group, an ethyl group, a methoxy group, an ethoxy group, a trifluoromethyl group, a trifluoromethoxy group, a perfluoroalkyl group having 2 to 10 carbon atoms, a perfluoroalkoxy group having 2 to 10 carbon atoms, a cyano group, a deuterium atom, a fluorine atom, a phenyl group optionally substituted with a fluorine atom, a biphenylyl group optionally substituted with a fluorine atom, a naphthyl group optionally substituted with a fluorine atom, a phenanthryl group which may be substituted with a fluorine atom, a pyridyl group which may be substituted with a fluorine atom, a carbazolyl group which may be substituted with a fluorine atom, a dibenzothienyl group which may be substituted with a fluorine atom, and a dibenzofuranyl group which may be substituted with a fluorine atom.

Due to having a high glass transition temperature and being able to suppress formation of a metal film on the film surface, Ar¹ to Ar¹⁴ more preferably has the following substituents. It should be noted that F represents a fluorine atom, v represents an integer of 0 to 5, w represents an integer of 0 to 4, x represents an integer of 0 to 3, y represents an integer of 0 to 2, and z represents an integer of 0 to 1.

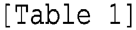

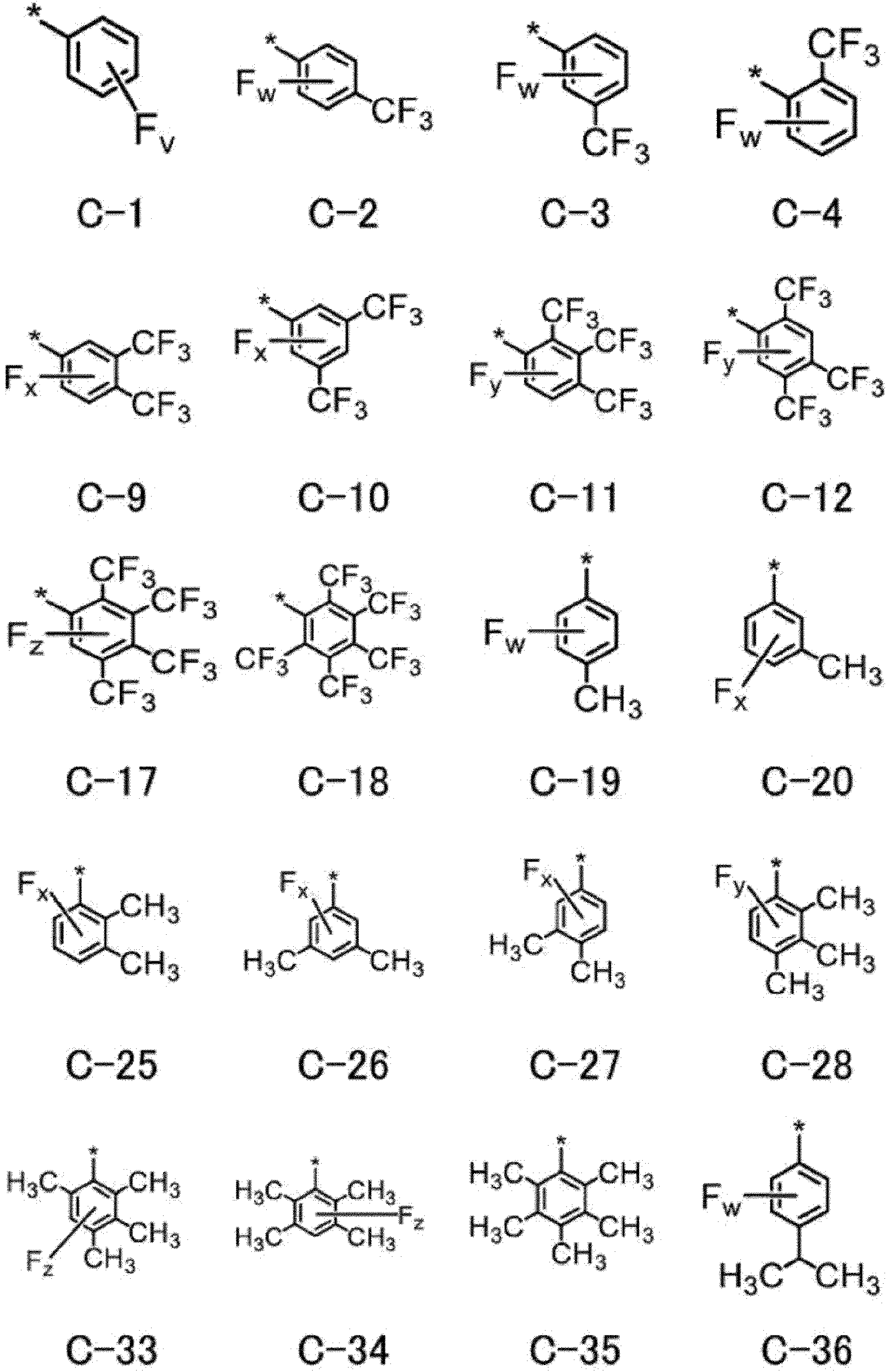

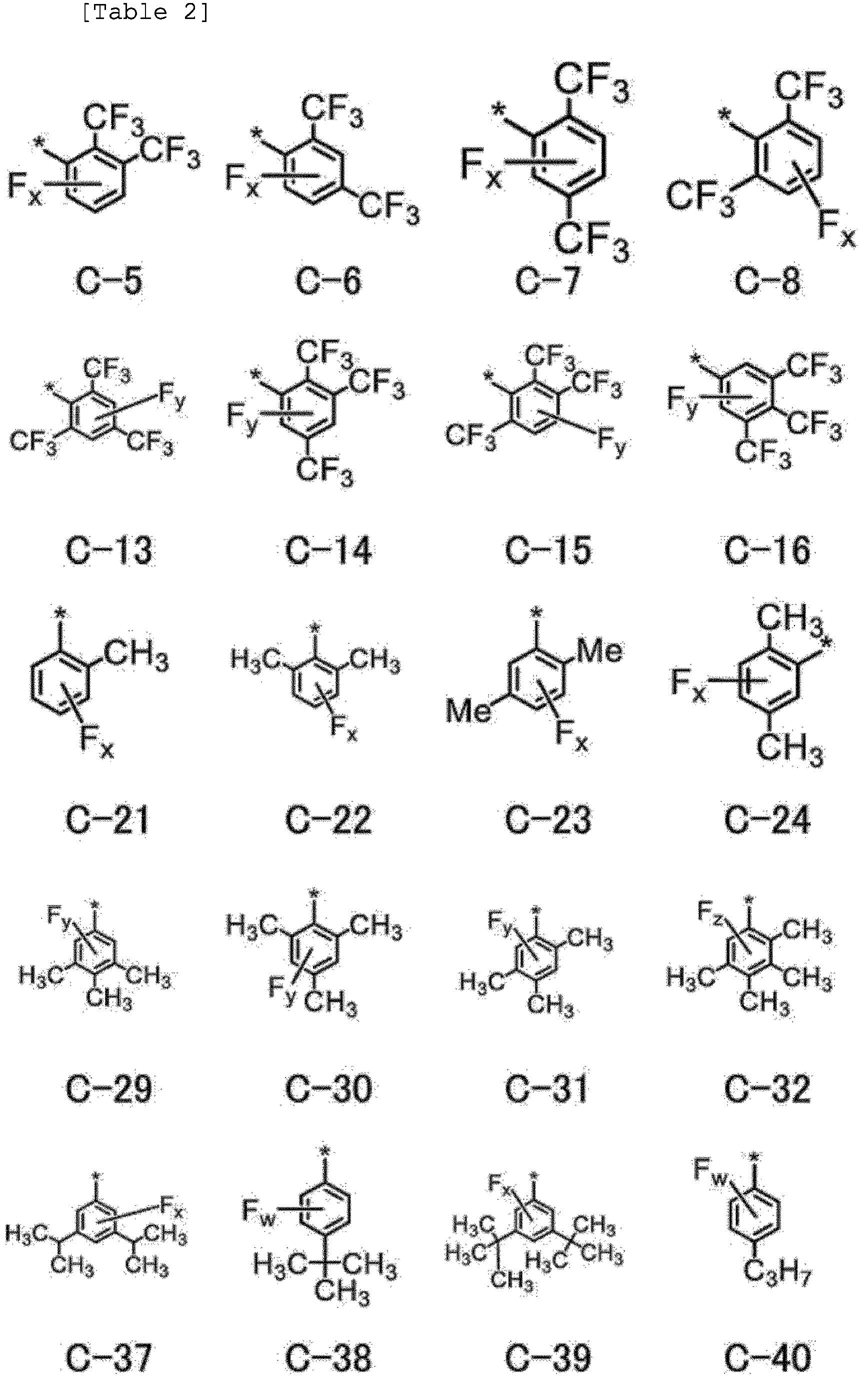

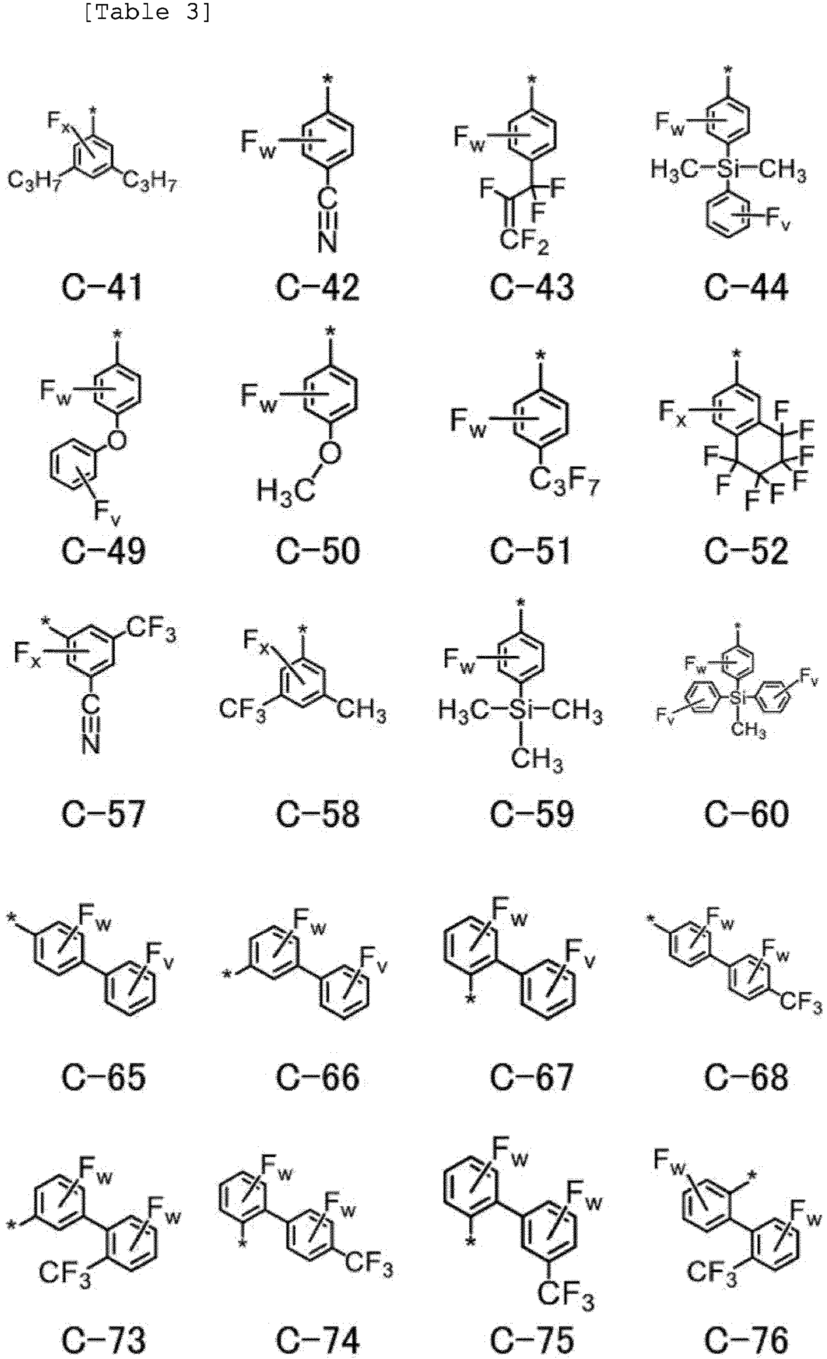

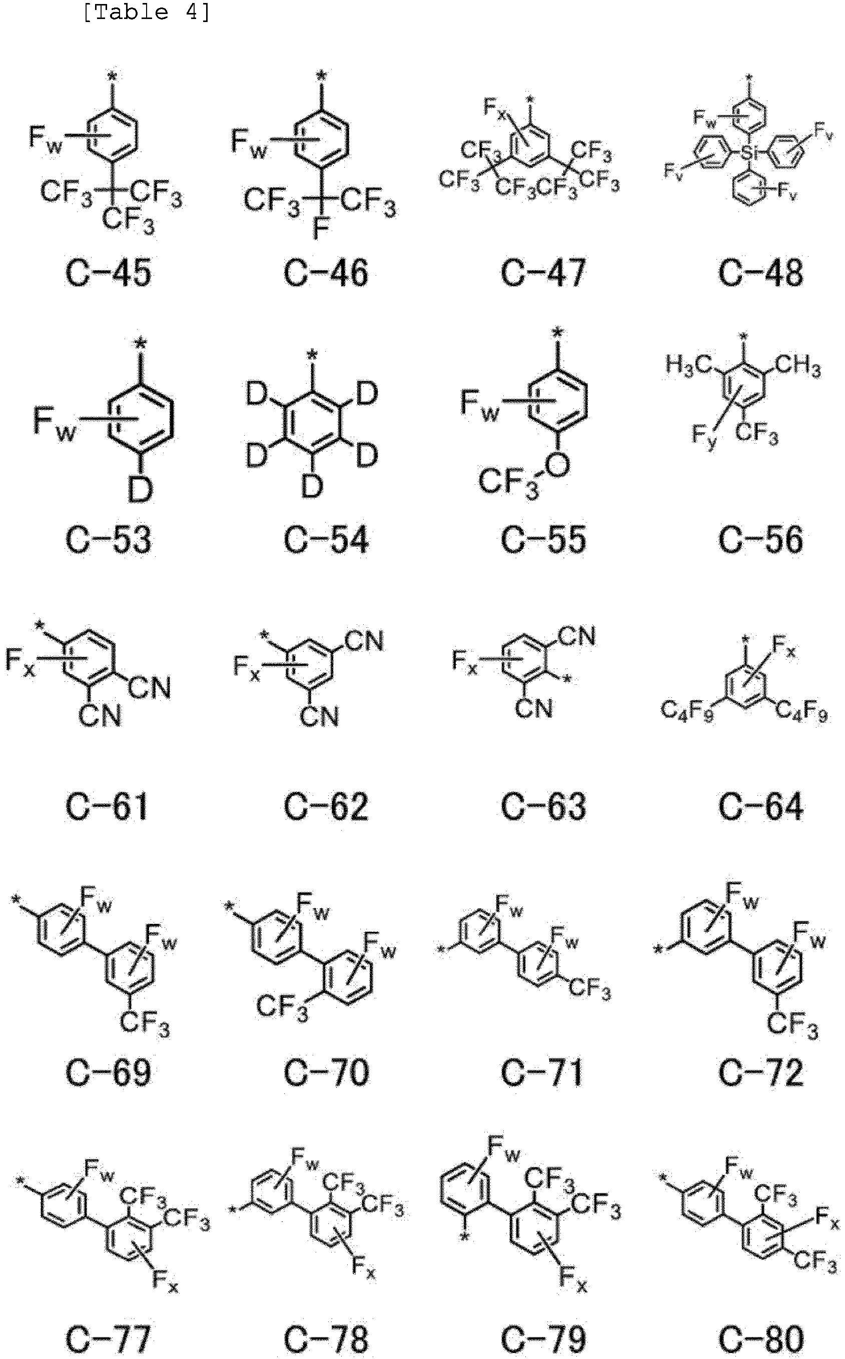

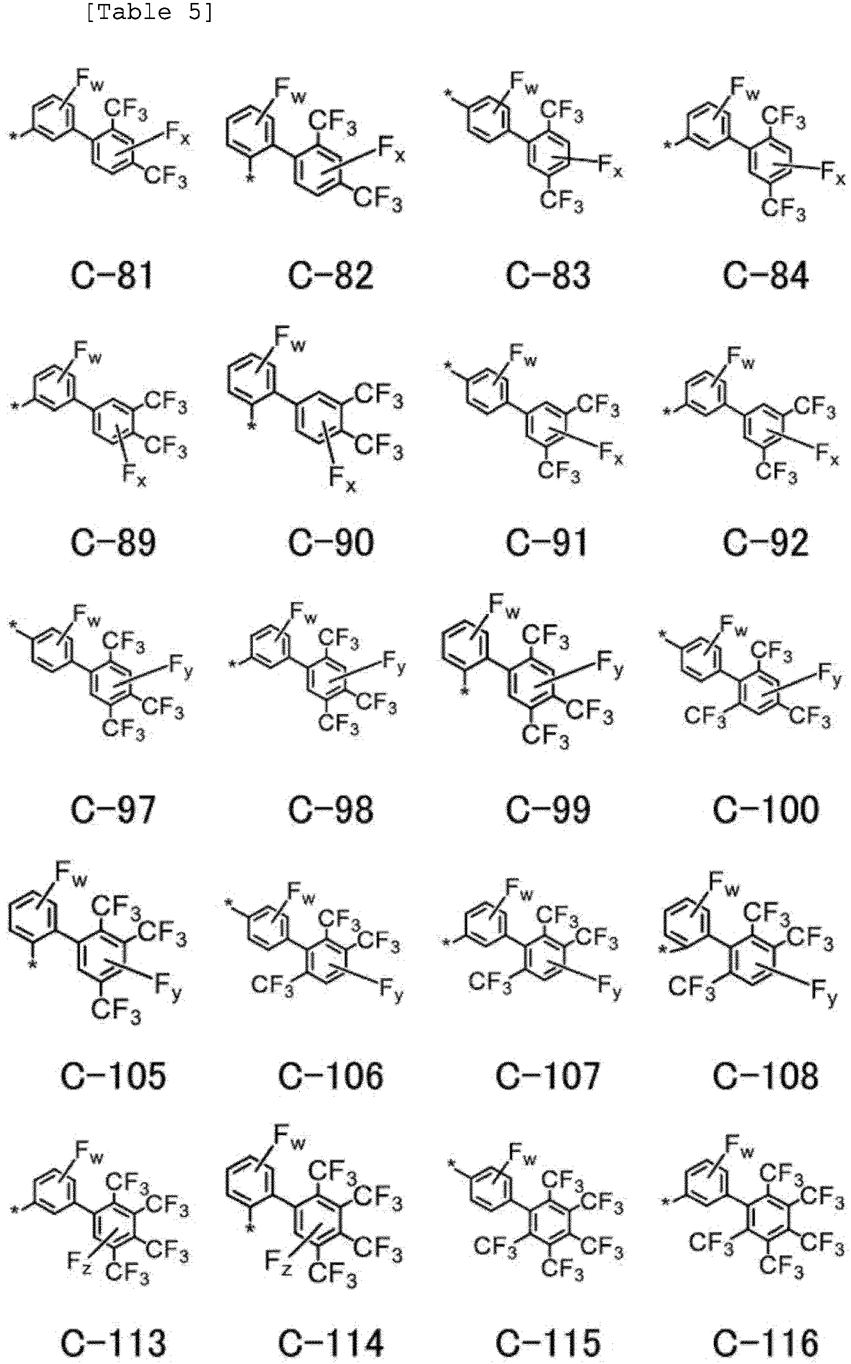

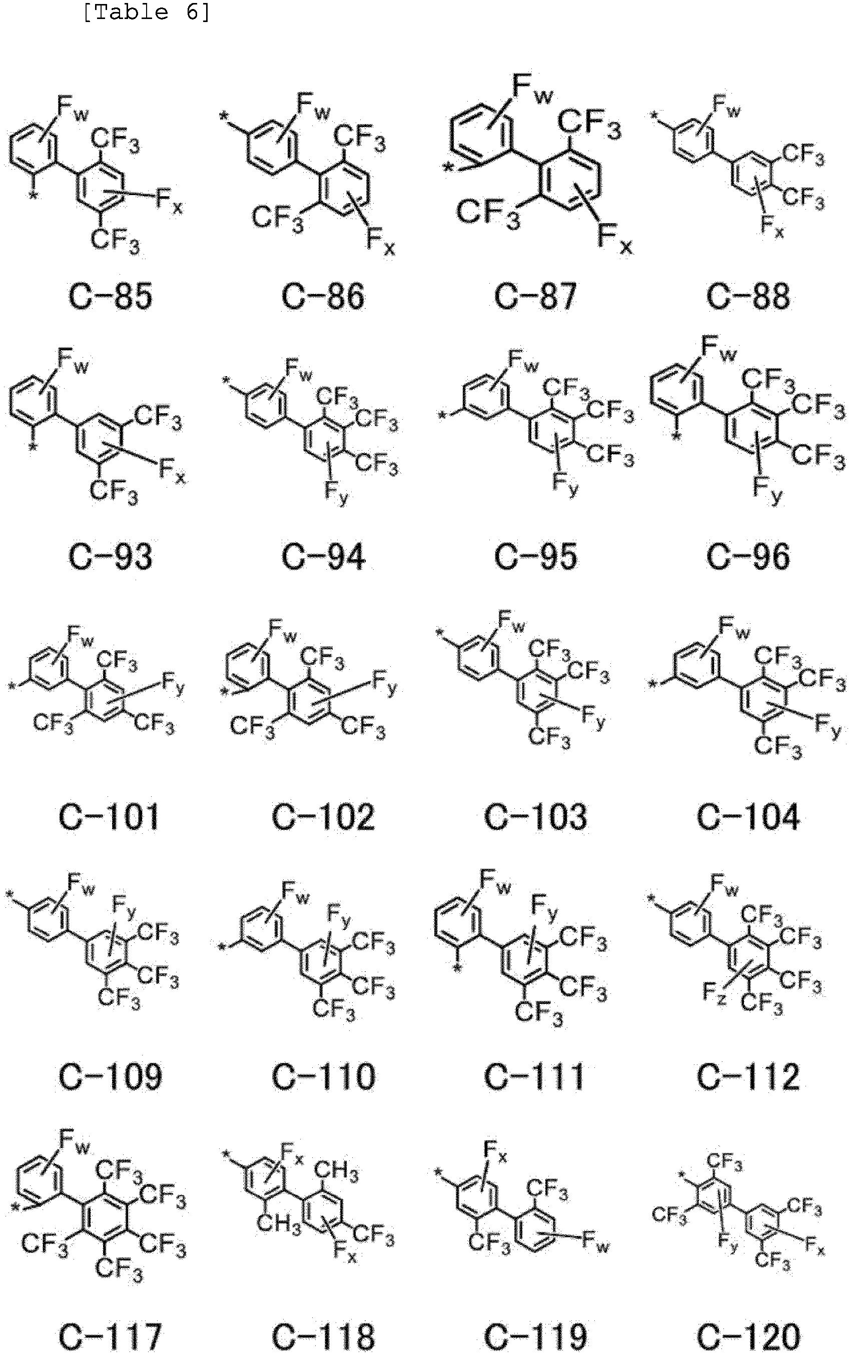

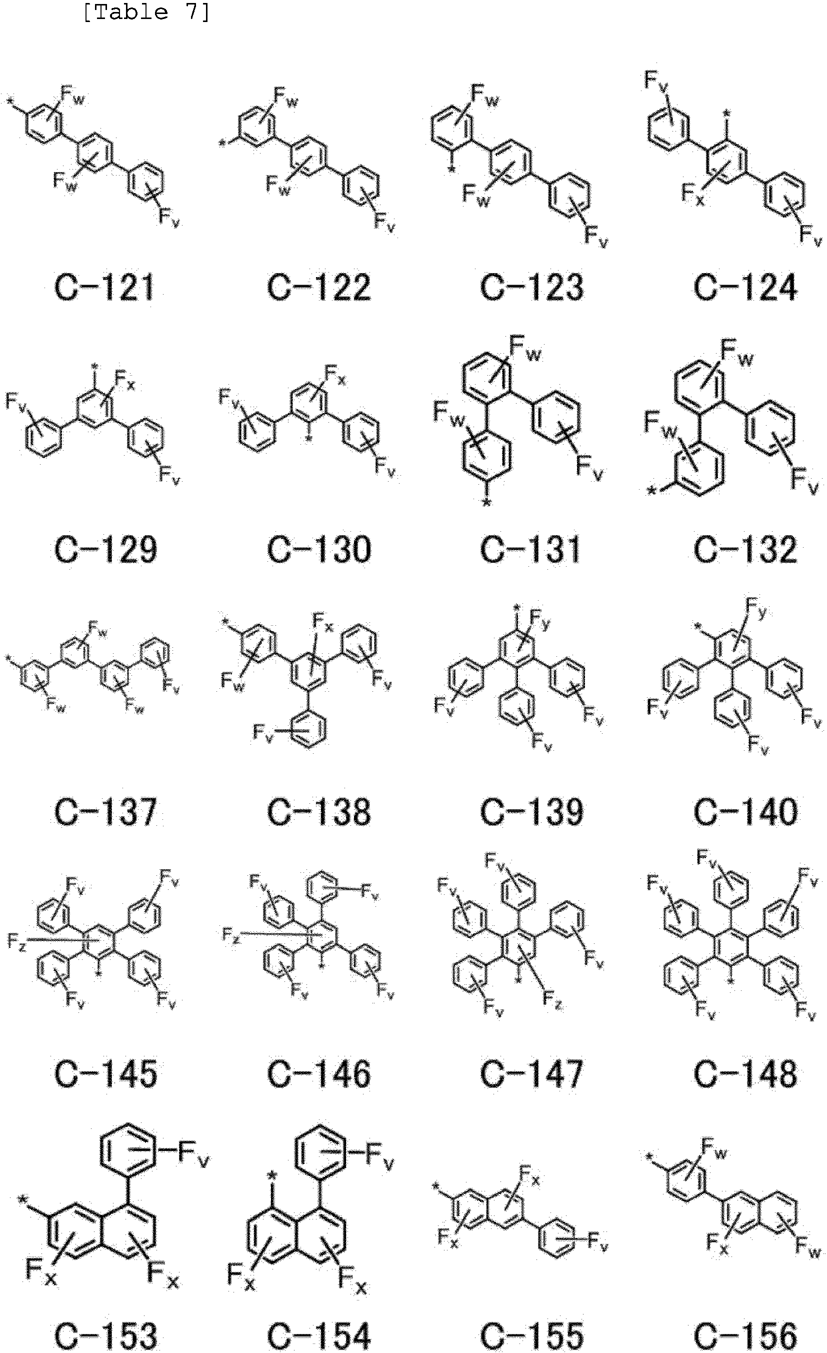

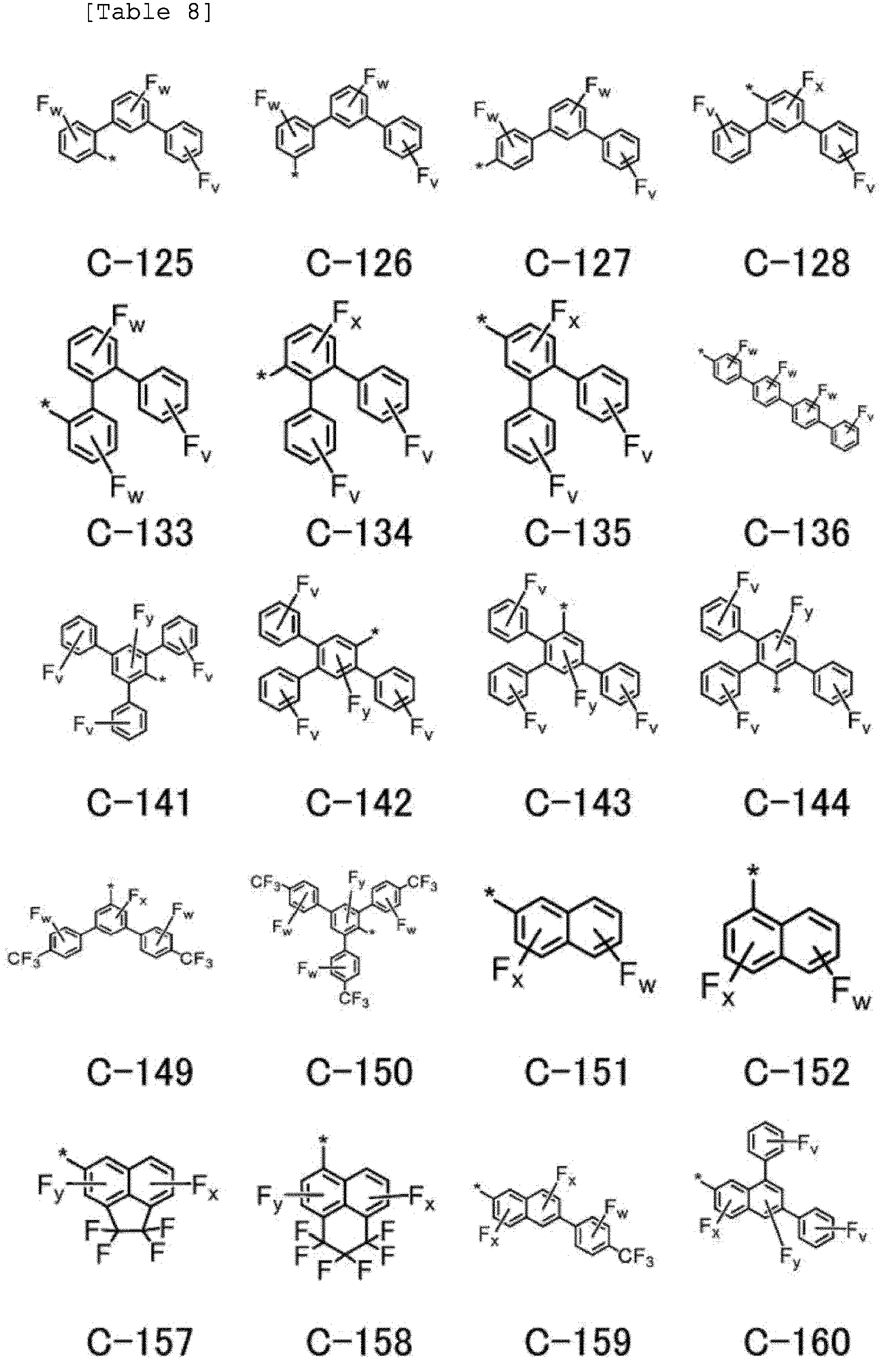

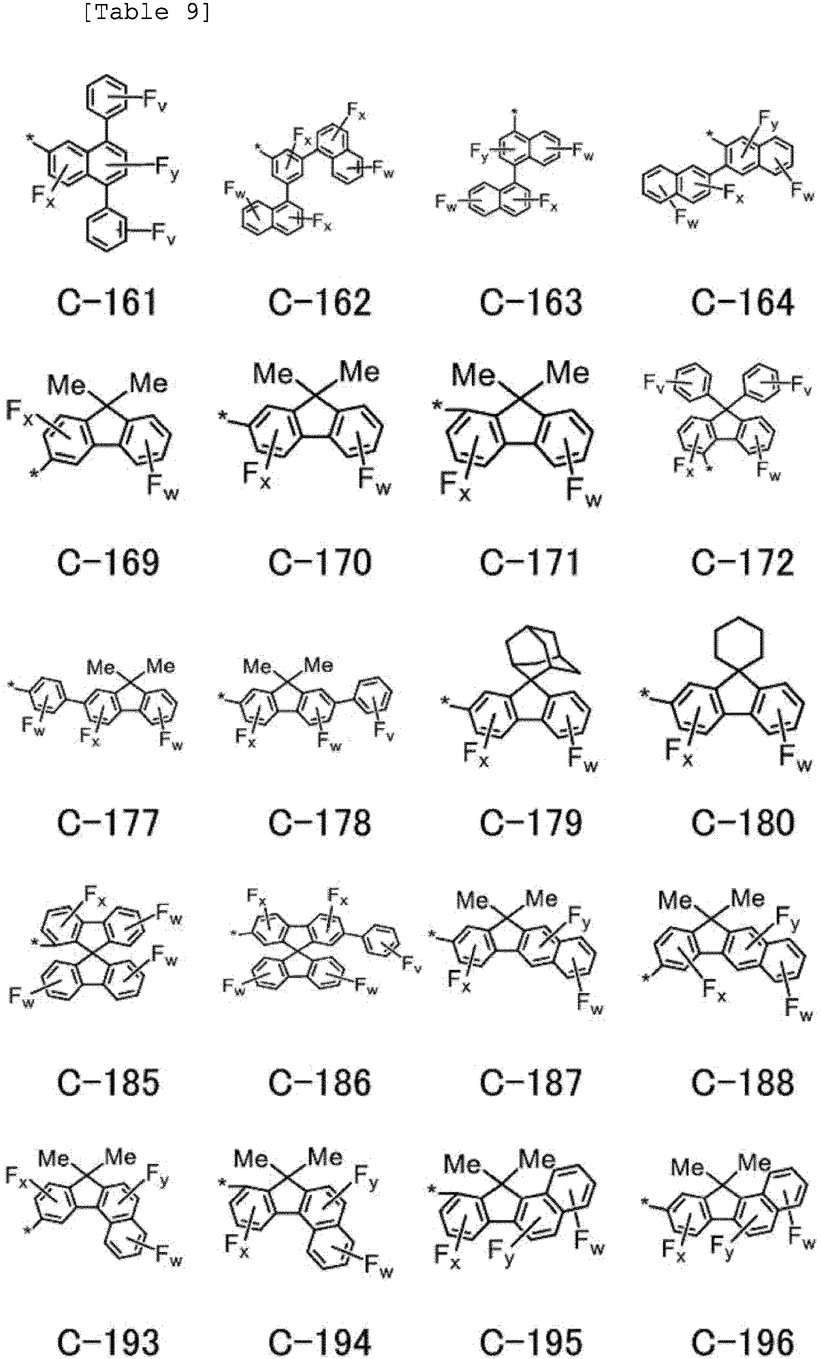

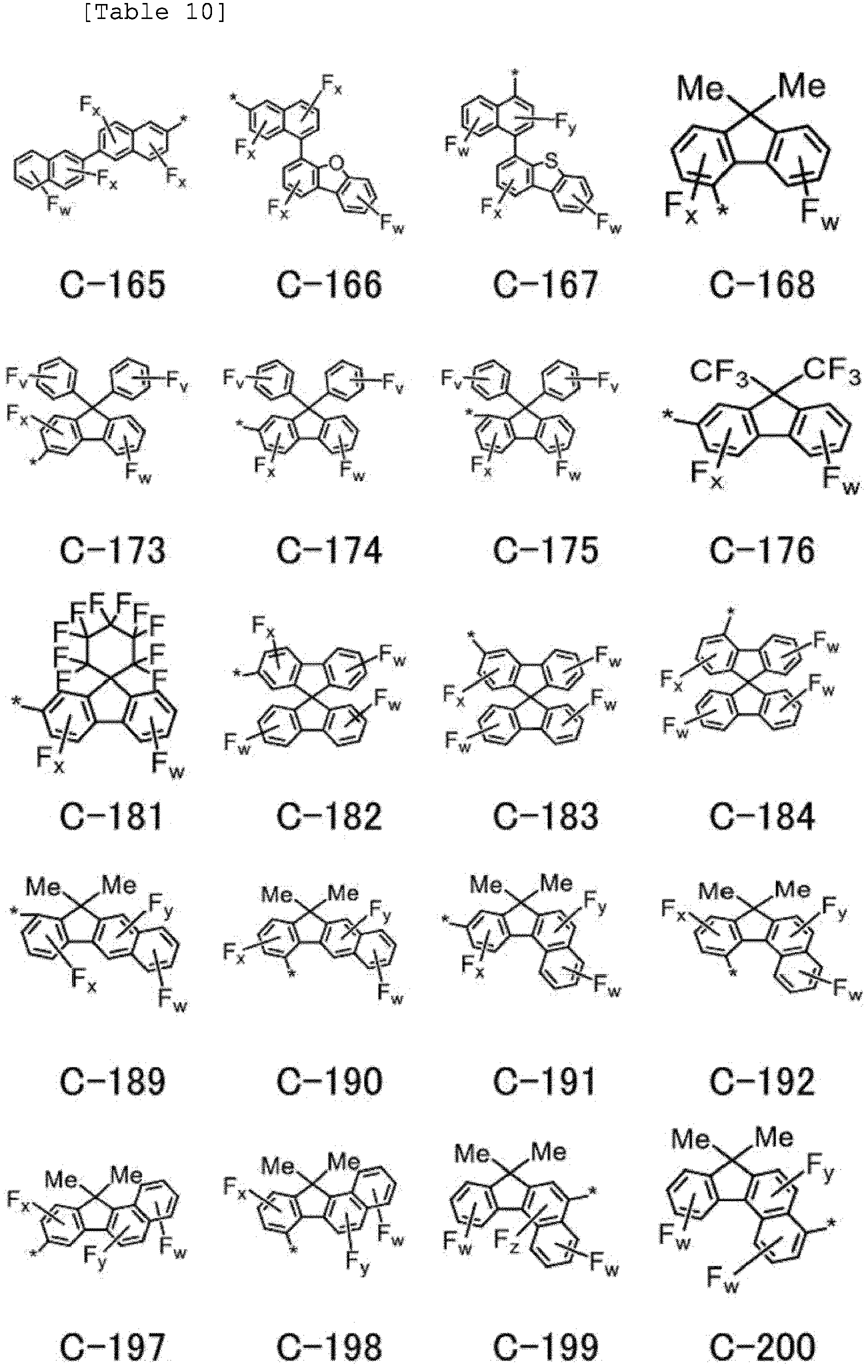

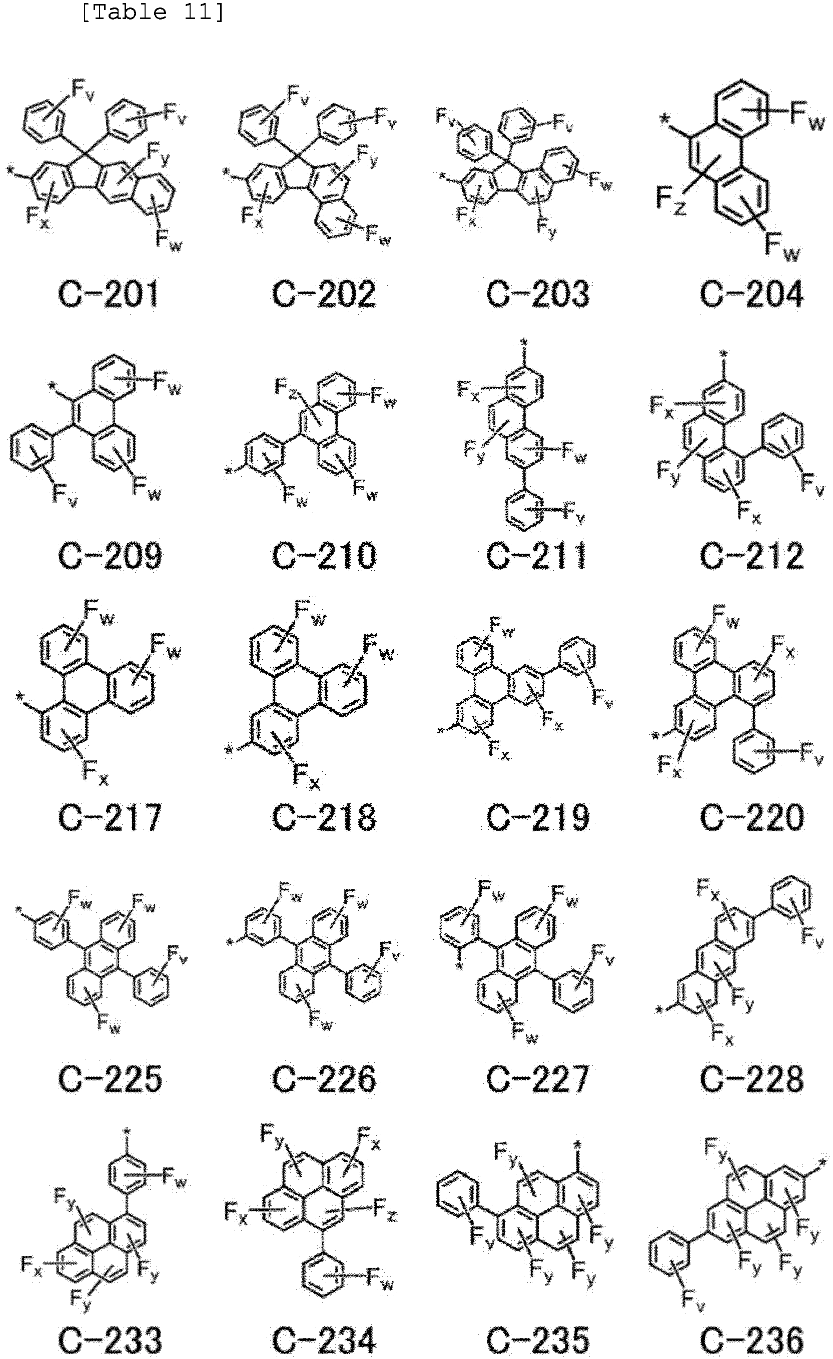

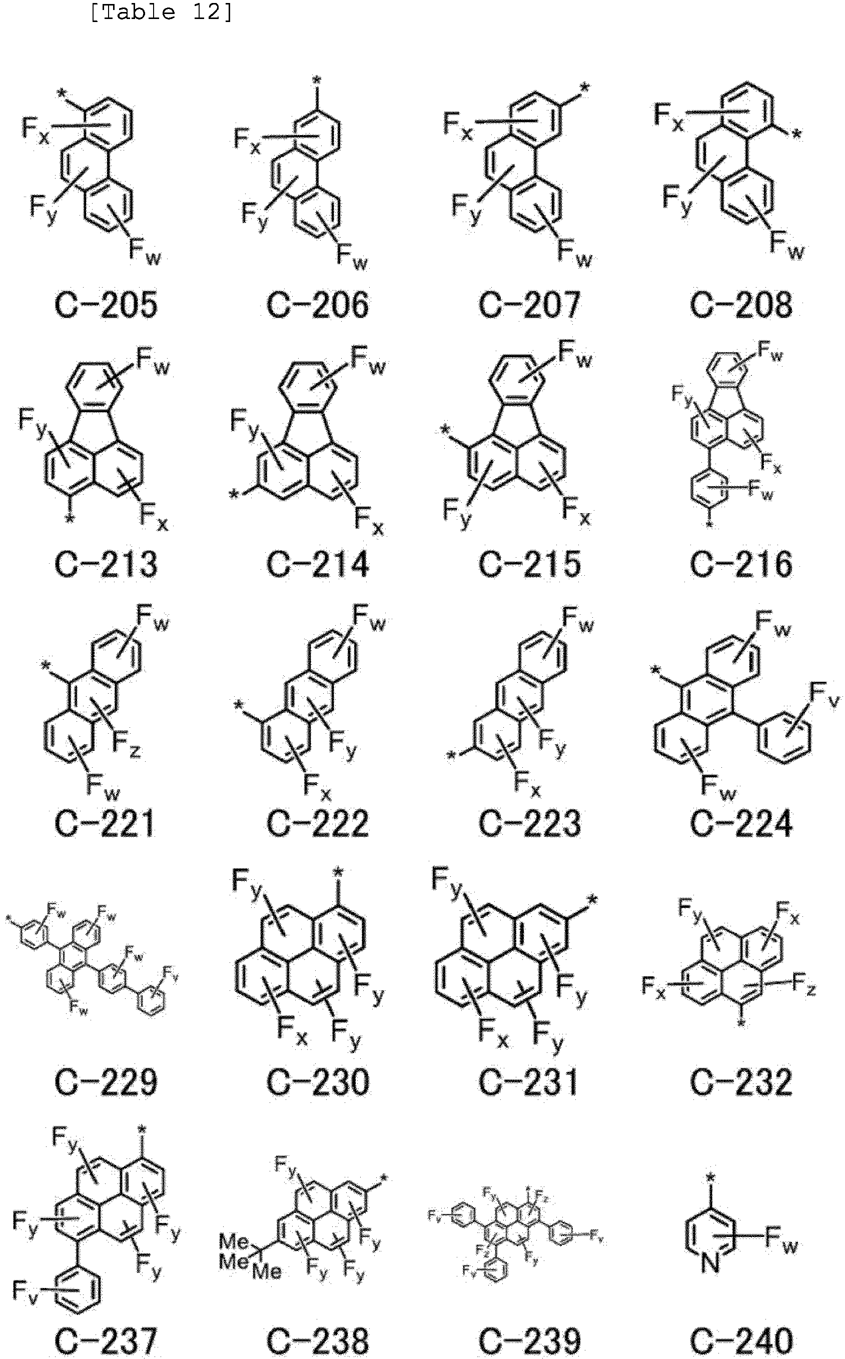

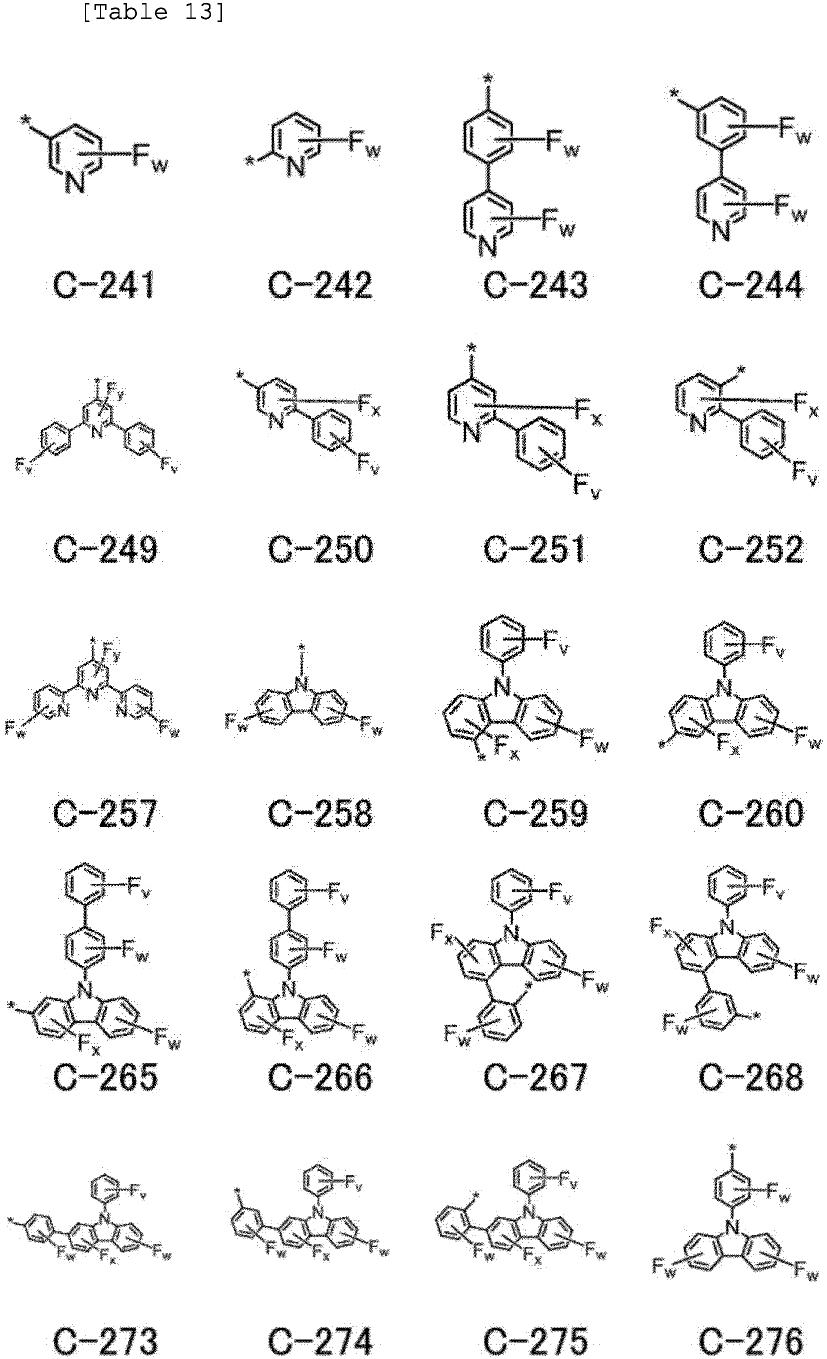

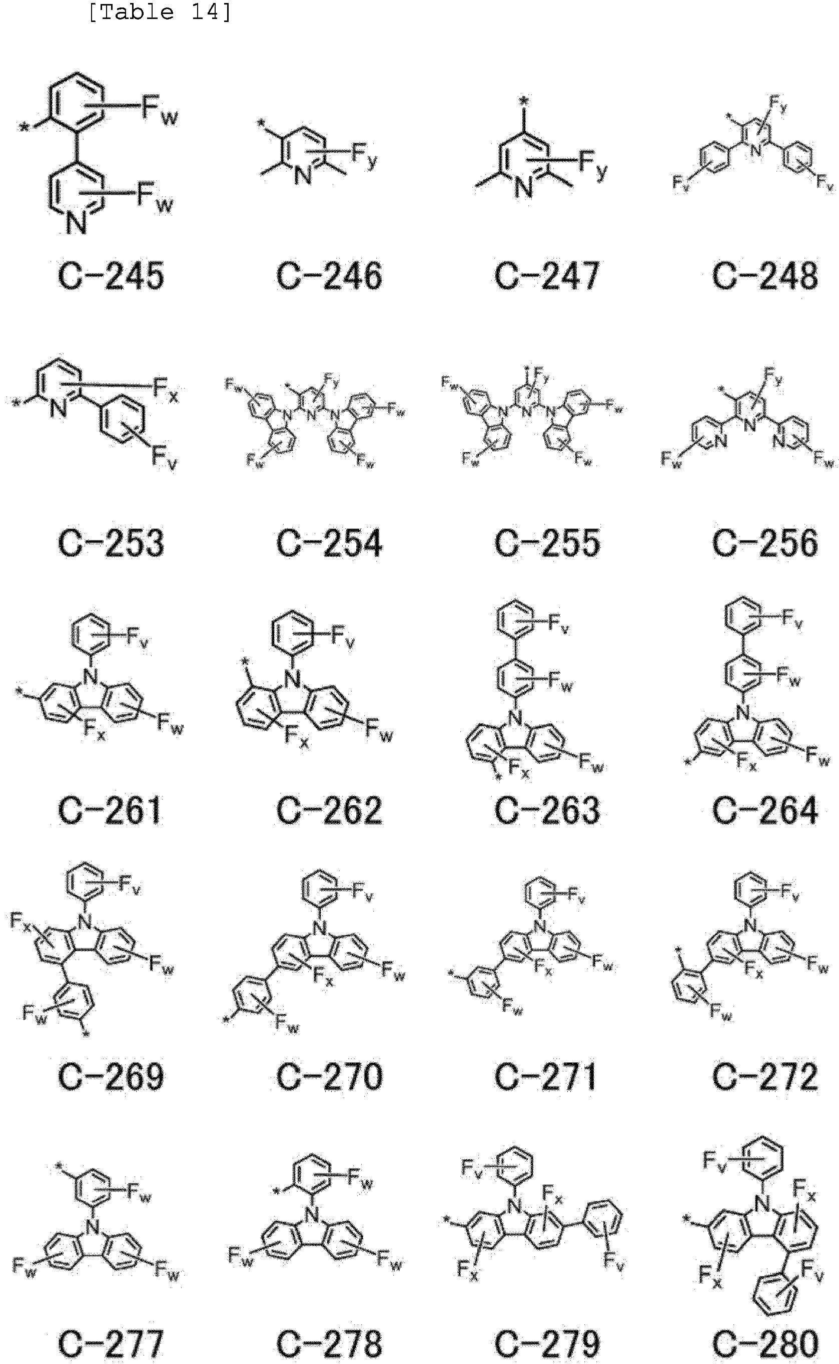

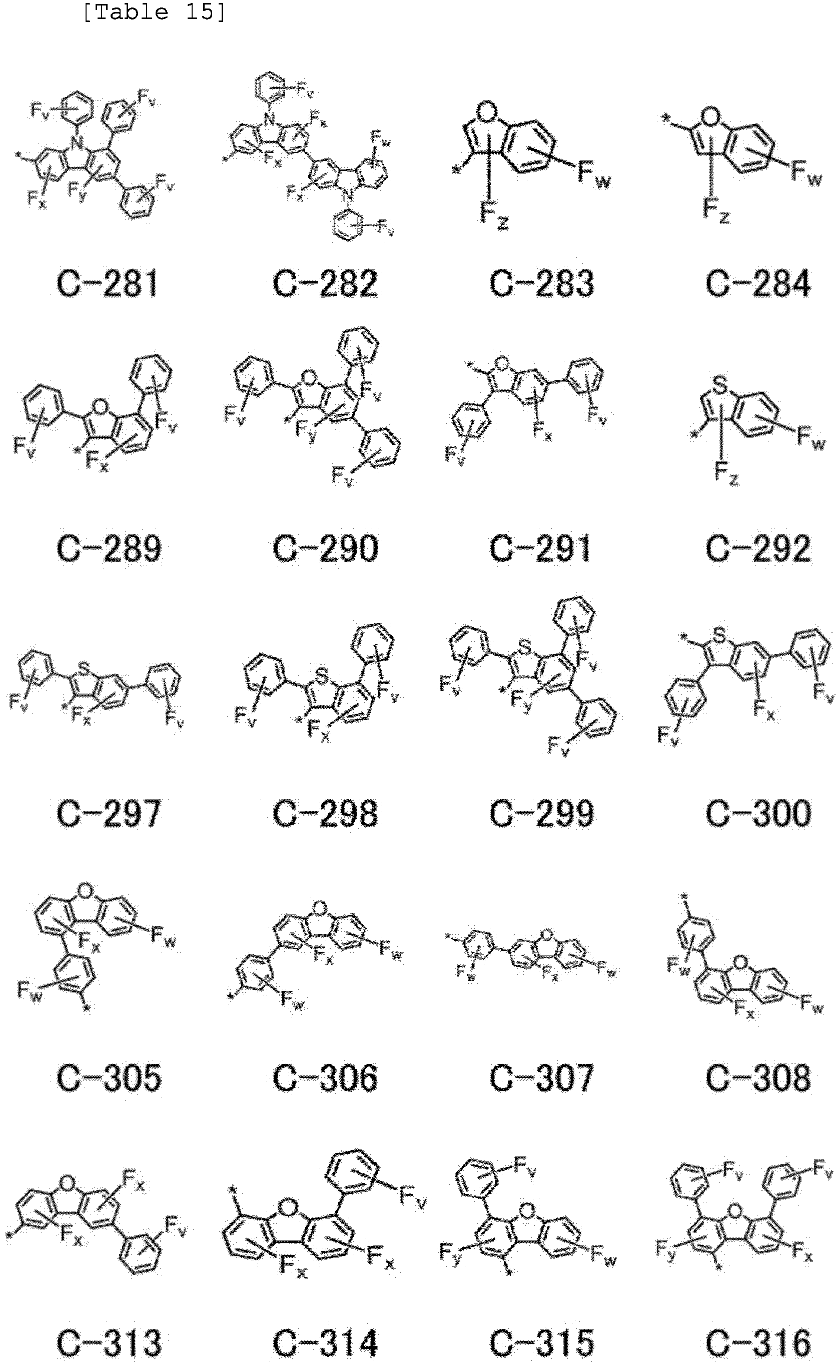

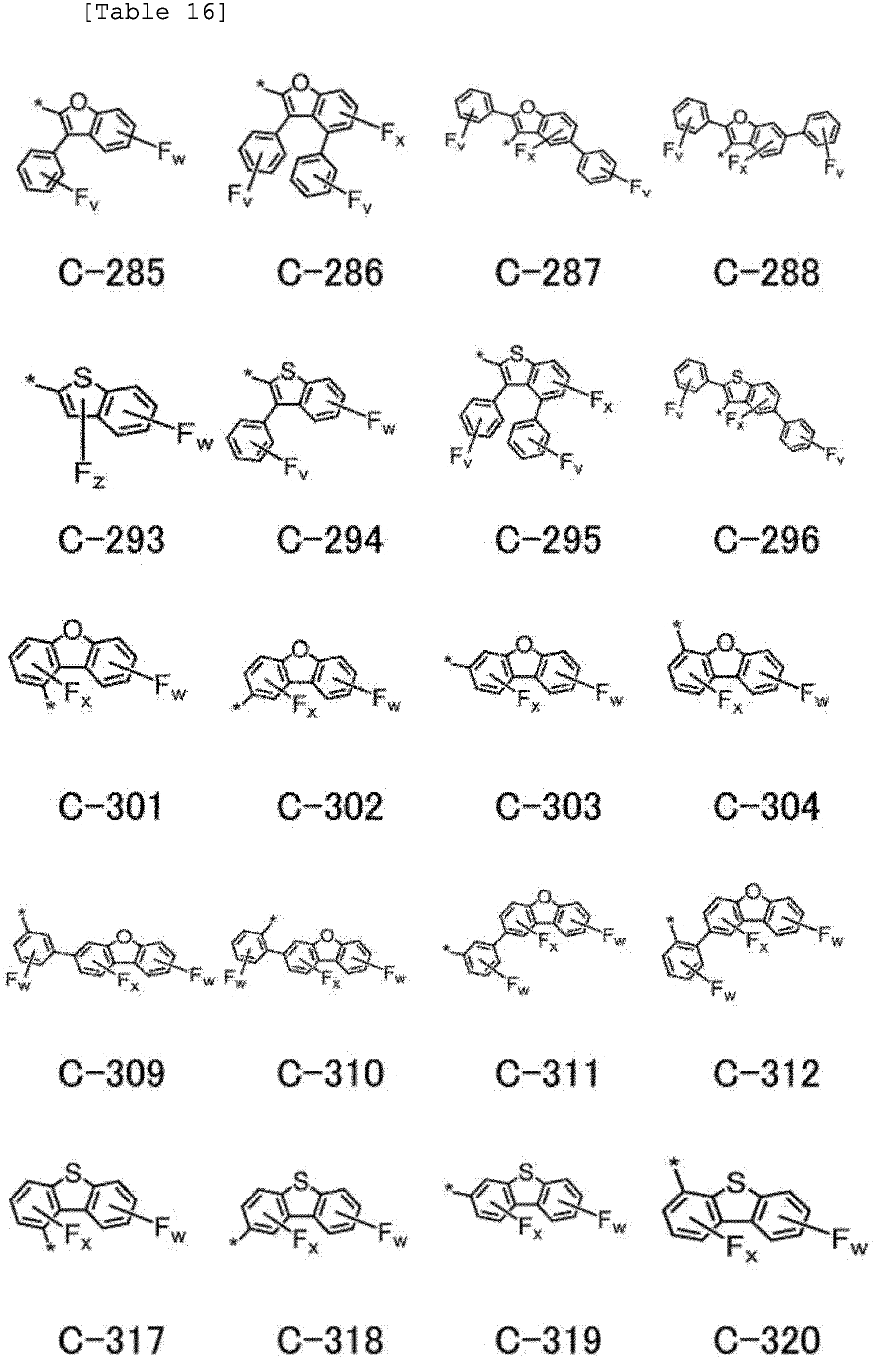

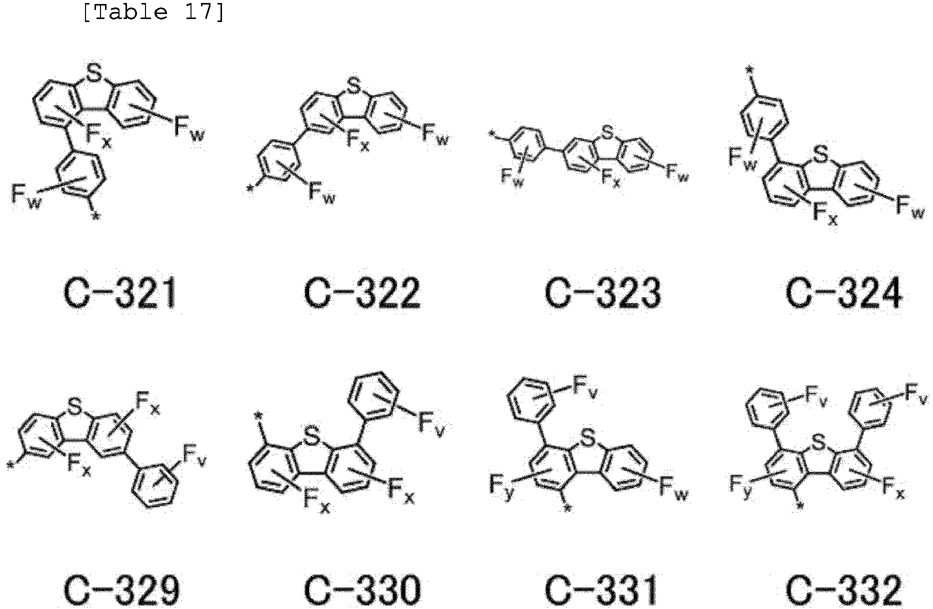

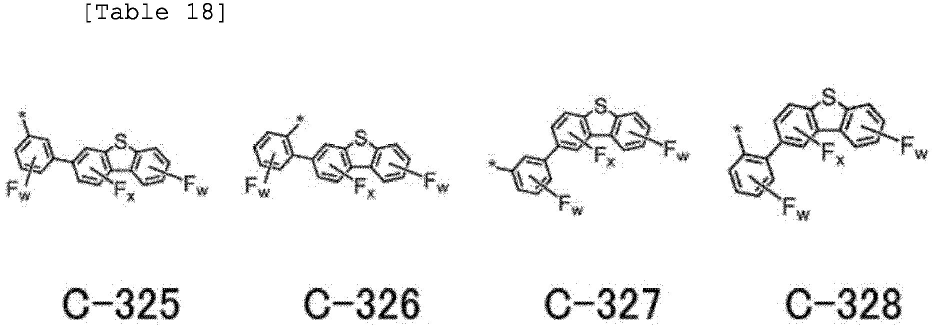

Due to having a high glass transition temperature and being able to suppress formation of a metal film on the film surface, Ar¹ to Ar¹⁴ each independently more preferably represent
(i') a phenyl group, a biphenylyl group, a terphenylyl group, a naphthyl group, a fluorenyl group, a spirobifluorenyl group, a phenanthryl group, a fluoranthenyl group, a triphenylenyl group, an anthryl group, a pyrenyl group, a pyridyl group, a carbazolyl group, a dibenzofuranyl group, or a dibenzothienyl group; or
(ii') a group in which the group represented by (i') is substituted with one or more groups selected from the group consisting of a methyl group, a trifluoromethyl group, a perfluoroalkyl group having 2 to 10 carbon atoms, a fluorine atom, and a phenyl group optionally substituted with a fluorine atom.

Due to having a high glass transition temperature and being able to suppress formation of a metal film on the film surface, Ar¹ to Ar¹⁴ each particularly preferably independently represent
a phenyl group, a phenyl group having at least one fluorine atom, a phenyl group having at least one trifluoromethyl group, a biphenylyl group, a biphenylyl group having at least one fluorine atom, a biphenylyl group having at least one trifluoromethyl group, a terfenylyl group, a terfenylyl group having at least one fluorine atom, a terfenylyl group having at least one trifluoromethyl group, a naphthyl group, a 9,9-dimethylfluorenyl group, a 9,9-diphenylfluorenyl group, a spirobiflurenyl group, a phenanthryl group, a carbazol-9-yl group, a 9-phenylcarbazolyl group, a dibenzofuranyl group, or a dibenzothienyl group.

Although specific examples of L¹ to L¹⁸ are not particularly limited, they include a 1,4-phenylene group, 1,3-phenylene group, 1,2-phenylene group, monofluoro -1,4-phenylene group, difluoro-1,4-phenylene group, trifluoro-1,4-phenylene group, tetrafluoro-1,4-phenylene group, monofluoro-1,4-phenylene group, monofluoro-1,3-phenylene group, difluoro -1,3-phenylene group, trifluoro-1,3-phenylene group, tetrafluoro-1,3-phenylene group, monofluoro-1,2-phenylene group, difluoro-1,2-phenylene group, trifluoro-1,2-phenylene group, tetrafluoro-1,2-phenylene group; 3-trifluoromethyl-1,2-phenylene group, 4-trifluoromethyl-1,2-phenylene group, 2-trifluoromethyl-1,3-phenylene group, 4-trifluoromethyl-1,3-phenylene group, 5-trifluoromethyl-1,3-phenylene group, 2-trifluoromethyl-1,4-phenylene group, 4,4'-biphenylene group, 4,3'-biphenylene group, 4,2'-biphenylene group, 3,3'-biphenylene group; 3,2'-biphenylylene group, 2,2'-biphenylylene group, monofluoro-4,4'-biphenylylene group, difluoro-4,4'-biphenylylene group, trifluoro-4,4'-biphenylylene group, tetrafluoro-4,4'-biphenylylene group, pentafluoro-4,4'-biphenylylene group, hexafluoro-4,4'-biphenylylene group; heptafluoro-4,4'-biphenylylene group, octafluoro-4,4'-biphenylylene group, monofluoro-4,3'-biphenylylene group, difluoro-4,3'-biphenylylene group, trifluoro-4,3'-biphenylylene group, tetrafluoro-4,3'-biphenylylene group, pentafluoro-4,3'-biphenylylene group, hexafluoro-4,3'-biphenylylene group, heptafluoro-4,3'-biphenylylene group; octafluoro-4,3'-biphenylylene group, monofluoro-4,2'-biphenylylene group, difluoro-4,2'-biphenylylene group, trifluoro-4,2'-biphenylylene group, tetrafluoro-4,2'-biphenylylene group, pentafluoro-4,2'-biphenylylene group, hexafluoro-4,2'-biphenylylene group, heptafluoro-4,2'-biphenylylene group, octafluoro-4,2'-biphenylylene group; monofluoro-3,3'-biphenylylene group, difluoro-3,3'-biphenylylene group, trifluoro-3,3'-biphenylylene group, tetrafluoro-3,3'-biphenylylene group, pentafluoro-3,3'-biphenylylene group, hexafluoro-3,3'-biphenylylene group; heptafluoro-3,3'-biphenylylene group, octafluoro-3,3'-biphenylylene group, monofluoro-3,2'-biphenylylene group, difluoro-3,2'-biphenylylene group, trifluoro-3,2'-biphenylylene group, tetrafluoro-3,2'-biphenylylene group, pentafluoro-3,2'-biphenylylene group, hexafluoro-3,2'-biphenylylene group, heptafluoro-3,2'-biphenylylene group; octafluoro-3,2'-biphenylylene group, monofluoro-2,2'-biphenylylene group, difluoro-2,2'-biphenylylene group, trifluoro-2,2'-biphenylylene group, tetrafluoro-2,2'-biphenylylene group, pentafluoro-2,2'-biphenylylene group, hexafluoro-2,2'-biphenylylene group, heptafluoro-2,2'-biphenylylene group, octafluoro-2,2'-biphenylylene group; 2,2'-dimethyl-4,4'-biphenylene group, 2,2'-di (trifluoromethyl) -4,4'-biphenylene group 4,4"-p-terphenylene group, perfluoro-4,4"-p-terphenylene group, 4,3"-p-terphenylene group, perfluoro-4,3"-p-terphenylene group, 4,2"-p-terphenylene group, perfluoro-4,2"-p-terphenylene group, 3,3"-p-terphenylene group; perfluoro-3,3"-p-terphenylene group, 3,2"-p-terphenylene group, perfluoro-3,2"-p-terphenylene group, 2,2"-p-terphenylene group, perfluoro-2,2"-p-terphenylene group, 4,2'-p-terphenylene group, perfluoro-4,2'-p-terphenylene group, 4,3'-p-terphenylene group, perfluoro-4,3'-p-terphenylene group, 3,2'-p-terphenylene group; perfluoro-3,2'-p-terphenylene group, 3,3'-p-terphenylene group, perfluoro-3,3'-p-terphenylene group, 2,3'-p-terphenylene group, perfluoro-2,3'-p-terphenylene group; 2,2'- P-terphenylene group, perfluoro-2,2'-p-terphenylene group, 4,4"-m-terphenylene group, 4,3"-m-terphenylene group, 4,2"-m-terphenylene group, 3,3"-m-terphenylene group, 3,2"-m-terphenylene group, 2,2‴-m-terphenylene group, 2,2"-m-terphenylene group, 4,2'-m-terphenylene group, 4,4'-m-terphenylene group, 4,5'-m-terphenylene group, 4,6'-m-terphenylene group; 3,2'-m-terphenylene group, 3,4'-m-terphenylene group, 3,5'-m-terphenylene group, 3,6'-m-terphenylene group, 2,2'-m-terphenylene group, 2,4'-m-terphenylene group, 2,5'-m-terphenylene group, 2,6'-m-terphenylene group, perfluoro - 4,4"-m-terphenylene group, perfluoro-4,3"-m-terphenylene group, perfluoro-4,2"-m-terphenylene group; perfluoro-3,3"-m-terphenylene group, perfluoro-3,2"-m-terphenylene group, perfluoro-2,2"-m-terphenylene group, perfluoro-4,2'-m-terphenylene group, perfluoro-4,4'-m-terphenylene group, perfluoro-4,5'-m-terphenylene group, perfluoro-4,6'-m-terphenylene group, perfluoro-3,2'-m-terphenylene group; perfluoro-3,4'-m-terphenylene group, perfluoro-3,5'-m-terphenylene group, perfluoro-3,6'-m-terphenylene group, perfluoro-2,2'-m-terphenylene group, perfluoro-2,4'-m-terphenylene group, perfluoro-2,5'-m-terphenylene group, perfluoro-2,6'-m-terphenylene group, 4,4"-o-terphenylene group, 4,3"-o-terphenylene group, 4,2"-o-terphenylene group, 3,3"-o-terphenylene group, 3,3"-o-terphenylene group, 2,2"-o-terphenylene group, 2,2' ''-o-terphenylene group, 4,3'-o-terphenylene group, 4,4'-o-terphenylene group, 4,5'-o-terphenylene group, 4,6'-o-terphenylene group, 3,3'-o-terphenylene group, 3,4'-o-terphenylene group, 3,5'-o-terphenylene group, 3,6'-o-terphenylene group, 3,6'-o-terphenylene group, 2,3'-o-terphenylene group, 2,4'-o-terphenylene group, 2,5'-o-terphenylene group, 2,6'-o-terphenylene group, perfluoro -4,4"-o-terphenylene group, perfluoro-4,3"-o-terphenylene group, perfluoro-4,2"-o-terphenylene group, perfluoro-3,3"-o-terphenylene group, perfluoro-3,2"-o-terphenylene group, perfluoro-2,2"-o-terphenylene group; perfluoro-4,3'-o-terphenylene group, perfluoro-4,4'-o-terphenylene group, perfluoro-4,5'-o-terphenylene group, perfluoro-4,6'-o-terphenylene group, perfluoro-3,3'-o-terphenylene group, perfluoro-3,4'-o-terphenylene group, perfluoro-3,5'-o-terphenylene group, perfluoro-3,6'-o-terphenylene group; perfluoro-2,3'-o-terphenylene group, perfluoro-2,4'-o-terphenylene group, perfluoro-2,5'-o-terphenylene group, perfluoro-2,6'-o-terphenylene group, 1,2-naphthylene group, 1,3-naphthylene group, 1,4-naphthylene group, monofluoro-1,4-naphthylene group, difluoro-1,4-naphthylene group, trifluoro-,4-naphthylene group, tetrafluoro-1,4-naphthylene group; hexafluoro-1,4-naphthylene group, 1,5-naphthylene group, 1,6-naphthylene group, 1,7-naphthylene group, 1,8-naphthylene group, 2,3-naphthylene group, 2,4-naphthylene group, 2,5-naphthylene group, 2,6-naphthylene group, monofluoro-2,6-naphthylene group, difluoro-2,6-naphthylene group, trifluoro-2,6-naphthylene group, tetrafluoro-2,6-naphthylene group, pentafluoro-2,6-naphthylene group; hexafluoro-2,6-naphthylene group, 2,7-naphthylene group, 2,8-naphthylene group, 9,9-dimethyl-fluorene-2,7-diyl group, 9,9-di (trifluoromethyl)-fluorene-2,7-diyl group, 9,9-di (pentafluoroethyl)-fluorene-2,7-diyl group, 9,9-di (heptafluoropropyl)-fluorene-2,7-diyl group, 9,9-di (nonafluorobutyl)-fluorene-2,7-diyl group, 9,9-di (perfluoropentyl)-fluorene-2,7-diyl group, and 9,9-di (perfluorohexyl)-fluorene-2,7-diyl group, 9,9-di (perfluoroheptyl)-fluorene-2,7-diyl group, 9,9-di (perfluorooctyl)-fluorene-2,7-diyl group, 9,9-dimethyl-fluorene-2,8-diyl group, 9,9-dimethyl-fluorene-2,4-diyl group, 9,9-dimethyl-fluorene-2,5-diyl group, 9,9-dimethyl-fluorene-2,6-diyl group; 9,9-dimethyl-fluorene-3,6-diyl group, 9,9-diphenyl-fluorene-2,7-diyl group, 9,9-di (pentafluorophenyl)-fluorene-2,7-diyl group, 9,9-diphenyl-fluorene-2,8-diyl group, 9,9-diphenyl-fluorene-2,4-diyl group, 9,9-diphenyl-fluorene-2,5-diyl group, 9,9-diphenyl-fluorene-2,6-diyl group; 9,9-diphenyl-fluorene-3,6-diyl group, spirobifluorene-2,7-diyl group, perfluorospirobifluorene-2,7-diyl group, spirobifluorene-2,8-diyl group, spirobifluorene-2,4-diyl group, spirobifluorene-2,5-diyl group, spirobifluorene-2,6-diyl group, spirobifluorene-3,6-diyl group, spirobifluorene-3,6-diyl group, phenanthrene-9,10-diyl group, phenanthrene-2,9-diyl group; phenanthrene-2,10-diyl group, phenanthrene-2,8-diyl group, phenanthrene-2,7-diyl group, phenanthrene-2,6-diyl group, phenanthrene-2,5-diyl group, phenanthrene-2,14-diyl group, phenanthrene-2,3-diyl group, phenanthrene-9,10-diyl group; phenanthrene-3,10-diyl group, phenanthrene-3,9-diyl group, phenanthrene-3,8-diyl group, phenanthrene-3,7-diyl group, phenanthrene-3,6-diyl group, phenanthrene-3,5-diyl group, phenanthrene-4,10-diyl group, phenanthrene-4,9-diyl group, phenanthrene-4,8-diyl group, phenanthrene-4,7-diyl group, phenanthrene-4,6-diyl group; triphenylene-1,3-diyl group, triphenylene-1,4-diyl group, triphenylene-1,5-diyl group, triphenylene-1,5-diyl group, triphenylene-1,6-diyl group, triphenylene-1,7-diyl group, triphenylene-1,8-diyl group, triphenylene-2,3-diyl group, triphenylene-2,4-diyl group, triphenylene-2,5-diyl group, triphenylene-2,6-diyl group, triphenylene-2,7-diyl group, triphenylene-3,5-diyl group, triphenylene-3,6-diyl group, triphenylene-4,6-diyl group, triphenylene-4,6-diyl group, pyrene-1,3-diyl group, pyrene-1,6-diyl group, pyrene-1,8-diyl group, pyrene-2,7-diyl group, anthracene-2,6-diyl group, anthracene-9,10-diyl group, perfluoroanthracene-9,10-diyl group, 9,10-diphenylanthracenediyl group, pyridine-2,3-diyl group; pyridine-2,4-diyl group, pyridine-2,5-diyl group, pyridine-2,6-diyl group, pyridine-2,6-diyl group, pyridine-3,4-diyl group, pyridine-3,5-diyl group, perfluoropyridine-2,3-diyl group, perfluoropyridine-2,4-diyl group, perfluoropyridine-2,5-diyl group, perfluoropyridine-2,6-diyl group, perfluoropyridine-3,4-diyl group, perfluoropyridine-3,5-diyl group, a dibenzofuran-2,7-diyl group, a dibenzofuran-4,5-diyl group, a dibenzothiophene-2,7-diyl group, a dibenzothiophene-4,5-diyl group, and a 9-phenylcarbazole-2,7-diyl group.

In Formulas (1) to (3), due to having a high glass transition temperature and being able to suppress the formation of a metal film on the surface of the film, L¹ to L¹⁸ each preferably independently represent
(iii) a phenylene group, biphenylylene group, terphenylene group, naphthylene group, pyridylene group, or fluorenylene group;
(iv) the group represented by (iii) is a methyl group, an ethyl group, a methoxy group, an ethoxy group, a trifluoromethyl group, a trifluoromethoxy group, a perfluoroalkyl group having 2 to 10 carbon atoms, a perfluoroalkoxy group having 2 to 10 carbon atoms, a cyano group, a deuterium atom, a fluorine atom, a phenyl group optionally substituted with a fluorine atom, a biphenylyl group optionally substituted with a fluorine atom, a naphthyl group optionally substituted with a fluorine atom, a phenanthryl group optionally substituted with a fluorine atom, a pyridyl group optionally substituted with a fluorine atom, a carbazolyl group optionally substituted with a fluorine atom, a dibenzothienyl group optionally substituted with a fluorine atom, and a dibenzofuranyl group optionally substituted with a fluorine atom; or
(v) a single bond.

Due to having a high glass transition temperature and being able to suppress formation of a metal film on the film surface, L¹ to L¹⁸ more preferably has the following substituents. It should be noted that F represents a fluorine atom, v represents an integer of 0 to 5, w represents an integer of 0 to 4, x represents an integer of 0 to 3, y represents an integer of 0 to 2, and z represents an integer of 0 to 1.

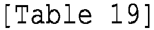

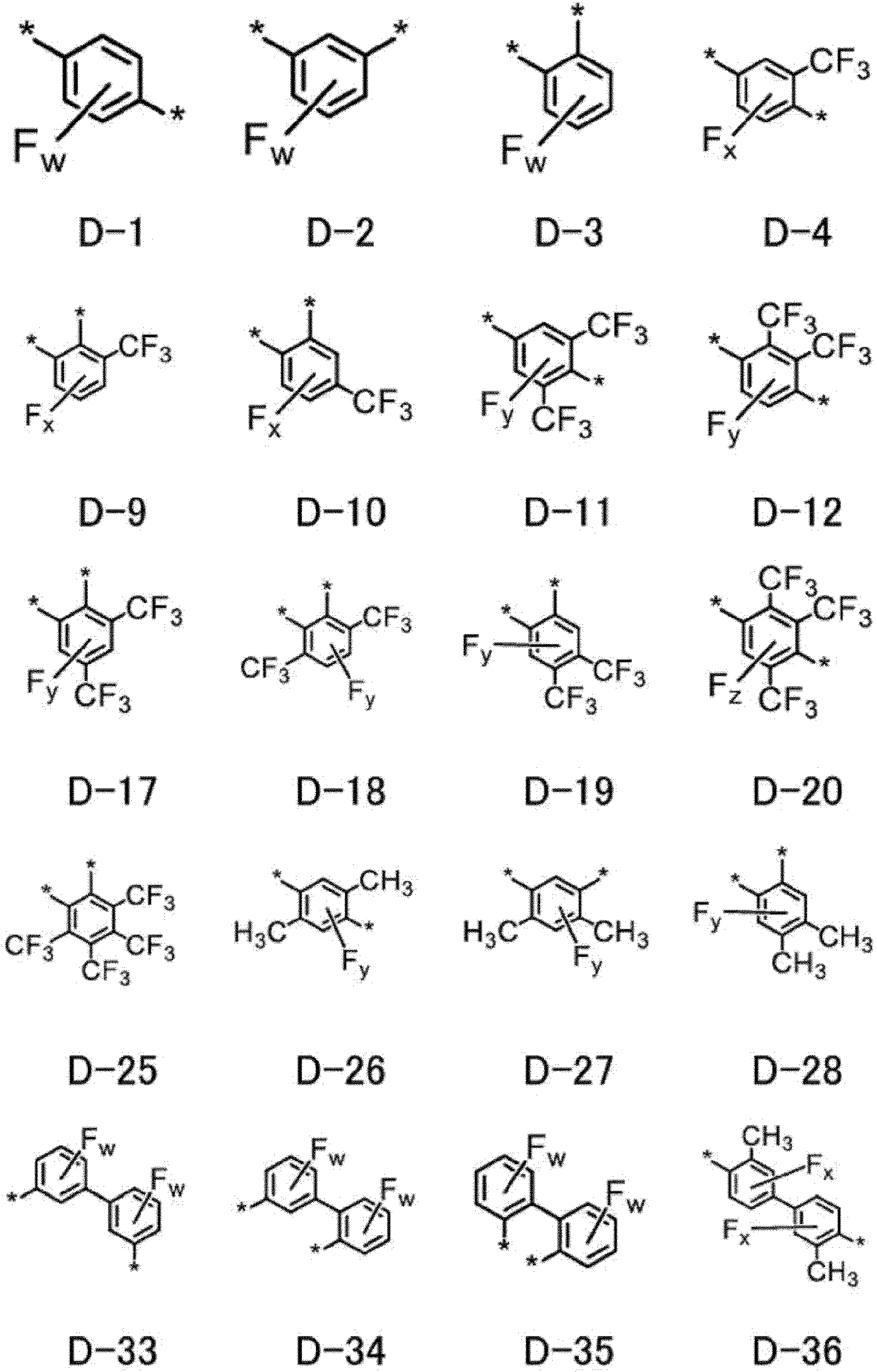

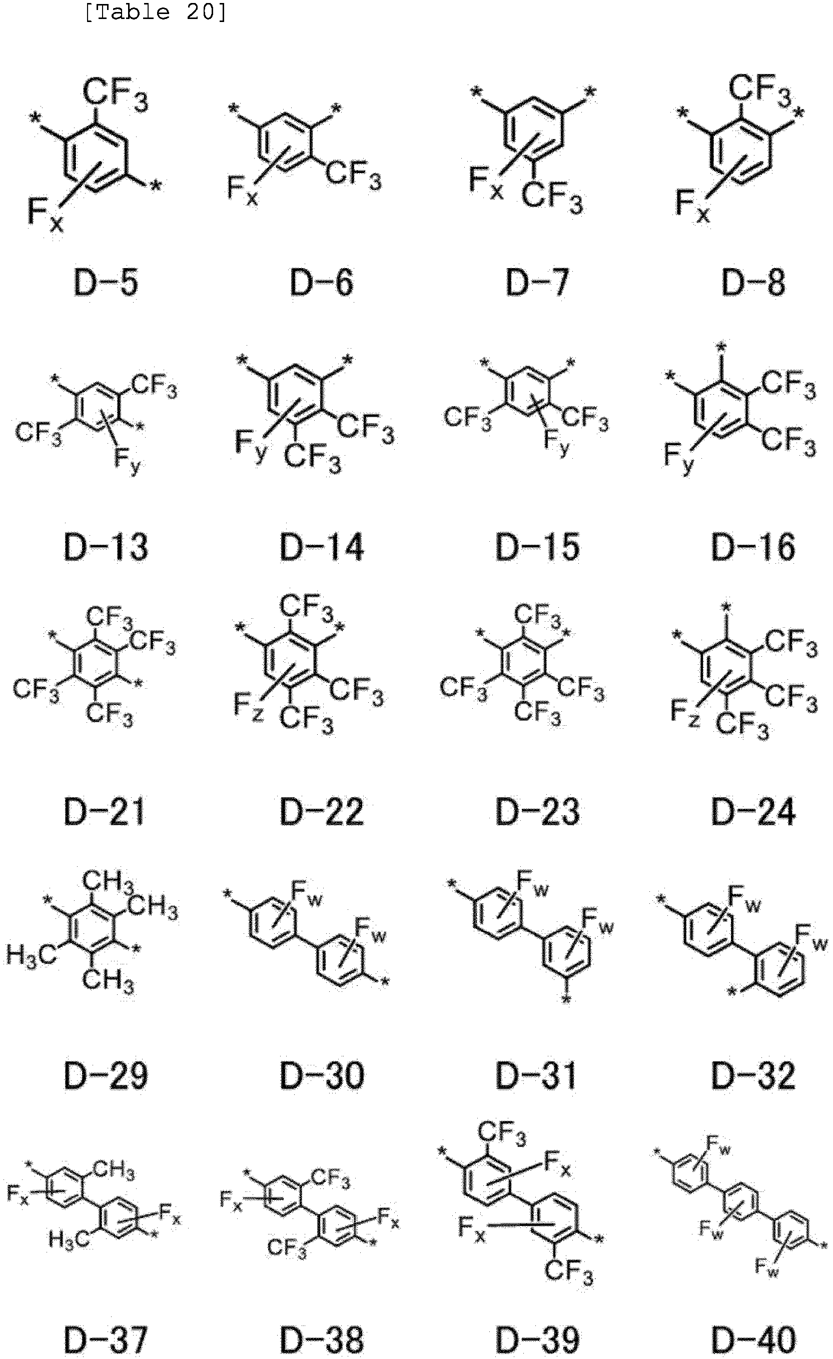

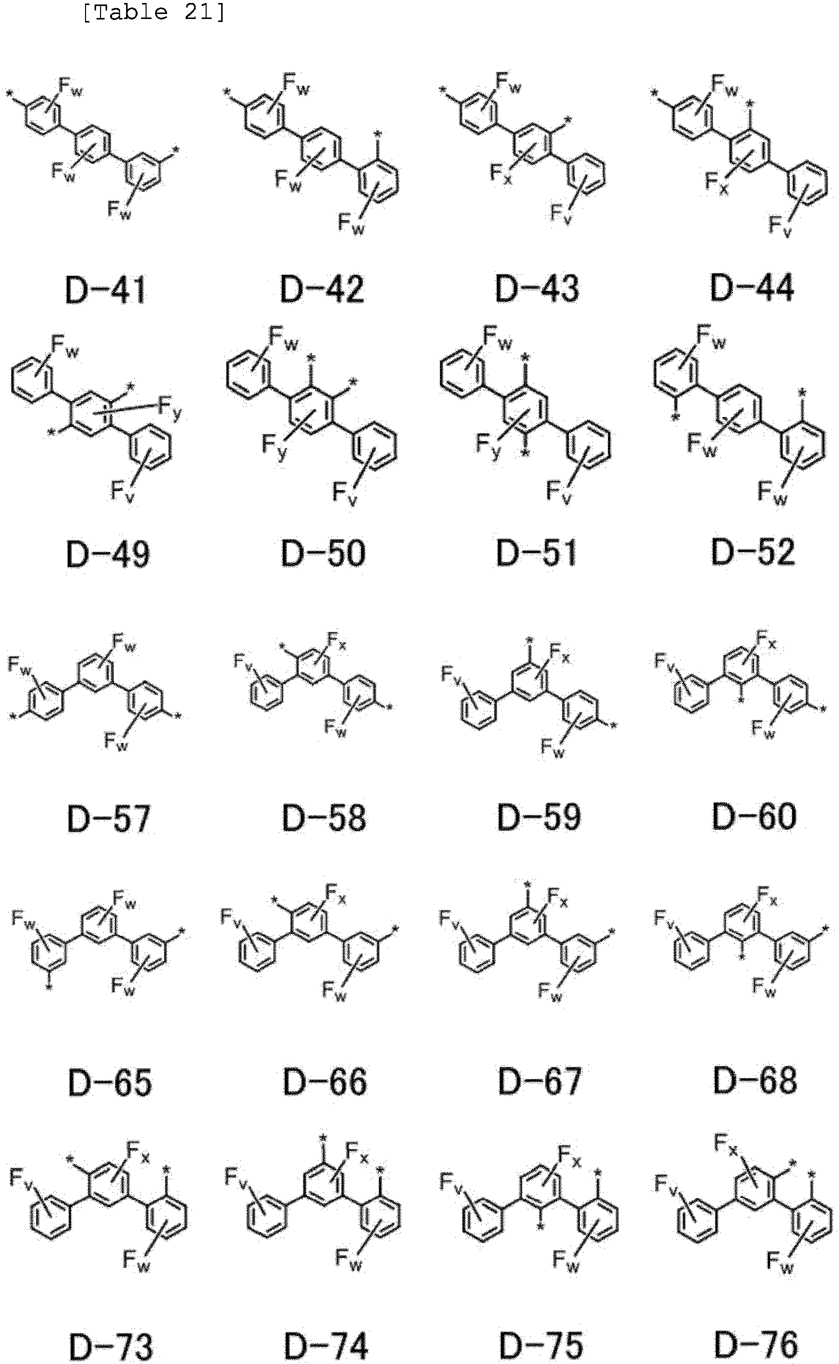

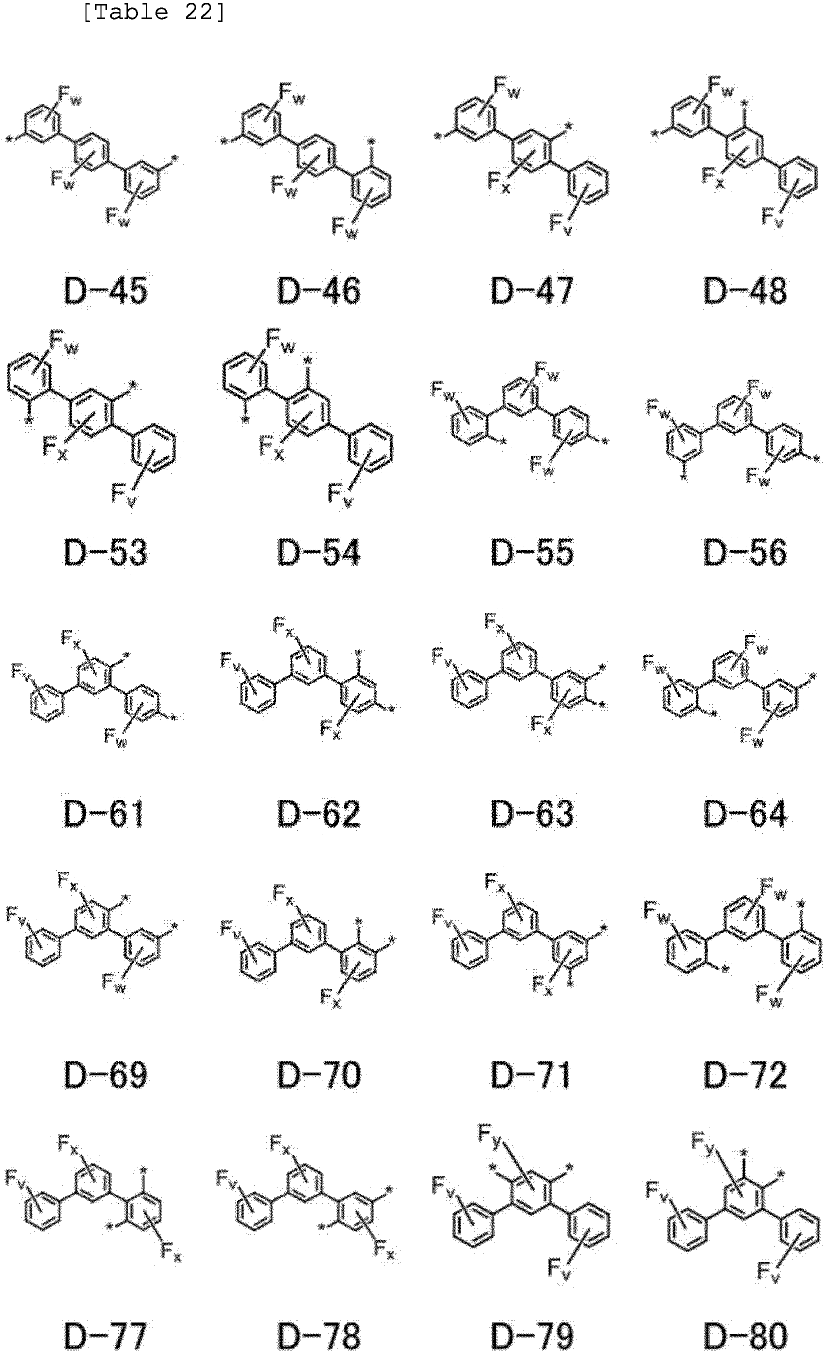

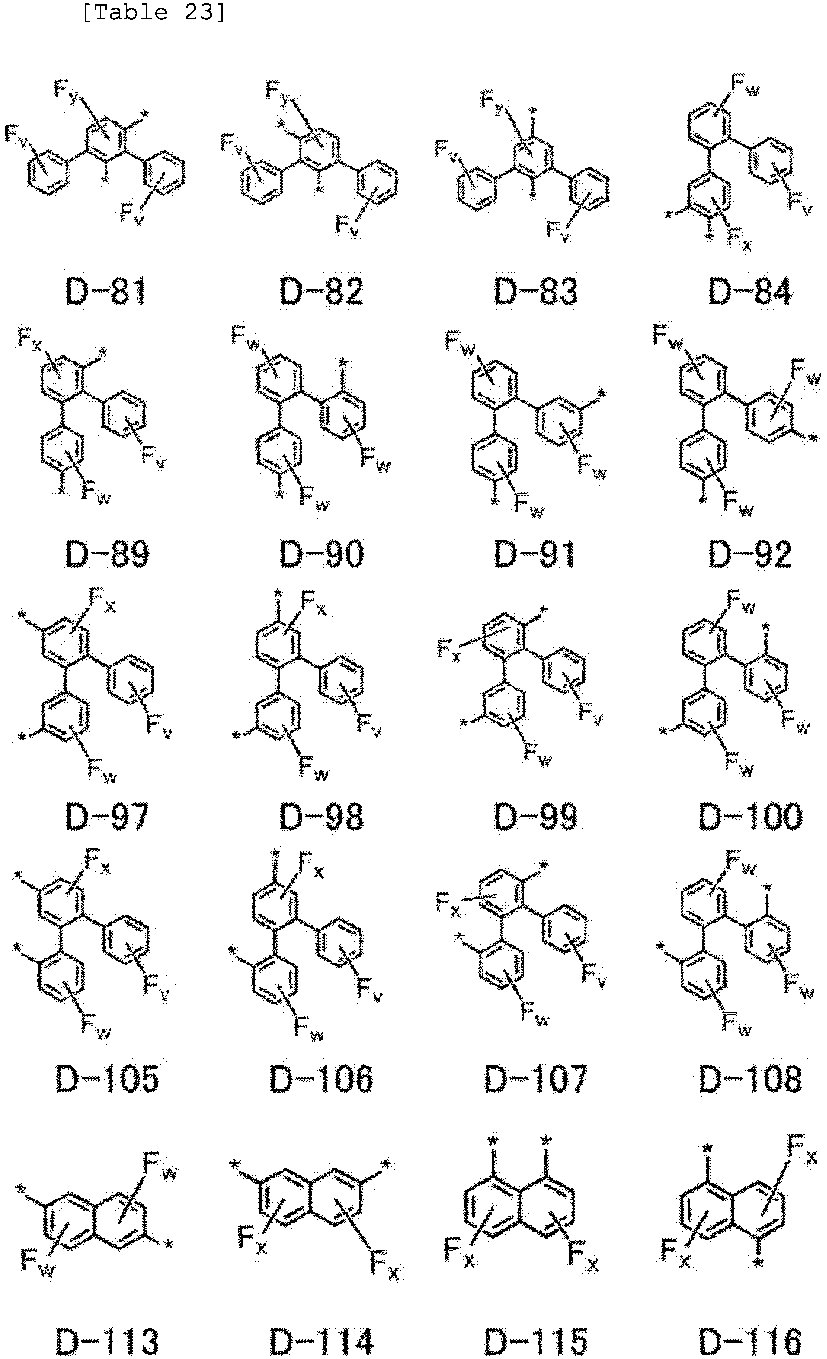

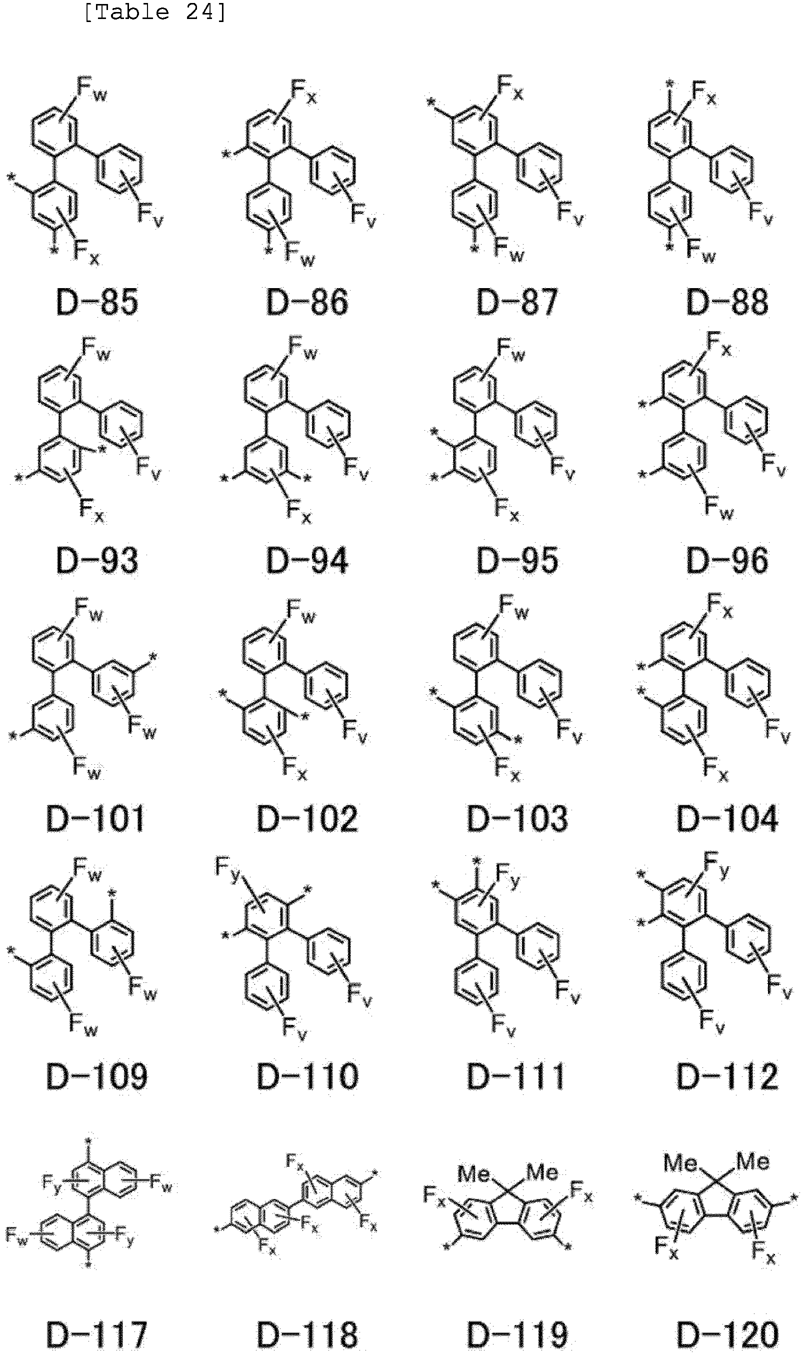

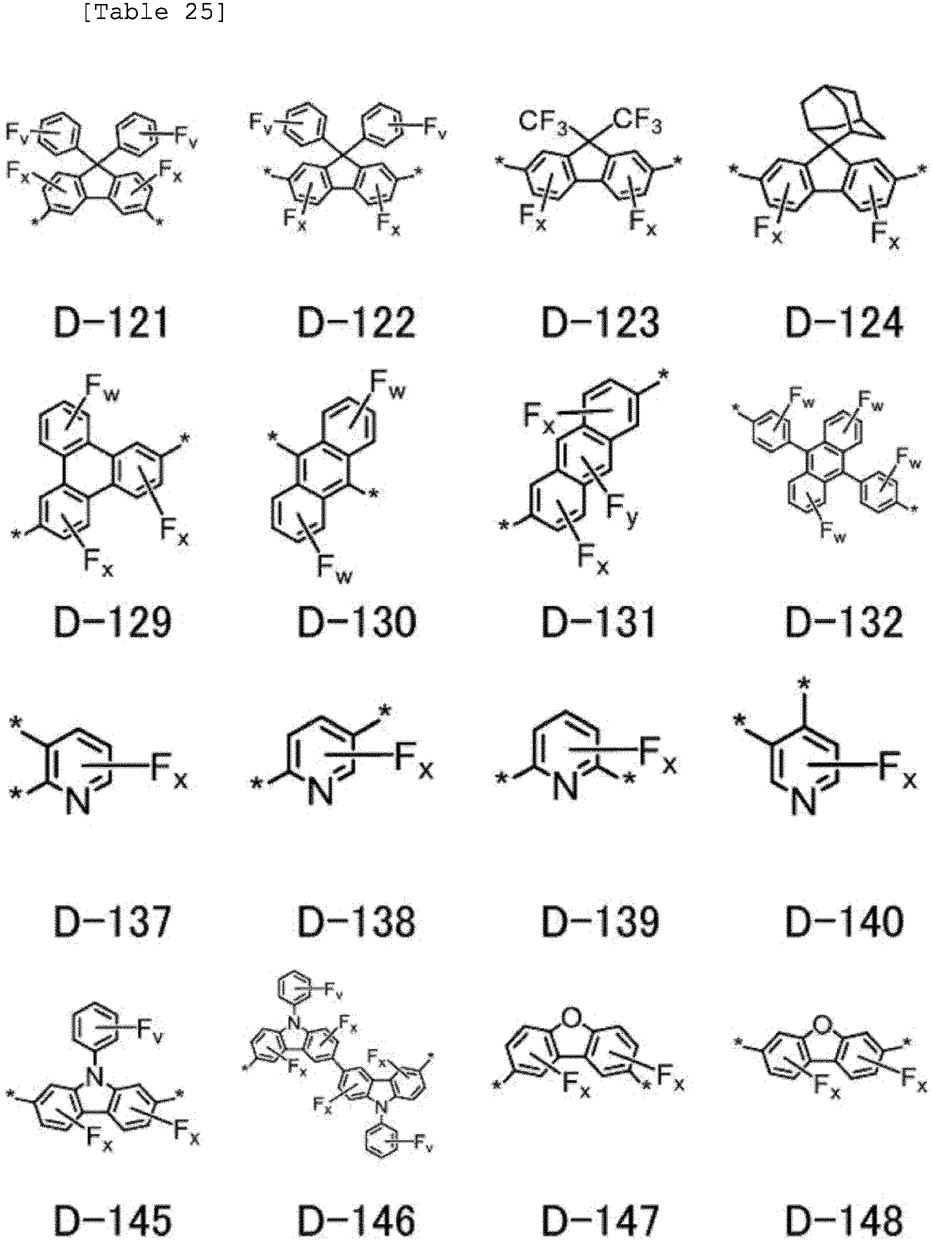

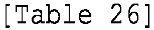

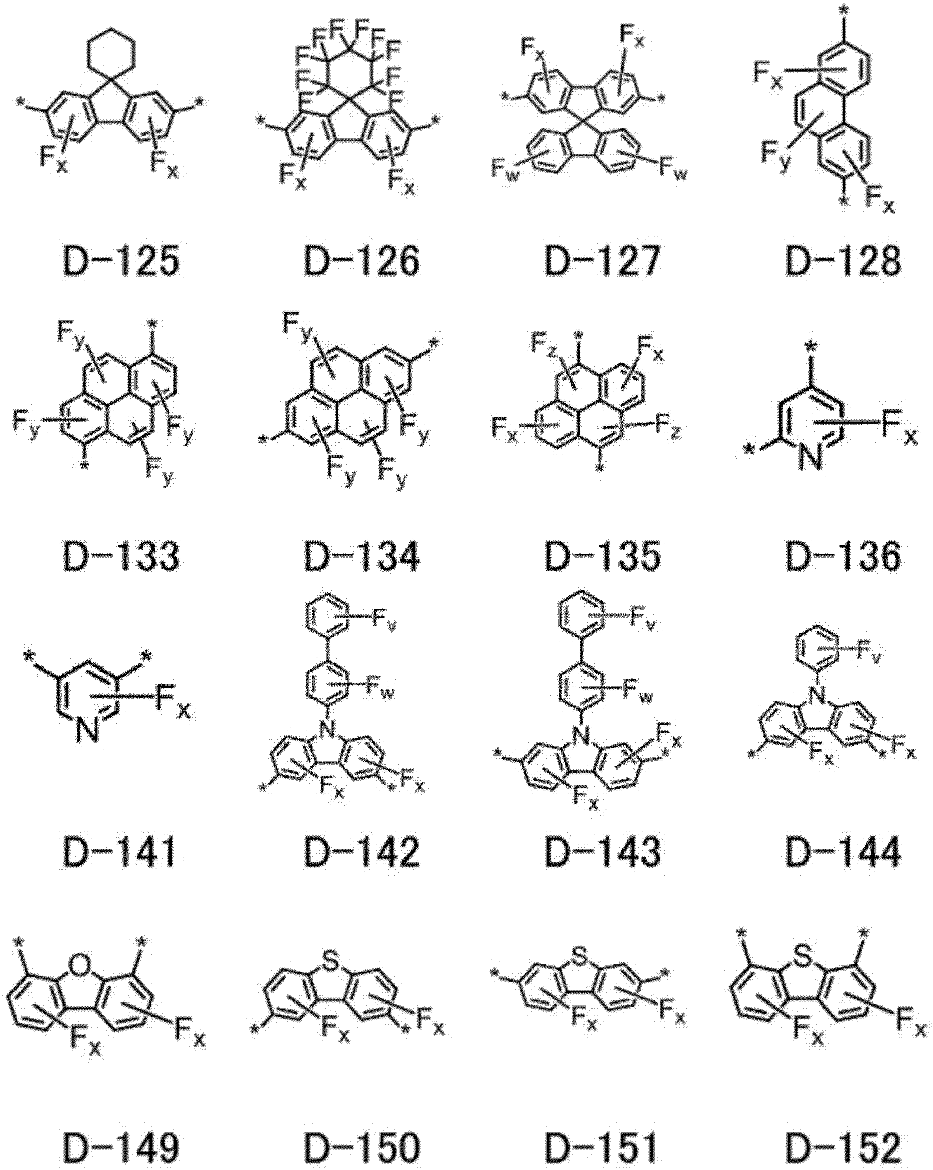

In Formulas (1) to (3), due to having a high glass transition temperature and being able to suppress the formation of a metal film on the surface of the film, L¹ to L¹⁸ each more preferably independently represent:
(iii') a phenylene group, a biphenylylene group, a terphenylylene group, a naphthylene group, a pyridylene group, or a fluorenylene group;
(iv') a group in which the group represented by (iii) is substituted with one or more groups selected from the group consisting of a methyl group, a trifluoromethyl group, a fluorine atom and a phenyl group optionally substituted with a fluorine atom; or
(v') a single bond.

In Formulas (1) to (3), since L¹ to L¹⁸ have a high glass transition temperature and can suppress formation of a metal film on the film surface, each independently represent a phenylene group, a phenylene group having at least one fluorine atom, a phenylene group having at least one trifluoromethyl group, a biphenylylene group, a biphenylylene group having at least one fluorine atom, a biphenylylene group having at least one trifluoromethyl group, a terphenylylene group, a terphenylylene group having at least one fluorine atom, a terphenylylene group having at least one trifluoromethyl group, a naphthylene group, or a single bond.

Although the specific examples of the divalent X are not particularly limited, they include: a 1,4-phenylene group, 1,3-phenylene group, 1,2-phenylene group, monofluoro-1,4-phenylene group, difluoro-1,4-phenylene group, trifluoro-1,4-phenylene group, tetrafluoro-1,4-phenylene group, monofluoro-1,3-phenylene group, difluoro-1,3-phenylene group, trifluoro-1,3-phenylene group, tetrafluoro-1,3-phenylene group, monofluoro-1,2-phenylene group, difluoro-1,2-phenylene group, trifluoro-1,2-phenylene group, tetrafluoro-1,2-phenylene group, 4,4'-biphenylylene group, 4,3'-biphenylylene group, 4,2'-biphenylylene group, 3,3'-biphenylylene group, 3,2'-biphenylylene group, 2,2'-biphenylylene group, monofluoro-4,4'-biphenylylene group, difluoro-4,4'-biphenylylene group, trifluoro-4,4'-biphenylylene group, tetrafluoro-4,4'-biphenylylene group, pentafluoro-4,4'-biphenylylene group, hexafluoro-4,4'-biphenylylene group, heptafluoro-4,4'-biphenylylene group, octafluoro-4,4'-biphenylylene group, monofluoro-4,3'-biphenylylene group; difluoro-4,3'-biphenylylene group, trifluoro-4,3'-biphenylylene group, tetrafluoro-4,3'-biphenylylene group, pentafluoro-4,3'-biphenylylene group, hexafluoro-4,3'-biphenylylene group, heptafluoro-4,3'-biphenylylene group, octafluoro-4,3'-biphenylylene group, monofluoro-4,2'-biphenylylene group, difluoro-4,2'-biphenylylene group; trifluoro-4,2'-biphenylylene group, tetrafluoro-4,2'-biphenylylene group, pentafluoro-4,2'-biphenylylene group, hexafluoro-4,2'-biphenylylene group, heptafluoro-4,2'-biphenylylene group, octafluoro-4,2'-biphenylylene group, monofluoro-3,3'-biphenylylene group, difluoro-3,3'-biphenylylene group, trifluoro-3,3'-biphenylylene group; tetrafluoro-3,3'-biphenylylene group, pentafluoro-3,3'-biphenylylene group, hexafluoro-3,3'-biphenylylene group, heptafluoro-3,3'-biphenylylene group, octafluoro-3,3'-biphenylylene group, monofluoro-3,2'-biphenylylene group, difluoro-3,2'-biphenylylene group, trifluoro-3,2'-biphenylylene group, tetrafluoro-3,2'-biphenylylene group; pentafluoro-3,2'-biphenylylene group, hexafluoro-3,2'-biphenylylene group, heptafluoro-3,2'-biphenylylene group, octafluoro-3,2'-biphenylylene group, monofluoro-2,2'-biphenylylene group, difluoro-2,2'-biphenylylene group, trifluoro-2,2'-biphenylylene group, tetrafluoro-2,2'-biphenylylene group, pentafluoro-2,2'-biphenylylene group; hexafluoro-2,2'-biphenylylene group, heptafluoro-2,2'-biphenylylene group, octafluoro-2,2'-biphenylylene group, 2,2'-dimethyl-4,4'-biphenylylene group, 2,2'-di (trifluoromethyl)-4,4'-biphenylylene group 4,4"-p-terphenylene group, perfluoro-4,4"-p-terphenylene group, 4,3"-p-terphenylene group, perfluoro-4,3"-p-terphenylene group, 4,2"-p-terphenylene group, perfluoro-4,2"-p-terphenylene group, 3,3"-p-terphenylene group, perfluoro-3,3"-p-terphenylene group, 3,2"-p-terphenylene group, perfluoro-3,2"-p-terphenylene group, 2,2"-p-terphenylene group, perfluoro-2,2"-p-terphenylene group; and 4,2'-p-terphenylene group, perfluoro-4,2'-p-terphenylene group, 4,3'-p-terphenylene group, perfluoro-4,3'-p-terphenylene group, 3,2'-p-terphenylene group, perfluoro-3,2'-p-terphenylene group, 3,3'-p-terphenylene group, 3,3'-p-terphenylene group, perfluoro-3,3'-p-terphenylene group, 2,3'-p-terphenylene group, perfluoro-2,3'-p-terphenylene group; 2,2'-p-terphenylene group, perfluoro-2,2'-p-terphenylene group, 4,4"-m-terphenylene group; 4,3"-m-terphenylene group, 4,2"-m-terphenylene group, 3,3"-m-terphenylene group, 3,2"-m-terphenylene group, 2,2"-m-terphenylene group, 2,2"-m-terphenylene group, 4,2'-m-terphenylene group, 4,4'-m-terphenylene group, 4,5'-m-terphenylene group, 4,6'-m-terphenylene group, 3,2'-m-terphenylene group, 3,4'-m-terphenylene group, 3,5'-m-terphenylene group; 3,6'-m-terphenylene group, 2,2'-m-terphenylene group, 2,2'-m-terphenylene group, 2,4'-m-terphenylene group, 2,5'-m-terphenylene group, 2,6'-m-terphenylene group, perfluoro - 4,4"-m-terphenylene group, perfluoro-4,3"-m-terphenylene group, perfluoro-4,2"-m-terphenylene group, perfluoro-3,3"-m-terphenylene group, perfluoro-3,2''-m-terphenylene group, perfluoro-3,2"-m-terphenylene group; perfluoro-2,2"-m-terphenylene group, perfluoro-4,2'-m-terphenylene group, perfluoro-4,4'-m-terphenylene group, perfluoro-4,5'-m-terphenylene group, perfluoro-4,6'-m-terphenylene group, perfluoro-3,2'-m-terphenylene group, perfluoro-3,4'-m-terphenylene group, perfluoro-3,5'-m-terphenylene group; perfluoro-3,6'-m-terphenylene group, perfluoro-2,2'-m-terphenylene group, perfluoro-2,4'-m-terphenylene group, perfluoro-2,5'-m-terphenylene group, perfluoro-2,6'-m-terphenylene group, 4,4"-o-terphenylene group, 4,3"-o-terphenylene group, 4,2"-o-terphenylene group, 3,3"-o-terphenylene group, 3,2"-o-terphenylene group, and 3,2"-o-terphenylene group, 2,2"-o-terphenylene group, 4,3'-o-terphenylene group, 4,4'-o-terphenylene group, 4,5'-o-terphenylene group, 4,6'-o-terphenylene group, 3,3'-o-terphenylene group, 3,4'-o-terphenylene group, 3,5'-o-terphenylene group, 3,6'-o-terphenylene group, 2,3'-o-terphenylene group, 2,4'-o-terphenylene group, 2,5'-o-terphenylene group; 2,6'-o-terphenylene group, perfluoro-4,4"-o-terphenylene group, perfluoro-4,3"-o-terphenylene group, perfluoro-4,2"-o-terphenylene group, perfluoro-3,3"-o-terphenylene group, perfluoro-3,2"-o-terphenylene group, perfluoro-2,2"-o-terphenylene group, perfluoro-4,3'-o-terphenylene group; Perfluoro-4,4'-o-terphenylene group, perfluoro-4,5'-o-terphenylene group, perfluoro-4,6'-o-terphenylene group, perfluoro-3,3'-o-terphenylene group, perfluoro-3,4'-o-terphenylene group, perfluoro-3,5'-o-terphenylene group, perfluoro-3,6'-o-terphenylene group, perfluoro-2,3'-o-terphenylene group; perfluoro-2,4'-o-terphenylene group, perfluoro-2,5'-o-terphenylene group, perfluoro-2,6'-o-terphenylene group, 1,2-naphthylene group, 1,3-naphthylene group, 1,4-naphthylene group, monofluoro-1,4-naphthylene group, difluoro-1,4-naphthylene group, trifluoro-1,4-naphthylene group, tetrafluoro-1,4-naphthylene group, hexafluoro-1,4-naphthylene group; 1,5-naphthylene group, 1,6-naphthylene group, 1,7-naphthylene group, 1,8-naphthylene group, 2,3-naphthylene group, 2,4-naphthylene group, 2,5-naphthylene group, 2,6-naphthylene group, monofluoro-2,6-naphthylene group, difluoro-2,6-naphthylene group, trifluoro - 2,6-naphthylene group, tetrafluoro-2,6-naphthylene group, pentafluoro-2,6-naphthylene group, hexafluoro-2,6-naphthylene group; 2,7-naphthylene group, 2,8-naphthylene group, 9,9-dimethyl-fluorene-2,7-diyl group, 9,9-di (trifluoromethyl)-fluorene-2,7-diyl group, 9,9-di (pentafluoroethyl)-fluorene-2,7-diyl group, 9,9-di (heptafluoropropyl)-fluorene-2,7-diyl group, 9,9-di (nonafluorobutyl)-fluorene-2,7-diyl group, 9,9-di (perfluoropentyl)-fluorene-2,7-diyl, 9,9-di (perfluorohexyl)-fluorene-2,7-diyl, 9,9-di (perfluoroheptyl)-fluorene-2,7-diyl, 9,9-di (perfluorooctyl)-fluorene-2,7-diyl, 9,9-dimethyl-fluorene-2,8-diyl, 9,9-dimethyl-fluorene-2,4-diyl, 9,9-dimethyl-fluorene-2,5-diyl group, 9,9-dimethyl-fluorene -2,6-diyl group, 9,9-dimethyl-fluorene-3,6-diyl group, 9,9-diphenyl-fluorene-2,7-diyl group, 9,9-di (pentafluorophenyl)-fluorene-2,7-diyl group, 9,9-diphenyl-fluorene-2,8-diyl group, 9,9-diphenyl-fluorene-2,4-diyl group; 9,9-diphenyl-fluorene-2,5-diyl group, 9,9-diphenyl-fluorene-2,6-diyl group, 9,9-diphenyl-fluorene-3,6-diyl group, spirobifluorene-2,7-diyl group, perfluorospirobifluorene-2,7-diyl group, spirobifluorene-2,8-diyl group, spirobifluorene-2,4-diyl group, spirobifluorene-2,5-diyl group, spirobifluorene-2,6-diyl group; Spirobifluorene-3,6-diyl group, phenanthrene-9,10-diyl group, phenanthrene-2,9-diyl group, phenanthrene-2,10-diyl group, phenanthrene-2,8-diyl group, phenanthrene-2,7-diyl group, phenanthrene-2,6-diyl group, phenanthrene-2,5-diyl group, phenanthrene-2,14-diyl group, phenanthrene-2,3-diyl group; phenanthrene-9,10-diyl group, phenanthrene-3,10-diyl group, phenanthrene-3,9-diyl group, phenanthrene-3,9-diyl group, phenanthrene-3,8-diyl group, phenanthrene-3,7-diyl group, phenanthrene-3,6-diyl group, phenanthrene-3,5-diyl group, phenanthrene-4,10-diyl group, phenanthrene-4,9-diyl group, phenanthrene-4,8-diyl group, phenanthrene-4,7-diyl group, phenanthrene-4,6-diyl group, triphenylene-1,3-diyl group, triphenylene-1,4-diyl group, triphenylene-1,4-diyl group, triphenylene-1,5-diyl group, triphenylene-1,6-diyl group, triphenylene-1,7-diyl group, triphenylene-1,8-diyl group, triphenylene-2,3-diyl group, triphenylene-2,4-diyl group, triphenylene-2,5-diyl group, triphenylene-2,6-diyl group, triphenylene-2,7-diyl group, triphenylene-3,5-diyl group, triphenylene-3,6-diyl group, triphenylene-3,6-diyl group, triphenylene-4,6-diyl group, pyrene-1,3-diyl group, pyrene-1,6-diyl group, pyrene-1,8-diyl group, pyrene-2,7-diyl group, anthracene-2,6-diyl group, anthracene-9,10-diyl group, perfluoroanthracene-9,10-diyl group, 9,10-diphenylanthracenediyl group; pyridine-2,3-diyl group, pyridine-2,4-diyl group, pyridine-2,5-diyl group, pyridine-2,6-diyl group, pyridine-3,4-diyl group, pyridine-3,5-diyl group, perfluoropyridin-2,3-diyl group, perfluoropyridin-2,4-diyl group, perfluoropyridin-2,5-diyl group, perfluoropyridin-2,6-diyl group, perfluoropyridin-3,4-diyl group; perfluoropyridine-3,5-diyl, dibenzofuran-2,7-diyl, dibenzofuran-4,5-diyl, dibenzothiophene-2,7-diyl, dibenzothiophene-4,5-diyl, 9-phenylcarbazol-2,7-diyl, pyrimidine-2,4-diyl, pyrimidine-2,5-diyl, pyrimidine-4,6-diyl, pyrimidine-4,5-diyl, triazine-2,4-diyl, an adamantane-1,3-diyl group, an adamantane-2,2-diyl group, a methanediyl group, a silanediyl group, a cyclohexane-1,1-diyl group, a cyclohexane-1,2-diyl group, a cyclohexane-1,3-diyl group, and a cyclohexane-1,4-diyl group.

Although the specific examples of the trivalent X are not particularly limited, they can include: a phenyl-1,2,3-triyl group, monofluoro-phenyl-1,2,3-triyl group, difluoro-phenyl-1,2,3-triyl group, trifluoro-phenyl-1,2,3-triyl group, pheny - 1,2,4-triyl group, monofluorophenyl-1,2,4-triyl group, difluorophenyl-1,2,4-triyl group, trifluorophenyl-1,2,4-triyl group, phenyl-1,3,5-triyl group, monofluorophenyl-1,3,5-triyl group, difluorophenyl-1,3,5-triyl group, trifluorophenyl-1,3,5-triyl group, biphenyl-2,3,4-triyl group, monofluoro-biphenyl-2,3,4-triyl group, difluoro-biphenyl-2,3,4-triyl group, trifluoro-biphenyl-2,3,4-triyl group, tetrafluoro-biphenyl-2,3,4-triyl group, pentafluoro-biphenyl-2,3,4-triyl group, hexafluoro-biphenyl-2,3,4-triyl group, heptafluoro-biphenyl-2,3,4-triyl group, monofluoro-biphenyl-2,3,5-triyl group, difluoro-biphenyl-2,3,5-triyl group, trifluoro-biphenyl-2,3,5-triyl group, tetrafluoro-biphenyl-2,3,5-triyl group; Pentafluoro-biphenyl-2,3,5-triyl group, hexafluoro-biphenyl-2,3,5-triyl group, heptafluoro-biphenyl-2,3,5-triyl group, monofluoro-biphenyl-2,3,6-triyl group, difluoro-biphenyl-2,3,6-triyl group, trifluoro-biphenyl-2,3,6-triyl group, tetrafluoro-biphenyl-2,3,6-triyl group, pentafluoro-biphenyl-2,3,6-triyl group; hexafluoro-biphenyl-2,3,6-triyl group, heptafluoro-biphenyl-2,3,6-triyl group, monofluoro-biphenyl-2,3,2'-triyl group, difluoro-biphenyl-2,3,2'-triyl group, trifluoro-biphenyl-2,3,2'-triyl group, tetrafluoro-biphenyl-2,3,2'-triyl group, pentafluoro-biphenyl-2,3,2'-triyl group, hexafluoro-biphenyl-2,3,2'-triyl group, heptafluoro-biphenyl-2,3,2'-triyl group, monofluoro-biphenyl-2,3,3'-triyl group, difluoro-biphenyl-2,3,3'-triyl group, trifluoro-biphenyl-2,3,3'-triyl group, tetrafluoro-biphenyl-2,3,3'-triyl group, pentafluoro-biphenyl-2,3,3'-triyl group, hexafluoro-biphenyl-2,3,3'-triyl group, heptafluoro-biphenyl-2,3,3'-triyl group, monofluoro-biphenyl-2,3,4'-triyl group, difluoro-biphenyl-2,3,4'-triyl group, trifluoro-biphenyl-2,3,4'-triyl group, tetrafluoro-biphenyl-2,3,4'-triyl group, pentafluoro-biphenyl-2,3,4'-triyl group; Hexafluoro-biphenyl-2,3,4'-triyl group, heptafluoro-biphenyl-2,3,4'-triyl group, monofluoro-biphenyl-2,3,5-triyl group, difluoro-biphenyl-2,3,5-triyl group, trifluoro-biphenyl-2,3,5-triyl group, tetrafluoro-biphenyl-2,3,5-triyl group, pentafluoro-biphenyl-2,3,5-triyl group, hexafluoro-biphenyl-2,3,5-triyl group, heptafluoro-biphenyl-2,3,5-triyl group, monofluoro-biphenyl-2,3,6-triyl group, difluoro-biphenyl-2,3,6-triyl group, trifluoro-biphenyl-2,3,6-triyl group, tetrafluoro-biphenyl-2,3,6-triyl group, pentafluoro-biphenyl-2,3,6-triyl group, hexafluoro-biphenyl-2,3,6-triyl group, heptafluoro-biphenyl-2,3,6-triyl group, monofluoro-biphenyl-2,3,2'-triyl group, difluoro-biphenyl-2,3,2'-triyl group, trifluoro-biphenyl-2,3,2'-triyl group, tetrafluoro-biphenyl-2,3,2'-triyl group, pentafluoro-biphenyl-2,3,2'-triyl group, hexafluoro-biphenyl-2,3,2'-triyl group, heptafluoro-biphenyl-2,3,2'-triyl group, monofluoro-biphenyl-2,3,3'-triyl group, difluoro-biphenyl-2,3,3'-triyl group, trifluoro-biphenyl-2,3,3'-triyl group, tetrafluoro-biphenyl-2,3,3'-triyl group, pentafluoro-biphenyl-2,3,3'-triyl group, hexafluoro-biphenyl-2,3,3'-triyl group, heptafluoro-biphenyl-2,3,3'-triyl group, monofluoro-biphenyl-2,3,4'-triyl group, difluoro-biphenyl-2,3,4'-triyl group, trifluoro-biphenyl-2,3,4'-triyl group, tetrafluoro-biphenyl-2,3,4'-triyl group, pentafluoro-biphenyl-2,3,4'-triyl group, hexafluoro-biphenyl-2,3,4'-triyl group, heptafluoro-biphenyl-2,3,4'-triyl group; monofluoro-biphenyl-2,5,2'-triyl group, difluoro-biphenyl-2,5,2'-triyl group, trifluoro-biphenyl-2,5,2'-triyl group, tetrafluoro-biphenyl-2,5,2'-triyl group, pentafluoro-biphenyl-2,5,2'-triyl group, hexafluoro-biphenyl-2,5,2'-triyl group, heptafluoro-biphenyl-2,5,2'-triyl group; Monofluoro-biphenyl-2,5,3'-triyl group, difluoro-biphenyl-2,5,3'-triyl group, trifluoro-biphenyl-2,5,3'-triyl group, tetrafluoro-biphenyl-2,5,3'-triyl group, pentafluoro-biphenyl-2,5,3'-triyl group, hexafluoro-biphenyl-2,5,3'-triyl group, heptafluoro-biphenyl-2,5,3'-triyl group, monofluoro-biphenyl-2,5,4'-triyl group, difluoro-biphenyl-2,5,4'-triyl group, trifluoro-biphenyl-2,5,4'-triyl group, tetrafluoro-biphenyl-2,5,4'-triyl group, pentafluoro-biphenyl-2,5,4'-triyl group, hexafluoro-biphenyl-2,5,4'-triyl group, heptafluoro-biphenyl-2,5,4'-triyl group; Monofluoro-biphenyl-2,6,2'-triyl group, difluoro-biphenyl-2,6,2'-triyl group, trifluoro-biphenyl-2,6,2'-triyl group, tetrafluoro-biphenyl-2,6,2'-triyl group, pentafluoro-biphenyl-2,6,2'-triyl group, hexafluoro-biphenyl-2,6,2'-triyl group, heptafluoro-biphenyl-2,6,2'-triyl group, monofluoro-biphenyl-2,6,3'-triyl group, difluoro-biphenyl-2,6,3'-triyl group, trifluoro-biphenyl-2,6,3'-triyl group, tetrafluoro-biphenyl-2,6,3'-triyl group, pentafluoro-biphenyl-2,6,3'-triyl group, hexafluoro-biphenyl-2,6,3'-triyl group, heptafluoro-biphenyl-2,6,3'-triyl group, monofluoro-biphenyl-2,6,4'-triyl group, difluoro-biphenyl-2,6,4'-triyl group, trifluoro-biphenyl-2,6,4'-triyl group, tetrafluoro-biphenyl-2,6,4'-triyl group, pentafluoro-biphenyl-2,6,4'-triyl group, hexafluoro-biphenyl-2,6,4'-triyl group, heptafluoro-biphenyl-2,6,4'-triyl group; monofluoro-biphenyl-3,4,5-triyl group, difluoro-biphenyl-3,4,5-triyl group, trifluoro-biphenyl-3,4,5-triyl group, tetrafluoro-biphenyl-3,4,5-triyl group, pentafluoro-biphenyl-3,4,5-triyl group, hexafluoro-biphenyl-3,4,5-triyl group, heptafluoro-biphenyl-3,4,5-triyl group, monofluoro-biphenyl-3,4,2'-triyl group, difluoro-biphenyl-3,4,2'-triyl group, trifluoro-biphenyl-3,4,2'-triyl group, tetrafluoro-biphenyl-3,4,2'-triyl group, pentafluoro-biphenyl-3,4,2'-triyl group, hexafluoro-biphenyl-3,4,2'-triyl group, heptafluoro-biphenyl-3,4,2'-triyl group, monofluoro-biphenyl-3,4,3'-triyl group, difluoro-biphenyl-3,4,3'-triyl group, trifluoro-biphenyl-3,4,3'-triyl group, tetrafluoro-biphenyl-3,4,3'-triyl group, pentafluoro-biphenyl-3,4,3'-triyl group, hexafluoro-biphenyl-3,4,3'-triyl group, heptafluoro-biphenyl-3,4,3'-triyl group, monofluoro-biphenyl-3,4,4'-triyl group, difluoro-biphenyl-3,4,4'-triyl group, trifluoro-biphenyl-3,4,4'-triyl group, tetrafluoro-biphenyl-3,4,4'-triyl group, pentafluoro-biphenyl-3,4,4'-triyl group, hexafluoro-biphenyl-3,4,4'-triyl group, heptafluoro-biphenyl-3,4,4'-triyl group, monofluoro-biphenyl-3,5,2'-triyl group, difluoro-biphenyl-3,5,2'-triyl group, trifluoro-biphenyl-3,5,2'-triyl group, tetrafluoro-biphenyl-3,5,2'-triyl group, pentafluoro-biphenyl-3,5,2'-triyl group, hexafluoro-biphenyl-3,5,2'-triyl group, heptafluoro-biphenyl-3,5,2'-triyl group, monofluoro-biphenyl-3,5,3'-triyl group, difluoro-biphenyl-3,5,3'-triyl group, trifluoro-biphenyl-3,5,3'-triyl group, tetrafluoro-biphenyl-3,5,3'-triyl group, pentafluoro-biphenyl-3,5,3'-triyl group, hexafluoro-biphenyl-3,5,3'-triyl group, heptafluoro-biphenyl-3,5,3'-triyl group, monofluoro-biphenyl-3,5,4'-triyl group, difluoro-biphenyl-3,5,4'-triyl group, trifluoro-biphenyl-3,5,4'-triyl group, tetrafluoro-biphenyl-3,5,4'-triyl group, pentafluoro-biphenyl-3,5,4'-triyl group, hexafluoro-biphenyl-3,5,4'-triyl group, heptafluoro-biphenyl-3,5,4'-triyl group; o-terphenyltriyl group, monofluoro-o-terphenyltriyl group, difluoro-o-terphenyltriyl group, trifluoro-o-terphenyltriyl group, tetrafluoro-o-terphenyltriyl group, pentafluoro-o-terphenyltriyl group, hexafluoro-o-terphenyltriyl group, heptafluoro-o-terphenyltriyl group, octafluoro-o-terphenyltriyl group, nonafluoro-o-terphenyltriyl group, decafluoro-o-terphenyltriyl group, perfluoro-o-terphenyltriyl group, m-terphenyltriyl group, monofluoro-m-terphenyltriyl group, difluoro-m-terphenyltriyl group, trifluoro-m-terphenyltriyl group, tetrafluoro-m-terphenyltriyl group, pentafluoro-m-terphenyltriyl group, hexafluoro-m-terphenyltriyl groups, heptafluoro-m-terphenyltriyl groups, octafluoro-m-terphenyltriyl groups, nonafluoro-m-terphenyltriyl groups, decafluoro-m-terphenyltriyl groups, perfluoro-m-terphenyltriyl groups, p-terphenyltriyl groups, monofluoro-p-terphenyltriyl groups, difluoro-p-terphenyltriyl groups. Trifluoro-p-terphenyltriyl group, tetrafluoro-p-terphenyltriyl group, pentafluoro-p-terphenyltriyl group, hexafluoro-p-terphenyltriyl group, heptafluoro-p-terphenyltriyl group, octafluoro-p-terphenyltriyl group, nonafluoro-p-terphenyltriyl group, decafluoro-p-terphenyltriyl group, perfluoro-p-terphenyltriyl group, naphthalene-1,2,8-triyl group, monofluoro-naphthalene-1,2,8-triyl group, difluoro-naphthalene-1,2,8-triyl group, trifluoro-naphthalene-1,2,8-triyl group, tetrafluoro-naphthalene-1,2,8-triyl group, pentafluoro-naphthalene-1,2,8-triyl group, naphthalene-1,3,8-triyl group, monofluoro-naphthalene-1,3,8-triyl group, difluoro-naphthalene-1,3,8-triyl, trifluoro-naphthalene-1,3,8-triyl, tetrafluoro-naphthalene-1,3,8-triyl, pentafluoro-naphthalene-1,3,8-triyl, naphthalene-1,4,8-triyl, monofluoro-naphthalene-1,4,8-triyl, difluoro-naphthalene-1,4,8-triyl, trifluoro-naphthalene-1,4,8-triyl, tetrafluoro-naphthalene-1,4,8-triyl group, pentafluoro-naphthalene-1,4,8-triyl group, naphthalene-1,2,3-triyl group, monofluoro-naphthalene-1,2,3-triyl group, difluoro-naphthalene-1,2,3-triyl group, trifluoro-naphthalene-1,2,3-triyl group, tetrafluoro-naphthalene-1,2,3-triyl group; pentafluoro-naphthalene-1,2,3-triyl group, naphthalene-1,2,4-triyl group, monofluoro-naphthalene-1,2,4-triyl group, difluoro-naphthalene-1,2,4-triyl group, trifluoro-naphthalene-1,2,4-triyl group, tetrafluoro-naphthalene-1,2,4-triyl group, pentafluoro-naphthalene-1,2,4-triyl group, naphthalene-1,2,5-triyl group, monofluoro-naphthalene-1,2,5-triyl group, difluoro-naphthalene-1,2,5-triyl group, trifluoro-naphthalene-1,2,5-triyl group, tetrafluoro-naphthalene-1,2,5-triyl group, pentafluoro-naphthalene-1,2,5-triyl group, naphthalene-1,2,6-triyl group, monofluoro-naphthalene-1,2,6-triyl group, difluoro-naphthalene-1,2,6-triyl group, trifluoro-naphthalene-1,2,6-triyl group, tetrafluoro-naphthalene-1,2,6-triyl group, pentafluoro-naphthalene-1,2,6-triyl group, naphthalene-1,2,7-triyl group, monofluoro-naphthalene-1,2,7-triyl group, difluoro-naphthalene-1,2,7-triyl group, trifluoro-naphthalene-1,2,7-triyl group, tetrafluoro-naphthalene-1,2,7-triyl group, pentafluoro-naphthalene-1,2,7-triyl group, naphthalene-2,3,6-triyl group, monofluoro-naphthalene-2,3,6-triyl group, difluoro-naphthalene-2,3,6-triyl group, trifluoro-naphthalene-2,3,6-triyl group, tetrafluoro-naphthalene-2,3,6-triyl group, pentafluoro-naphthalene-2,3,6-triyl group, naphthalene-2,3,5-triyl group, monofluoro-naphthalene-2,3,5-triyl group, difluoro-naphthalene-2,3,5-triyl group, trifluoro-naphthalene-2,3,5-triyl group, tetrafluoro-naphthalene-2,3,5-triyl group, pentafluoro-naphthalene-2,3,5-triyl group, naphthalene-1,3,5-triyl group, monofluoro-naphthalene-1,3,5-triyl group, difluoro-naphthalene-1,3,5-triyl group, trifluoro-naphthalene-1,3,5-triyl group; tetrafluoro-naphthalene-1,3,5-triyl group, pentafluoro-naphthalene-1,3,5-triyl group, naphthalene-1,3,6-triyl group, monofluoro-naphthalene-1,3,6-triyl group, difluoro-naphthalene-1,3,6-triyl group, trifluoro-naphthalene-1,3,6-triyl group, tetrafluoro-naphthalene-1,3,6-triyl group, pentafluoro-naphthalene-1,3,6-triyl group, naphthalene-1,4,6-triyl group, monofluoro-naphthalene-1,4,6-triyl group, difluoro-naphthalene-1,4,6-triyl group, trifluoro-naphthalene-1,4,6-triyl group, tetrafluoro-naphthalene-1,4,6-triyl group, pentafluoro-naphthalene-1,4,6-triyl group, 9,9-dimethyl-fluorene-triyl group, 9,9-di (trifluoromethyl)-fluorene-triyl group, 9,9-di (pentafluoroethyl)-fluorene-triyl group, 9,9-di (heptafluoropropyl)-fluorene-triyl group, 9,9-di (nonafluorobutyl)-fluorene-triyl group, 9,9-di (perfluoropentyl)-fluorene-triyl group, 9,9-di (perfluorohexyl)-fluorene-triyl group, 9,9-di (perfluoroheptyl)-fluorene-triyl group, 9,9-di (perfluorooctyl)-fluorene-triyl group, 9,9-diphenyl-fluorenetriyl group, 9,9-di (pentafluorophenyl)-fluorene-triyl group, spirobifluorene-triyl group, perfluorospirobifluorene-triyl group, spirobifluorene-triyl group, phenanthrene-triyl group, monofluoro-phenanthrene-triyl group, difluoro-phenanthrene-triyl group, trifluoro-phenanthrene-triyl group, tetrafluoro-phenanthrene-triyl group, pentafluoro-phenanthrene-triyl group, hexasulfuro-phenanthrene-triyl group, triphenylenetriyl group, anthracene-triyl group, perfluoroanthracene-triyl group, 9,10-diphenylanthracene-triyl group, pyridine-2,3,4-triyl group, monofluoropyridine-2,3,4-triyl group, difluoropyridine-2,3,4-triyl group, pyridine-2,3,5-triyl group, monofluoropyridin-2,3,5-triyl group, difluoropyridin-2,3,5-triyl group, pyridine-2,3,6-triyl group, monofluoropyridin-2,3,6-triyl group, difluoropyridin-2,3,6-triyl group, pyridine-3,4,5-triyl group, monofluoropyridin-3,4,5-triyl group, difluoropyridine-3,4,5-triyl group, dibenzofuran-triyl group, dibenzothiophene-triyl group, dibenzothiophene-triyl group, 9-phenylcarbazole-triyl group, pyrimidine-2,4,6-triyl group, pyrimidine-4,5,6-triyl group, pyrimidine-2,4,5-triyl group, triazine-2,4,6-triyl group, adamantane-1,3,5-triyl group, methanetriyl group, silane triyl group, and cyclohexane-1,3,5-triyl group.

Although the specific examples of tetravalent X not particularly limited, they can include a phenyl-1,2,3,4-tetrayl group, monofluoro-phenyl-1,2,3,4-tetrayl group, difluoro-phenyl-1,2,3,4-tetrayl group, difluoro-phenyl-1,2,3,4-tetrayl group, phenyl-1,2,3,5-tetrayl group, monofluoro-phenyl-1,2,3,5-tetrayl group, difluorophenyl-1,2,3,5-tetrayl group, phenyl-1,2,4,5-tetrayl group, monofluoro-phenyl-1,2,4,5-tetrayl group, difluoro-phenyl-1,2,4,5-tetrayl group, 5-trifluoromethyl-phenyl-1,2,3,4-tetrayl group, 5-trifluoromethyl-6-fluoro-phenyl -1,2,3,4-tetrayl group, 5,6-ditrifluoromethyl-phenyl-1,2,3,4-tetrayl group, 4-trifluoromethyl-phenyl-1,2,3,5-tetrayl group, 4-trifluoromethyl-6-fluoro-phenyl-1,2,3,5-tetrayl group, 4,6-ditrifluoromethyl-phenyl-1,2,3,5-tetrayl group; 3-trifluoromethyl-phenyl-1,2,4,5-tetrayl group, 3-trifluoromethyl-6-fluoro-phenyl-1,2,4,5-tetrayl group, 3,6-ditrifluoromethyl-phenyl-1,2,4,5-tetrayl group, biphenyl-2,3,4,3'-tetrayl group, monofluoro-biphenyl -2,3,4,3'-tetrayl group, difluoro-biphenyl -2,3,4,3'-tetrayl group, trifluoro-biphenyl -2,3,4,3'-tetrayl group, tetrafluoro-biphenyl - 2,3,4,3'-tetrayl group, pentafluoro-biphenyl -2,3,4,3'-tetrayl group, hexafluoro-biphenyl -2,3,4,3'-tetrayl group, biphenyl-2,3,5,3'-tetrayl group, monofluoro-biphenyl-2,3,5,3'-tetrayl group, difluoro-biphenyl-2,3,5,3'-tetrayl group, trifluoro-biphenyl-2,3,5,3'-tetrayl group; tetrafluoro-biphenyl-2,3,5,3'-tetrayl group, pentafluoro-biphenyl-2,3,5,3'-tetrayl group, hexafluoro-biphenyl-2,3,5,3'-tetrayl group, biphenyl-2,4,5,3'-tetrayl group, monofluoro-biphenyl-2,4,5,3'-tetrayl group, difluoro-biphenyl-2,4,5,3'-tetrayl group, trifluoro-biphenyl-2,4,5,3'-tetrayl group, tetrafluoro-biphenyl-2,4,5,3'-tetrayl group, pentafluoro-biphenyl-2,4,5,3'-tetrayl group, hexafluoro-biphenyl-2,4,5,3'-tetrayl group, biphenyl-3,4,5,3'-tetrayl group, monofluoro-biphenyl-3,4,5,3'-tetrayl group, difluoro-biphenyl-3,4,5,3'-tetrayl group, trifluoro-biphenyl-3,4,5,3'-tetrayl group, tetrafluoro-biphenyl-3,4,5,3'-tetrayl group, pentafluoro-biphenyl-3,4,5,3'-tetrayl group, hexafluoro-biphenyl-3,4,5,3'-tetrayl group, biphenyl-2,3,4,5-tetrayl group, monofluoro-biphenyl-2,3,4,5-tetrayl group, difluoro-biphenyl-2,3,4,5-tetrayl group, trifluoro-biphenyl-2,3,4,5-tetrayl group; tetrafluoro-biphenyl-2,3,4,5-tetrayl group, pentafluoro-biphenyl-2,3,4,5-tetrayl group, hexafluoro-biphenyl-2,3,4,5-tetrayl group, biphenyl-2,3,6,3'-tetrayl group, monofluoro-biphenyl-2,3,6,3'-tetrayl group, difluoro-biphenyl-2,3,6,3'-tetrayl group, trifluoro-biphenyl-2,3,6,3'-tetrayl group; Tetrafluoro-biphenyl-2,3,6,3'-tetrayl group, pentafluoro-biphenyl-2,3,6,3'-tetrayl group, hexafluoro-biphenyl-2,3,6,3'-tetrayl group, biphenyl-2,4,6,3'-tetrayl group, monofluoro-biphenyl-2,4,6,3'-tetrayl group, difluoro-biphenyl-2,4,6,3'-tetrayl group, trifluoro-biphenyl-2,4,6,3'-tetrayl group, tetrafluoro-biphenyl-2,4,6,3'-tetrayl group, pentafluoro-biphenyl-2,4,6,3'-tetrayl group, hexafluoro-biphenyl-2,4,6,3'-tetrayl group, biphenyl-2,3,4,6-tetrayl group, monofluoro-biphenyl-2,3,4,6-tetrayl group, difluoro-biphenyl-2,3,4,6-tetrayl group, trifluoro-biphenyl-2,3,4,6-tetrayl group, tetrafluoro-biphenyl-2,3,4,6-tetrayl group, pentafluoro-biphenyl-2,3,4,6-tetrayl group, hexafluoro-biphenyl-2,3,4,6-tetrayl group, biphenyl-2,3,5,6-tetrayl group, monofluoro-biphenyl-2,3,5,6-tetrayl group, difluoro-biphenyl-2,3,5,6-tetrayl group, trifluoro-biphenyl-2,3,5,6-tetrayl group, tetrafluoro-biphenyl-2,3,5,6-tetrayl group, pentafluoro-biphenyl-2,3,5,6-tetrayl group, hexafluoro-biphenyl-2,3,5,6-tetrayl group, biphenyl-2,5,2',3'-tetrayl group, monofluoro-biphenyl-2,5,2',3'-tetrayl group, difluoro-biphenyl-2,5,2',3'-tetrayl group, trifluoro-biphenyl-2,5,2',3'-tetrayl group, 3'-tetrayl group, tetrafluoro-biphenyl-2,5,2',3'-tetrayl group, pentafluoro-biphenyl-2,5,2',3'-tetrayl group, hexafluoro-biphenyl-2,5,2',3'-tetrayl group, biphenyl-2,4,2',5'-tetrayl group, monofluoro-biphenyl-2,4,2',5'-tetrayl group, difluoro-biphenyl-2,4,2',5'-tetrayl group, trifluoro-biphenyl-2,4,2',5'-tetrayl group, tetrafluoro-biphenyl-2,4,2',5'-tetrayl group, pentafluoro-biphenyl-2,4,2',5'-tetrayl group, hexafluoro-biphenyl-2,4,2',5'-tetrayl group, 5'-tetrayl group, biphenyl-2,5,3',4'-tetrayl group, monofluoro-biphenyl-2,5,3',4'-tetrayl group, difluoro-biphenyl-2,5,3',4'-tetrayl group, trifluoro-biphenyl-2,5,3',4'-tetrayl group, tetrafluoro-biphenyl-2,5,3',4'-tetrayl group, pentafluoro-biphenyl-2,5,3',4'-tetrayl group, 4'-tetrayl group, hexafluoro-biphenyl-2,5,3',4'-tetrayl group, biphenyl-2,3,4,2'-tetrayl group, monofluoro-biphenyl-2,3,4,2'-tetrayl group, difluoro-biphenyl-2,3,4,2'-tetrayl group, trifluoro-biphenyl-2,3,4,2'-tetrayl group, tetrafluoro-biphenyl-2,3,4,2'-tetrayl group, pentafluoro-biphenyl-2,3,4,2'-tetrayl group; Hexafluoro-biphenyl-2,3,4,2'-tetrayl group, biphenyl-2,5,2',5'-tetrayl group, monofluoro-biphenyl-2,5,2',5'-tetrayl group, difluoro-biphenyl-2,5,2',5'-tetrayl group, trifluoro-biphenyl-2,5,2',5'-tetrayl group, tetrafluoro-biphenyl-2,5,2',5'-tetrayl group, pentafluoro-biphenyl-2,5,2',5'-tetrayl group, 5'-tetrayl group, hexafluoro-biphenyl-2,5,2',5'-tetrayl group, biphenyl-2,5,3',5'-tetrayl group, monofluoro-biphenyl-2,5,3',5'-tetrayl group, difluoro-biphenyl-2,5,3',5'-tetrayl group, trifluoro-biphenyl-2,5,3',5'-tetrayl group, tetrafluoro-biphenyl-2,5,3',5'-tetrayl group, 5'-tetrayl group, pentafluoro-biphenyl-2,5,3',5'-tetrayl group, hexafluoro-biphenyl-2,5,3',5'-tetrayl group, biphenyl-2,3,5,2'-tetrayl group, monofluoro-biphenyl-2,3,5,2'-tetrayl group, difluoro-biphenyl-2,3,5,2'-tetrayl group, trifluoro-biphenyl-2,3,5,2'-tetrayl group, tetrafluoro-biphenyl-2,3,5,2'-tetrayl group, pentafluoro-biphenyl-2,3,5,2'-tetrayl group, hexafluoro-biphenyl-2,3,5,2'-tetrayl group, biphenyl-2,4,5,2'-tetrayl group, monofluoro-biphenyl -2,4,5,2'-tetrayl group, difluoro-biphenyl-2,4,5,2'-tetrayl group, trifluoro-biphenyl-2,4,5,2'-tetrayl group, tetrafluoro-biphenyl-2,4,5,2'-tetrayl group, pentafluoro-biphenyl-2,4,5,2'-tetrayl group, hexafluoro-biphenyl-2,4,5,2'-tetrayl group, biphenyl-3,4,5,2'-tetrayl group, monofluoro-biphenyl-3,4,5,2'-tetrayl group, difluoro-biphenyl-3,4,5,2'-tetrayl group, trifluoro-biphenyl-3,4,5,2'-tetrayl group, tetrafluoro-bipheny -3,4,5,2'-tetrayl group, pentafluoro-biphenyl-3,4,5,2'-tetrayl group, hexafluoro-biphenyl-3,4,5,2'-tetrayl group, biphenyl-2,5,2',6'-tetrayl group, monofluoro-biphenyl-2,5,2',6'-tetrayl group, difluoro-biphenyl-2,5,2',6'-tetrayl group, 6'-tetrayl group, trifluoro-biphenyl-2,5,2',6'-tetrayl group, tetrafluoro-biphenyl-2,5,2',6'-tetrayl group, pentafluoro-biphenyl-2,5,2',6'-tetrayl group, hexafluoro-bipheny -2,5,2',6'-tetrayl group, biphenyl-2,3,6,2'-tetrayl group, monofluoro-biphenyl-2,3,6,2'-tetrayl group, difluoro-biphenyl-2,3,6,2'-tetrayl group, trifluoro-biphenyl-2,3,6,2'-tetrayl group, tetrafluoro-biphenyl-2,3,6,2'-tetrayl group, pentafluoro-biphenyl-2,3,6,2'-tetrayl group, hexafluoro-biphenyl-2,3,6,2'-tetrayl group, biphenyl-2,4,6,2'-tetrayl group, monofluoro-biphenyl-2,4,6,2'-tetrayl group, difluoro-biphenyl-2,4,6,2'-tetrayl group, trifluoro-biphenyl-2,4,6,2'-tetrayl group, tetrafluoro-biphenyl-2,4,6,2'-tetrayl group, pentafluoro-biphenyl-2,4,6,2'-tetrayl group, hexafluoro-biphenyl-2,4,6,2'-tetrayl group, biphenyl-2,3,3',4'-tetrayl group, monofluoro-biphenyl-2,3,3',4'-tetrayl group, difluoro-biphenyl-2,3,3',4'-tetrayl group, trifluoro-biphenyl-2,3,3',4'-tetrayl group, tetrafluoro-biphenyl-2,3,3',4'-tetrayl group, pentafluoro-biphenyl-2,3,3',4'-tetrayl group, hexafluoro-biphenyl-2,3,3',4'-tetrayl group, 4'-tetrayl group, biphenyl-2,4,3',4'-tetrayl group, monofluoro-biphenyl-2,4,3',4'-tetrayl group, difluoro-biphenyl-2,4,3',4'-tetrayl group, trifluoro-biphenyl-2,4,3',4'-tetrayl group, tetrafluoro-biphenyl-2,4,3',4'-tetrayl group, pentafluoro-biphenyl-2,4,3',4'-tetrayl group, 4'-tetrayl group, hexafluoro-biphenyl-2,4,3',4'-tetrayl group, biphenyl-3,4,3',4'-tetrayl group, monofluoro-biphenyl-3,4,3',4'-tetrayl group, difluoro-biphenyl-3,4,3',4'-tetrayl group, trifluoro-biphenyl-3,4,3',4'-tetrayl group, tetrafluoro-biphenyl-3,4,3',4'-tetrayl group, 4'-tetrayl group, pentafluoro-biphenyl-3,4,3',4'-tetrayl group, hexafluoro-biphenyl-3,4,3',4'-tetrayl group, biphenyl-2,3,4,4'-tetrayl group, monofluoro-biphenyl-2,3,4,4'-tetrayl group, difluoro-biphenyl-2,3,4,4'-tetrayl group, trifluoro-biphenyl-2,3,4,4'-tetrayl group, tetrafluoro-biphenyl-2,3,4,4'-tetrayl group, pentafluoro-biphenyl-2,3,4,4'-tetrayl group, hexafluoro-biphenyl-2,3,4,4'-tetrayl group, biphenyl-3,4,3',5'-tetrayl group, monofluoro-biphenyl-3,4,3',5'-tetrayl group, difluoro-biphenyl-3,4,3',5'-tetrayl group, trifluoro-biphenyl-3,4,3',5'-tetrayl group, tetrafluoro-biphenyl-3,4,3',5'-tetrayl group, pentafluoro-biphenyl-3,4,3',5'-tetrayl group, 5'-tetrayl group, hexafluoro-biphenyl-3,4,3',5'-tetrayl group, biphenyl-2,3,5,4'-tetrayl group, monofluoro-biphenyl-2,3,5,4'-tetrayl group, difluoro-biphenyl-2,3,5,4'-tetrayl group, trifluoro-biphenyl-2,3,5,4'-tetrayl group, tetrafluoro-biphenyl-2,3,5,4'-tetrayl group, pentafluoro-biphenyl-2,3,5,4'-tetrayl group, hexafluoro-biphenyl-2,3,5,4'-tetrayl group, biphenyl-2,4,5,4'-tetrayl group, monofluoro-biphenyl-2,4,5,4'-tetrayl group, difluoro-biphenyl-2,4,5,4'-tetrayl group, trifluoro-biphenyl-2,4,5,4'-tetrayl group, tetrafluoro-biphenyl-2,4,5,4'-tetrayl group, pentafluoro-biphenyl-2,4,5,4'-tetrayl group, hexafluoro-biphenyl-2,4,5,4'-tetrayl group, biphenyl-3,4,5,4'-tetrayl group, monofluoro-biphenyl-3,4,5,4'-tetrayl group, difluoro-biphenyl-3,4,5,4'-tetrayl group, trifluoro-biphenyl-3,4,5,4'-tetrayl group, tetrafluoro-biphenyl-3,4,5,4'-tetrayl group, pentafluoro-biphenyl-3,4,5,4'-tetrayl group, hexafluoro-biphenyl-3,4,5,4'-tetrayl group, biphenyl-2,6,3',4'-tetrayl group, monofluoro-biphenyl-2,6,3',4'-tetrayl group, difluoro-biphenyl-2,6,3',4'-tetrayl group, trifluoro-biphenyl-2,6,3',4'-tetrayl group, tetrafluoro-biphenyl-2,6,3',4'-tetrayl group, pentafluoro-biphenyl-2,6,3',4'-tetrayl group, hexafluoro-biphenyl-2,6,3',4'-tetrayl group, 4'-tetrayl group, biphenyl-2,3,6,4'-tetrayl group, monofluoro-biphenyl-2,3,6,4-tetrayl group, difluoro-biphenyl-2,3,6,4-tetrayl group, trifluoro-biphenyl-2,3,6,4-tetrayl group, tetrafluoro-biphenyl-2,3,6,4-tetrayl group, pentafluoro-biphenyl-2,3,6,4-tetrayl group, hexafluoro-biphenyl-2,3,6,4-tetrayl group, biphenyl-2,4,6,4'-tetrayl group, monofluoro-biphenyl-2,4,6,4-tetrayl group, difluoro-biphenyl-2,4,6,4-tetrayl group, trifluoro-biphenyl-2,4,6,4-tetrayl group, tetrafluoro-biphenyl-2,4,6,4-tetrayl group, pentafluoro-biphenyl-2,4,6,4-tetrayl group; Hexafluoro-biphenyl-2,4,6,4-tetrayl group, biphenyl-2,3,2',4'-tetrayl group, monofluoro-biphenyl-2,3,2',4'-tetrayl group, difluoro-biphenyl-2,3,2',4'-tetrayl group, trifluoro-biphenyl-2,3,2',4'-tetrayl group; tetrafluoro-biphenyl-2,3,2',4'-tetrayl group, pentafluoro-biphenyl-2,3,2',4'-tetrayl group, hexafluoro-biphenyl-2,3,2',4'-tetrayl group, biphenyl-2,4,2',4'-tetrayl group, monofluoro-biphenyl-2,4,2',4'-tetrayl group, difluoro-biphenyl-2,4,2',4'-tetrayl group, trifluoro-biphenyl-2,4,2',4'-tetrayl group, 4'-tetrayl group, tetrafluoro-biphenyl-2,4,2',4'-tetrayl group, pentafluoro-biphenyl-2,4,2',4'-tetrayl group, hexafluoro-biphenyl-2,4,2',4'-tetrayl group, biphenyl-3,5,2',4'-tetrayl group, monofluoro-biphenyl-3,5,2',4'-tetrayl group, difluoro-biphenyl-3,5,2',4'-tetrayl group, 4'-tetrayl group, trifluoro-biphenyl-3,5,2',4'-tetrayl group, tetrafluoro-biphenyl-3,5,2',4'-tetrayl group, pentafluoro-biphenyl-3,5,2',4'-tetrayl group, hexafluoro-biphenyl-3,5,2',4'-tetrayl group, biphenyl-2,4,2',6'-tetrayl group, monofluoro-biphenyl-2,4,2',6-tetrayl group, difluoro-biphenyl-2,4,2',6-tetrayl group, trifluoro-biphenyl-2,4,2',6-tetrayl group, tetrafluoro-biphenyl-2,4,2',6-tetrayl group, pentafluoro-biphenyl-2,4,2',6-tetrayl group, hexafluoro-biphenyl-2,4,2',6-tetrayl group, biphenyl-2,3,2',3'-tetrayl group, monofluoro-biphenyl-2,3,2',3'-tetrayl group, difluoro-biphenyl-2,3,2',3'-tetrayl group, trifluoro-biphenyl-2,3,2',3'-tetrayl group, tetrafluoro-biphenyl-2,3,2',3'-tetrayl group, pentafluoro-biphenyl-2,3,2',3'-tetrayl group, hexafluoro-biphenyl-2,3,2',3'-tetrayl group, biphenyl-2,3,3',5'-tetrayl group, monofluoro-biphenyl-2,3,3',5'-tetrayl group, difluoro-biphenyl-2,3,3',5'-tetrayl group, trifluoro-biphenyl-2,3,3',5'-tetrayl group, tetrafluoro-biphenyl-2,3,3',5'-tetrayl group, pentafluoro-biphenyl-2,3,3',5'-tetrayl group, hexafluoro-biphenyl-2,3,3',5'-tetrayl group; biphenyl-2,3,2',6'-tetrayl group, monofluoro-biphenyl-2,3,2',6'-tetrayl group, difluoro-biphenyl-2,3,2',6'-tetrayl group, trifluoro-biphenyl-2,3,2',6'-tetrayl group, tetrafluoro-biphenyl-2,3,2',6'-tetrayl group, pentafluoro-biphenyl-2,3,2',6'-tetrayl group, hexafluoro-biphenyl-2,3,2',6'-tetrayl group, 6'-tetrayl group, biphenyl - 3,5,2',6'-tetrayl group, monofluoro-biphenyl-3,5,2',6'-tetrayl group, difluoro-biphenyl-3,5,2',6'-tetrayl group, trifluoro-biphenyl-3,5,2',6'-tetrayl group, tetrafluoro-biphenyl-3,5,2',6'-tetrayl group, pentafluoro-biphenyl-3,5,2',6'-tetrayl group, 6'-tetrayl group, hexafluoro-biphenyl-3,5,2',6'-tetrayl group, biphenyl-2,6,2',6'-tetrayl group, monofluoro-biphenyl-2,6,2',6'-tetrayl group, difluoro-biphenyl-2,6,2',6'-tetrayl group, trifluoro-biphenyl-2,6,2',6'-tetrayl group, tetrafluoro-biphenyl-2,6,2',2',6'-tetrayl group, pentafluoro-biphenyl-2,6,2',6'-tetrayl group, hexafluoro-biphenyl-2,6,2',6'-tetrayl group, biphenyl-3,5,3',5'-tetrayl group, monofluoro-biphenyl-3,5,3',5'-tetrayl group, difluoro-biphenyl-3,5,3',5'-tetrayl group, trifluoro-biphenyl-3,5,3',trifluoro-biphenyl-3,5,3',5'-tetrayl group, tetrafluoro-biphenyl-3,5,3',5'-tetrayl group, pentafluoro-biphenyl-3,5,3',5'-tetrayl group, hexafluoro-biphenyl-3,5,3',5'-tetrayl group, o-terphenyl tetrayl group, monofluoro-o-terphenyl tetrayl group, difluoro-o-terphenyl tetrayl group, trifluoro-o-terphenyl tetrayl group, tetrafluoro-o-terphenyl tetrayl group, pentafluoro-o-terphenyl tetrayl group, hexafluoro-o-terphenyl tetrayl group, heptafluoro-o-terphenyl tetrayl group, octafluoro-o-terphenyl tetrayl group, nonafluoro-o-terphenyl tetrayl group, decafluoro-o-terphenyl tetrayl group, m-terphenyl tetrayl group, monofluoro-m-terphenyl tetrayl group, difluoro-m-terphenyl tetrayl group, trifluoro-m-terphenyl tetrayl group, tetrafluoro-m-terphenyl tetrayl group, pentafluoro-m-terphenyl tetrayl group, hexafluoro-m-terphenyl tetrayl group, heptafluoro-m-terphenyl tetrayl group, octafluoro-m-terphenyl tetrayl group, nonafluoro-m-terphenyl tetrayl group, decafluoro-m-terphenyl tetrayl group, perfluoro-m-terphenyl tetrayl group, p-terphenyl tetrayl group, monofluoro-p-terphenyl tetrayl group, difluoro-p-terphenyl tetrayl group, difluoro-p-terphenyl tetrayl group, trifluoro-p-terphenyl tetrayl group, tetrafluoro-p-terphenyl tetrayl group, pentafluoro-p-terphenyl tetrayl group, hexafluoro-p-terphenyl tetrayl group, heptafluoro-p-terphenyl tetrayl group, octafluoro-p-terphenyl tetrayl group, nonafluoro-p-terphenyl tetrayl group, decafluoro-p-terphenyl tetrayl group, perfluoro-p-terphenyl tetrayl group, naphthalene-1,4,5,8-tetrayl group, monofluoro-naphthalene-1,4,5,8-tetrayl group, difluoro-naphthalene-1,4,5,8-tetrayl group, trifluoro-naphthalene-1,4,5,8-tetrayl group, tetrafluoro-1,4,5,8-naphthalene-tetrayl group, naphthalene-2,3,6,7-tetrayl group, monofluoro-naphthalene-2,3,6,7-tetrayl group, difluoro-naphthalene-2,3,6,7-tetrayl group, trifluoro-naphthalene-2,3,6,7-tetrayl group, tetrafluoro-2,3,6,7-naphthalene-tetrayl group, 9,9-dimethyl-fluorene-tetrayl group, 9,9-di (trifluoromethyl)-fluorene-tetrayl group, 9,9-di (pentafluoroethyl)-fluorene-tetrayl group, 9,9-di (heptafluoropropyl)-fluorene-tetrayl, 9,9-di (nonafluorobutyl)-fluorene-tetrayl, 9,9-di (perfluoropentyl)-fluorene-tetrayl, 9,9-di (perfluorohexyl)-fluorene-tetrayl, 9,9-di (perfluoroheptyl)-fluorene-tetrayl, 9,9-di (perfluoroheptyl)-fluorene-tetrayl, 9,9-di (perfluorooctyl)-fluorene-tetrayl. 9,9-diphenyl-fluorene-tetrayl group, 9,9-di (pentafluorophenyl)-fluorene-tetrayl group, spirobifluorene-tetrayl group, perfluorospirobifluorene-tetrayl group, spirobifluorene-tetrayl group, phenanthrene-tetrayl group, monofluoro-phenanthrene-tetrayl group, difluoro-phenanthrene-tetrayl group, trifluoro-phenanthrene-tetrayl group, tetrafluoro-phenanthrene-tetrayl group, pentafluoro-phenanthrene-tetrayl group, triphenylene-tetrayl group, anthracene-tetrayl group, perfluoroanthracene-tetrayl group, 9,10-diphenylanthracene-tetrayl group, pyridine-2,3,4,5-tetrayl group, monofluoropyridine-2,3,4,5-tetrayl group, pyridine-2,3,4,6-tetrayl group, monofluoropyridine-2,3,4,6 tetrayl group, pyridine-2,3,5,6 tetrayl group, monofluoropyridine-2,3,5,6 tetrayl group, pyrimidine-2,4,5,6 tetrayl group, pyrimidine-2,4,5,6-triyl group, dibenzofuran-tetrayl group, dibenzothiophene-tetrayl group, 9-phenylcarbazole-tetrayl group, adamantane-1,3,5,7-tetrayl group, methanetetrayl group, silane tetrayl group, and cyclohexane-1,1,4,4-tetrayl groups.

In Formulas (1) to (3), due to having a high glass transition temperature and being able to suppress the formation of a metal film on the surface of the film, X preferably represents:
(vi) a phenylene group, biphenylene group, terphenylylene group, naphthylene group, fluorenylene group, spirobifluorenylene group, benzofluorenylene group, phenanthrene group, fluoranthenylene group, triphenylenylene group, anthracenediyl group, pyridylene group, pyrimidinediyl group, triazinediyl group, carbazoldiyl group, benzoflanyl group, benzothiophenediyl group, dibenzofuryl group, adamantanediyl group, methylene group, silanediyl group, cyclohexylene groups, phenyltriyl group, biphenyltriyl groups, terphenyltriyl group, naphthylyl groups, fluorenetriyl group, spirobiflurenetriyl group, benzofluorenetriyl groups, phenanthrenetriyl group, fluoranthenetriyl group, triphenylenetriyl groups, anthracenetriyl group, pyrenetriyl groups, pyridinetriyl group, pyrimidinetriyl groups, triazinetriyl grous, arbazole triyl group, benzofuran triyl group, benzothiophene triyl group, dibenzofuran triyl group, adamantane triyl group, methane triyl group, silane triyl group, cyclohexane triyl group, phenyl tetrayl group, biphenyl tetrayl group, terphenyl tetrayl group, naphthyl tetrayl group, fluorene tetrayl group, spirobifluorene tetrayl group, benzofluorene tetrayl group, phenanthrene tetrayl group, fluoranthene tetrayl group, triphenylene tetrayl group, anthracene tetrayl group, pyrene tetrayl group, pyridine tetrayl group, pyrimidine tetrayl group, carbazole tetrayl group, benzofuran tetrayl group, benzothiophene tetrayl group, dibenzofuran tetrayl group, adamantane tetrayl group, methane tetrayl group, silane tetrayl group, or cyclohexane tetrayl group; or
(vii) a group in which the group represented by (vi) is substituted with one or more groups selected from the group consisting of a methyl group, an ethyl group, a methoxy group, an ethoxy group, a trifluoromethyl group, a trifluoromethoxy group, a perfluoroalkyl group having 2 to 10 carbon atoms, a perfluoroalkoxy group having 2 to 10 carbon atoms, a cyano group, a deuterium atom, a fluorine atom, a phenyl group optionally substituted with a fluorine atom, a biphenylyl group optionally substituted with a fluorine atom, a naphthyl group optionally substituted with a fluorine atom, a phenanthryl group which may be substituted with a fluorine atom, a pyridyl group which may be substituted with a fluorine atom, a carbazolyl group which may be substituted with a fluorine atom, a dibenzothienyl group which may be substituted with a fluorine atom, and a dibenzofuranyl group which may be substituted with a fluorine atom.

Due to having a high glass transition temperature and being able to suppress formation of a metal film on the film surface, X more preferably has the following substituents. It should be noted that F represents a fluorine atom, v represents an integer of 0 to 5, w represents an integer of 0 to 4, x represents an integer of 0 to 3, y represents an integer of 0 to 2, and z represents an integer of 0 to 1.

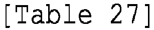

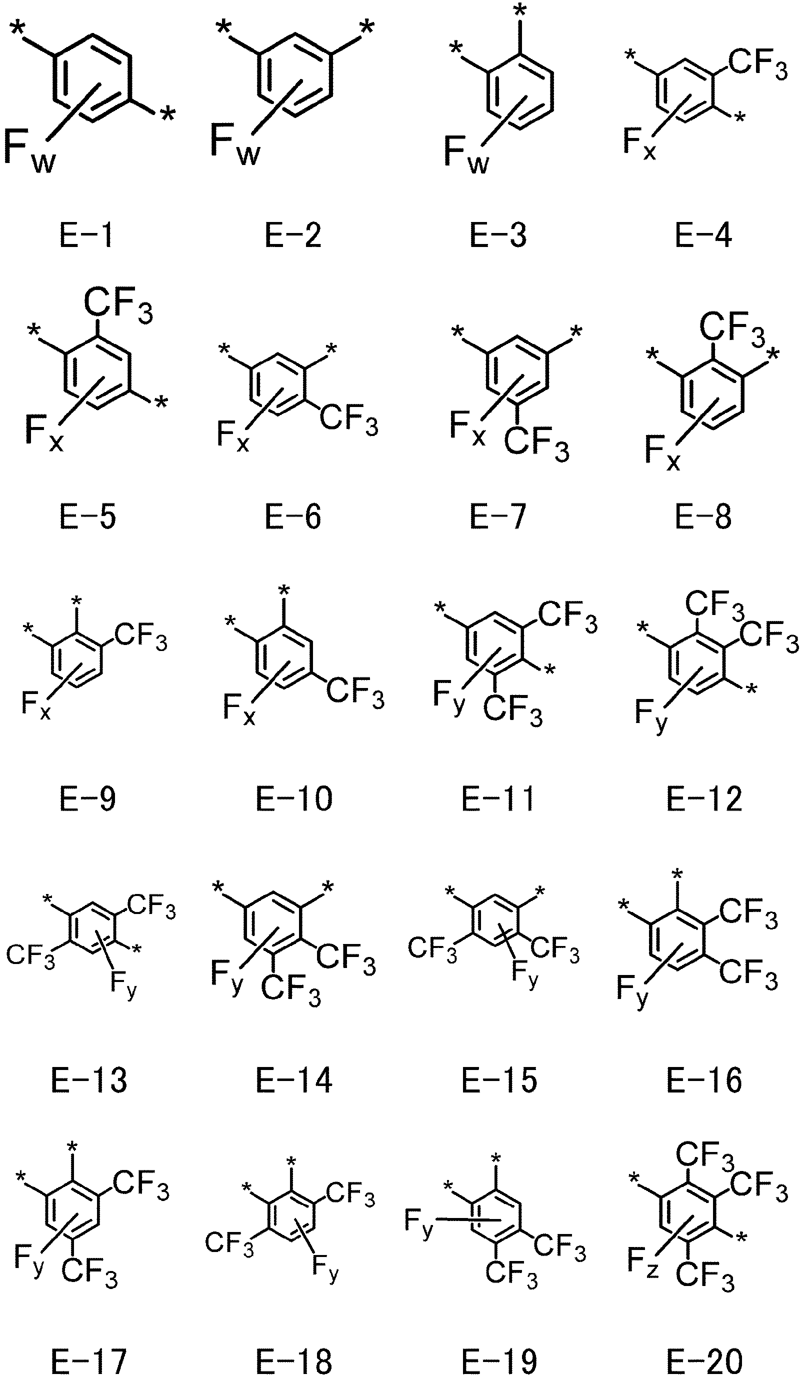

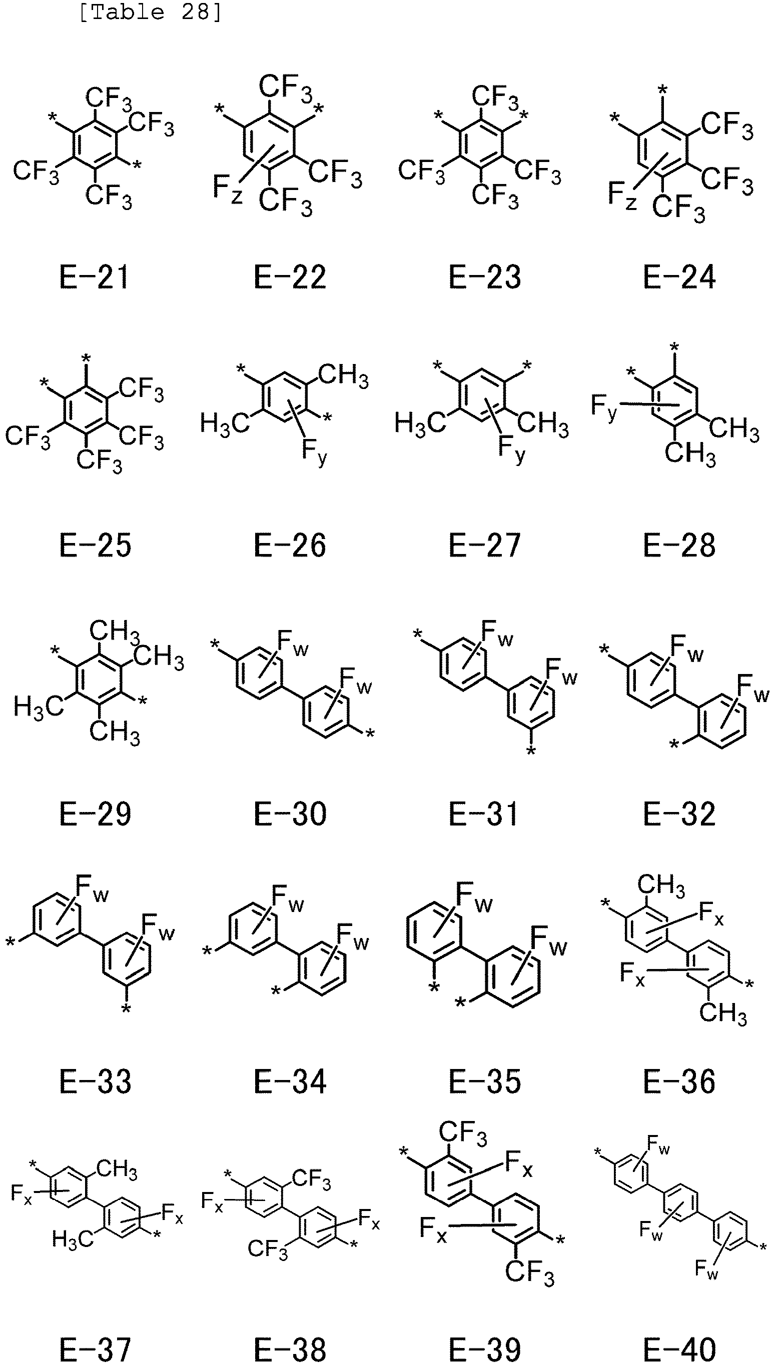

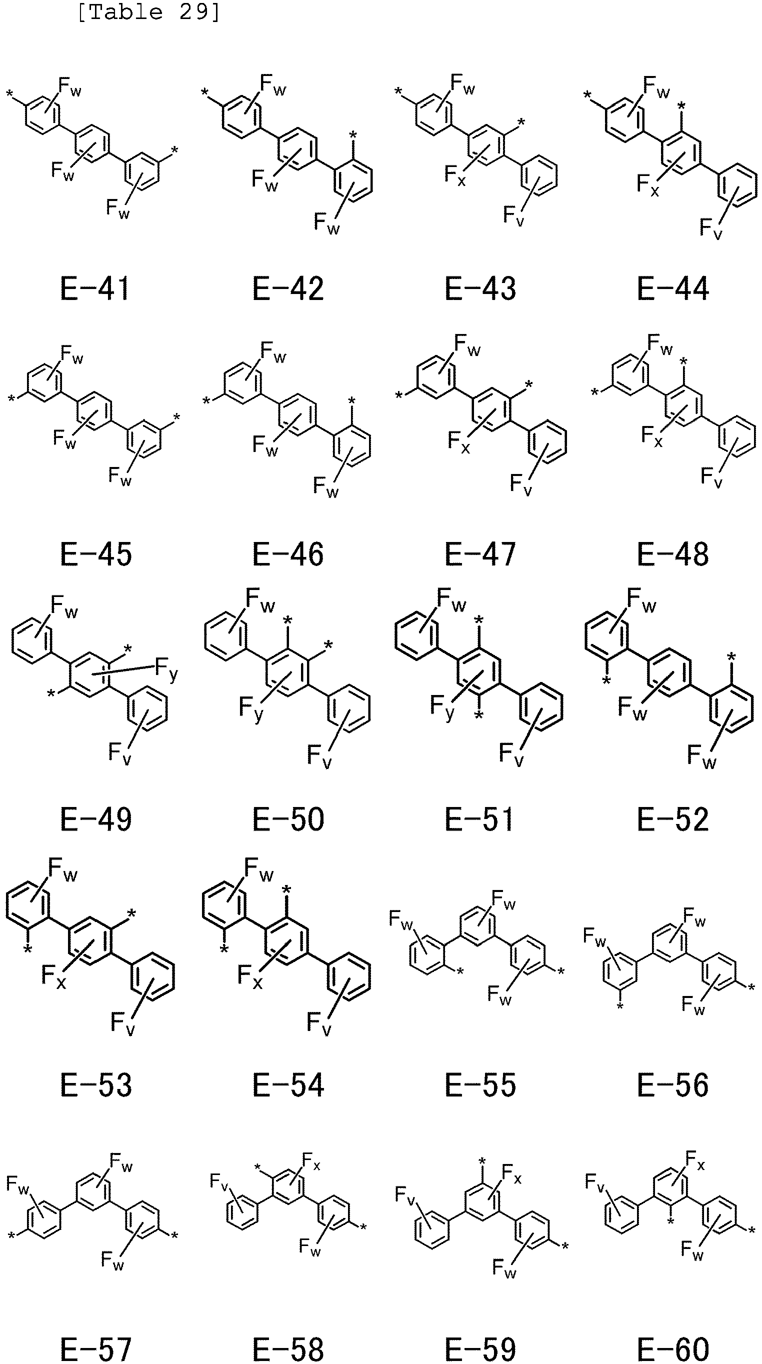

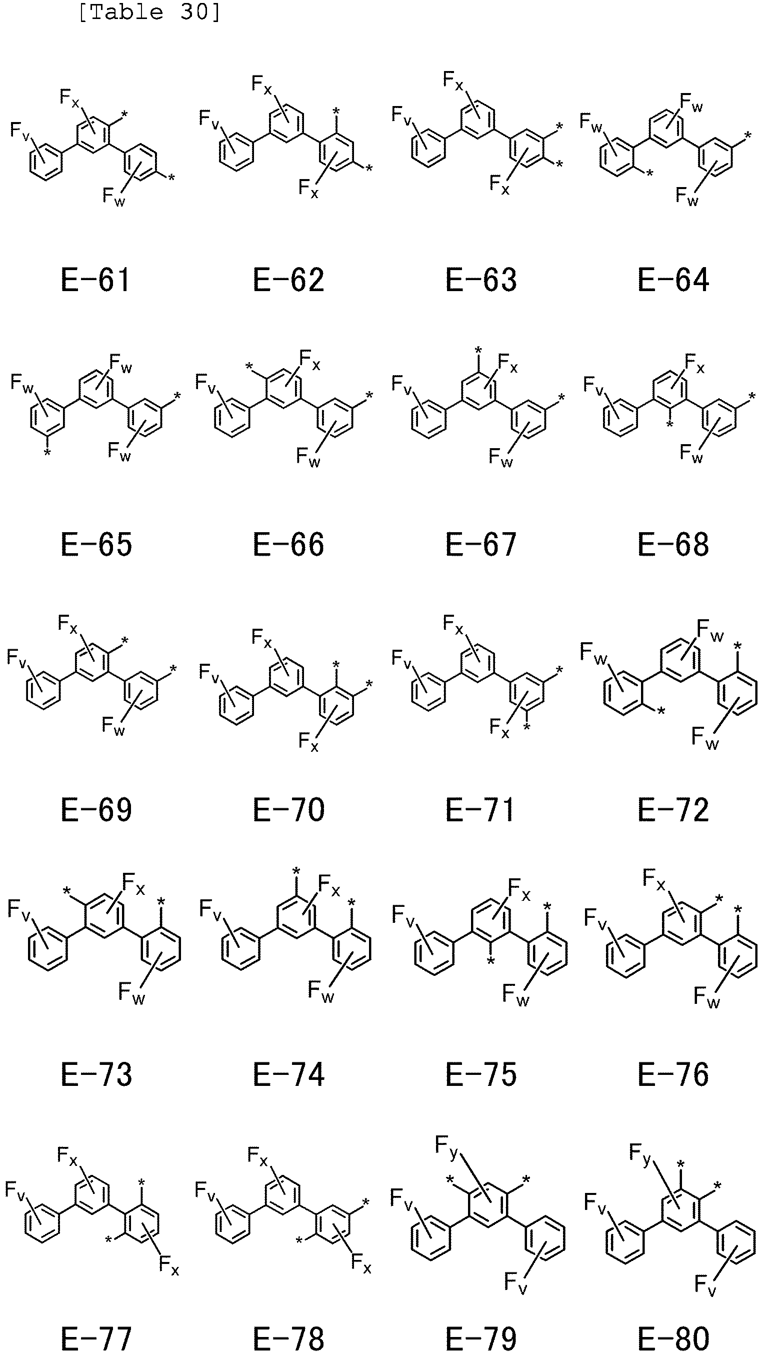

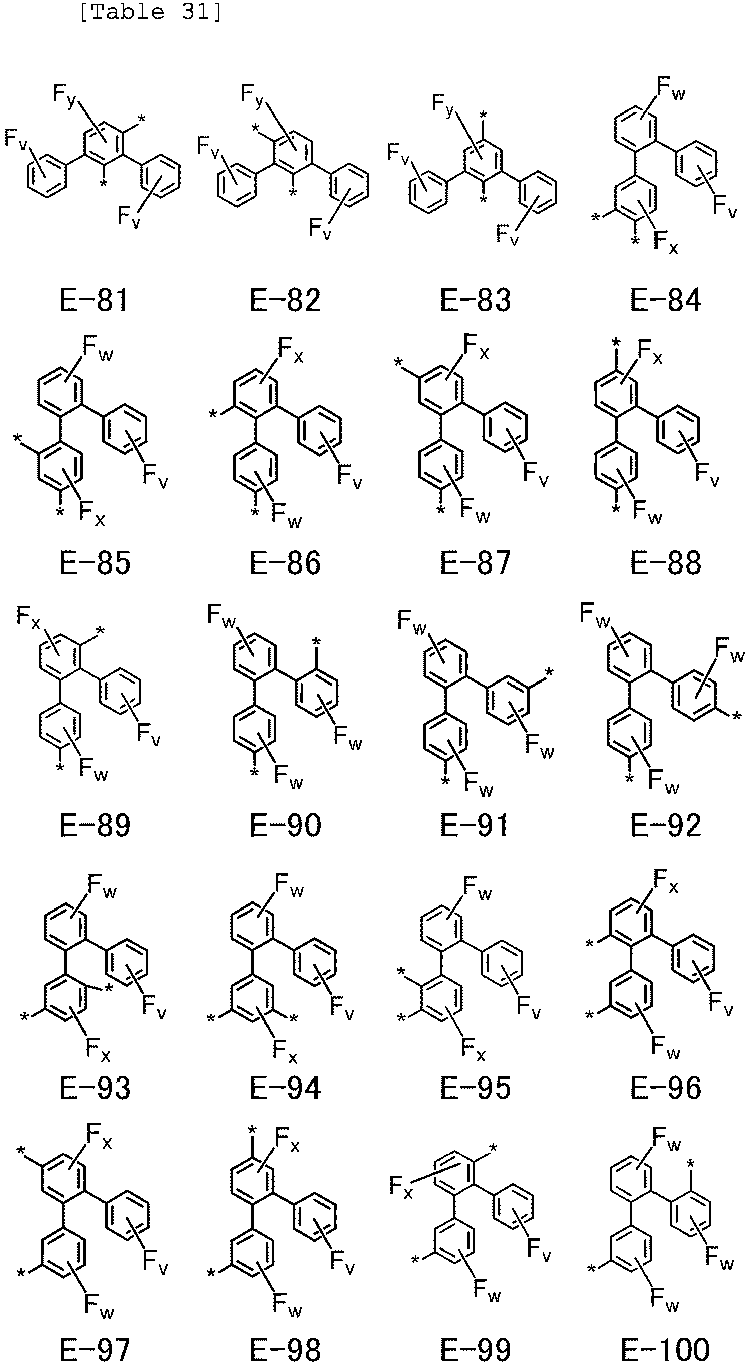

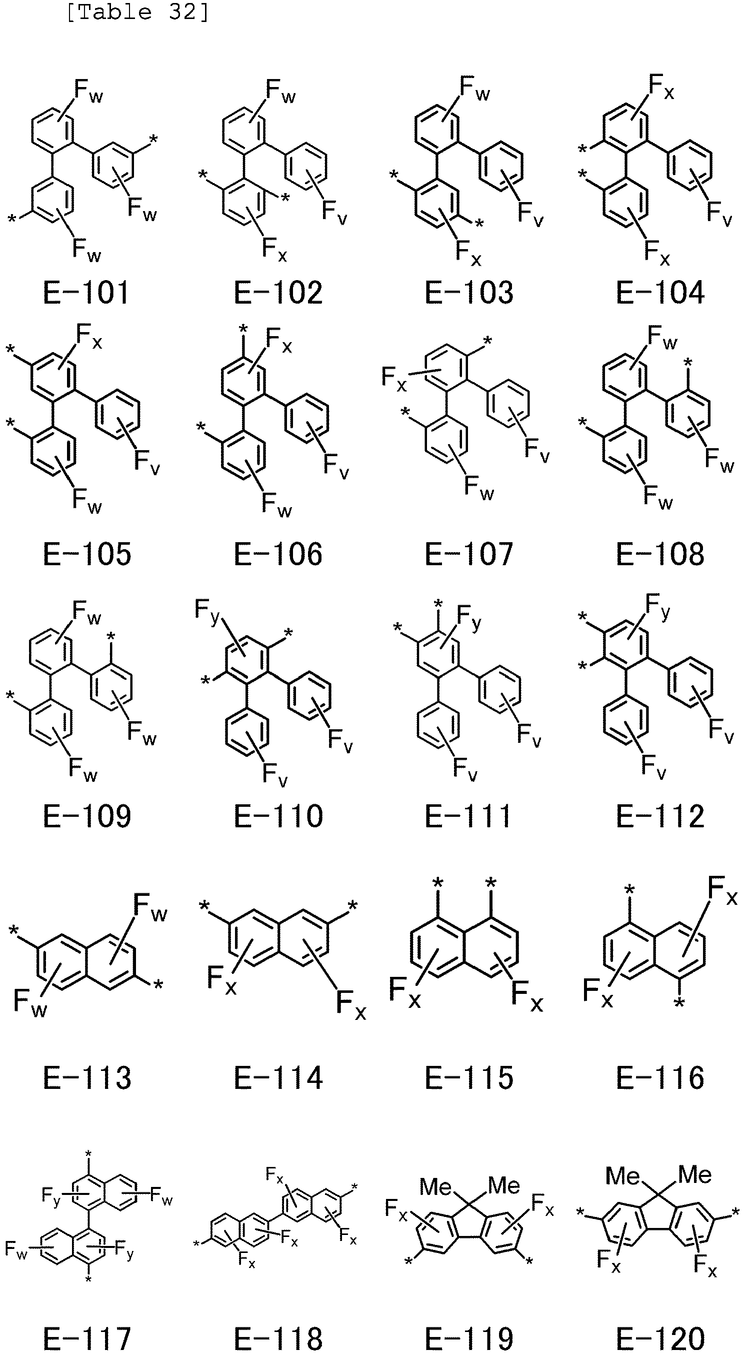

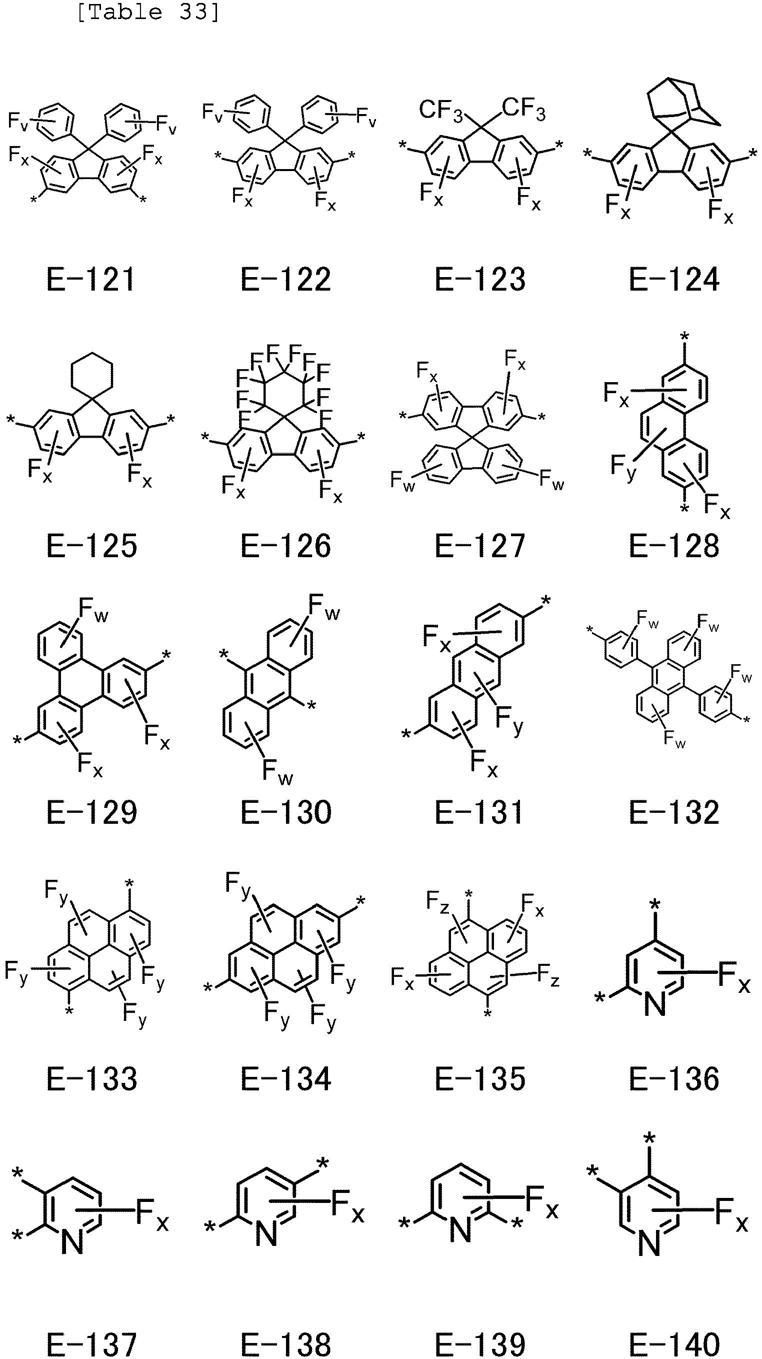

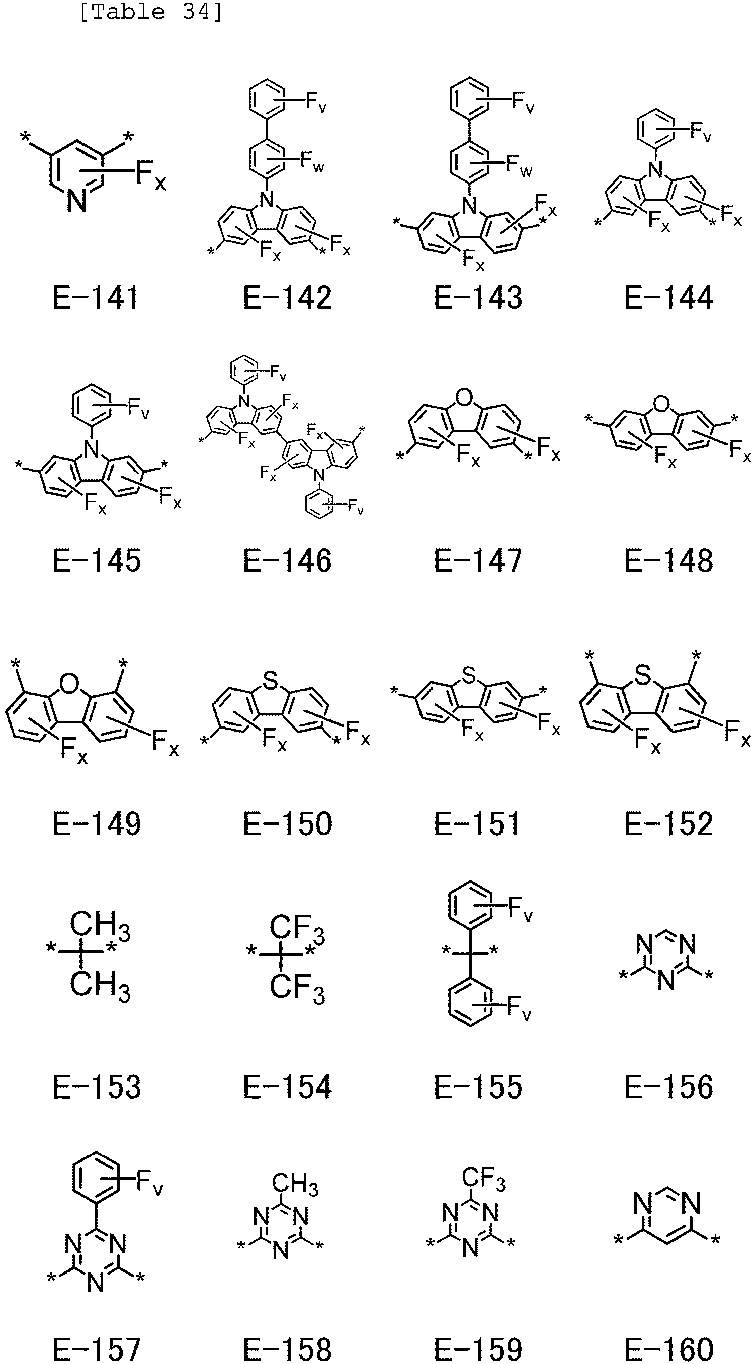

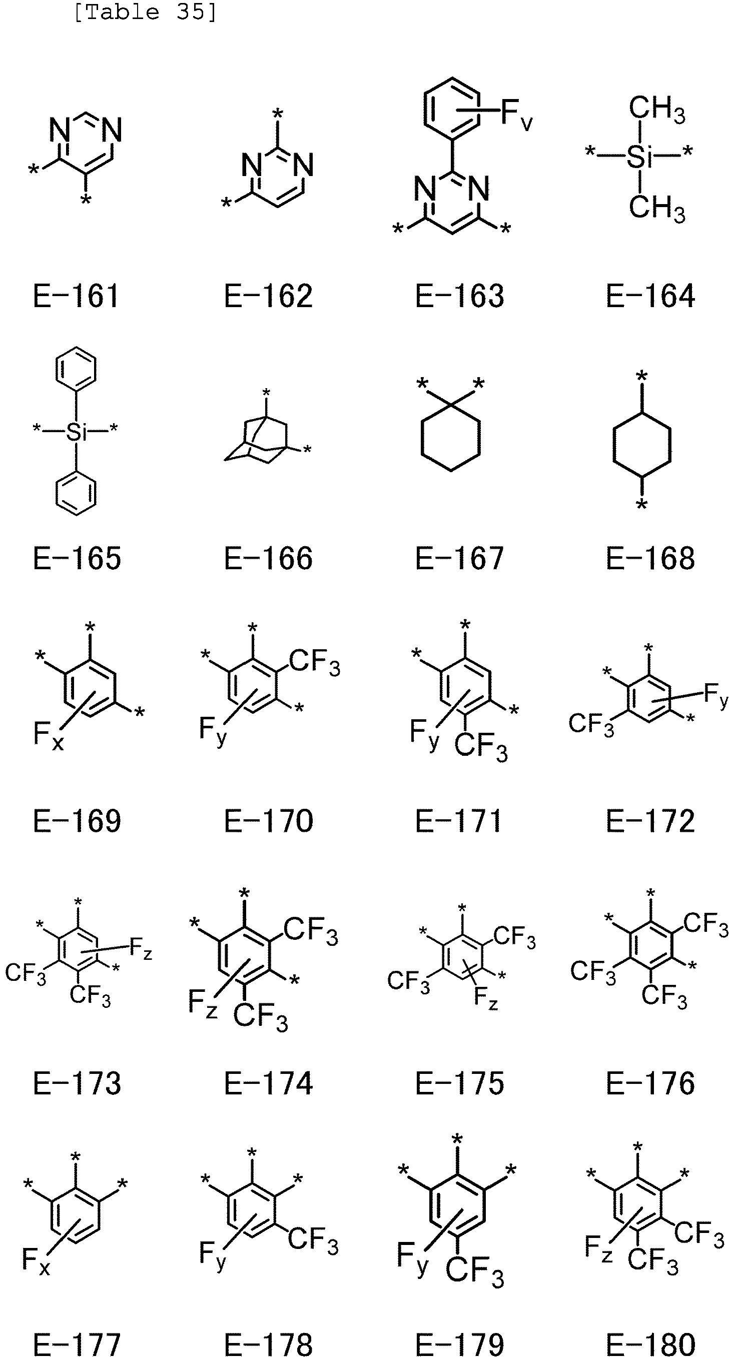

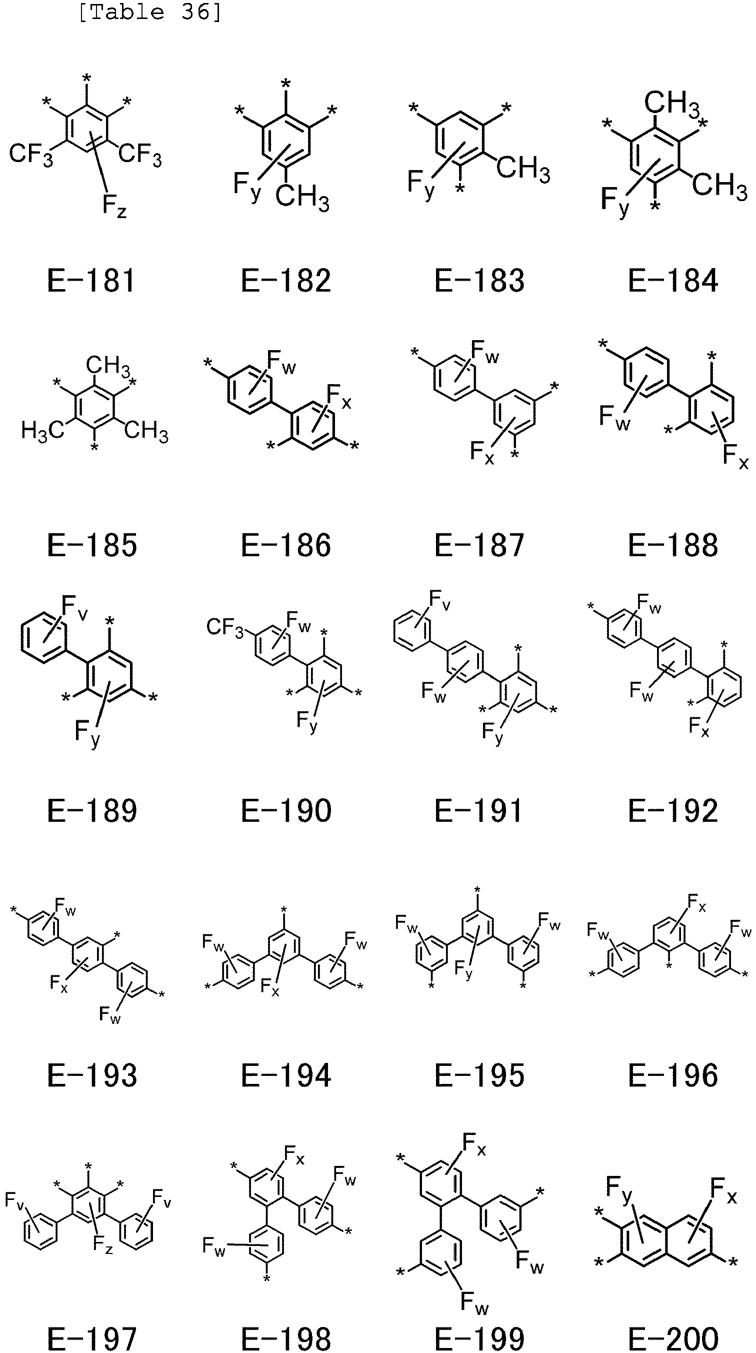

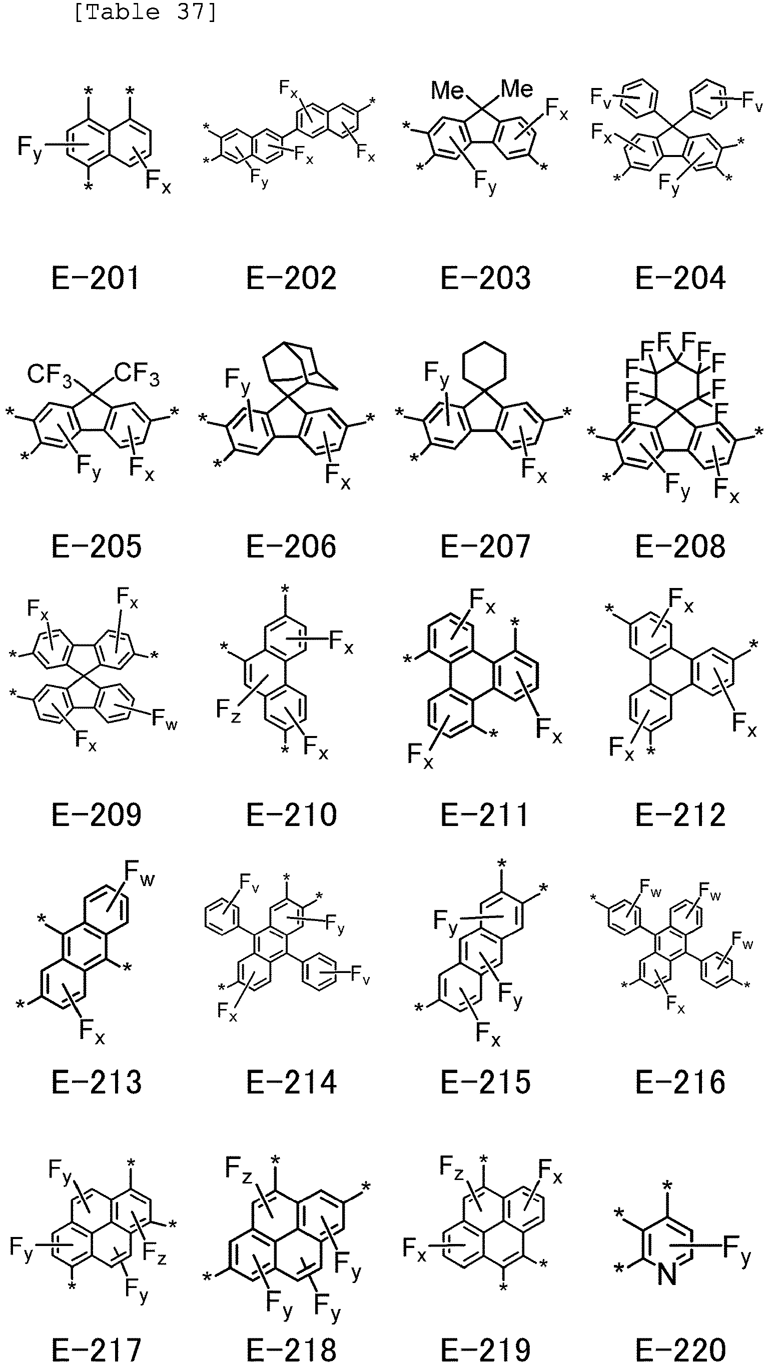

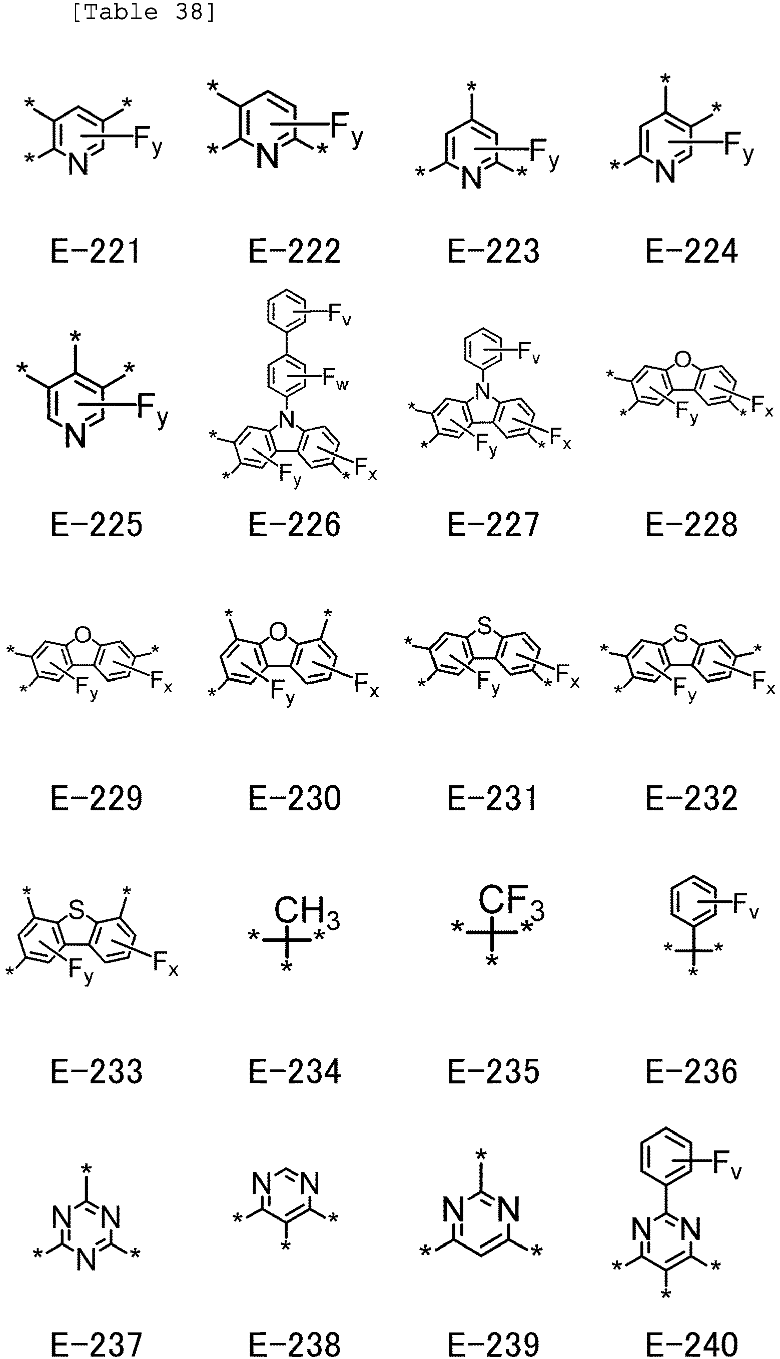

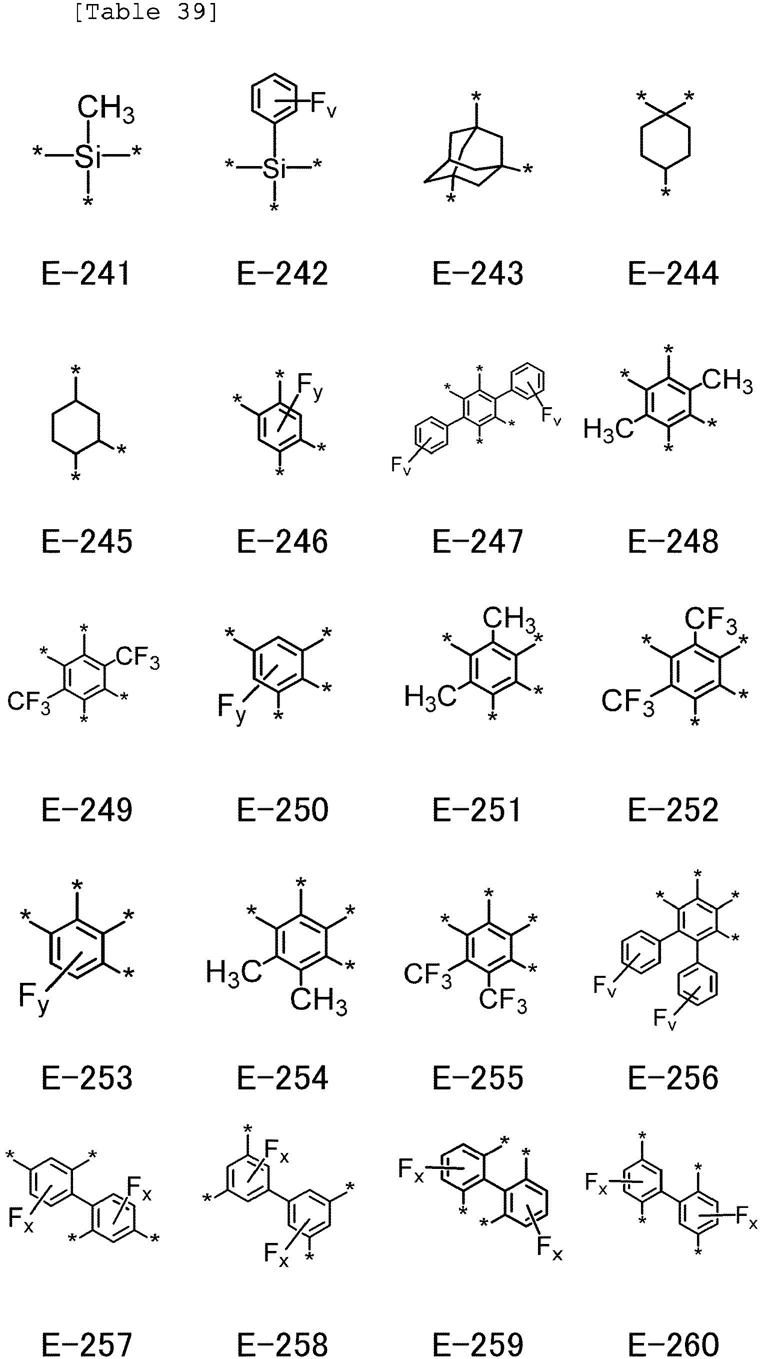

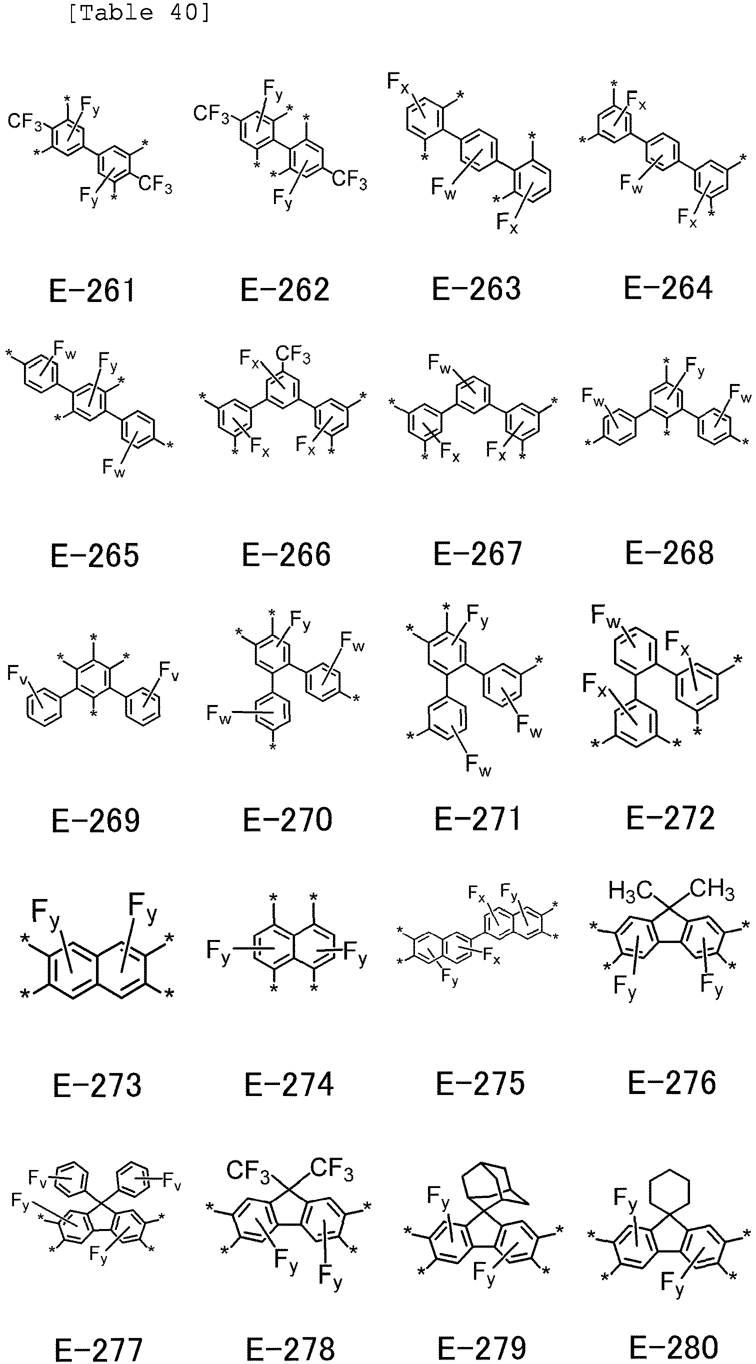

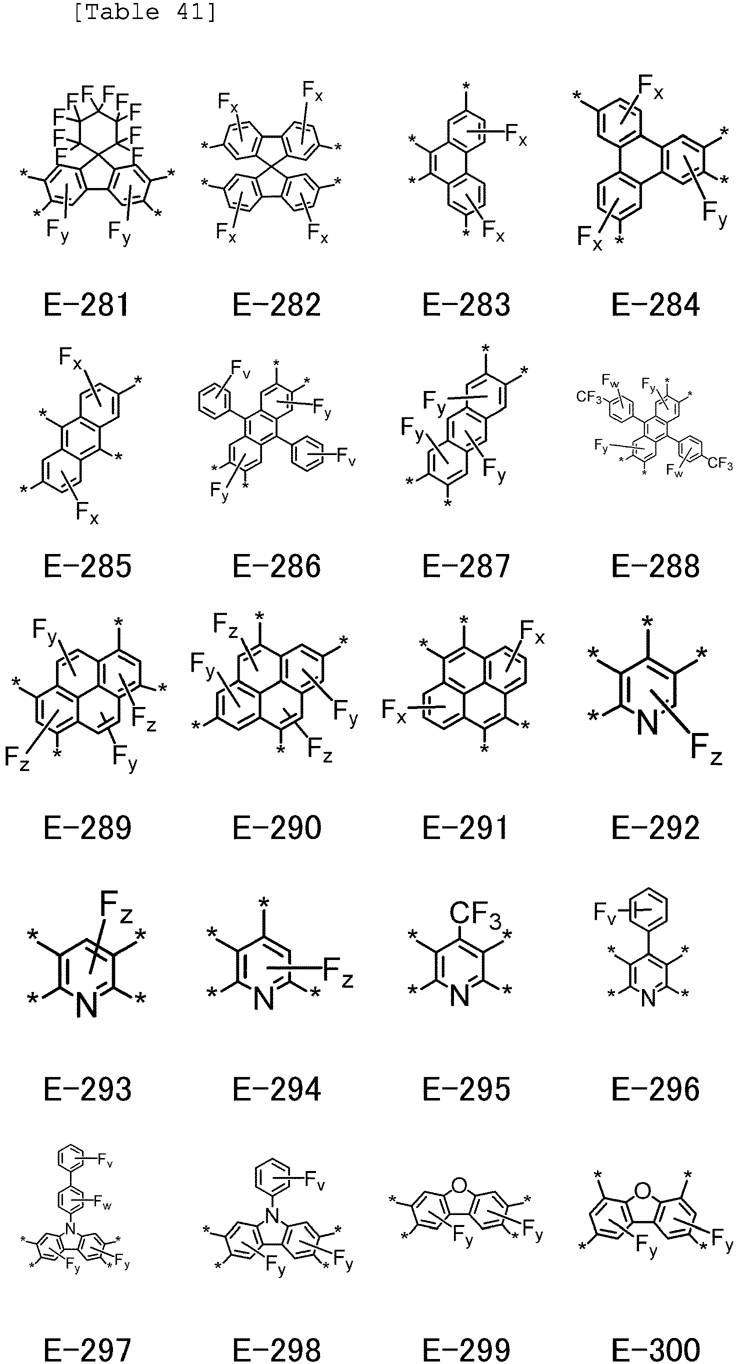

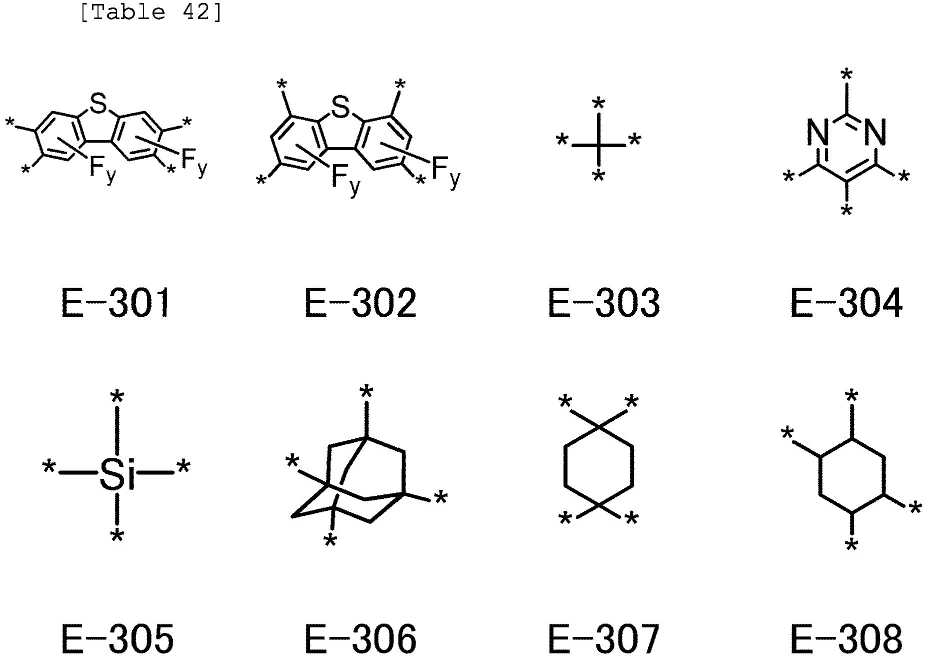

Due to having a high glass transition temperature and being able to suppress the formation of a metal film on the surface of the film, X even more preferably represents
(vi') a phenylene group, biphenylene group, terphenylylene group, naphthylene group, fluorenylene group, spirobifluorenylene group, benzofluorenylene group, phenanthrene group, fluoranthenylene group, triphenylenylene group, anthracenediyl group, pyridylene group, pyrimidinediyl group, triazinediyl group, carbazolediyl group, benzofuryl group, benzothiophenediyl group, dibenzofuryl group, adamantanediyl group, methylene group, silanediyl group, cyclohexylene group, phenyltriyl group, biphenyltriyl group, terphenyltriyl group, naphthylyl group, fluorenetriyl group, spirobifluorenetriyl group, benzofluorenetriyl group, phenanthrenetriyl group, fluoranthenetriyl group, triphenylenetriyl group, anthracenetriyl group, pyrenetriyl group, pyridinetriyl group, pyrimidinetriyl group, triazinetriyl group, carbazole triyl group, benzofuran triyl group, benzothiophene triyl group, dibenzofuran triyl group, adamantane triyl group, methane triyl group, silane triyl group, cyclohexane triyl group, phenyl tetrayl group, biphenyl tetrayl group, terphenyl tetrayl group, naphthyl tetrayl group, fluorene tetrayl group, spirobifluorene tetrayl group, benzofluorene tetrayl group, phenanthrene tetrayl group, fluoranthene tetrayl group, triphenylene tetrayl group, anthracene tetrayl group, pyrene tetrayl group, pyridine tetrayl group, pyrimidine tetrayl group, carbazole tetrayl group, benzofuran tetrayl group, benzothiophene tetrayl group, dibenzofuran tetrayl group, adamantane tetrayl group, methane tetrayl group, silane tetrayl group, or cyclohexane tetrayl group; or
(vii') a group in which the group represented by (vi ') is substituted with one or more groups selected from the group consisting of a methyl group, a trifluoromethyl group, a perfluoroalkyl group having 2 to 10 carbon atoms, a fluorine atom, and a phenyl group optionally substituted with a fluorine atom.

Due to having a high glass transition temperature is high and being able to suppress the formation of a metal thin film on a film surface, X is particularly preferably a phenylene group, a phenylene group having at least one fluorine atom, a phenylene group having at least one trifluoromethyl group, a biphenylene group, a biphenylene group having at least one fluorine atom, a biphenylene group having at least one trifluoromethyl group, a terphenylylene group, a terphenylylene group having at least one fluorine atom, a terphenylylene group having at least one fluorine atom, a terphenylylene group, a naphthylene group, fluorenylene group, a spirobifluorenylene group, a benzofluorenylene group, a phenanthrene group, a fluoranthenylene group, a triphenylenylene group, an anthracenediyl group, a pyridylene group, a pyrimidinediyl group, a triazinediyl group, a carbazolediyl group, a benzofuryl group, a benzothiophenediyl group, a dibenzofuryl group, an adamantanediyl group, a methylene group, a silanediyl group, a cyclohexylene group; a phenyltriyl group, a phenyltriyl group having at least one fluorine atom, a phenyltriyl group having at least one trifluoromethyl group, a biphenyltriyl group, a biphenyltriyl group having at least one fluorine atom, a biphenyltriyl group having at least one trifluoromethyl group, a terphenyltriyl group, a terphenyltriyl group having at least one fluorine atom, a terphenyltriyl group having at least one trifluoromethyl group, a naphthylyl group, a fluorenetriyl group, a spirobifluorenetriyl group, a benzofluorenetriyl group, a phenanthrenetriyl group, a fluoranthenetriyl group, a triphenylenetriyl group, an anthracenetriyl group, a pyrenetriyl group, a pyridinetriyl group, a pyrimidinetriyl group, a triazinetriyl group, a carbazoletriyl group, benzofurantriyl group, a benzothiophenetriyl group, a dibenzofurantriyl group, an adamantanetriyl group, a methanetriyl group, a silanetriyl group, a cyclohexanetriyl group;
a phenyl tetrayl group, a phenyl tetrayl group having at least one fluorine atom, a phenyl tetrayl group having at least one trifluoromethyl group, a biphenyl tetrayl group, a biphenyl tetrayl group having at least one fluorine atom, a biphenyl tetrayl group having at least one trifluoromethyl group, a terphenyl tetrayl group, a terphenyl tetrayl group having at least one fluorine atom, a terphenyl tetrayl group, a naphthyl tetrayl group, a fluorene tetrayl group, a spirobiflurene tetrayl group, a benzofluorene tetrayl group, a phenanthrene tetrayl group, a fluoranthene tetrayl group, a triphenylene tetrayl group, an anthracene tetrayl group, a pyrene tetrayl group, a pyridine tetrayl group, a pyrimidine tetrayl group, a carbazole tetrayl group having at least one trifluoromethyl group, a benzofuran tetrayl group, a benzothiophene tetrayl group, a dibenzofuran tetrayl group, an adamantane tetrayl group, a methane tetrayl group, a silane tetrayl group, or a cyclohexane tetrayl group.

Due to being able to suppress the formation of a metal film on the surface of the film, the metal patterning material preferably has one or more groups selected from the group consisting of a perfluorophenyl group, a perfluorotolyl group, a perfluorodimethylphenyl group, and a perfluorobiphenylyl group in the molecule.

Due to being able to suppress the formation of a metal film on the film surface, the metal patterning material preferably has the proportion of the number of fluorine atoms to carbon atoms in the molecular structure of the metal patterning material larger than 1:4, more preferably larger than 1:3, and still more preferably larger than 1:2. From the viewpoint of the glass transition temperature and the sublimation temperature, the proportion is more preferable in the order of from 1:2 to 2:1, from 1:2 to 1.9:1, from 1:2 to 1.8:1, from 1:2 to 1.7:1, from 1:2 to 1.6 :1, and from 1:2 to 1.5:1.

In addition, the metal patterning material is preferably a compound represented by Formula (4), (5) or (6).

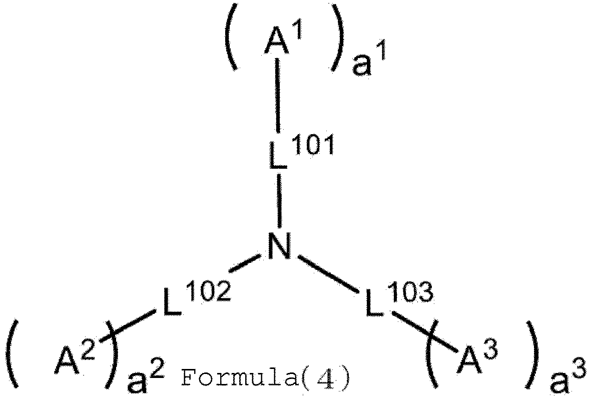

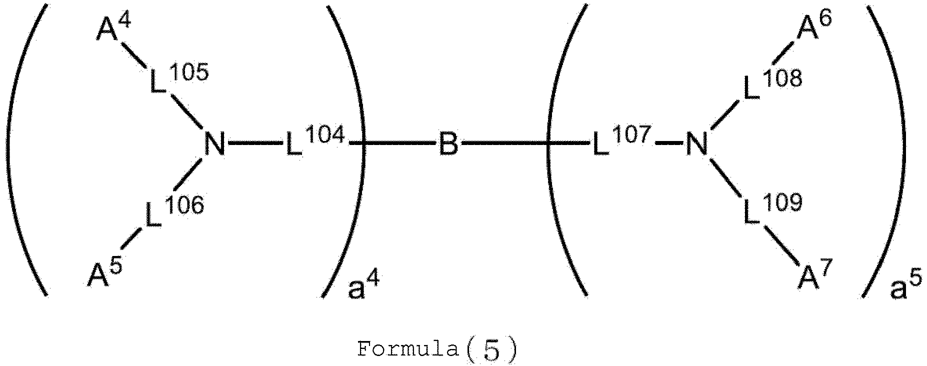

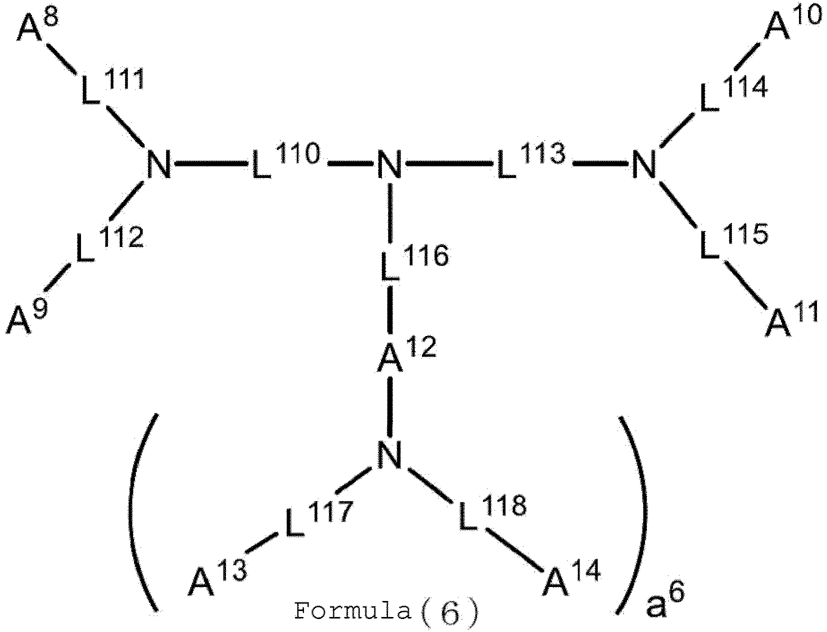

In the formula,
a¹, a² and a³ each independently represent an integer of 1 to 3;
a⁴ and a⁵ each independently represent an integer of 1 or 2;
a⁶ represents an integer of 0 or 1;
A¹ to A¹⁴ each independently represent
an optionally substituted monocyclic, linked or condensed aromatic hydrocarbon group having 6 to 25 carbon atoms;
an optionally substituted monocyclic, linked, or condensed heterocyclic heteroaromatic group having 3 to 25 carbon atoms; or
optionally substituted cyclic alkyl group having 3 to 25 carbon atoms;
provided that
at least one of A¹ to A³ is independently an optionally substituted cyclic alkyl group having 3 to 25 carbon atoms;
at least one of A⁴ to A⁷ is independently an optionally substituted cyclic alkyl group having 3 to 25 carbon atoms;
when a⁶ is 0, at least one of A⁸ to A¹¹ is independently an optionally substituted cyclic alkyl group having 3 to 25 carbon atoms;
when a⁶ is 1, at least one of A⁸ to A¹⁴ is independently an optionally substituted cyclic alkyl group having 3 to 25 carbon atoms;
L¹⁰¹ to L¹¹⁸ each independently represent
an optionally substituted monocyclic, linked, or condensed divalent aromatic hydrocarbon group having 6 to 25 carbon atoms;
an optionally substituted monocyclic, linked or condensed divalent heteroaromatic group having of 3 to 25 carbon atoms;
   or
a single bond;
B represents
an optionally substituted monocyclic, linked or condensed di-to tetravalent aromatic hydrocarbon group having ring of 6 to 25 carbon atoms;
an optionally substituted monocyclic, linked or condensed di-to tetravalent heteroaromatic group having ring of 3 to 25 carbon atoms;
an optionally substituted linear, branched or cyclic di- to tetravalent alkyl group having 1 to 10 carbon atoms; or
an optionally substituted di- to tetravalent silicon atom.

When A¹ to A¹⁴ are substituted aromatic hydrocarbon groups, substituted heteroaromatic groups, or substituted cyclic alkyl groups, each of these groups preferably is independently substituted with one or more groups selected from the group consisting of substituted by:
a linear, branched or cyclic alkyl group having 1 to 18 carbon atoms which may be substituted with a fluorine atom,
a linear, branched, or cyclic alkoxy group having 1 to 18 carbon atoms which may be substituted with a fluorine atom,
an aromatic hydrocarbon group having 6 to 20 carbon atoms which may be substituted with a fluorine atom;
a heteroaromatic group having 3 to 20 carbon atoms which may be substituted with a fluorine atom;
a cyano group,
a fluorine atom and
a deuterium atom;
when L¹⁰¹ to L¹¹⁸ are substituted divalent aromatic hydrocarbon groups or substituted divalent heteroaromatic groups, each of these groups preferably is independently substituted with one or more groups selected from the group consisting of:
   a linear, branched, or cyclic alkyl group having 1 to 18 carbon atoms which may be substituted with a fluorine atom;
   a linear, branched, or cyclic alkoxy group having 1 to 18 carbon atoms which may be substituted with a fluorine atom;
   an aromatic hydrocarbon group having 6 to 20 carbon atoms which may be substituted with a fluorine atom;
   a heteroaromatic group having 3 to 20 carbon atoms which may be substituted with a fluorine atom;
   a cyano group,
   a fluorine atom, and
   a deuterium atom;
   when B is a substituted di- to tetravalent aromatic hydrocarbon group, a substituted di- to tetravalent heteroaromatic group, a substituted di- to tetravalent alkyl group, or a substituted di- to tetravalent silicon atom, each of these groups preferably is independently substituted by one or more groups selected from the group consisting of:
      a linear, branched, or cyclic alkyl group having 1 to 18 carbon atoms which may be substituted with a fluorine atom;
      a linear, branched, or cyclic alkoxy group having 1 to 18 carbon atoms which may be substituted with a fluorine atom;
      an aromatic hydrocarbon group having 6 to 20 carbon atoms which may be substituted with a fluorine atom;
      a heteroaromatic group having 3 to 20 carbon atoms which may be substituted with a fluorine atom;
      a cyano group,
      a fluorine atoms and
      a deuterium atom.

As the condensed cyclic alkyl group having 3 to 25 carbon atoms, for example, a cyclobutyl group, a cyclopentyl group, a cyclohexyl group, a cycloheptyl group, a cyclooctyl group, an adamantyl group, a noradamantyl group, a norbornyl group, a diamantyl group, and a decahydronaphthalene group can be exemplified.

It should be noted that, as described above, the condensed cyclic alkyl group having 3 to 25 carbon atoms may have a substituent. When they have a substituent, it is preferably substituted by one or more groups selected from the group consisting of a linear, branched, or cyclic alkyl group having 1 to 18 carbon atoms which may be substituted with a fluorine atom, a linear, branched, or cyclic alkoxy group having 1 to 18 carbon atoms which may be substituted with a fluorine atom, an aromatic hydrocarbon group having 6 to 20 carbon atoms which may be substituted with a fluorine atom, a heteroaromatic group having 3 to 20 carbon atoms which may be substituted with a fluorine atom, a cyano group, a fluorine atom or a deuterium atom. In this case, the number of substituents is not particularly limited.

In Formula (4), (5) or (6), a monocyclic, linked, or condensed aromatic hydrocarbon group having 6 to 25 carbon atoms; a monocyclic, linked, or condensed heteroaromatic group having 3 to 25 carbon atoms; a monocyclic, linked, or condensed aromatic hydrocarbon group having 6 to 25 carbon atoms; a monocyclic, linked or condensed divalent heteroaromatic group having 3 to 25 carbon atoms; a monocyclic, linked, or condensed di- to tetravalent aromatic hydrocarbon group having 6 to 25 carbon atoms; a monocyclic, linked, or condensed di- to tetravalent heteroaromatic group having 3 to 25 carbon atoms; a linear, branched or cyclic di-to tetravalent alkyl group having 1 to 10 carbon atoms; a linear, branched, or cyclic alkyl group having 1 to 18 carbon atoms which may be substituted with a fluorine atom; a linear, branched, or cyclic alkoxy group having 1 to 18 carbon atoms which may be substituted with a fluorine atom; an aromatic hydrocarbon group having 6 to 20 carbon atoms which may be substituted with a fluorine atom; a heteroaromatic group having 3 to 20 carbon atoms which may be substituted with a fluorine atom; an aromatic hydrocarbon group having 6 to 25 carbon atoms; a monocyclic, linked, or condensed heteroaromatic group having 3 to 25 carbon atoms; a monocyclic, linked, or condensed aromatic hydrocarbon group having 6 to 25 carbon atoms; a monocyclic, linked or condensed divalent heteroaromatic group having 3 to 25 carbon atoms; a monocyclic, linked, or condensed di- to tetravalent aromatic hydrocarbon group having 6 to 25 carbon atoms; a monocyclic, linked, or condensed di- to tetravalent heteroaromatic group having 3 to 25 carbon atoms; a linear, branched or cyclic di-to tetravalent alkyl groups having 1 to 10 carbon atoms; a linear, branched, or cyclic alkyl group having 1 to 18 carbon atoms which may be substituted with a fluorine atom; a linear, branched, or cyclic alkoxy group having 1 to 18 carbon atoms which may be substituted with a fluorine atom; an aromatic hydrocarbon group having 6 to 20 carbon atoms which may be substituted with a fluorine atom; a heteroaromatic group having 3 to 20 carbon atoms which may be substituted with a fluorine atom; are synonymous. From the viewpoint of being able to suppress the formation of the metal film on the surface of the film, the number of carbon atoms in the condensed aromatic hydrocarbon group in Formulas (4) to (6) is preferably 6 to 15. Specifically, it is preferably from 6 to 15, more preferably from 6 to 14, and still more preferably from 6 to 13.

In Formulas (4) to (6), due A¹ to A¹⁴ having a high glass transition temperature and being able to suppress the formation of a metal film on the film surface, they preferably each independently represent:
(viii) a phenyl group, biphenylyl group, terphenylyl group, naphthyl group, fluorenyl group, spirobifluorenyl group, benzofluorenyl group, phenanthryl group, fluoranthyl group, fluoranthenyl group, triphenylenyl group, anthryl group, pyrenyl group, pyridyl group, carbazolyl group, benzofuranyl group, benzothienyl group, dibenzofuranyl group, dibenzofuranyl group, dibenzothienyl group, cyclopentyl group, cyclohexyl group, diamantyl group, adamantyl group, noradamantyl group, norbornyl group or decahydronaphthalene group; or
(ix) a group in which the group represented by (viii) is substituted with one or more groups selected from the group consisting of: a methyl group, an ethyl group, a methoxy group, an ethoxy group, a trifluoromethyl group, a trifluoromethoxy group, a perfluoroalkyl group having 2 to 10 carbon atoms, a perfluoroalkoxy group having 2 to 10 carbon atoms, a cyano group, a deuterium atom, a fluorine atom, a phenyl group optionally substituted with a fluorine atom, a biphenylyl group optionally substituted with a fluorine atom, a naphthyl group optionally substituted with a fluorine atom, a phenanthryl group which may be substituted with a fluorine atom, a pyridyl group which may be substituted with a fluorine atom, a carbazolyl group which may be substituted with a fluorine atom, a dibenzothienyl group which may be substituted with a fluorine atom, and a dibenzofuranyl group which may be substituted with a fluorine atom.

In Formulas (4) to (6), due A¹ to A¹⁴ having a high glass transition temperature and being able to suppress the formation of a metal film on the film surface, they more preferably each independently represent
(viii') a phenyl group, a biphenylyl group, a terphenylyl group, a naphthyl group, a fluorenyl group, a spirobifluorenyl group, a phenanthryl group, a fluoranthenyl group, a triphenylenyl group, an anthryl group, a pyrenyl group, a pyridyl group, a carbazolyl group, a dibenzofuranyl group, a dibenzothienyl group, a cyclopentyl group, a cyclohexyl group, a diamantyl group, an adamantyl group, a noradamantyl group, a norbornyl group, or a decahydronaphthalene group; or
(ix') a group in which the group represented by (viii ') is substituted with one or more groups selected from the group consisting of a methyl group, a trifluoromethyl group, a perfluoroalkyl group having 2 to 10 carbon atoms, a fluorine atom, and a phenyl group optionally substituted with a fluorine atom.

Due to A¹ to A¹⁴ having a high glass transition temperature and being able to suppress the formation of a metal film on the film surface, they even more preferably each independently represent
a phenyl group, a phenyl group having at least one fluorine atom, a phenyl group having at least one trifluoromethyl group, a biphenylyl group, a biphenylyl group having at least one fluorine atom, a biphenylyl group having at least one trifluoromethyl group, a terfenylyl group, a terfenylyl group having at least one fluorine atom, a terfenyl group having at least one trifluoromethyl group, a naphthyl group, a 9,9-dimethylfluorenyl group, a 9,9-diphenylfluorenyl group, a spirobiflurenyl group, a phenanthryl group, a carbazol-9-yl group, a 9-phenylcarbazolyl group, a dibenzofuranyl group, a dibenzothienyl group, a cyclohexyl group, a cyclohexyl group having at least one fluorine atom, a cyclohexyl group having at least one trifluoromethyl group, an adamantyl group, an adamantyl group having at least one fluorine atom, or an adamantyl group having at least one trifluoromethyl group.

In Formulas (4) to (6), due to L¹⁰¹ to L¹¹⁸ having a high glass transition temperature and being able to suppress the formation of a metal film on the film surface, they preferably each independently represent
(x) a phenylene group, biphenylylene group, terphenylene group, naphthylene group, pyridylene group, or fluorenylene group;
(xi) a group in which the group represented by (x) is substituted with one or more groups selected from the group consisting of a methyl group, an ethyl group, a methoxy group, an ethoxy group, a trifluoromethyl group, a trifluoromethoxy group, a perfluoroalkyl group having 2 to 10 carbon atoms, a perfluoroalkoxy group having 2 to 10 carbon atoms, a cyano group, a deuterium atom, a fluorine atom, a phenyl group optionally substituted with a fluorine atom, a biphenylyl group optionally substituted with a fluorine atom, a naphthyl group optionally substituted with a fluorine atom, a phenanthryl group optionally substituted with a fluorine atom, a pyridyl group optionally substituted with a fluorine atom, a carbazolyl group optionally substituted with a fluorine atom, a dibenzothienyl group optionally substituted with a fluorine atom, and a dibenzofuranyl group optionally substituted with a fluorine atom; or
(xii) a single bond.

Due to L¹⁰¹ to L¹¹⁸ having a high glass transition temperature and being able to suppress the formation of a metal film on the film surface, they more preferably each independently represent
(x') a phenylene group, a biphenylylene group, a terphenylylene group, a naphthylene group, a pyridylene group, or a fluorenylene group;
(xi') a group in which the group represented by (x') is substituted with one or more groups selected from the group consisting of a methyl group, a trifluoromethyl group, a fluorine atom, and a phenyl group optionally substituted with a fluorine atom; or
(xii') a single bond.

Due to L¹⁰¹ to L¹¹⁸ having a high glass transition temperature and being able to suppress the formation of a metal film on the film surface, they even more preferably are each independently: a phenylene group, a phenylene group having at least one fluorine atom, a phenylene group having at least one trifluoromethyl group, a biphenylylene group, a biphenylylene group having at least one fluorine atom, a biphenylylene group having at least one trifluoromethyl group, a terphenylylene group, a terphenylylene group having at least one fluorine atom, a terphenylylene group having at least one trifluoromethyl group, a naphthylene group, or a single bond.

In Formulas (4) to (6), due to having a high glass transition temperature and being able to suppress the formation of a metal film on the film surface, B preferably represents
(xiii) a phenylene group, biphenylene group, terphenylylene group, naphthylene group, fluorenylene group, spirobifluorenylene group, benzofluorenylene group, phenanthrene group, fluoranthenylene group, triphenylenylene group, anthracenediyl group, pyridylene group, pyrimidinediyl group, triazinediyl group, carbazoldiyl group, benzoflanyl group, benzothiophenediyl group, dibenzofuryl group, adamantanediyl group, methylene group, silanediyl group, cyclohexylene group, phenyltriyl group, biphenyltriyl group, terphenyltriyl group, naphthylyl group, fluorenetriyl group, spirobiflurenetriyl group, benzofluorenetriyl group, phenanthrenetriyl group, fluoranthenetriyl group, triphenylenetriyl group, anthracenetriyl group, pyrenetriyl group, pyridinetriyl group, pyrimidinetriyl group, triazinetriyl group, carbazole triyl group, benzofuran triyl group, benzothiophene triyl group, dibenzofuran triyl group, adamantane triyl group, methane triyl group, silane triyl group, cyclohexane triyl group, phenyl tetrayl group, biphenyl tetrayl group, terphenyl tetrayl group, naphthyl tetrayl group, fluorene tetrayl group, spirobifluorene tetrayl group, benzofluorene tetrayl group, phenanthrene tetrayl group, fluoranthene tetrayl group, triphenylene tetrayl group, anthracene tetrayl group, pyrene tetrayl group, pyridine tetrayl group, pyrimidine tetrayl group, carbazole tetrayl group, benzofuran tetrayl group, benzothiophene tetrayl group, dibenzofuran tetrayl group, adamantane tetrayl group, methane tetrayl group, silane tetrayl group, or cyclohexane tetrayl group; or
(xiv) a group in which the group represented by (xiii) is substituted with one or more groups selected from the group consisting of: a methyl group, an ethyl group, a methoxy group, an ethoxy group, a trifluoromethyl group, a trifluoromethoxy group, a perfluoroalkyl group having 2 to 10 carbon atoms, a perfluoroalkoxy group having 2 to 10 carbon atoms, a cyano group, a deuterium atom, a fluorine atom, a phenyl group optionally substituted with a fluorine atom, a biphenylyl group optionally substituted with a fluorine atom, a naphthyl group optionally substituted with a fluorine atom, a phenanthryl group which may be substituted with a fluorine atom, a pyridyl group which may be substituted with a fluorine atom, a carbazolyl group which may be substituted with a fluorine atom, a dibenzothienyl group which may be substituted with a fluorine atom, and a dibenzofuranyl group which may be substituted with a fluorine atom.

Due to having a high glass transition temperature and being able to suppress the formation of a metal film on the film surface, B more preferably represents
(xii') a phenylene group, a biphenylene group, a terphenylylene group, a naphthylene group, a fluorenylene group, a spirobifluorenylene group, a benzofluorenylene group, a phenanthrene group, a fluoranthenylene group, a triphenylenylene group, an anthracenediyl group, a pyridylene group, a pyrimidinediyl group, a triazinediyl group, a carbazolediyl group, a benzofuryl group, a benzothiophenediyl group, a dibenzofuryl group, an adamantanediyl group, a methylene group, a silanediyl group, cyclohexylene group, a phenyltriyl group, biphenyltriyl group, a terphenyltriyl group, naphthylyl group, a fluorenetriyl group, a spirobifluorenetriyl group, a benzofluorenetriyl group, a phenanthrenetriyl group, a fluoranthenetriyl group, a triphenylenetriyl group, an anthracenetriyl group, a pyrenetriyl group, a pyridinetriyl group, a pyrimidinetriyl group, a triazinetriyl group, a carbazole triyl group, a benzofuran triyl group, a benzothiophene triyl group, a dibenzofuran triyl group, an adamantane triyl group, a methane triyl group, a silane triyl group, a cyclohexane triyl group, a phenyl tetrayl group, a biphenyl tetrayl group, a terphenyl tetrayl group, a naphthyl tetrayl group, a fluorene tetrayl group, a spirobifluorene tetrayl group, a benzofluorene tetrayl group, a phenanthrene tetrayl group, a fluoranthene tetrayl group, a triphenylene tetrayl group, an anthracene tetrayl group, a pyrene tetrayl group, a pyridine tetrayl group, a pyrimidine tetrayl group, a carbazole tetrayl group, a benzofuran tetrayl group, a benzothiophene tetrayl group, a dibenzofuran tetrayl group, an adamantane tetrayl group, a methane tetrayl group, a silane tetrayl group, or a cyclohexane tetrayl group; or
(xiv') a group in which the group represented by (xiii') is substituted with one or more groups selected from the group consisting of: a methyl group, a trifluoromethyl group, a perfluoroalkyl group having 2 to 10 carbon atoms, a fluorine atom, and a phenyl group optionally substituted with a fluorine atom.

Due to having a high glass transition temperature and being able to suppress the formation of a metal film on the film surface, B is even more preferably: a phenylene group, a phenylene group having at least one fluorine atom, a phenylene group having at least one trifluoromethyl group, a biphenylene group, a biphenylene group having at least one fluorine atom, a biphenylene group having at least one trifluoromethyl group, a terphenylylene group, a terphenylylene group having at least one fluorine atom, a terphenylylene group having at least one fluorine atom, a terphenylylene group, a naphthylene group, a fluorenylene group, a spirobifluorenylene group, a benzofluorenylene group, a phenanthrene group, a fluoranthenylene group, a triphenylenylene group, an anthracenediyl group, a pyridylene group, a pyrimidinediyl group, a triazinediyl group, a carbazolediyl group, a benzofuryl group, a benzothiophenediyl group, a dibenzofuryl group, an adamantanediyl group, a methylene group, a silanediyl group, or a cyclohexylene group;
a phenyltriyl group, phenyltriyl group having at least one fluorine atom, phenyltriyl group having at least one trifluoromethyl group, biphenyltriyl group, biphenyltriyl group having at least one fluorine atom, biphenyltriyl group having at least one trifluoromethyl group, terphenyltriyl group, terphenyltriyl group having at least one fluorine atom, terphenyltriyl group having at least one trifluoromethyl group, naphthylyl group, fluorenetriyl group, spirobifluorenetriyl group, benzofluorenetriyl group, phenanthrenetriyl group, fluoranthenetriyl group, triphenylenetriyl group, anthracenetriyl group, pyrenetriyl group, pyridinetriyl group, pyrimidinetriyl group, triazinetriyl group, carbazoletriyl group, benzofurantriyl group, benzothiophenetriyl group, dibenzofurantriyl group, adamantanetriyl group, methanetriyl group, silanetriyl group, or cyclohexanetriyl group; or
a phenyl tetrayl group, phenyl tetrayl group having at least one fluorine atom, phenyl tetrayl group having at least one trifluoromethyl group, biphenyl tetrayl group, biphenyl tetrayl group having at least one fluorine atom, biphenyl tetrayl group having at least one trifluoromethyl group, terphenyl tetrayl group, terphenyl tetrayl group having at least one fluorine atom, terphenyl tetrayl group, naphthyl tetrayl group, fluorene tetrayl group, spirobiflurene tetrayl group, benzofluorene tetrayl group, phenanthrene tetrayl group, fluoranthene tetrayl group, triphenylene tetrayl group, anthracene tetrayl group, pyrene tetrayl group, pyridine tetrayl group, pyrimidine tetrayl group, carbazole tetrayl group having at least one trifluoromethyl group, benzofuran tetrayl group, benzothiophene tetrayl group, dibenzofuran tetrayl group, adamantane tetrayl group, methane tetrayl group, silane tetrayl group, or cyclohexane tetrayl group.

Due to being able to suppress the formation of a metal film on the surface of the film, the metal patterning material preferably has one or more groups selected from the group consisting of a perfluorophenyl group, a perfluorotolyl group, a perfluorodimethylphenyl group, and a perfluorobiphenylyl group in the molecule.

Due to being able to suppress the formation of a metal film on the film surface, the metal patterning material more preferably has a proportion of the number of fluorine atoms to carbon atoms in the molecular structure of the metal patterning material larger than 1:4, more preferably larger than 1:3, and still more preferably larger than 1:2. From the viewpoint of the glass transition temperature and the sublimation temperature, the proportion is more preferable in the order of from 1:2 to 2:1, from 1:2 to 1.9:1, from 1:2 to 1.8:1, from 1:2 to 1.7:1, from 1:2 to 1.6:1, and from 1:2 to 1.5:1.

### [Amine Compounds]

An amine compound according to one embodiment of the present disclosure is represented by Formula (7) or (8):

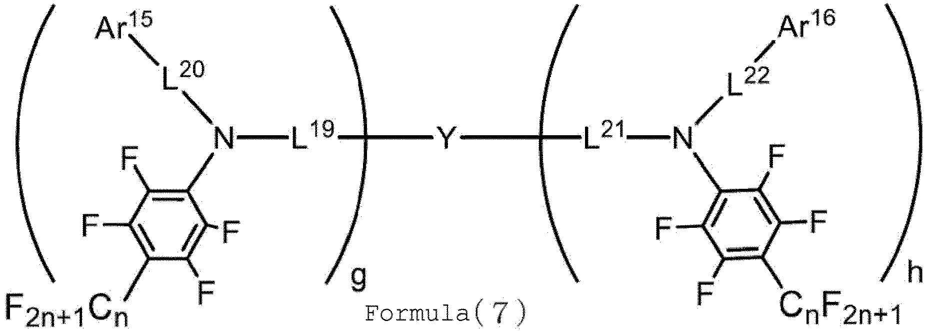

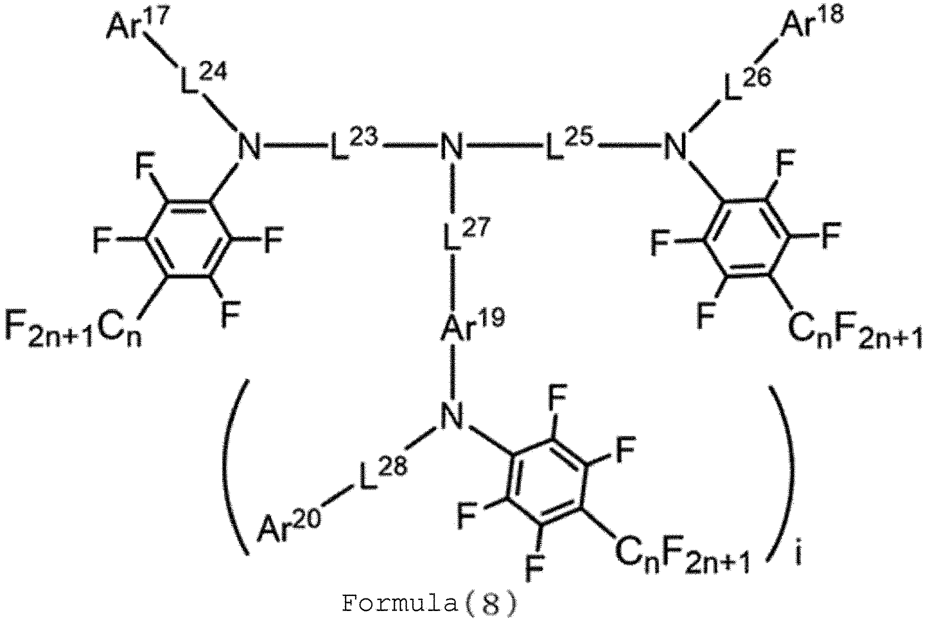

In the Formula,
Ar¹⁵ to Ar²⁰ each independently represent
an optionally substituted monocyclic, linked or condensed aromatic hydrocarbon group having 6 to 25 carbon atoms, or an optionally substituted monocyclic, linked, or condensed heteroaromatic group of 3 to 25 carbon atoms;
L¹⁹ to L²⁸ each independently represent
an optionally substituted monocyclic, linked, or condensed monocyclic divalent aromatic hydrocarbon group having 6 to 25 carbon atoms,
a monocyclic, linked or condensed divalent heteroaromatic group having an optionally substituted ring having 3 to 25 carbon atoms, or
a single bond;
Y represents
an optionally substituted monocyclic, linked or condensed di-to tetravalent aromatic hydrocarbon group having 6 to 25 carbon atoms,
an optionally substituted monocyclic, linked or condensed di-to tetravalent heteroaromatic group having 3 to 25 carbon atoms,
an optionally substituted linear, branched or cyclic di- to tetravalent alkyl groups of 1 to 10 carbon atoms, or
an optionally substituted di- to tetravalent silicon atom;
n represents an integer of 1 to 18;
g and h each independently represent an integer of 1 or 2; and i represents an integer of 0 or 1.

When Ar¹⁵ to Ar²⁰ are substituted aromatic hydrocarbon groups or substituted heteroaromatic groups, each of these groups preferably is independently substituted with one or more groups selected from the group consisting of:
a linear, branched, or cyclic alkyl group having 1 to 18 carbon atoms which may be substituted with a fluorine atom,
a linear, branched, or cyclic alkoxy group having 1 to 18 carbon atoms which may be substituted with a fluorine atom,
an aromatic hydrocarbon group having 6 to 20 carbon atoms which may be substituted with a fluorine atom,
a heteroaromatic group having 3 to 20 carbon atoms which may be substituted with a fluorine atom,
a cyano group,
a fluorine atom, and
a deuterium atom;
when L¹⁹ to L²⁸ are substituted divalent aromatic hydrocarbon groups or substituted divalent heteroaromatic groups, each of these groups preferably is independently substituted with one or more groups selected from the group consisting of:
   a linear, branched, or cyclic alkyl group having 1 to 18 carbon atoms which may be substituted with a fluorine atom,
   a linear, branched, or cyclic alkoxy group having 1 to 18 carbon atoms which may be substituted with a fluorine atom, an aromatic hydrocarbon group having 6 to 20 carbon atoms which may be substituted with a fluorine atom,
   a heteroaromatic group having 3 to 20 carbon atoms which may be substituted with a fluorine atom,
   a cyano group,
   a fluorine atom, and
   a deuterium atoms;
   when Y is a substituted di- to tetravalent aromatic hydrocarbon group, a substituted di- to tetravalent heteroaromatic group, a substituted di- to tetravalent alkyl group, or a substituted di- to tetravalent silicon atom, each of these groups preferably is independently substituted with one or more groups selected from the group consisting of:
      a linear, branched, or cyclic alkyl group having 1 to 18 carbon atoms which may be substituted with a fluorine atom,
      a linear, branched, or cyclic alkoxy group having 1 to 18 carbon atoms which may be substituted with a fluorine atom,
      an aromatic hydrocarbon group having 6 to 20 carbon atoms which may be substituted with a fluorine atom,
      a heteroaromatic group having 3 to 20 carbon atoms which may be substituted with a fluorine atom,
      a cyano group,
      a fluorine atom, and
      a deuterium atom.

In Formulas (7) and (8), a monocyclic, linked, or condensed aromatic hydrocarbon group having 6 to 25 carbon atoms; a monocyclic, linked, or condensed heteroaromatic group having 3 to 25 carbon atoms; a monocyclic, linked, or condensed di- to tetravalent aromatic hydrocarbon group having 6 to 25 carbon atoms; a monocyclic, linked, or condensed di-to tetravalent heteroaromatic group having 3 to 25 carbon atoms; a linear, branched or cyclic 2 to tetravalent alkyl groups having 1 to 10 carbon atoms; a linear, branched, or cyclic alkyl group having 1 to 18 carbon atoms which may be substituted with a fluorine atom; a linear, branched, or cyclic alkoxy group having 1 to 18 carbon atoms which may be substituted with a fluorine atom; an aromatic hydrocarbon group having 6 to 20 carbon atoms which may be substituted with a fluorine atom; a heteroaromatic group having 3 to 20 carbon atoms which may be substituted with a fluorine atom; an aromatic hydrocarbon group having 6 to 25 carbon atoms; a monocyclic, linked, or condensed heteroaromatic group having 3 to 25 carbon atoms; a monocyclic, linked, or condensed di- to tetravalent aromatic hydrocarbon group having 6 to 25 carbon atoms; a monocyclic, linked, or condensed di- to tetravalent heteroaromatic group having 3 to 25 carbon atoms; a linear, branched or cyclic di- to tetravalent alkyl groups having 1 to 10 carbon atoms; a linear, branched, or cyclic alkyl group having 1 to 18 carbon atoms which may be substituted with a fluorine atom; a linear, branched, or cyclic alkoxy group having 1 to 18 carbon atoms which may be substituted with a fluorine atom; an aromatic hydrocarbon group having 6 to 20 carbon atoms which may be substituted with a fluorine atom; and a heteroaromatic group having 3 to 20 carbon atoms which may be substituted with a fluorine atom are synonymous. From the viewpoint of suppressing the formation of the metal film on the surface of the film, the number of carbon atoms of the condensed aromatic hydrocarbon group in Formulas (7) and (8) is preferably 6 or more and 15 or less. Specifically, it is preferably from 6 to 15, more preferably from 6 to 14, and still more preferably from 6 to 13. In Formula (7) and Formula (8), the number of nitrogen atoms contained in the heteroaromatic group (monocyclic, linked, or condensed heteroaromatic group having 3 to 25 carbon atoms; monocyclic, linked, or condensed di- to tetravalent heteroaromatic group having 3 to 25 carbon atoms; heteroaromatic group having 3 to 20 carbon atoms optionally substituted with a fluorine atom) is preferably three or less.

In Formulas (7) and (8), due to having a high glass transition temperature and being able to suppress the formation of a metal film on the surface of the film, Ar¹⁵ to Ar²⁰ preferably each independently represent
(xv) a phenyl group, a biphenylyl group, a terphenylyl group, a naphthyl group, a fluorenyl group, a spirobifluorenyl group, a benzofluorenyl group, a phenanthryl group, a fluoranthenyl group, a triphenylenyl group, an anthryl group, a pyrenyl group, a pyridyl group, a carbazolyl group, a benzofuranyl group, a benzothienyl group, a dibenzofuranyl group, or a dibenzothienyl group; or
(xvi) a group in which the group represented by (xv) is substituted with one or more groups selected from the group consisting of: a methyl group, an ethyl group, a methoxy group, an ethoxy group, a trifluoromethyl group, a trifluoromethoxy group, a perfluoroalkyl group having 2 to 10 carbon atoms, a perfluoroalkoxy group having 2 to 10 carbon atoms, a cyano group, a deuterium atom, a fluorine atom, a phenyl group optionally substituted with a fluorine atom, a biphenylyl group optionally substituted with a fluorine atom, a naphthyl group optionally substituted with a fluorine atom, a group a phenanthryl group which may be substituted with a fluorine atom, a pyridyl group which may be substituted with a fluorine atom, a carbazolyl group which may be substituted with a fluorine atom, a dibenzothienyl group which may be substituted with a fluorine atom, and a dibenzofuranyl group which may be substituted with a fluorine atom.

Due to having high glass transition temperature and being able to suppress the formation of a metal film on the surface of the film, Ar¹⁵ to Ar²⁰ more preferably each independently represent:
(xv') a phenyl group, biphenylyl group, terphenylyl group, naphthyl group, fluorenyl group, spirobifluorenyl group, phenanthryl group, fluoranthenyl group, triphenylenyl group, anthryl group, pyrenyl group, pyridyl group, carbazolyl group, dibenzofuranyl group, or dibenzothienyl group; or
(xvi ') a group in which the group represented by (xv') is substituted with one or more groups selected from the group consisting of: a methyl group, a trifluoromethyl group, a perfluoroalkyl group having 2 to 10 carbon atoms, a fluorine atom, and a phenyl group optionally substituted with a fluorine atom.

Due to having a high glass transition temperature and being able to suppress formation of a metal film on a film surface, Ar¹⁵ to Ar²⁰ more preferably each independently represent a phenyl group, a phenyl group having at least one fluorine atom, a phenyl group having at least one trifluoromethyl group, a biphenylyl group, a biphenylyl group having at least one fluorine atom, a biphenylyl group having at least one trifluoromethyl group, a terphenylyl group, a terfenylyl group having at least one fluorine atom, a terphenylyl group having at least one trifluoromethyl group, a naphthyl group, a 9,9-dimethylfluorenyl group, a 9,9-diphenylfluorenyl group, a spirobiflurenyl group, a phenanthryl group, a carbazol-9-yl group, a 9-phenylcarbazolyl group, a dibenzofuranyl group, or a dibenzothienyl group.

In Formulas (7) and (8), due to having a high glass transition temperature and being able to suppress the formation of a metal film on the film surface, L¹⁹ to L²⁸ preferably each independently represent:
(xvii) a phenylene group, biphenylylene group, terphenylene group, naphthylene group, pyridylene group, or fluorenylene group;
(xviii) a group in which the group represented by (xvii) is substituted with one or more groups selected from the group consisting of: a methyl group, an ethyl group, a methoxy group, an ethoxy group, a trifluoromethyl group, a trifluoromethoxy group, a perfluoroalkyl group having 2 to 10 carbon atoms, a perfluoroalkoxy group having 2 to 10 carbon atoms, a cyano group, a deuterium atom, a fluorine atom, a phenyl group optionally substituted with a fluorine atom, a biphenylyl group optionally substituted with a fluorine atom, a naphthyl group optionally substituted with a fluorine atom, a phenanthryl group optionally substituted with a fluorine atom, a pyridyl group optionally substituted with a fluorine atom, a carbazolyl group optionally substituted with a fluorine atom, a dibenzothienyl group optionally substituted with a fluorine atom, and a dibenzofuranyl group optionally substituted with a fluorine atom; or
(xix) a single bond.

Due to having a high glass transition temperature and being able to suppress the formation of a metal film on the film surface, L¹⁹ to L²⁸ more preferably each independently represent:
(xvii') a phenylene group, a biphenylylene group, a terphenylylene group, a naphthylene group, a pyridylene group or a fluorenylene group;
(xviii') a group in which the group represented by (xvii ') is substituted with one or more groups selected from the group consisting of a methyl group, a trifluoromethyl group, a fluorine atom and a phenyl group optionally substituted with a fluorine atom; or
(xix') a single bond.

Due to having a high glass transition temperature and being able to suppress the formation of a metal film on the film surface, L¹⁹ to L²⁸ more preferably each independently represent a phenylene group, a phenylene group having at least one fluorine atom, a phenylene group having at least one trifluoromethyl group, a biphenylylene group, a biphenylylene group having at least one fluorine atom, a biphenylylene group having at least one trifluoromethyl group, a terphenylylene group, a terphenylylene group having at least one fluorine atom, a terphenylylene group having at least one trifluoromethyl group, a naphthylene group, or a single bond.

In Formula (7), due to having a high glass transition temperature and being able to suppress the formation of a metal film on the film surface, Y preferably represents:
(xx) a phenylene group, biphenylene group, terphenylylene group, naphthylene group, fluorenylene group, spirobifluorenylene group, benzofluorenylene group, phenanthrene group, fluoranthenylene group, triphenylenylene group, anthracenediyl group, pyridylene group, pyrimidinediyl group, triazinediyl group, carbazoldiyl group, benzoflanyl group, benzothiophenediyl group, dibenzofuryl group, adamantanediyl group, methylene group, silanediyl group, cyclohexylene group, phenyltriyl group, biphenyltriyl group, terphenyltriyl group, naphthylyl group, fluorenetriyl group, spirobiflurenetriyl group, benzofluorenetriyl groups, phenanthrenetriyl group, fluoranthenetriyl group, triphenylenetriyl group, anthracenetriyl group, pyrenetriyl group, pyridinetriyl group, pyrimidinetriyl group, triazinetriyl group, carbazole triyl group, benzofuran triyl group, benzothiophene triyl group, dibenzofuran triyl group, adamantane triyl group, methane triyl group, silane triyl group, cyclohexane triyl group, phenyl tetrayl group, biphenyl tetrayl group, terphenyl tetrayl group, naphthyl tetrayl group, fluorene tetrayl group, spirobifluorene tetrayl group, benzofluorene tetrayl group, phenanthrene tetrayl group, fluoranthene tetrayl group, triphenylene tetrayl group, anthracene tetrayl group, pyrene tetrayl group, pyridine tetrayl group, pyrimidine tetrayl group, carbazole tetrayl group, benzofuran tetrayl group, benzothiophene tetrayl group, dibenzofuran tetrayl group, adamantane tetrayl group, methane tetrayl group, silane tetrayl group, or cyclohexane tetrayl group; or
(xxi) a group in which the group represented by (xx) is substituted with one or more groups selected from the group consisting of: a methyl group, an ethyl group, a methoxy group, an ethoxy group, a trifluoromethyl group, a trifluoromethoxy group, a perfluoroalkyl group having 2 to 10 carbon atoms, a perfluoroalkoxy group having 2 to 10 carbon atoms, a cyano group, a deuterium atom, a fluorine atom, a phenyl group optionally substituted with a fluorine atom, a biphenylyl group optionally substituted with a fluorine atom, a naphthyl group optionally substituted with a fluorine atom, a phenanthryl group which may be substituted with a fluorine atom, a pyridyl group which may be substituted with a fluorine atom, a carbazolyl group which may be substituted with a fluorine atom, a dibenzothienyl group which may be substituted with a fluorine atom, and a dibenzofuranyl group which may be substituted with a fluorine atom.

Due to having a high glass transition temperature and being able to suppress the formation of a metal film on the film surface, Y more preferably represents:
(xx') a phenylene group, a biphenylene group, a terphenylylene group, a naphthylene group, a fluorenylene group, a spirobifluorenylene group, a benzofluorenylene group, a phenanthrene group, a fluoranthenylene group, a triphenylenylene group, an anthracenediyl group, a pyridylene group, a pyrimidinediyl group, a triazinediyl group, a carbazolediyl group, a benzofuryl group, a benzothiophenediyl group, a dibenzofuryl group, an adamantanediyl group, a methylene group, a silanediyl group, a cyclohexylene group, a phenyltriyl group, a biphenyltriyl group, a terphenyltriyl group, a naphthylyl group, a fluorenetriyl group, a spirobifluorenetriyl group, a benzofluorenetriyl group, a phenanthrenetriyl group, a fluoranthenetriyl group, a triphenylenetriyl group, an anthracenetriyl group, a pyrenetriyl group, a pyridinetriyl group, a pyrimidinetriyl group, a triazinetriyl group, a carbazole triyl group, a benzofuran triyl group, a benzothiophene triyl group, a dibenzofuran triyl group, an adamantane triyl group, a methane triyl group, a silane triyl group, a cyclohexane triyl group, a phenyl tetrayl group, a biphenyl tetrayl group, a terphenyl tetrayl group, a naphthyl tetrayl group, a fluorene tetrayl group, a spirobifluorene tetrayl group, a benzofluorene tetrayl group, a phenanthrene tetrayl group, a fluoranthene tetrayl group, a triphenylene tetrayl group, an anthracene tetrayl group, a pyrene tetrayl group, a pyridine tetrayl group, a pyrimidine tetrayl group, a carbazole tetrayl group, a benzofuran tetrayl group, a benzothiophene tetrayl group, a dibenzofuran tetrayl group, an adamantane tetrayl group, a methane tetrayl group, a silane tetrayl group, or a cyclohexane tetrayl group; or
(xxi') a group in which the group represented by (xx') is substituted with one or more groups selected from the group consisting of a methyl group, a trifluoromethyl group, a perfluoroalkyl group having 2 to 10 carbon atoms, a fluorine atom, and a phenyl group optionally substituted with a fluorine atom.

Due to having a high glass transition temperature and being able to suppress the formation of a metal film on the film surface, Y still more preferably represents: a phenylene group, a phenylene group having at least one fluorine atom, a phenylene group having at least one trifluoromethyl group, a biphenylene group, a biphenylene group having at least one fluorine atom, a biphenylene group having at least one trifluoromethyl group, a terphenylylene group, a terphenylylene group having at least one fluorine atom, a terphenylylene group having at least one fluorine atom, a terphenylylene group, a naphthylene group, a fluorenylene group, a spirobifluorenylene group, a benzofluorenylene group, a phenanthrene group, a fluoranthenylene group, a triphenylenylene group, an anthracenediyl group, a pyridylene group, a pyrimidinediyl group, a triazinediyl group, a carbazolediyl group, a benzofuryl group, a benzothiophenediyl group, a dibenzofuryl group, an adamantanediyl group, a methylene group, a silanediyl group, a cyclohexylene group;
a phenyltriyl group, a phenyltriyl group having at least one fluorine atom, a phenyltriyl group having at least one trifluoromethyl group, a biphenyltriyl group, a biphenyltriyl group having at least one fluorine atom, a biphenyltriyl group having at least one trifluoromethyl group, a terphenyltriyl group, a terphenyltriyl group having at least one fluorine atom, a terphenyltriyl group having at least one trifluoromethyl group, a naphthylyl group, a fluorenetriyl group, a spirobifluorenetriyl group, a benzofluorenetriyl group, a phenanthrenetriyl group, a fluoranthenetriyl group, a triphenylenetriyl group, an anthracenetriyl group, a pyrenetriyl group, a pyridinetriyl group, a pyrimidinetriyl group, a triazinetriyl group, a carbazoletriyl group, a benzofurantriyl group, a benzothiophenetriyl group, a dibenzofurantriyl group, an adamantanetriyl group, a methanetriyl group, a silanetriyl group, a cyclohexanetriyl group; or
a phenyl tetrayl group, a phenyl tetrayl group having at least one fluorine atom, a phenyl tetrayl group having at least one trifluoromethyl group, a biphenyl tetrayl group, a biphenyl tetrayl group having at least one fluorine atom, a biphenyl tetrayl group having at least one trifluoromethyl group, a terphenyl tetrayl group, a terphenyl tetrayl group having at least one fluorine atom, a terphenyl tetrayl group, a naphthyl tetrayl group, a fluorene tetrayl group, a spirobiflurene tetrayl group, a benzofluorene tetrayl group, a phenanthrene tetrayl group, a fluoranthene tetrayl group, a triphenylene tetrayl group, an anthracene tetrayl group, a pyrene tetrayl group, a pyridine tetrayl group, a pyrimidine tetrayl group, a carbazole tetrayl group having at least one trifluoromethyl group, a benzofuran tetrayl group, a benzothiophene tetrayl group, a dibenzofuran tetrayl group, an adamantane tetrayl group, a methane tetrayl group, a silane tetrayl group, or a cyclohexane tetrayl group.

In Formulas (7) and (8), n represents an integer of 1 to 18. Due to having a high glass temperature and ease of raw material availability, n is preferably an integer of 1 to 8, more preferably an integer of 1 to 4, and still more preferably 1.

In addition, the amine compound according to one embodiment of the present disclosure is represented by Formula (9) or (10):

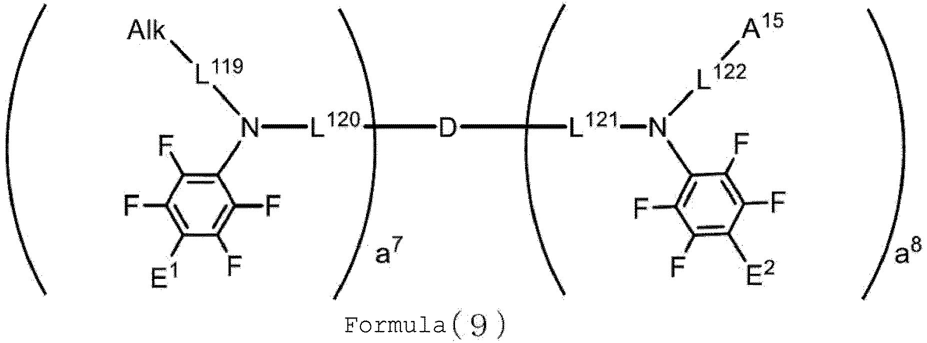

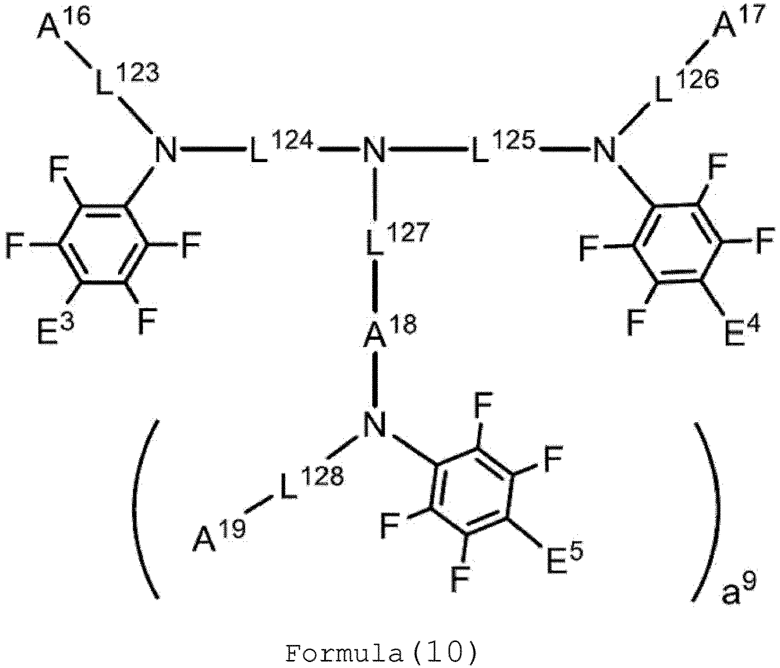

In the formula,
a⁷ represents an integer of 1 or 2;
a⁸ represents an integer of 0 to 2;
a⁹ represents an integer of 0 or 1; and
Alk represents an optionally substituted cyclic alkyl group having 3 to 25 carbon atoms. A¹⁵ to A¹⁹ each independently represent
an optionally substituted monocyclic, linked or condensed aromatic hydrocarbon group having 6 to 25 carbon atoms,
an optionally substituted monocyclic, linked, or condensed heterocyclic heteroaromatic group having 3 to 25 carbon atoms, or
an optionally substituted cyclic alkyl group having 3 to 25 carbon atoms;
provided that
when a⁹ is 0, at least one of A¹⁶ to A¹⁸ is independently an optionally substituted cyclic alkyl group having 3 to 25 carbon atoms;
when a⁹ is 1, at least one of A¹⁶ to A¹⁹ is independently an optionally substituted cyclic alkyl group having 3 to 25 carbon atoms;
L¹¹⁹ to L¹²⁸ each independently represent
an optionally substituted monocyclic, linked, or condensed monocyclic divalent aromatic hydrocarbon group having 6 to 25 carbon atoms,
an optionally substituted monocyclic, linked or condensed divalent heteroaromatic group having 3 to 25 carbon atoms, or a single bond;
D represents
an optionally substituted monocyclic, linked or condensed monocyclic 1 to tetravalent aromatic hydrocarbon group having 6 to 25 carbon atoms,
an optionally substituted monocyclic, linked or condensed monocyclic mono- to tetravalent heteroaromatic groups of 3 to 25 carbon atoms,
an optionally substituted linear, branched or cyclic mono- to tetravalent alkyl group having 1 to 10 carbon atoms, or
an optionally substituted 1 to tetravalent silicon atom; and each of E¹ to E⁵ independently represents
a fluorine atom or
a linear, branched or cyclic alkyl group having 1 to 18 carbon atoms which may be substituted with a fluorine atom.

When Alk is a substituted cyclic alkyl group, it is substituted by one or more groups selected from the group consisting of:
a linear, branched, or cyclic alkyl group having 1 to 18 carbon atoms which may be substituted with a fluorine atom;
a fluorine atom, and
a deuterium atom;
when A¹⁵ to A¹⁹ are substituted aromatic hydrocarbon groups, substituted heteroaromatic groups or substituted cyclic alkyl groups, each of these groups preferably is independently substituted with one or more groups selected from the group consisting of:
   a linear, branched, or cyclic alkyl group having 1 to 18 carbon atoms which may be substituted with a fluorine atom,
   a linear, branched, or cyclic alkoxy group having 1 to 18 carbon atoms which may be substituted with a fluorine atom,
   an aromatic hydrocarbon group having 6 to 20 carbon atoms which may be substituted with a fluorine atom,
   a heteroaromatic group having 3 to 20 carbon atoms which may be substituted with a fluorine atom,
   a cyano group,
   a fluorine atoms, and
   a deuterium atom;
   when L¹¹⁹ to L¹²⁸ are substituted divalent aromatic hydrocarbon groups or substituted divalent heteroaromatic groups, each of these groups preferably is independently substituted with one or more groups selected from the group consisting of:
      a linear, branched, or cyclic alkyl group having 1 to 18 carbon atoms which may be substituted with a fluorine atom,
      a linear, branched, or cyclic alkoxy group having 1 to 18 carbon atoms which may be substituted with a fluorine atom,
      an aromatic hydrocarbon group having 6 to 20 carbon atoms which may be substituted with a fluorine atom,
      a heteroaromatic group having 3 to 20 carbon atoms which may be substituted with a fluorine atom;
      a cyano group,
      a fluorine atoms, and
      a deuterium atom;
      when D is a substituted mono- to tetravalent aromatic hydrocarbon group, a substituted mono- to tetravalent heteroaromatic group, a substituted mono- to tetravalent alkyl group, or a substituted mono- to tetravalent silicon atom, each of these groups preferably is independently substituted by one or more groups selected from the group consisting of:
         a linear, branched, or cyclic alkyl group having 1 to 18 carbon atoms which may be substituted with a fluorine atom,
         a linear, branched, or cyclic alkoxy group having 1 to 18 carbon atoms which may be substituted with a fluorine atom,
         an aromatic hydrocarbon group having 6 to 20 carbon atoms which may be substituted with a fluorine atom,
         a heteroaromatic group having 3 to 20 carbon atoms which may be substituted with a fluorine atom,
         a cyano group,
         a fluorine atoms, and
         a deuterium atom.

As the linear, branched, or cyclic monovalent alkyl group having 1 to 10 carbon atoms include a cyclobutyl group, for example, a cyclopentyl group, a cyclohexyl group, a cycloheptyl group, a cyclooctyl group, an adamantyl group, a noradamantyl group, a norbornyl group, and a decahydronaphthalene group can be exemplified.

It should be noted that, as described above, the condensed cyclic alkyl group having 3 to 25 carbon atoms may have a substituent. When they have a substituent, it is preferably substituted by one or more groups selected from the group consisting of: a linear, branched, or cyclic alkyl group having 1 to 18 carbon atoms which may be substituted with a fluorine atom, a linear, branched, or cyclic alkoxy group having 1 to 18 carbon atoms which may be substituted with a fluorine atom, an aromatic hydrocarbon group having 6 to 20 carbon atoms which may be substituted with a fluorine atom, a heteroaromatic group having 3 to 20 carbon atoms which may be substituted with a fluorine atom, a cyano group, a fluorine atom, or a deuterium atom. In this case, the number of substituents is not particularly limited.

In Formulas (9) and (10), a monocyclic, linked, or condensed aromatic hydrocarbon group having 6 to 25 carbon atoms; a monocyclic, linked, or condensed heteroaromatic group having 3 to 25 carbon atoms; a monocyclic, linked, or condensed di- to tetravalent aromatic hydrocarbon group having 6 to 25 carbon atoms; a monocyclic, linked, or condensed di-to tetravalent heteroaromatic group having 3 to 25 carbon atoms; a linear, branched or cyclic di- to tetravalent alkyl groups having 1 to 10 carbon atoms; a linear, branched, or cyclic alkyl group having 1 to 18 carbon atoms which may be substituted with a fluorine atom; a linear, branched, or cyclic alkoxy group having 1 to 18 carbon atoms which may be substituted with a fluorine atom; an aromatic hydrocarbon group having 6 to 20 carbon atoms which may be substituted with a fluorine atom; a heteroaromatic group having 3 to 20 carbon atoms which may be substituted with a fluorine atom; an aromatic hydrocarbon group having 6 to 25 carbon atoms; a monocyclic, linked, or condensed heteroaromatic group having 3 to 25 carbon atoms; a monocyclic, linked, or condensed di- to tetravalent aromatic hydrocarbon group having 6 to 25 carbon atoms; a monocyclic, linked, or condensed di- to tetravalent heteroaromatic group having 3 to 25 carbon atoms; a linear, branched or cyclic di- to tetravalent alkyl groups having 1 to 10 carbon atoms; a linear, branched, or cyclic alkyl group having 1 to 18 carbon atoms which may be substituted with a fluorine atom; a linear, branched, or cyclic alkoxy group having 1 to 18 carbon atoms which may be substituted with a fluorine atom; an aromatic hydrocarbon group having 6 to 20 carbon atoms which may be substituted with a fluorine atom; a heteroaromatic group having 3 to 20 carbon atoms which may be substituted with a fluorine atom are synonymous. From the viewpoint of being able to suppress the formation of the metal film on the surface of the film, the number of carbon atoms of the condensed aromatic hydrocarbon group in Formulas (9) and (10) is preferably from 6 to 15. Specifically, it is preferably from 6 to 15, more preferably from 6 to 14, and still more preferably from 6 to 13.

In Formulas (9) and (10), a monovalent aromatic hydrocarbon group having 6 to 25 carbon atoms; a monocyclic, linked, or condensed monovalent heteroaromatic group having 3 to 25 carbon atoms; an aromatic hydrocarbon group having 6 to 25 carbon atoms; and a monocyclic, linked, or condensed heteroaromatic group having 3 to 25 carbon atoms shown in the above Formulas (1) to (3) are synonymous.

In Formulas (9) and (10), the condensed cyclic alkyl group having 3 to 25 carbon atoms is synonymous with the condensed cyclic alkyl group having 3 to 25 carbon atoms represented by the above Formulas (4) to (6).

In Formulas (9) and (10), due to having a high glass transition temperature and being able to suppress the formation of a metal film on the film surface, Alk preferably represents
(xxii) a cyclopentyl group, cyclohexyl group, diamantyl group, adamantyl group, noradamantyl group, norbornyl group, or decahydronaphthalene group; or
(xxiii) a group in which the group represented by (xxii) is substituted with one or more groups selected from the group consisting of a methyl group, a trifluoromethyl group, a perfluoroalkyl group having 2 to 10 carbon atoms, a fluorine atom, and a phenyl group optionally substituted with a fluorine atom. Due to having a high glass transition temperature and being able to suppress the formation of a metal film on the film surface, Alk more preferably represents (xxii') a cyclohexyl group, an adamantyl group, a norbornyl group, or a decahydronaphthalene group; or
(xxii') a group in which the group represented by (xxii') is substituted with one or more groups selected from the group consisting of a methyl group, a trifluoromethyl group, a perfluoroalkyl group having 2 to 10 carbon atoms, a fluorine atom, and a phenyl group optionally substituted with a fluorine atom.

Due to having a high glass transition temperature and being able to suppress the formation of a metal film on the film surface, Alk even more preferably represents a cyclohexyl group, a cyclohexyl group having at least one fluorine atom, a cyclohexyl group having at least one trifluoromethyl group, a cyclohexyl group having at least one trifluoromethyl group, an adamantyl group, an adamantyl group having at least one fluorine atom, and an adamantyl group having at least one trifluoromethyl group.

In Formulas (9) and (10), due to having high glass transition temperatures and being able to suppress formation of a metal film on the surface of the film, A¹⁵ to A¹⁹ preferably each independently represent:
(xxiv) a phenyl group, biphenylyl group, terphenylyl group, naphthyl group, fluorenyl group, spirobifluorenyl group, benzofluorenyl group, phenanthryl group, fluoranthyl group, fluoranthenyl group, triphenylenyl group, anthryl group, pyrenyl group, pyridyl group, carbazolyl group, benzofuranyl group, benzothienyl group, dibenzofuranyl group, dibenzofuranyl group, dibenzothienyl group, cyclopentyl group, cyclohexyl group, diamantyl group, adamantyl group, noradamantyl group, a norbornyl group or a decahydronaphthalene group; or
(xxv) a group in which the group represented by (xxiv) is substituted with one or more groups selected from the group consisting of a methyl group, an ethyl group, a methoxy group, an ethoxy group, a trifluoromethyl group, a trifluoromethoxy group, a perfluoroalkyl group having 2 to 10 carbon atoms, a perfluoroalkoxy group having 2 to 10 carbon atoms, a cyano group, a deuterium atom, a fluorine atom, a phenyl group optionally substituted with a fluorine atom, a biphenylyl group optionally substituted with a fluorine atom, a naphthyl group optionally substituted with a fluorine atom, a phenanthryl group which may be substituted with a fluorine atom, a pyridyl group which may be substituted with a fluorine atom, a carbazolyl group which may be substituted with a fluorine atom, a dibenzothienyl group which may be substituted with a fluorine atom, and a dibenzofuranyl group which may be substituted with a fluorine atom.

Due to having a high glass transition temperature and being able to suppress the formation of a metal film on the surface of the film, A¹⁵ to A¹⁹ more preferably each independently represent:
(xxiv') a phenyl group, biphenylyl group, terphenylyl group, naphthyl group, fluorenyl group, spirobifluorenyl group, phenanthryl group, fluoranthenyl group, triphenylenyl group, anthryl group, pyrenyl group, pyridyl group, carbazolyl group, dibenzofuranyl group, dibenzothienyl group, cyclopentyl group, cyclohexyl group, diamantyl group, adamantyl group, noradamantyl group, norbornyl group, or decahydronaphthalene group; or
(xxiv') a group in which the group represented by (xxv') is substituted with one or more groups selected from the group consisting of a methyl group, a trifluoromethyl group, a perfluoroalkyl group having 2 to 10 carbon atoms, a fluorine atom, and a phenyl group optionally substituted with a fluorine atom.

Due to having a high glass transition temperature and being able to suppress the formation of a metal film on the surface of the film, A¹⁵ to A¹⁹ even more preferably each independently represent:
a phenyl group, a phenyl group having at least one fluorine atom, a phenyl group having at least one trifluoromethyl group, a biphenylyl group, a biphenylyl group having at least one fluorine atom, a biphenylyl group having at least one trifluoromethyl group, a terfenylyl group, a terfenylyl group having at least one fluorine atom, a terfenyl group having at least one trifluoromethyl group, a naphthyl group, 9,9-dimethylfluorenyl group, a 9,9-diphenylfluorenyl group, a spirobiflurenyl group, phenanthryl group, a carbazol-9-yl group, a 9-phenylcarbazolyl group, a dibenzofuranyl group, a dibenzothienyl group, a cyclohexyl group, a cyclohexyl group having at least one fluorine atom, a cyclohexyl group having at least one trifluoromethyl group, an adamantyl group, an adamantyl group having at least one fluorine atom, or an adamantyl group having at least one trifluoromethyl group.

In Formulas (9) and (10), due to having a high glass transition temperature and being able to suppress the formation of a metal film on the surface of the film, L¹¹⁹ to L¹²⁸ preferably each independently represent:
(xxvi) a phenylene group, biphenylylene group, terphenylene group, naphthylene group, pyridylene group, or fluorenylene group;
(xxvii) a group in which the group represented by (xxvi) is substituted with one or more groups selected from the group consisting of a methyl group, an ethyl group, a methoxy group, an ethoxy group, a trifluoromethyl group, a trifluoromethoxy group, a perfluoroalkyl group having 2 to 10 carbon atoms, a perfluoroalkoxy group having 2 to 10 carbon atoms, a cyano group, a deuterium atom, a fluorine atom, a phenyl group optionally substituted with a fluorine atom, a biphenylyl group optionally substituted with a fluorine atom, a naphthyl group optionally substituted with a fluorine atom, a phenanthryl group optionally substituted with a fluorine atom, a pyridyl group optionally substituted with a fluorine atom, a carbazolyl group optionally substituted with a fluorine atom, a dibenzothienyl group optionally substituted with a fluorine atom, and a dibenzofuranyl group optionally substituted with a fluorine atom; or
(xxviii) a single bond.

Due to having a high glass transition temperature and being able to suppress the formation of a metal film on the surface of the film, L¹¹⁹ to L¹²⁸ more preferably each independently represent:
(xxvi') a phenylene group, a biphenylylene group, a terphenylylene group, a naphthylene group, a pyridylene group, or a fluorenylene group;
(xxvii') a group in which the group represented by (xxvi') is substituted with one or more groups selected from the group consisting of a methyl group, a trifluoromethyl group, a fluorine atom, and a phenyl group optionally substituted with a fluorine atom; or
(xxviii') a single bond.

Due to having a high glass transition temperature and being able to suppress the formation of a metal film on the surface of the film, L¹¹⁹ to L¹²⁸ even more preferably each independently represent: a phenylene group, a phenylene group having at least one fluorine atom, a phenylene group having at least one trifluoromethyl group, a biphenylylene group, a biphenylylene group having at least one fluorine atom, a biphenylylene group having at least one trifluoromethyl group, a terphenylylene group, a terphenylylene group having at least one fluorine atom, a terphenylylene group having at least one trifluoromethyl group, a naphthylene group, or a single bond.

In Formula (9), due to having a high glass transition temperature and being able to suppress the formation of a metal film on the surface of the film, D preferably represents:
(xxix) a phenyl group, a biphenyl group, a terphenylyl group, a naphthyl group, a fluorenyl group, a spirobifluorenyl group, a benzofluorenyl group, a phenanthryl group, a fluoranthenyl group, a triphenylyl group, an anthryl group, a pyridyl group, a pyrimidyl group, a triazine group, a carbazolyl group, a benzofuranyl group, a benzothionyl group, a dibenzofuranyl group, an adamantyl group, a methyl group, a silyl group, a cyclohexyl group, a phenylene group, a biphenylene group, a biphenylene group, a terphenylylene group, a naphthylene group, a fluorenylene group, a spirobifluorenylene group, a benzofluorenylene group, a phenanthrene group, a fluoranternylene group, a triphenylenylene group, an anthracenediyl group, a pyridylene group, a pyrimidinediyl group, a triazinediyl group, a carbazoldiyl group, a benzofuranyl group, a benzothiophenediyl group, a dibenzofuranyl group, an adamantanediyl group, a methylene group, a silanediyl group, a cyclohexylene group, a phenyltriyl group, a biphenyltriyl group, a terphenyltriyl group, a naphthylyl group, a fluorenetriyl group, a spirobifluorenetriyl group, a benzofluorenetriyl group, a phenanthrenetriyl group, a fluoranthenetriyl group, a triphenylenetriyl group, an anthracenetriyl group, a pyrenetriyl group, a pyridinetriyl group, a pyrimidinetriyl group, a triazinetriyl group, a carbazoletriyl group, a benzofuran triyl group, a benzothiophene triyl group, a dibenzofuran triyl group, an adamantane triyl group, a methane triyl group, a silane triyl group, a cyclohexane triyl group, a phenyl tetrayl group, a biphenyl tetrayl group, a terphenyl tetrayl group, a naphthyl tetrayl group, a fluorene tetrayl group, a spirobifluorene tetrayl group, a benzofluorene tetrayl group, a phenanthrene tetrayl group, a fluoranthene tetrayl group, a triphenylene tetrayl group, an anthracene tetrayl group, a pyrene tetrayl group, a pyridine tetrayl group, a pyrimidine tetrayl group, a carbazole tetrayl group, a benzofuran tetrayl group, a benzothiophene tetrayl group, a dibenzofuran tetrayl group, an adamantane tetrayl group, a methane tetrayl group, a silane tetrayl group, or a cyclohexane tetrayl group; or
(xxx) a group in which the group represented by (xxix) is substituted with one or more groups selected from the group consisting of: a methyl group, an ethyl group, a methoxy group, an ethoxy group, a trifluoromethyl group, a trifluoromethoxy group, a perfluoroalkyl group having 2 to 10 carbon atoms, a perfluoroalkoxy group having 2 to 10 carbon atoms, a cyano group, a deuterium atom, a fluorine atom, a phenyl group optionally substituted with a fluorine atom, a biphenylyl group optionally substituted with a fluorine atom, a naphthyl group optionally substituted with a fluorine atom, a phenanthryl group which may be substituted with a fluorine atom, a pyridyl group which may be substituted with a fluorine atom, a carbazolyl group which may be substituted with a fluorine atom, a dibenzothienyl group which may be substituted with a fluorine atom, and a dibenzofuranyl group which may be substituted with a fluorine atom.

Due to having a high glass transition temperature and being able to suppress the formation of a metal film on the surface of the film, D more preferably represents:
(xxix') a phenyl group, a biphenyl group, a terphenylyl group, a naphthyl group, a fluorenyl group, a spirobifluorenyl group, a benzofluorenyl group, a phenanthryl group, a fluoranthenyl group, a triphenylyl group, an anthryl group, a pyridyl group, a pyrimidyl group, a triazine group, a carbazolyl group, a benzofuranyl group, a benzothionyl group, a dibenzofuranyl group, a adamantyl group, a methyl group, a silyl group, a cyclohexyl group, a phenylene group, a biphenylene group, a terphenylylene group, a naphthylene group, a fluorenylene group, a spirobifluorenylene group, a benzofluorenylene group, a phenanthrene group, a fluoranternylene group, a triphenylenylene group, an anthracenediyl group, a pyridylene group, a pyrimidinediyl group, a triazinediyl group, a carbazoldiyl group, a benzofuranyl group, a benzothiophenediyl group, a dibenzofuranyl group, an adamantanediyl group, a methylene group, a silanediyl group, a cyclohexylene group, a phenyltriyl group, a biphenyltriyl group, a terphenyltriyl group, a naphthylyl group, a fluorenetriyl group, a spirobifluorenetriyl group, a benzofluorenetriyl group, a phenanthrenetriyl group, a fluoranthenetriyl group, a triphenylenetriyl group, an anthracenetriyl group, a pyrenetriyl group, a pyridinetriyl group, a pyrimidinetriyl group, a triazinetriyl group, a carbazoletriyl group, a benzofuran triyl group, a benzothiophene triyl group, a dibenzofuran triyl group, an adamantane triyl group, a methane triyl group, a silane triyl group, a cyclohexane triyl group, a phenyl tetrayl group, a biphenyl tetrayl group, a terphenyl tetrayl group, a naphthyl tetrayl group, a fluorene tetrayl group, a spirobifluorene tetrayl group, a benzofluorene tetrayl group, a phenanthrene tetrayl group, a fluoranthene tetrayl group, a triphenylene tetrayl group, an anthracene tetrayl group, a pyrene tetrayl group, a pyridine tetrayl group, a pyrimidine tetrayl group, a carbazole tetrayl group, a benzofuran tetrayl group, a benzothiophene tetrayl group, a dibenzofuran tetrayl group, an adamantane tetrayl group, a methane tetrayl group, a silane tetrayl group, or a cyclohexane tetrayl group; or
(xxx') a group in which the group represented by (xxix') is substituted with one or more groups selected from the group consisting of a methyl group, a trifluoromethyl group, a perfluoroalkyl group having 2 to 10 carbon atoms, a fluorine atom, and a phenyl group optionally substituted with a fluorine atom.

Due to having a high glass transition temperature and being able to suppress the formation of a metal film on the surface of the film, D even more preferably represents:
a phenyl group, a phenyl group having at least one fluorine atom, a phenyl group having at least one trifluoromethyl group, a biphenyl group, a biphenyl group having at least one fluorine atom, a biphenyl group having at least one trifluoromethyl group, a terphenylyl group, a terphenylyl group having at least one fluorine atom, a terphenylyl group having at least one trifluoromethyl group, a naphthyl group, a fluorenyl group, a spirobifluorenyl group, a benzofluorenyl group, a phenanthryl group, a fluoranthenyl group, a triphenylyl group, an anthryl group, a pyridyl group, a pyrimidyl group, a triazine group, a carbazolyl group, a benzofuranyl group, a benzothionyl group, a dibenzofuranyl group, an adamantyl group, a methyl group, a silyl group, or a cyclohexyl group;
a phenylene group, a phenylene group having at least one fluorine atom, a phenylene group having at least one trifluoromethyl group, a biphenylene group, a biphenylene group having at least one fluorine atom, a biphenylene group having at least one trifluoromethyl group, a terphenylylene group, a terphenylylene group having at least one fluorine atom, a terphenylylene group having at least one trifluoromethyl group, a naphthylene group, a fluorenylene group, a spirobiflurenylene group, a benzofluorenylene group, a phenanthrene group, a fluoranthenylene group, a triphenylenylene group, an anthracenediyl group, a pyridylene group, a pyrimidinediyl group, a triazinediyl group, a carbazoldiyl group, a benzofunyl group, a benzothiophenediyl group, a dibenzofuryl group, an adamantanediyl group, a methylene group, a silanediyl group, or a cyclohexylene group; a phenyltriyl group, a phenyltriyl group having at least one fluorine atom, a phenyltriyl group having at least one trifluoromethyl group, a biphenyltriyl group, a biphenyltriyl group having at least one fluorine atom, a biphenyltriyl group having at least one trifluoromethyl group, a terphenyltriyl group, a terphenyltriyl group having at least one fluorine atom, a terphenyltriyl group having at least one trifluoromethyl group, a naphthylyl group, a fluorenetriyl group, a spirobifluorenetriyl group, a benzofluorenetriyl group, a phenanthrenetriyl group, a fluoranthenetriyl group, a triphenylenetriyl group, an anthracenetriyl group, a pyrenetriyl group, a pyridinetriyl group, a pyrimidinetriyl group, a triazinetriyl group, a carbazoletriyl group, a benzofurantriyl group, a benzothiophenetriyl group, a dibenzofurantriyl group, a adamantanetriyl group, a methanetriyl group, a silanetriyl group, or a cyclohexanetriyl group; or
a phenyl tetrayl group, a phenyl tetrayl group having at least one fluorine atom, a phenyl tetrayl group having at least one trifluoromethyl group, a biphenyl tetrayl group, a biphenyl tetrayl group having at least one fluorine atom, a biphenyl tetrayl group having at least one trifluoromethyl group, a terphenyl tetrayl group, a terphenyl tetrayl group having at least one fluorine atom, a terphenyl tetrayl group, a naphthyl tetrayl group, a fluorene tetrayl group, a spirobiflurene tetrayl group, a benzofluorene tetrayl group, a phenanthrene tetrayl group, a fluoranthene tetrayl group, a triphenylene tetrayl group, an anthracene tetrayl group, a pyrene tetrayl group, a pyridine tetrayl group, a pyrimidine tetrayl group, a carbazole tetrayl group having at least one trifluoromethyl group, a benzofuran tetrayl group, a benzothiophene tetrayl group, a dibenzofuran tetrayl group, an adamantane tetrayl group, a methane tetrayl group, a silane tetrayl group, or a cyclohexane tetrayl group.

The amine compound according to one embodiment of the present disclosure described above can be used as a metal patterning material or a material for metal patterning.

### [Specific examples of metal patterning materials and amine compounds]

The metal patterning material according to one embodiment of the present disclosure and the amine compound according to one embodiment of the present disclosure are exemplified by compounds (A1) to (A769) below; however, the present disclosure is not to be limited to these compounds.

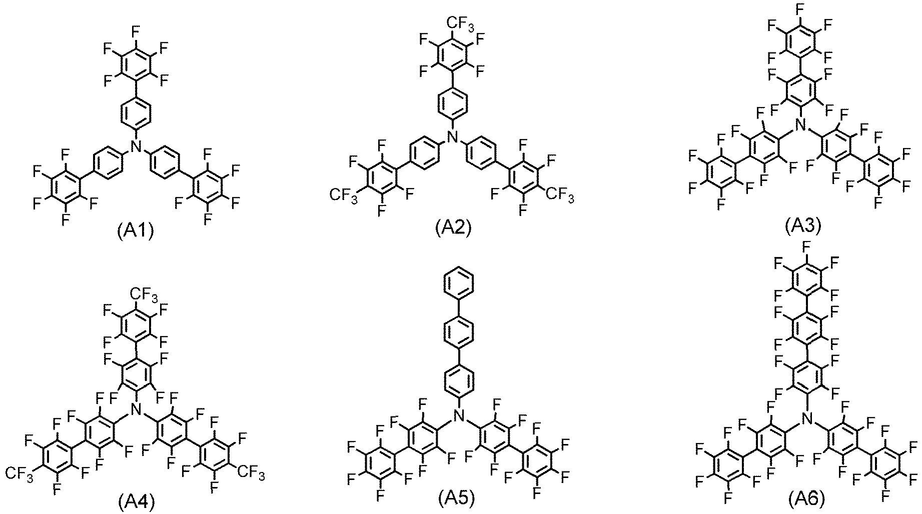

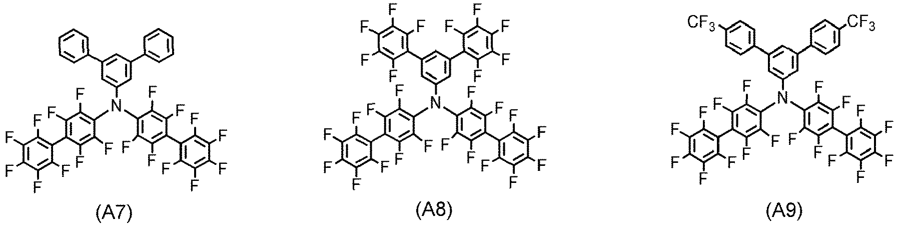

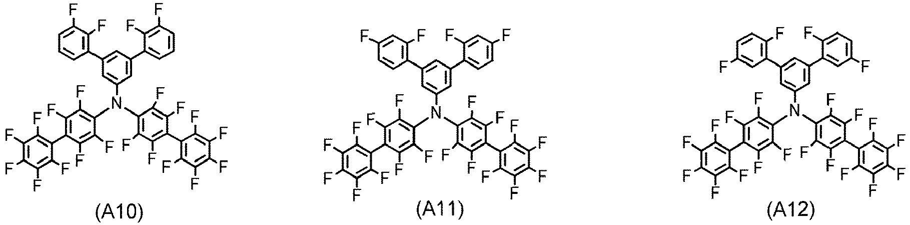

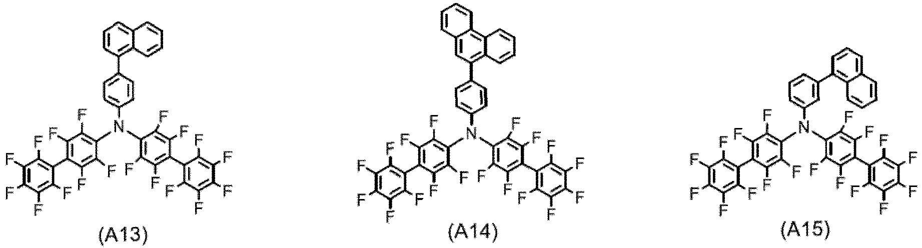

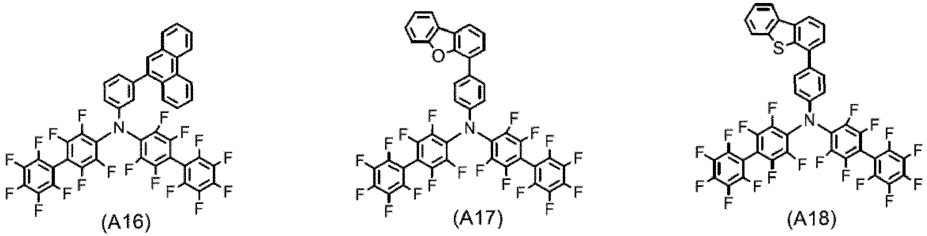

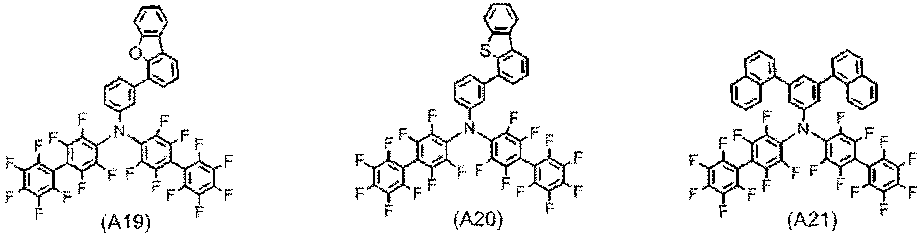

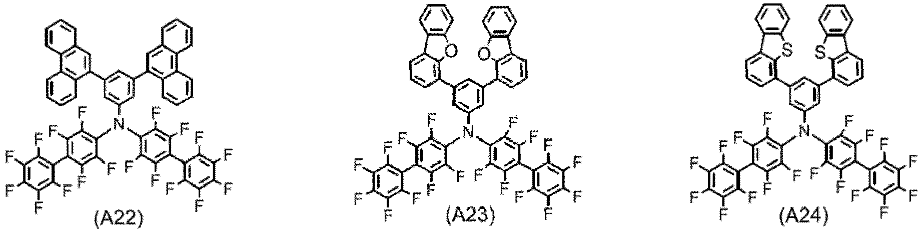

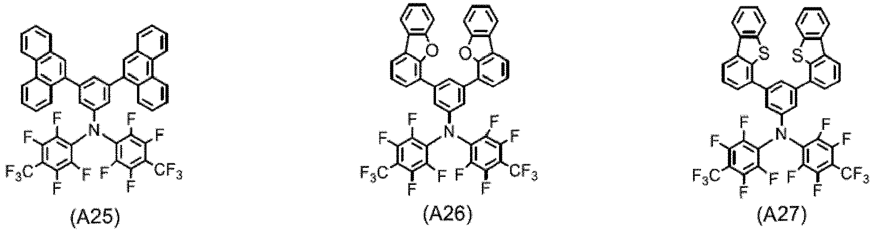

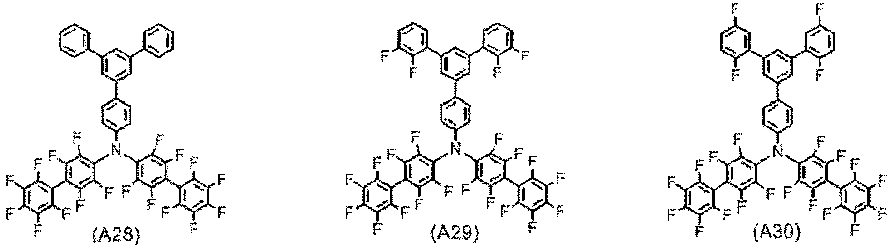

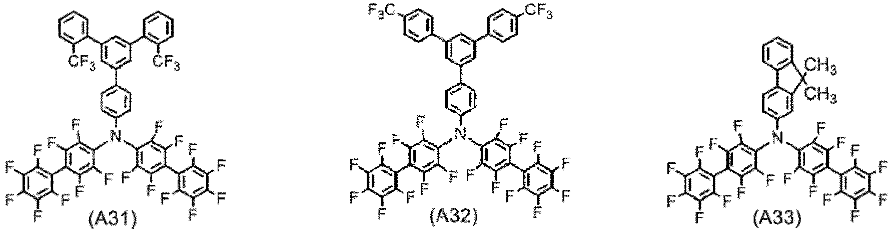

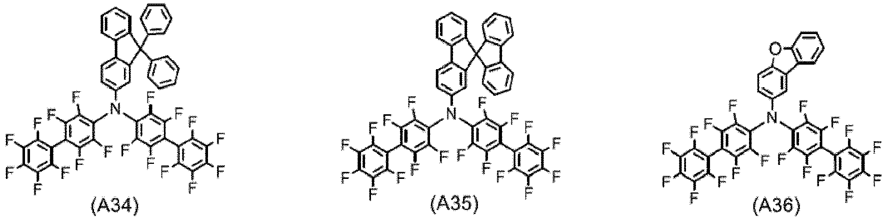

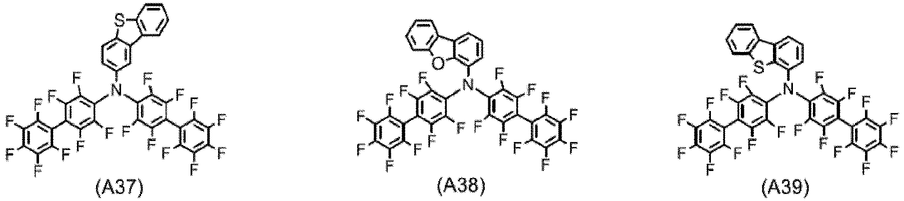

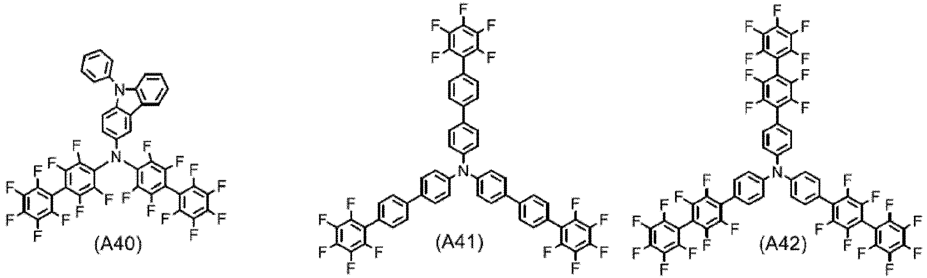

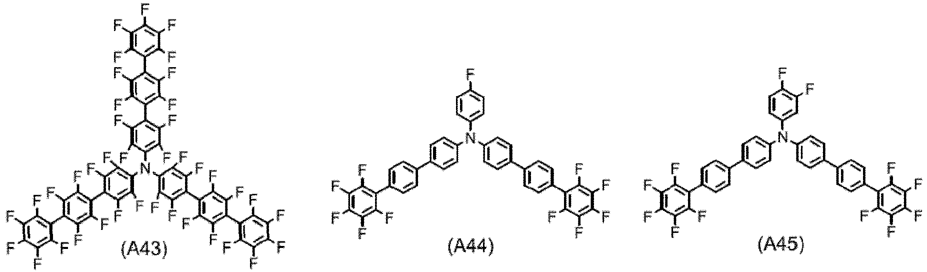

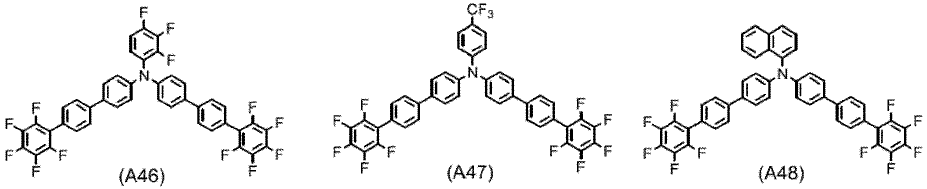

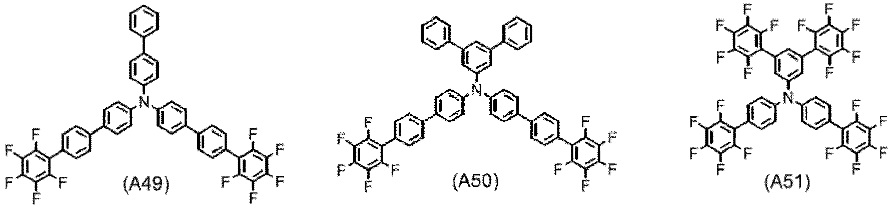

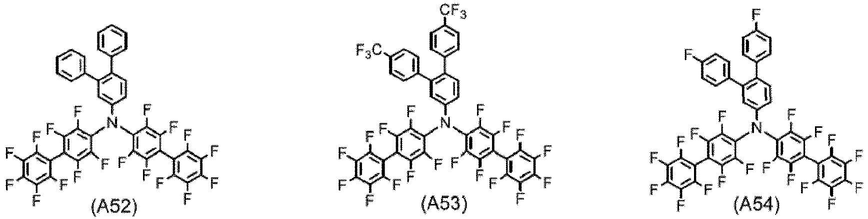

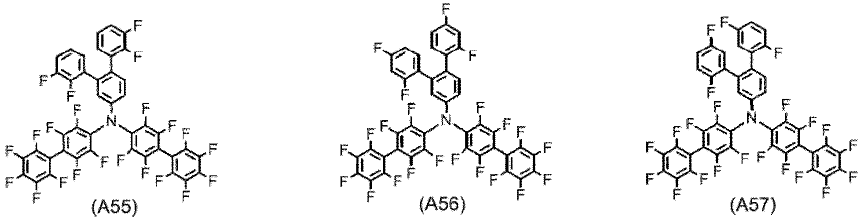

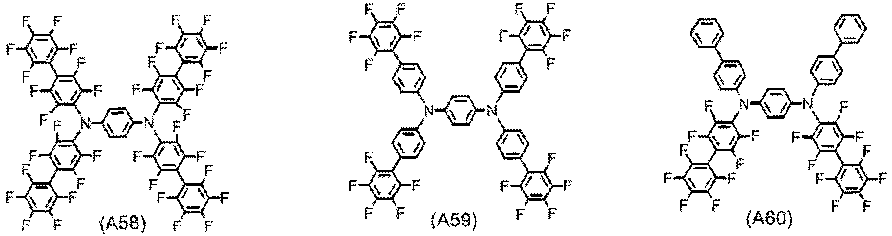

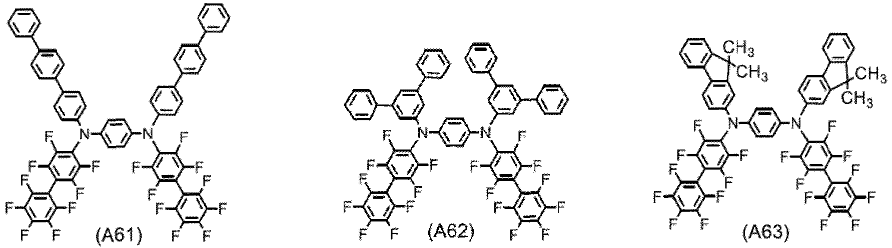

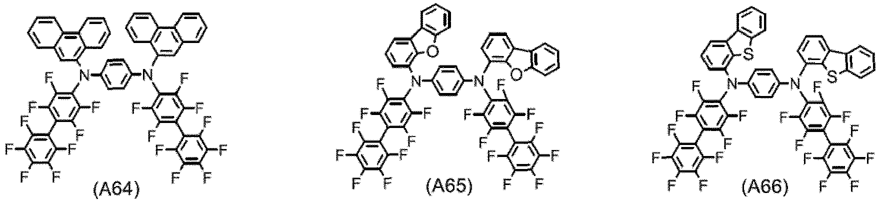

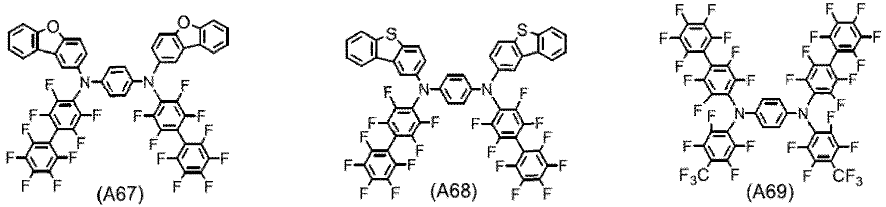

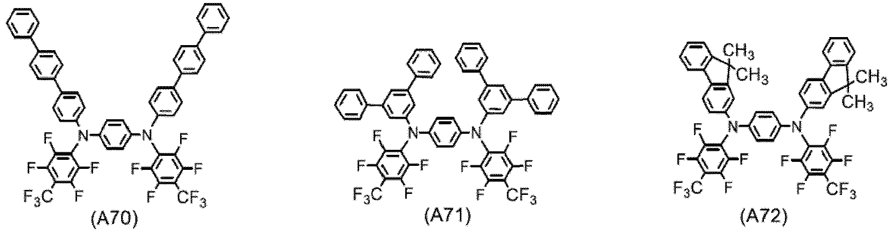

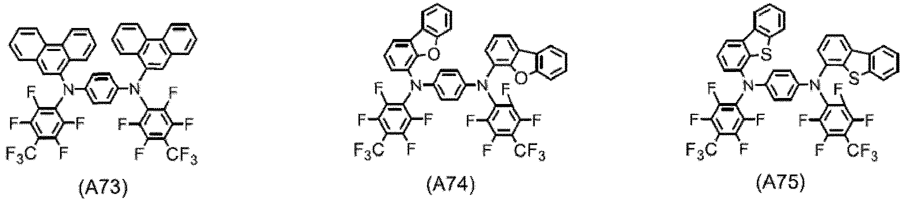

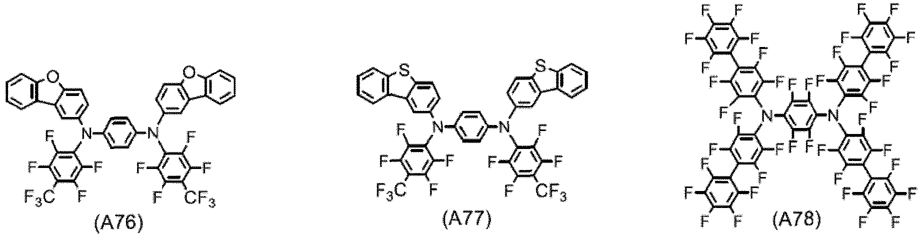

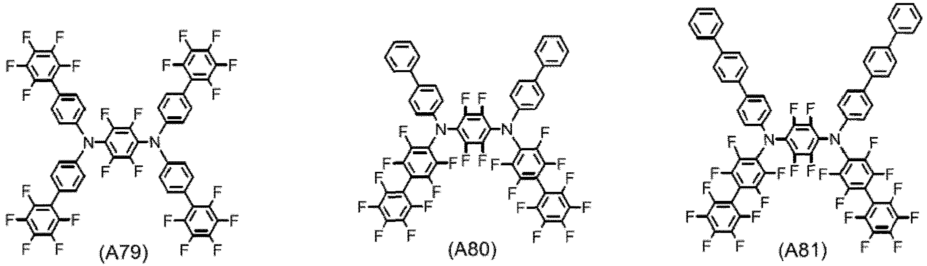

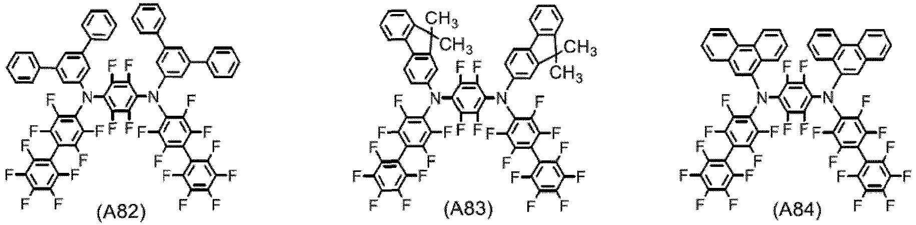

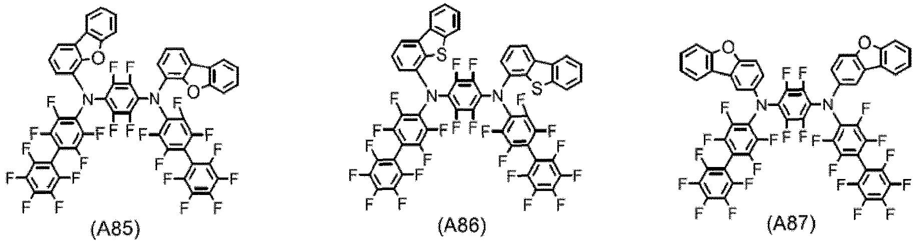

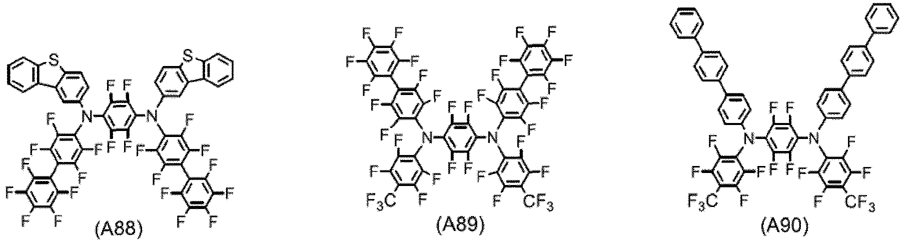

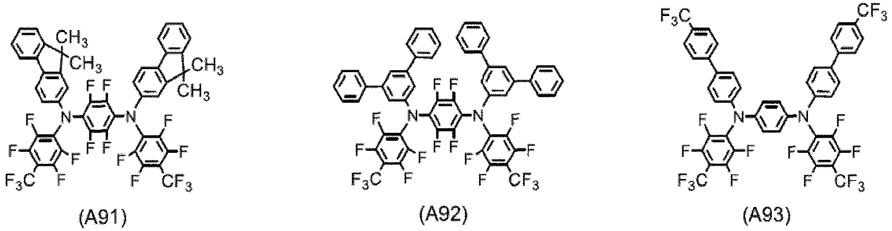

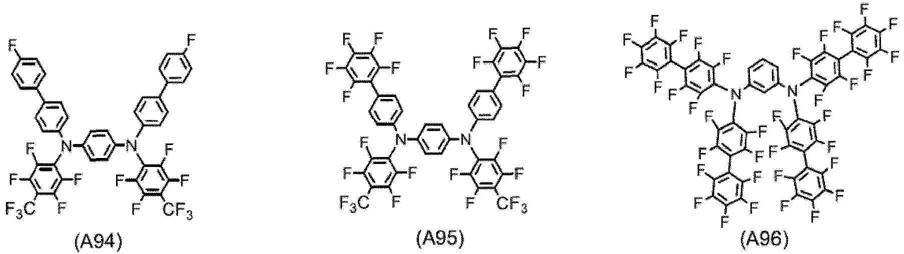

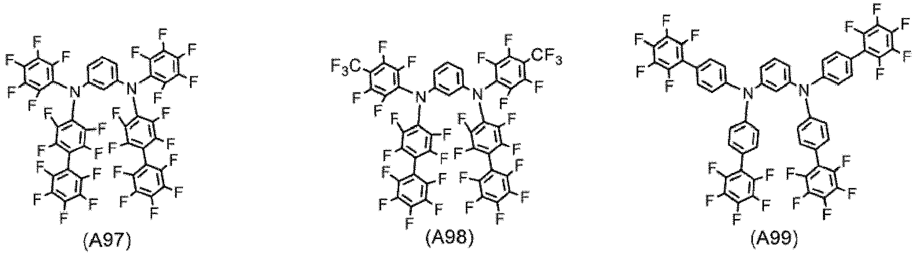

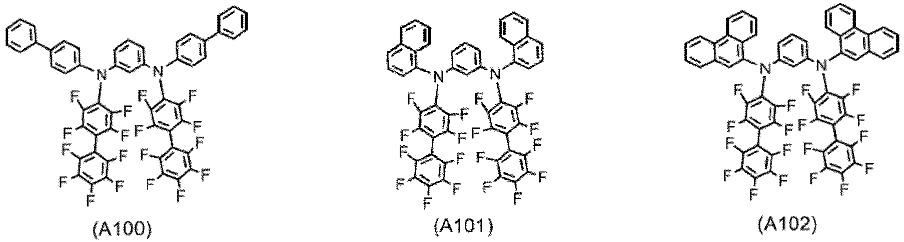

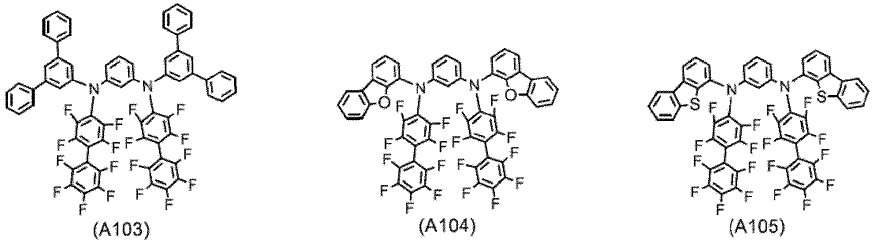

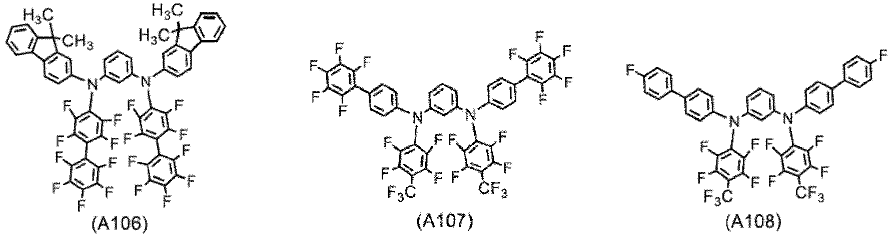

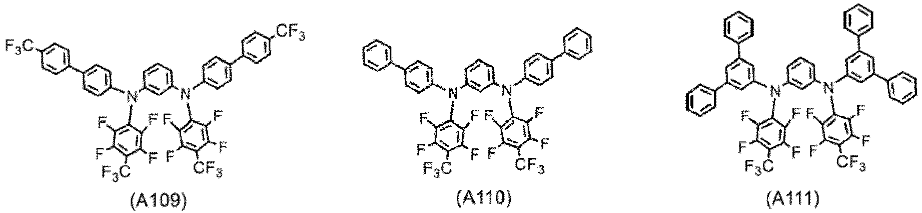

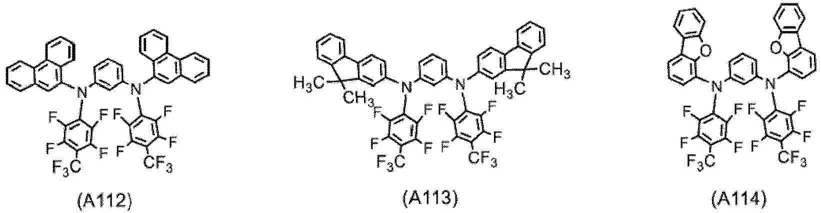

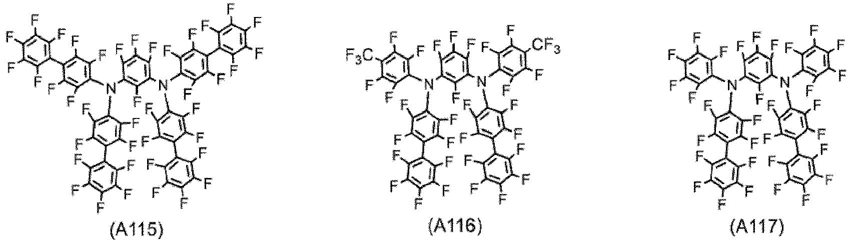

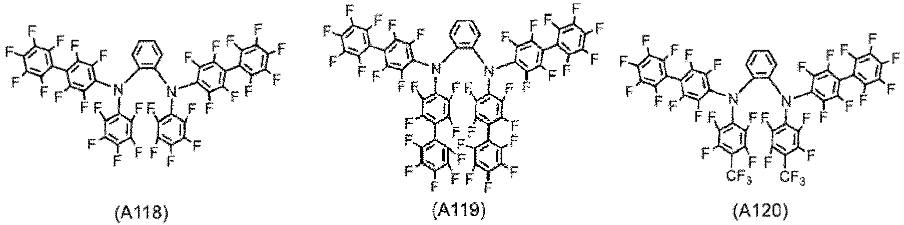

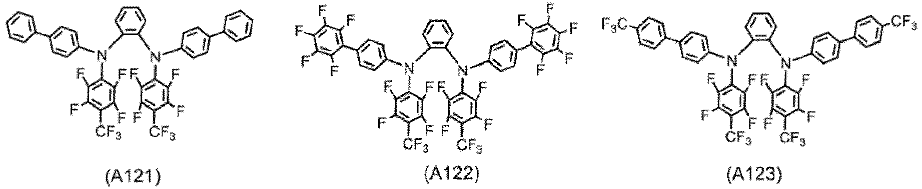

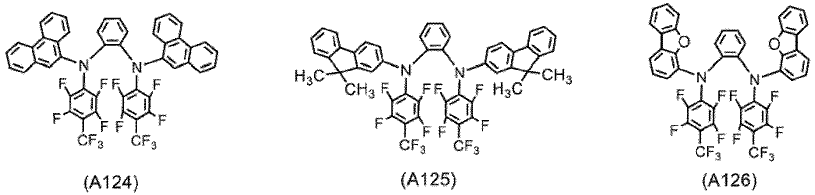

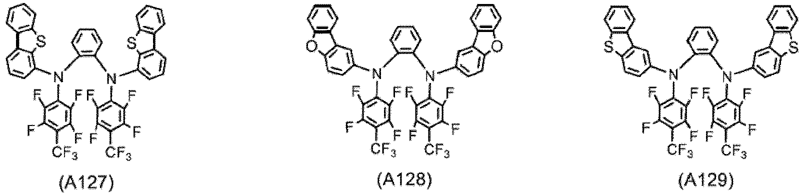

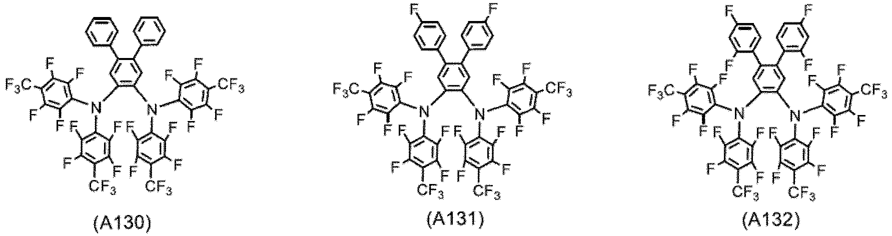

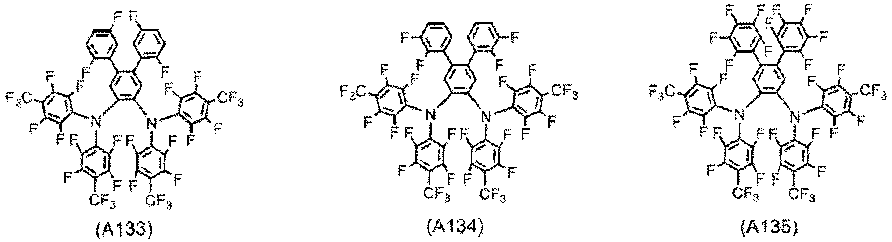

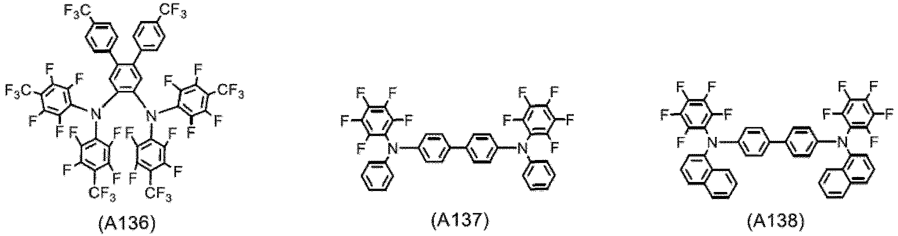

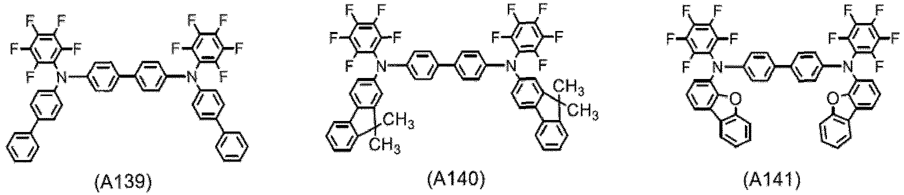

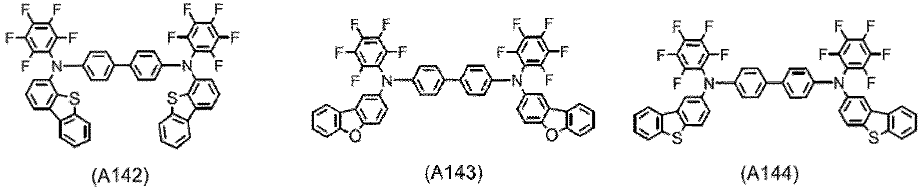

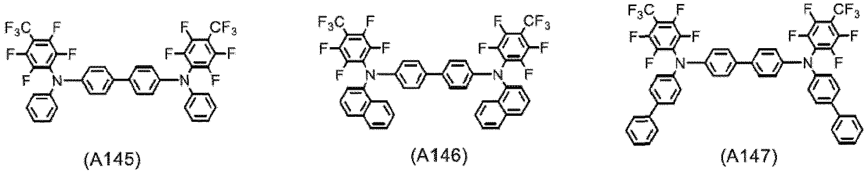

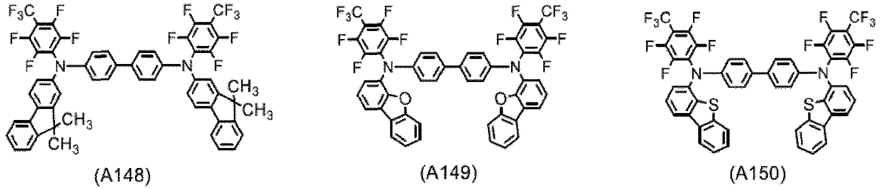

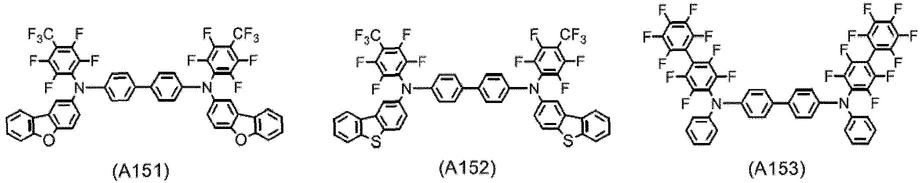

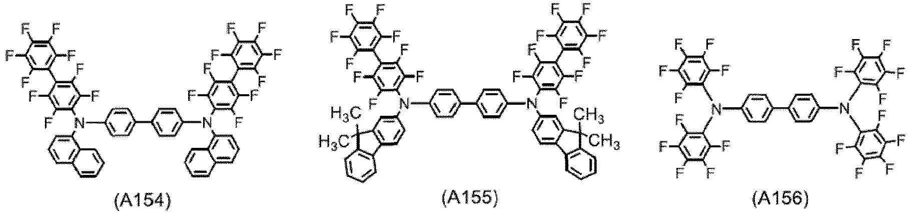

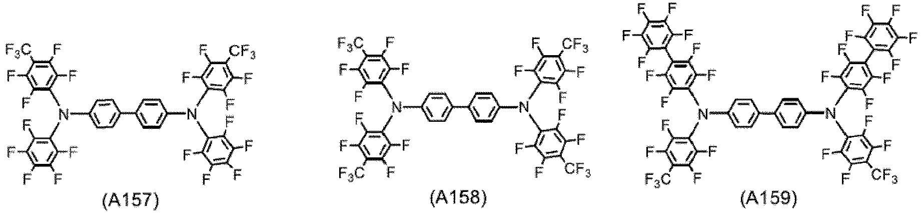

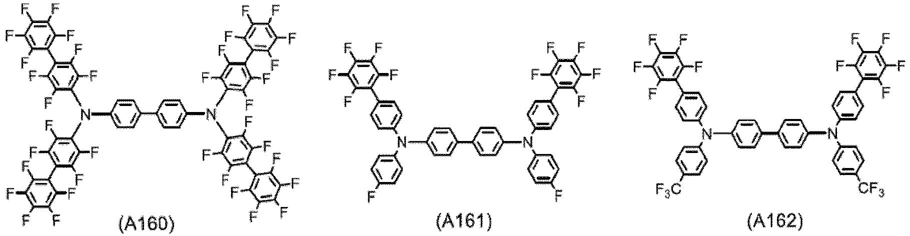

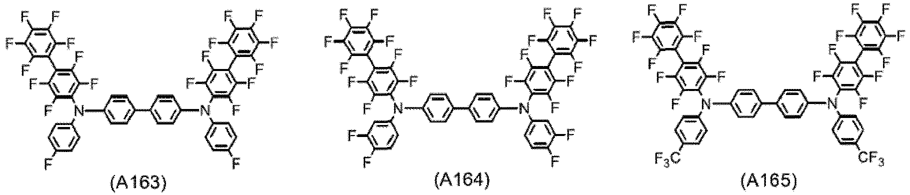

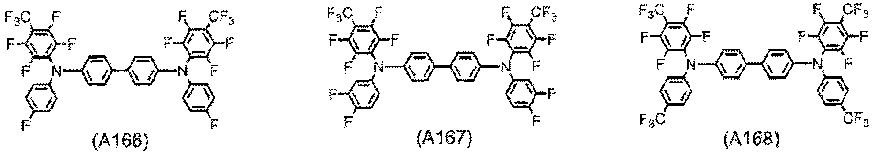

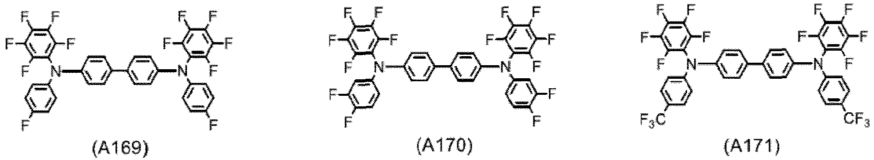

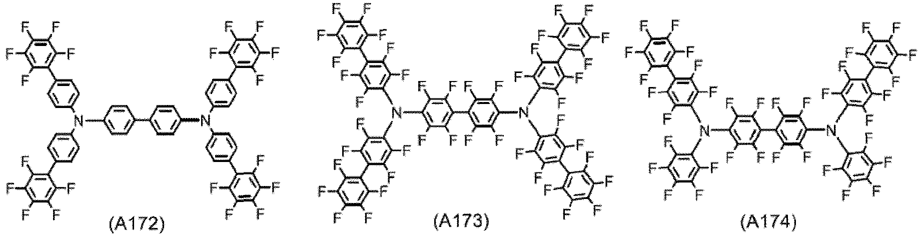

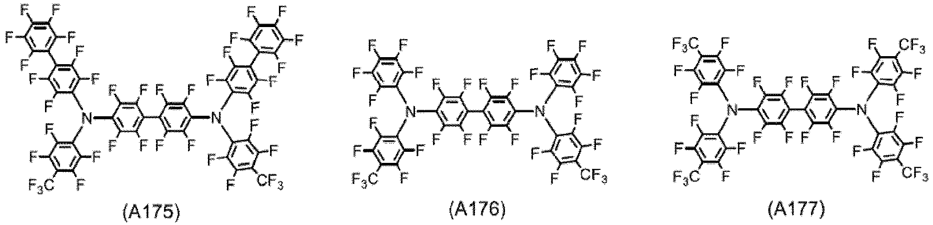

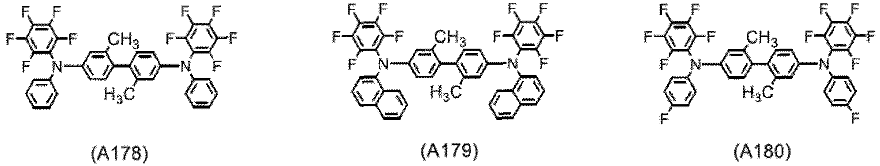

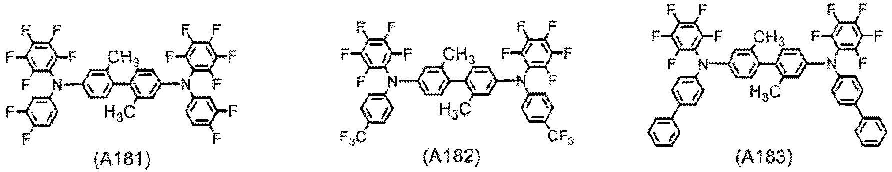

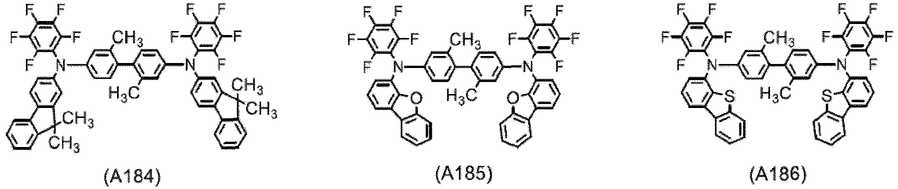

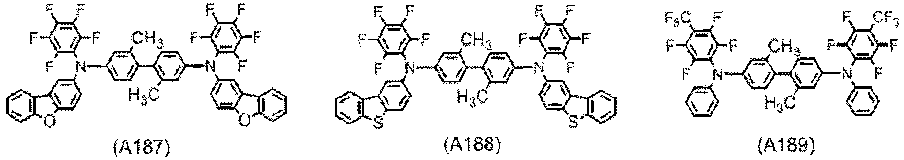

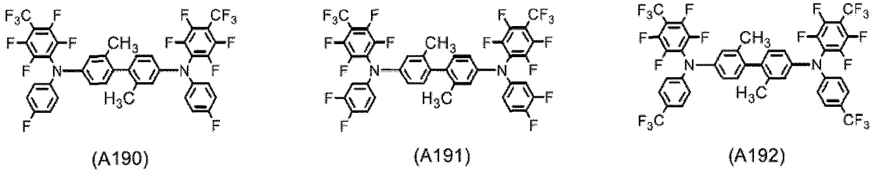

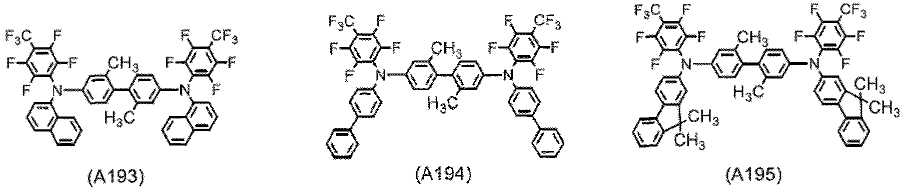

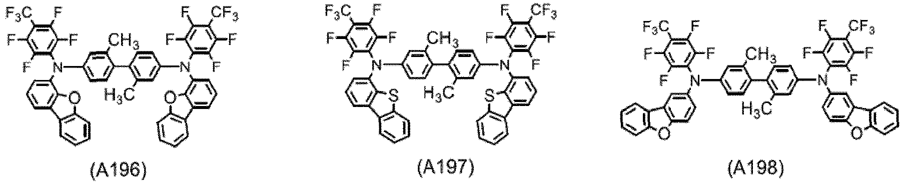

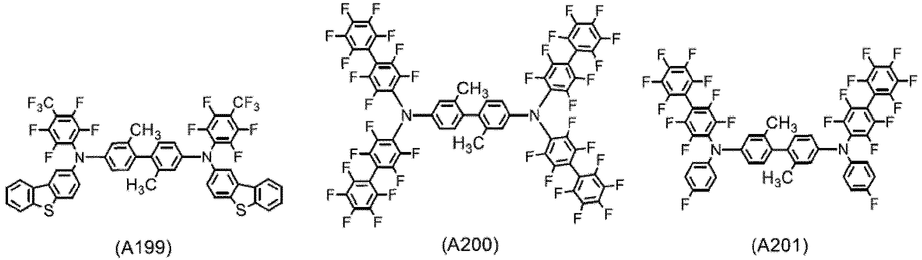

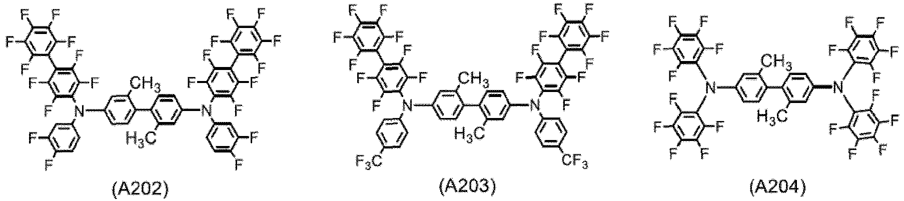

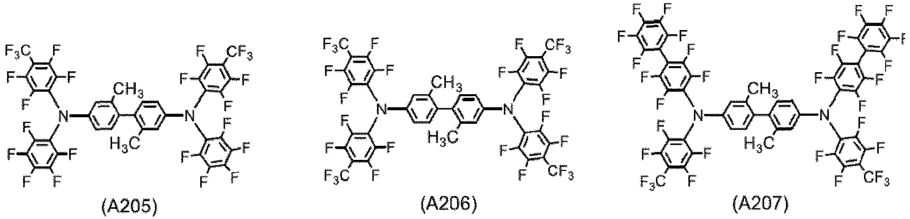

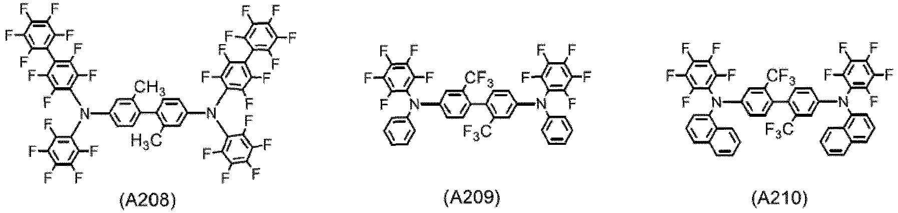

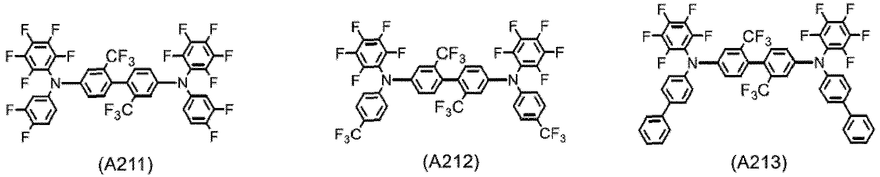

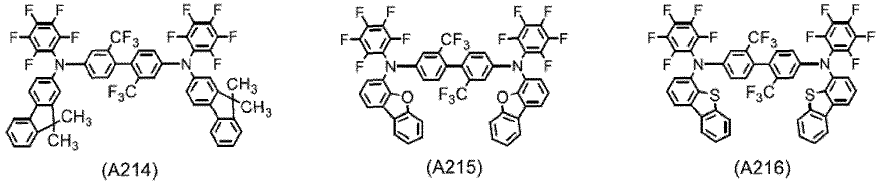

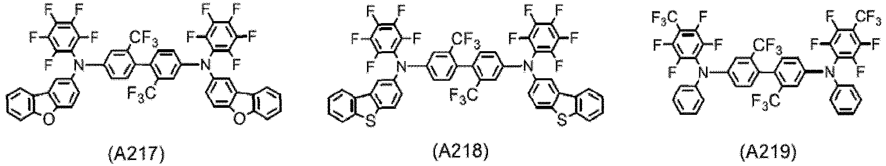

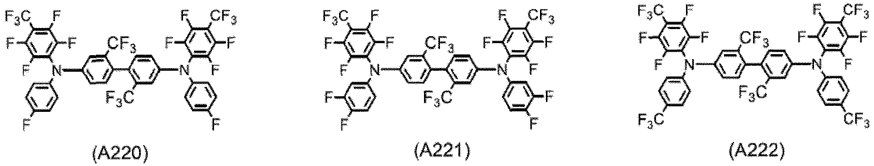

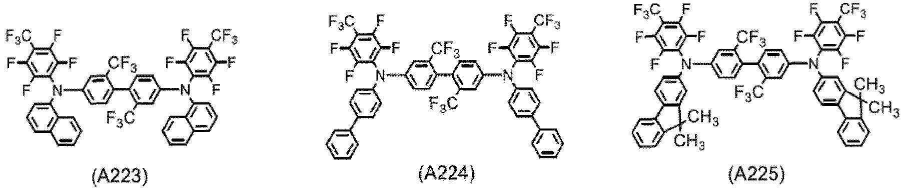

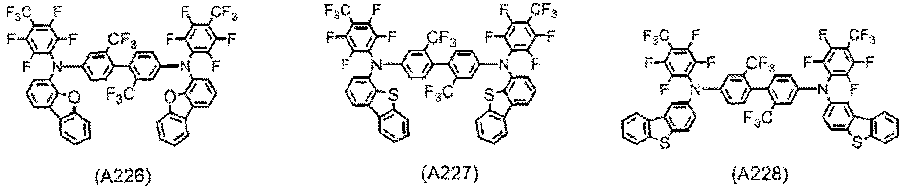

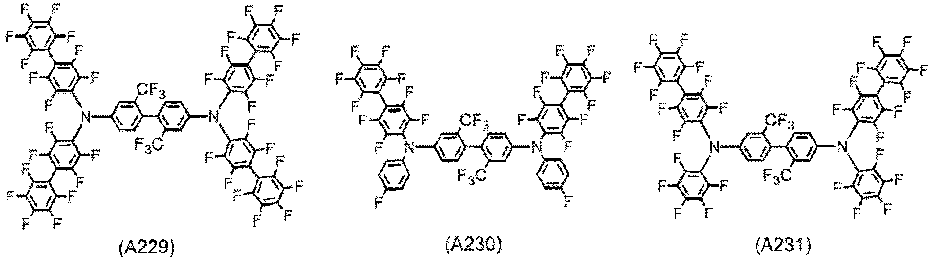

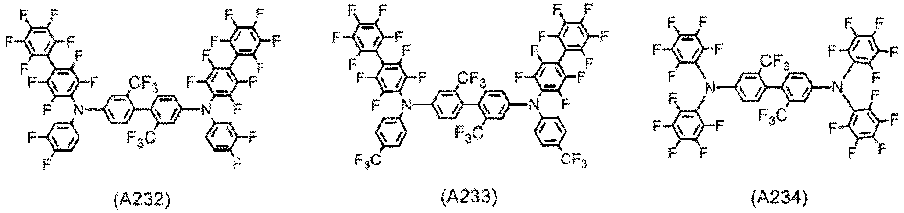

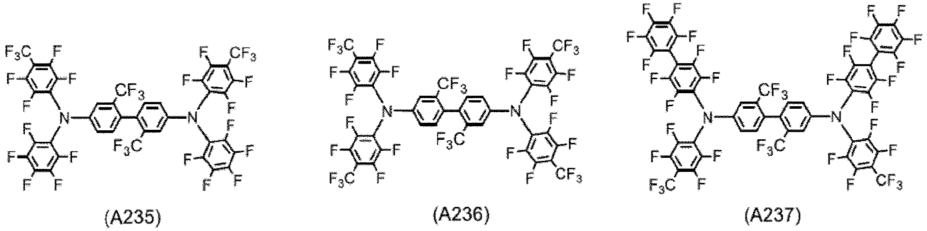

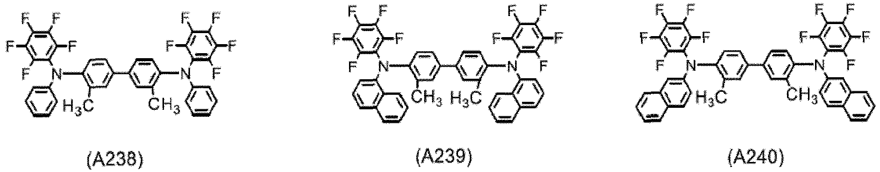

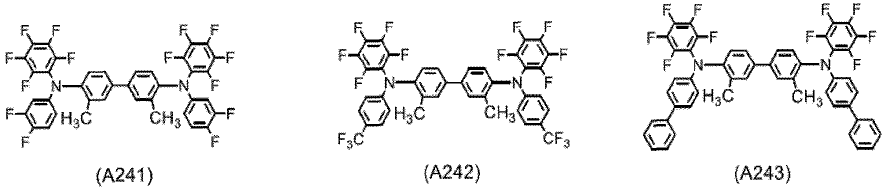

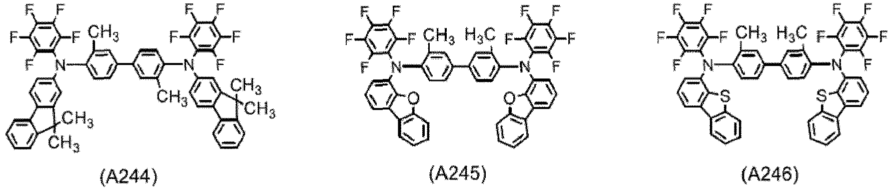

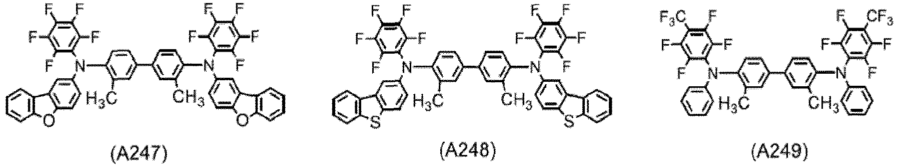

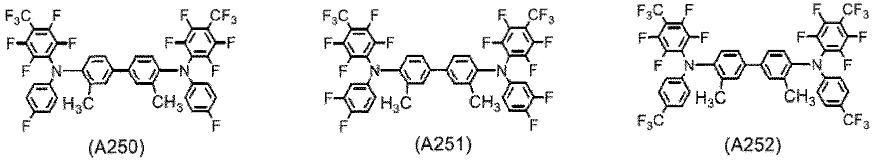

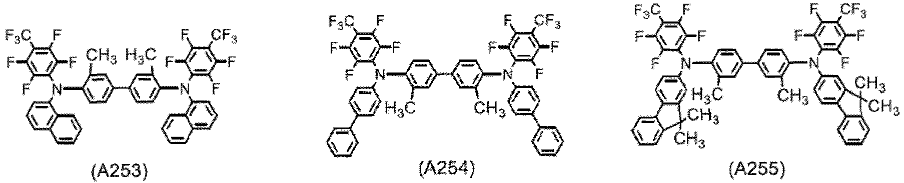

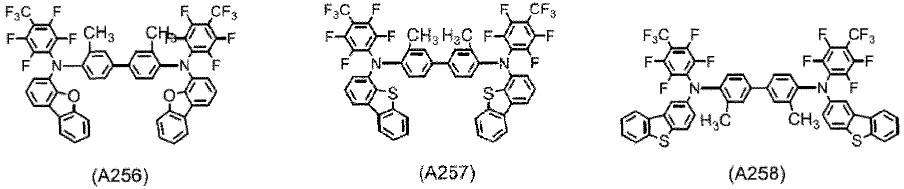

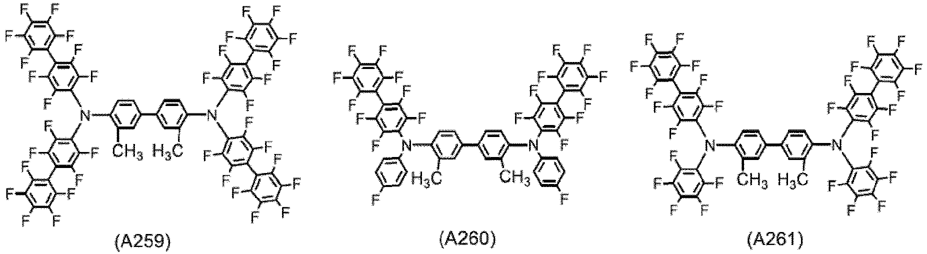

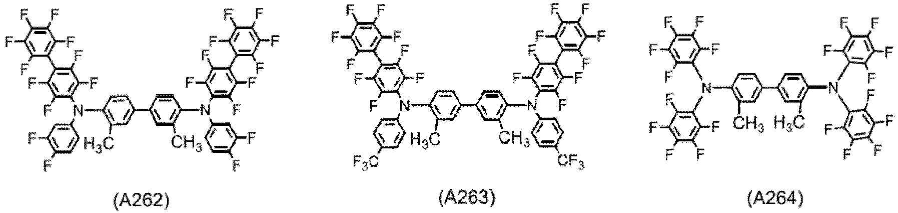

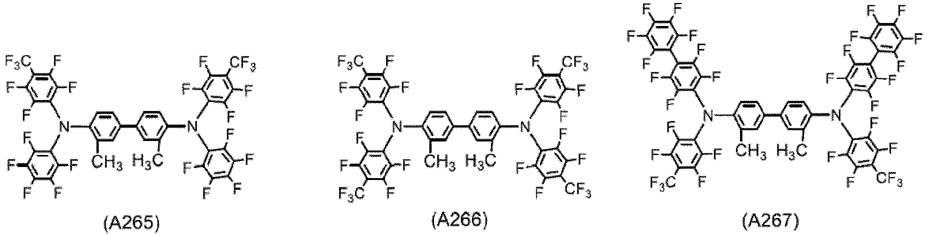

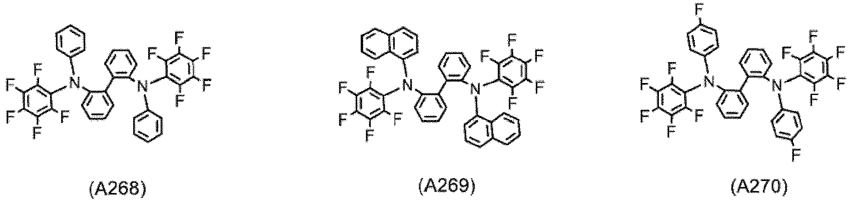

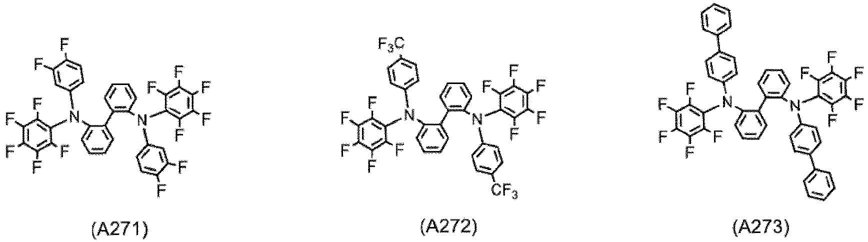

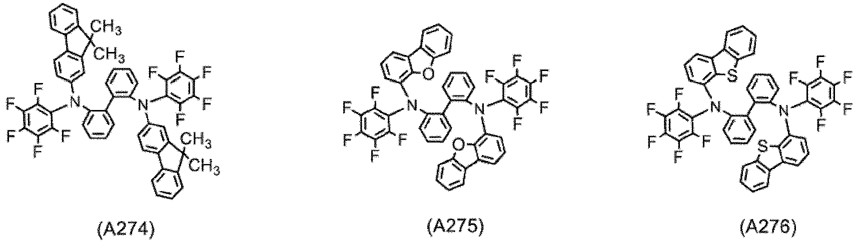

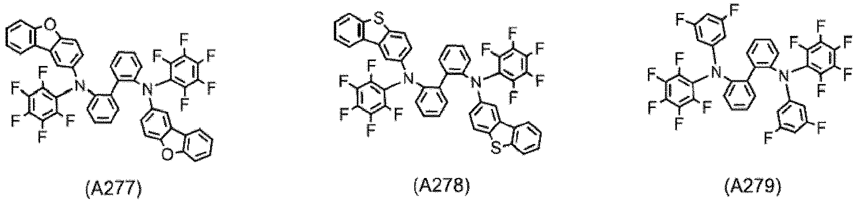

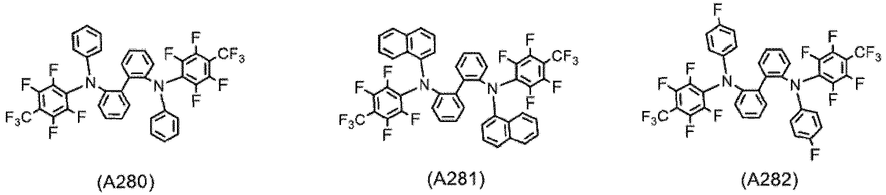

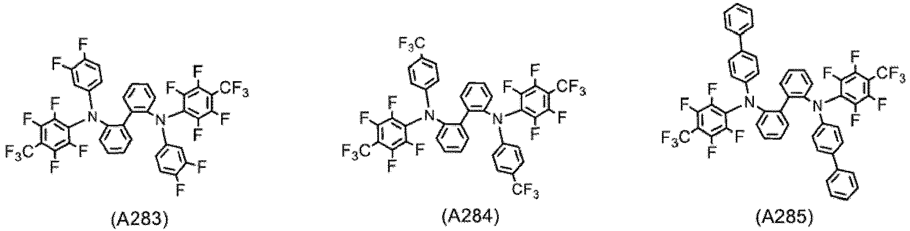

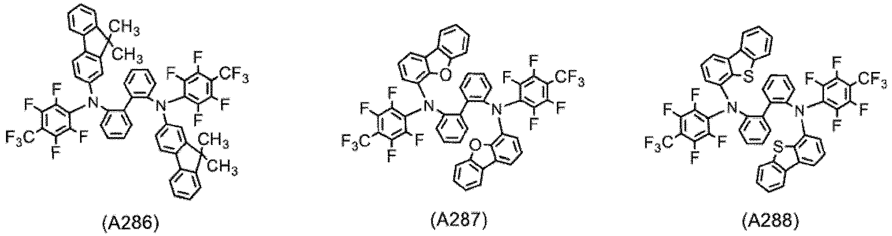

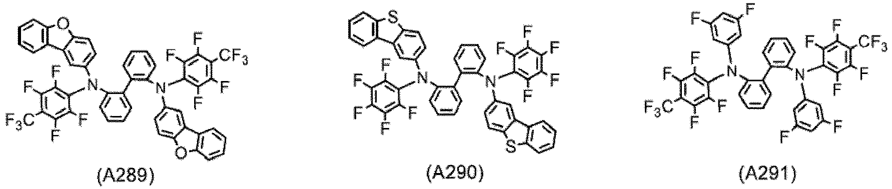

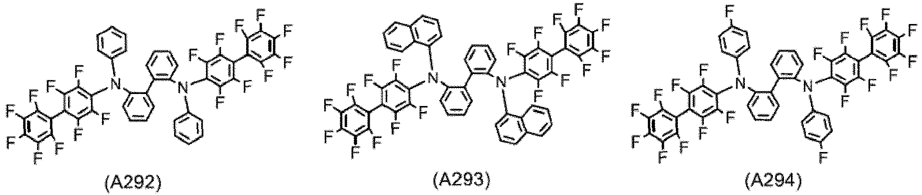

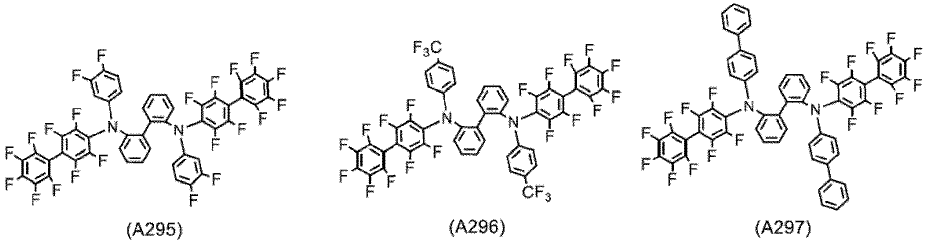

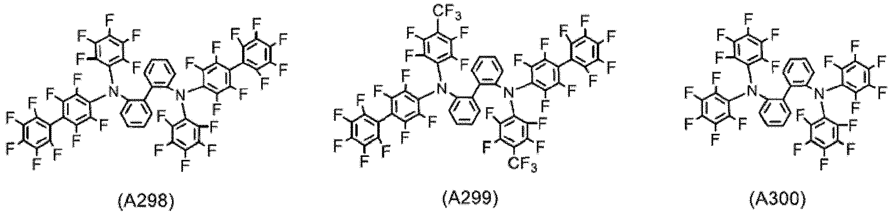

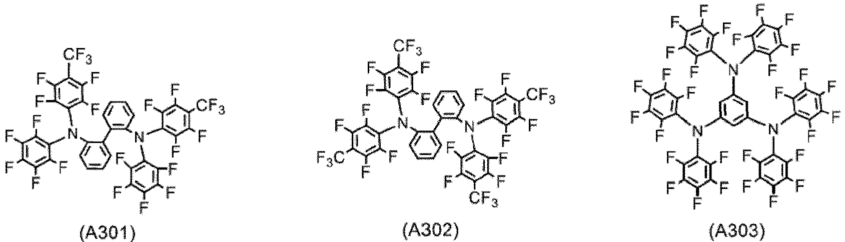

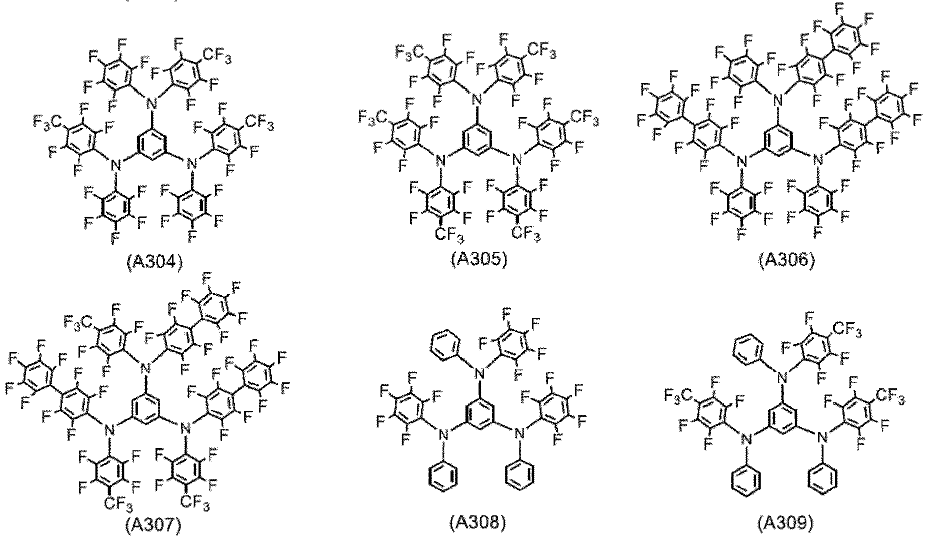

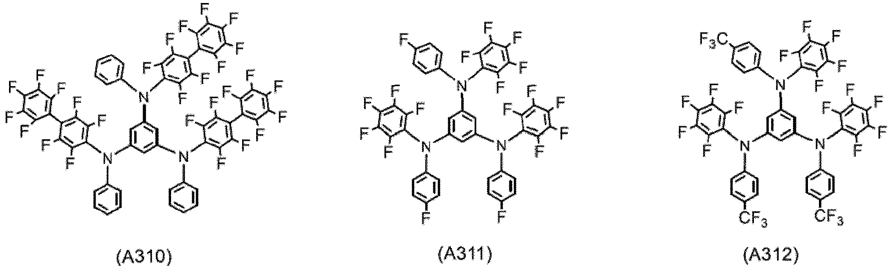

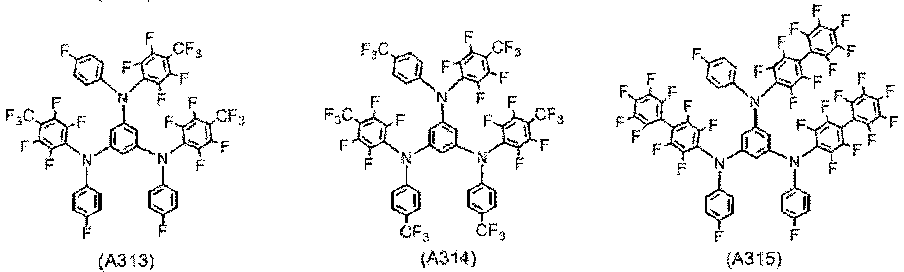

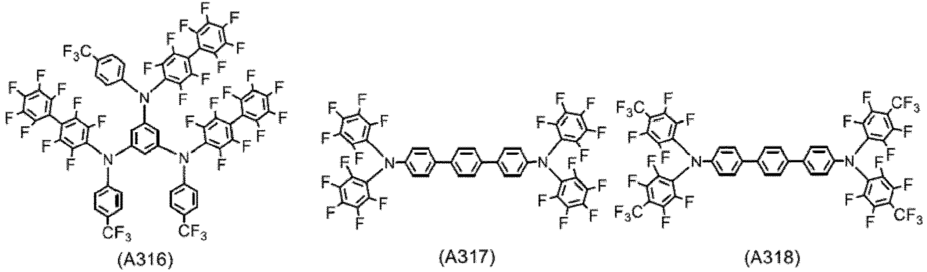

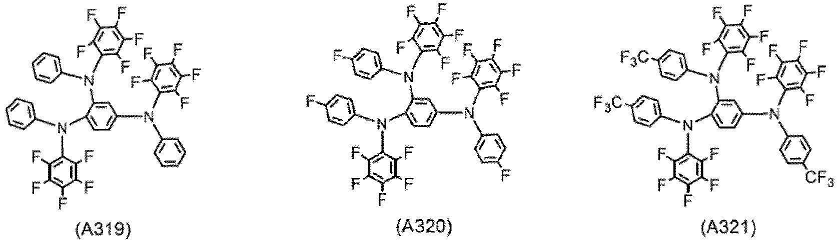

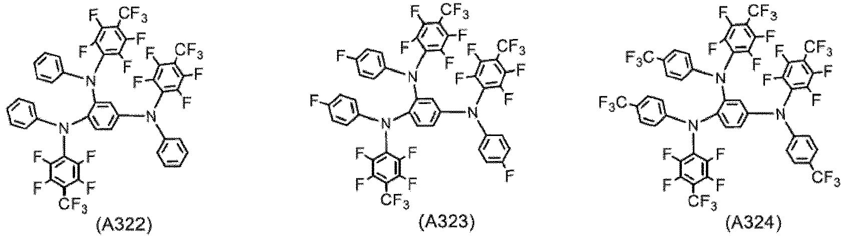

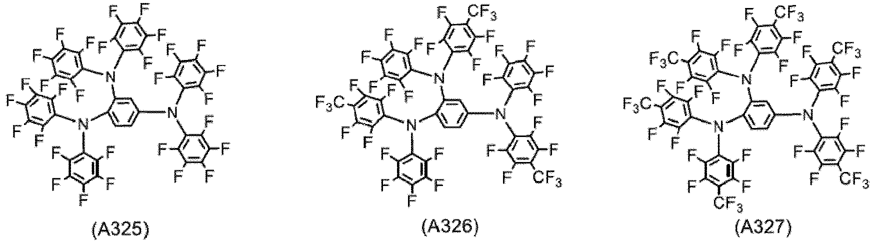

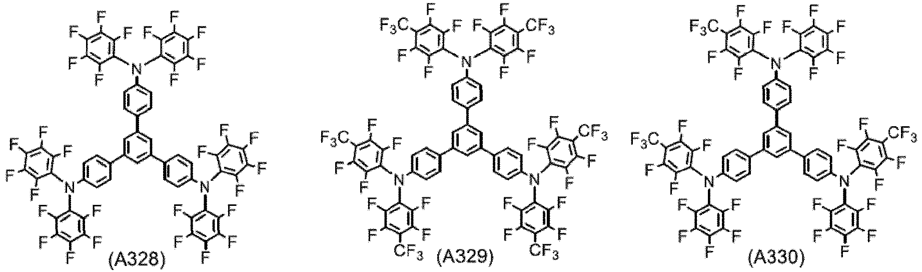

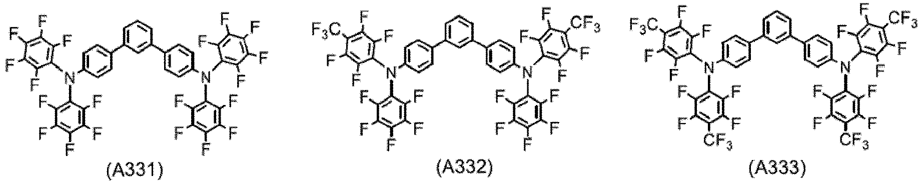

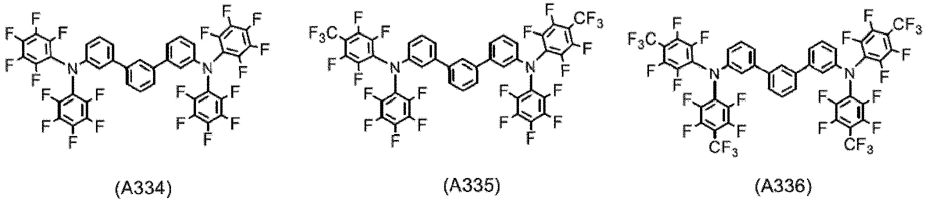

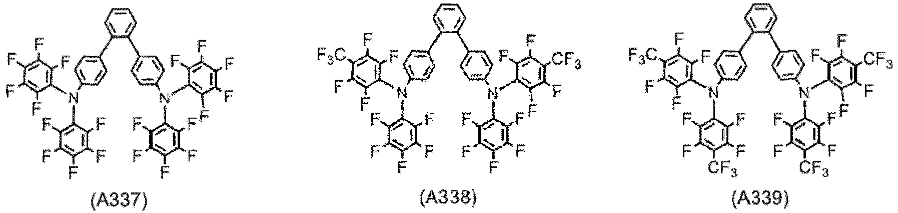

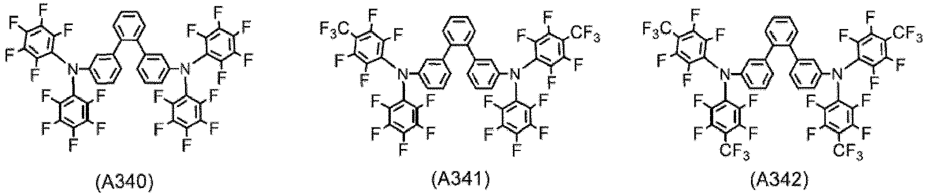

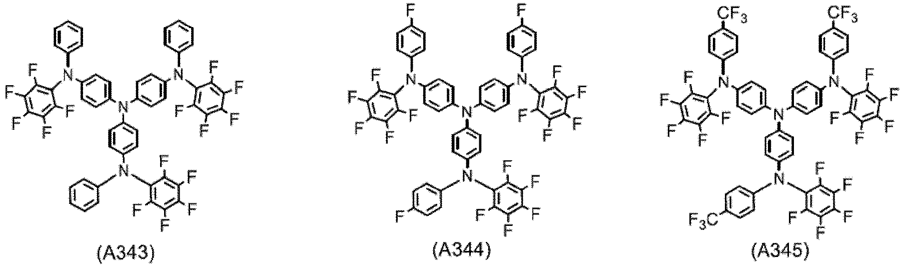

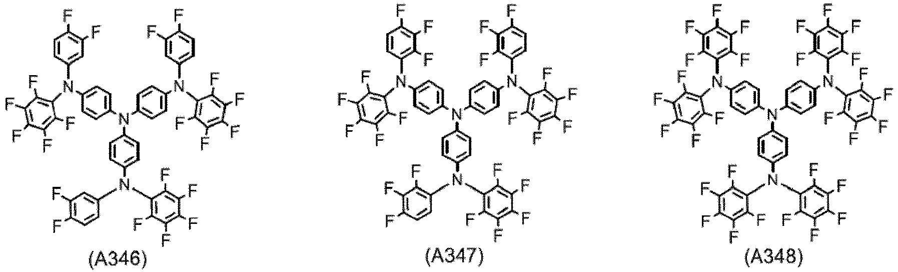

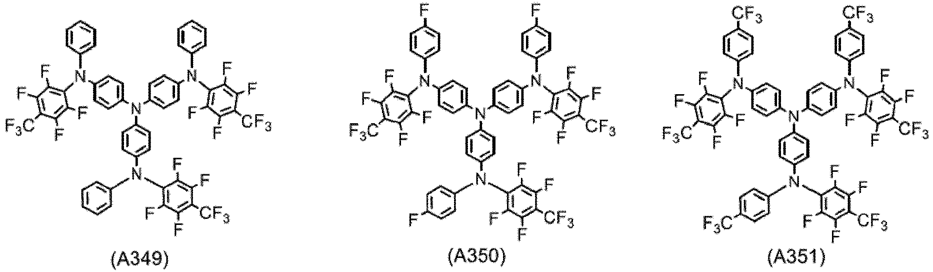

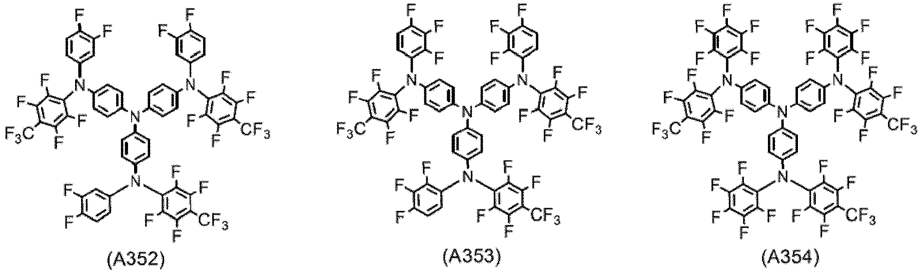

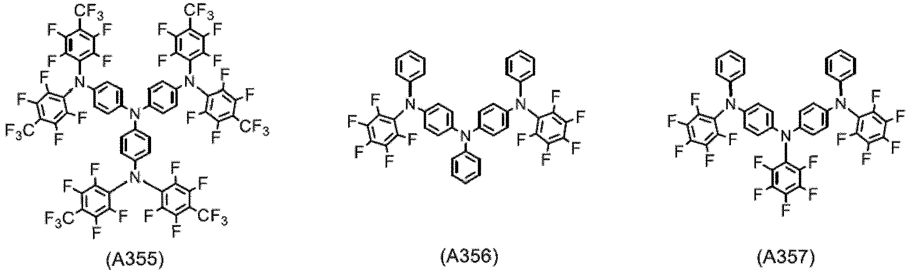

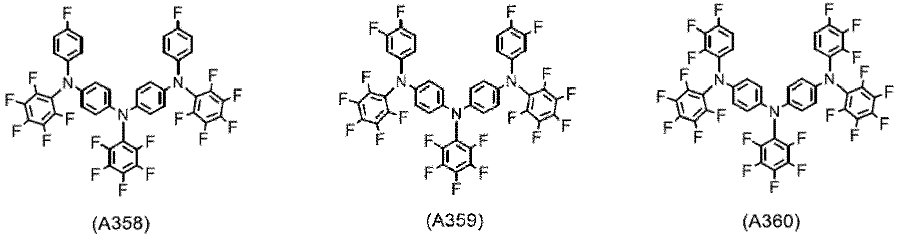

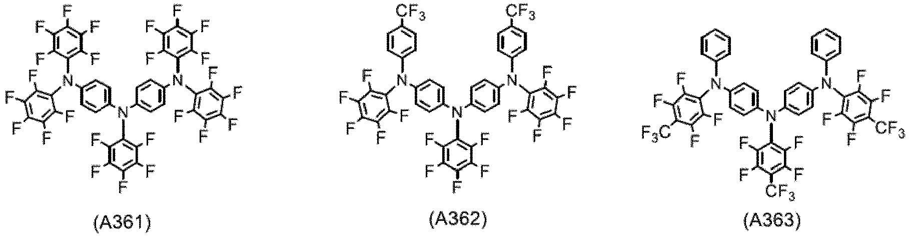

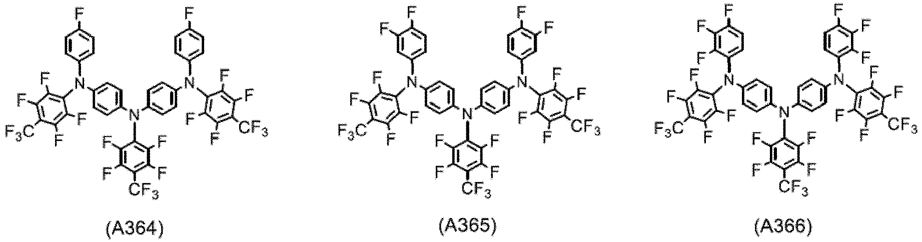

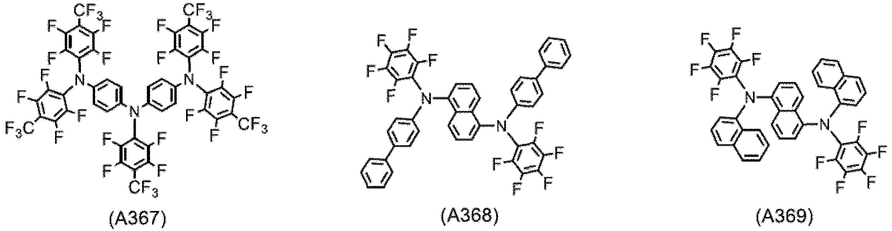

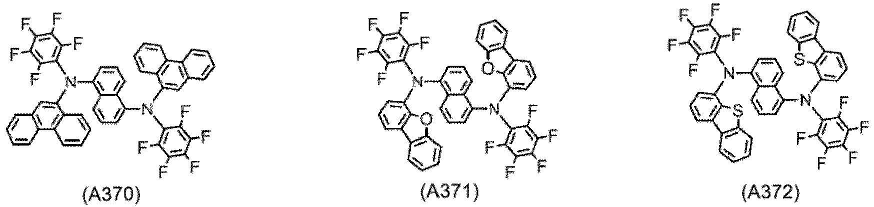

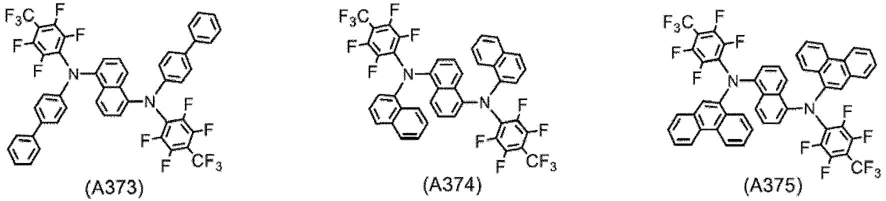

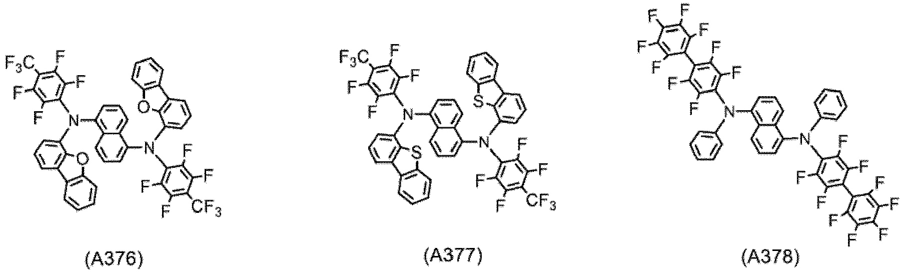

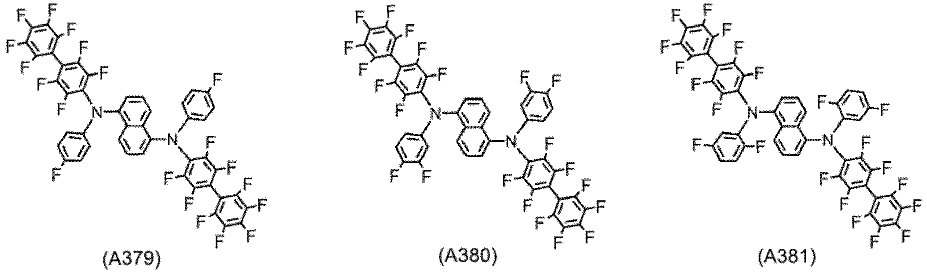

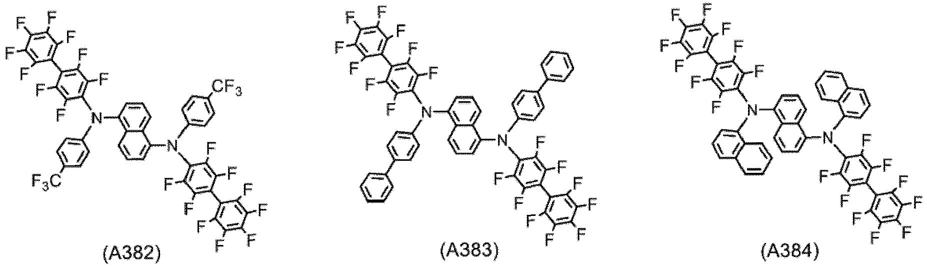

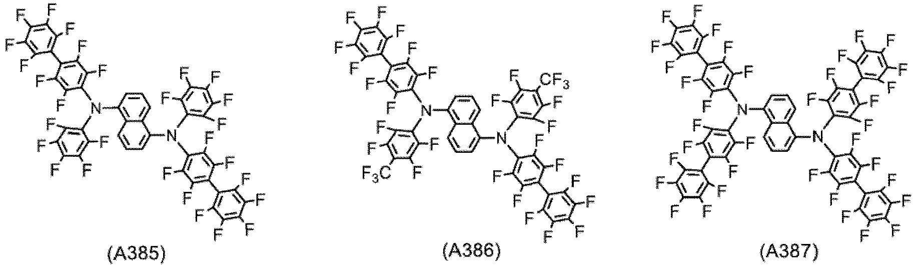

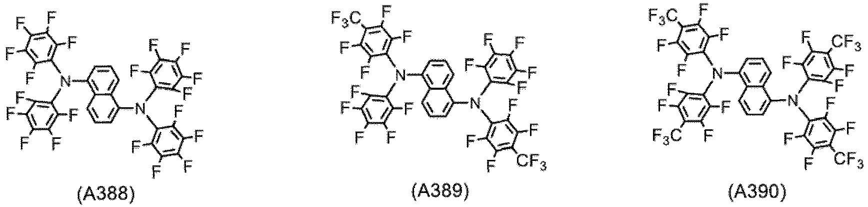

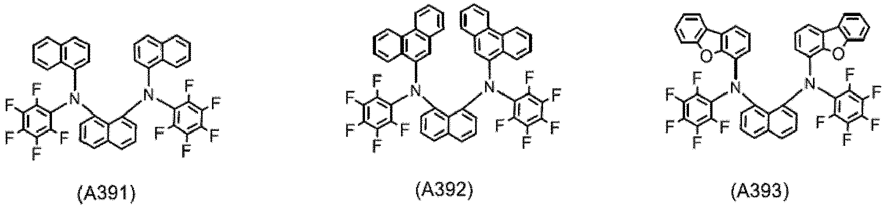

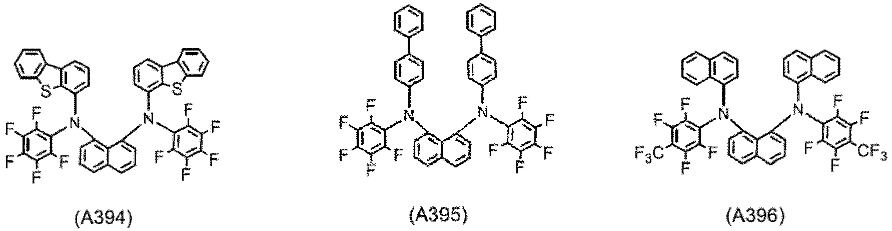

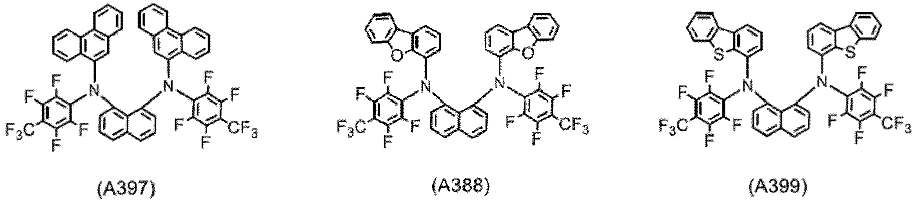

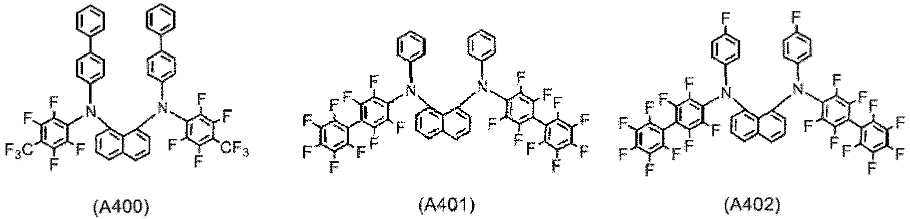

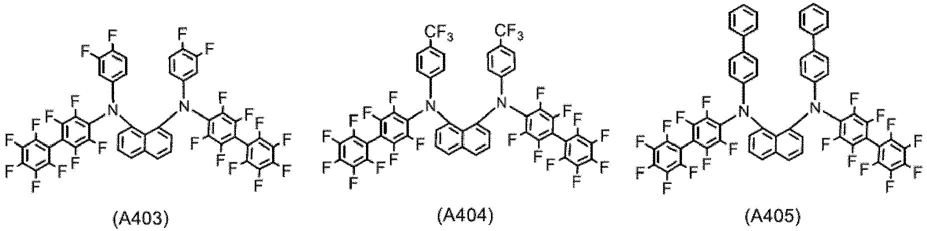

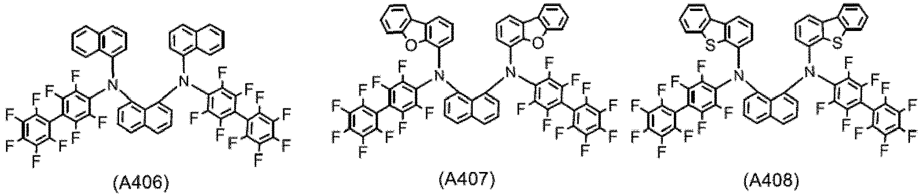

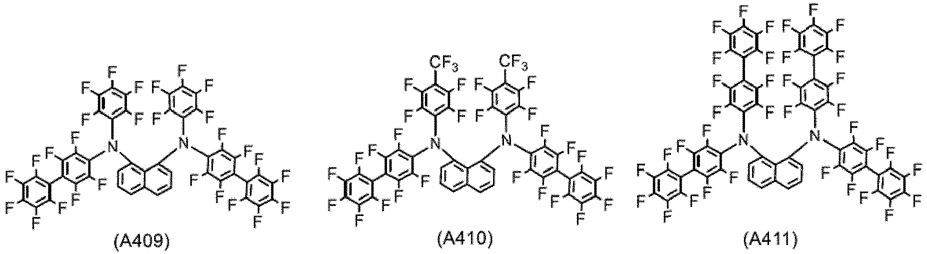

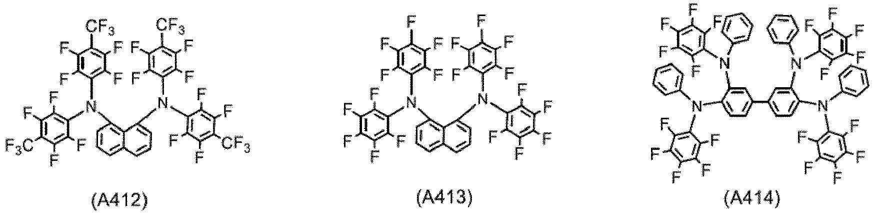

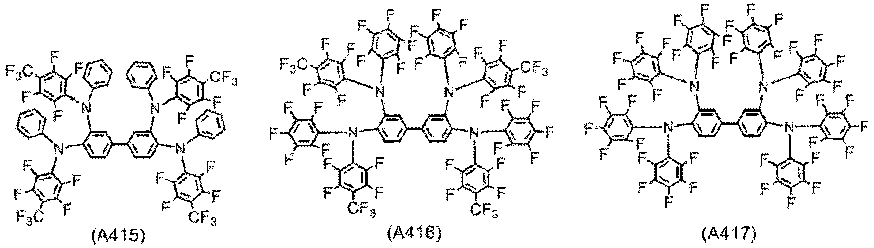

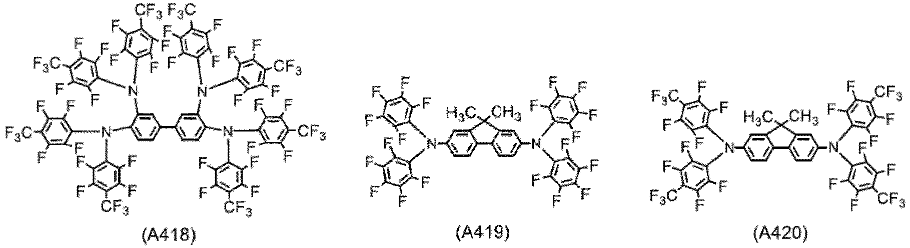

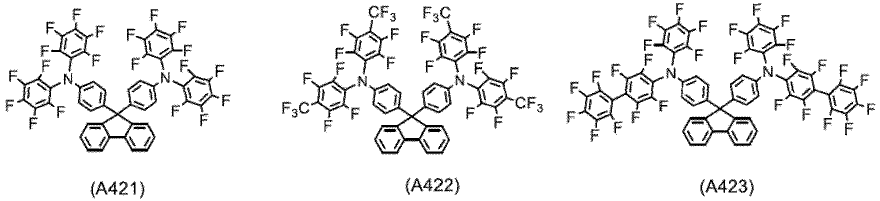

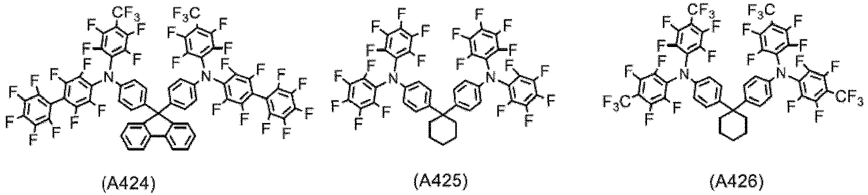

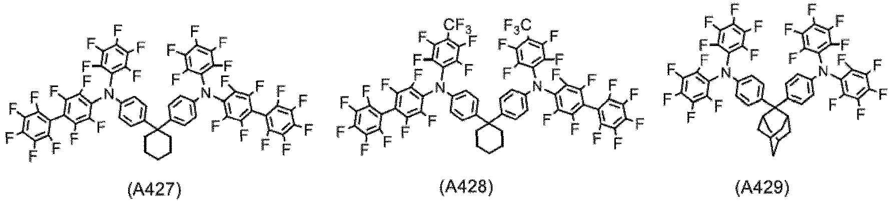

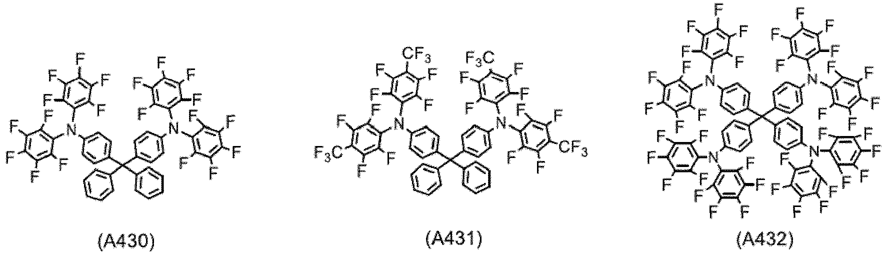

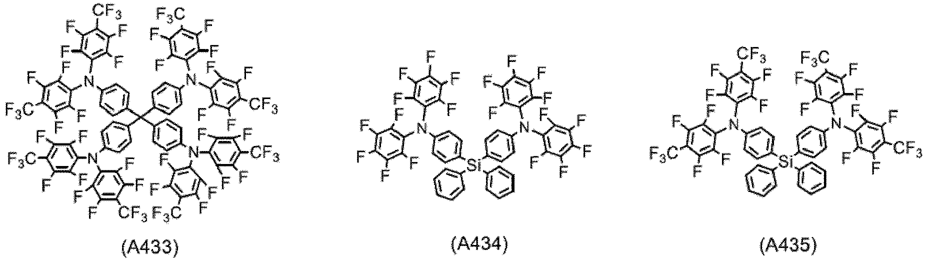

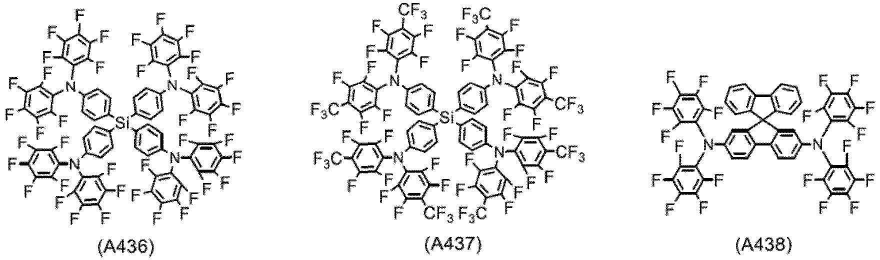

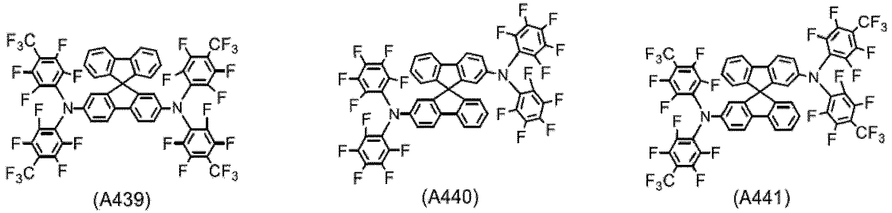

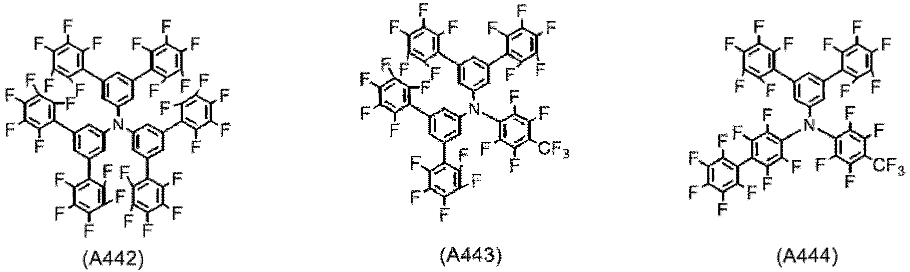

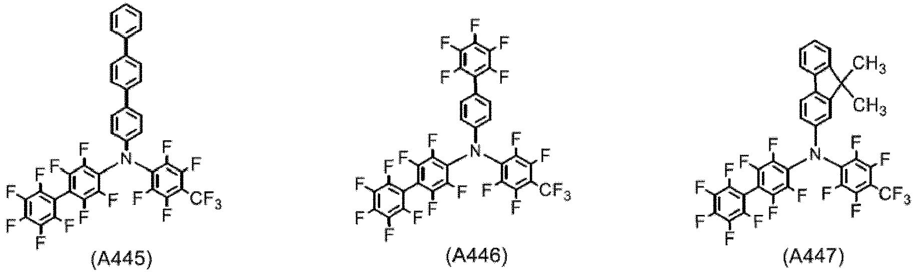

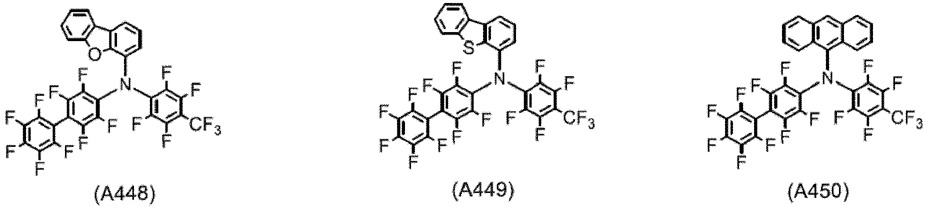

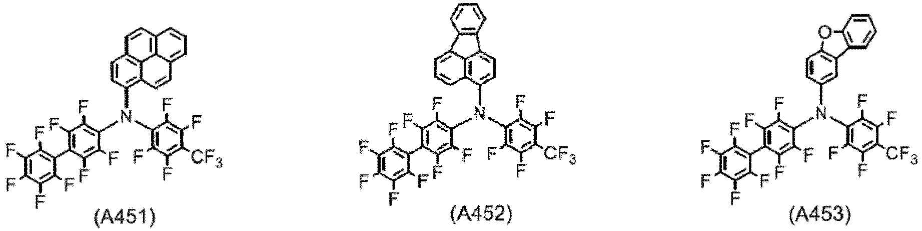

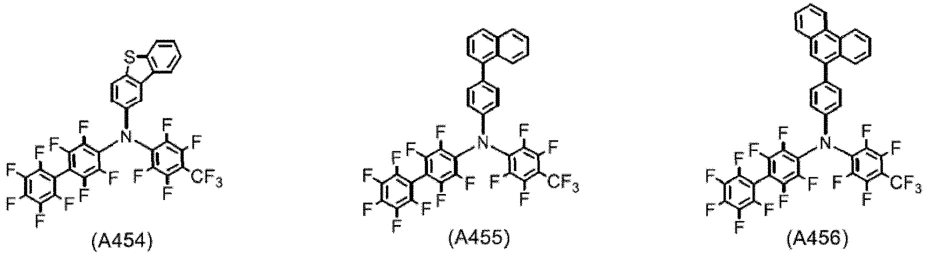

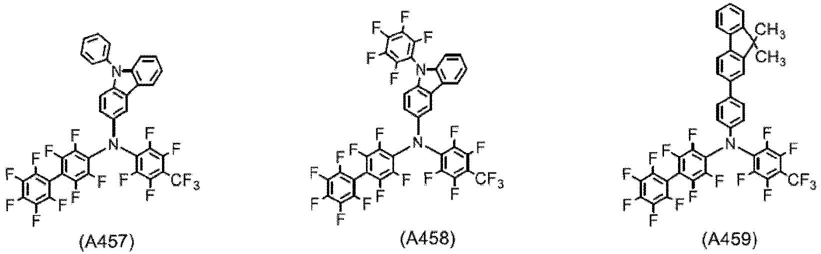

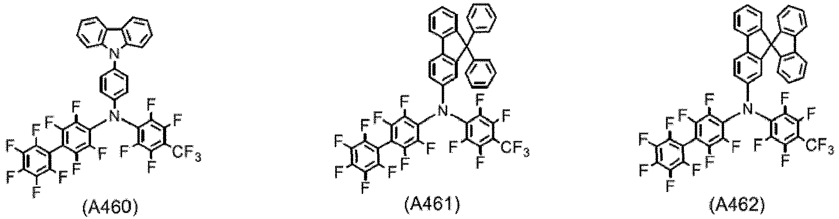

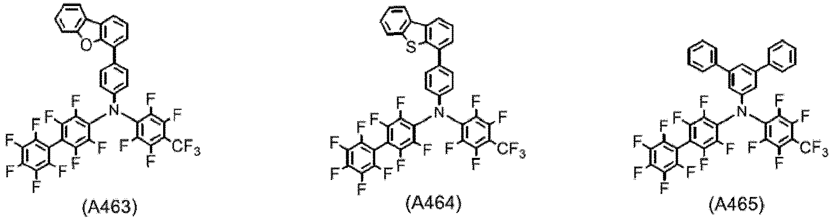

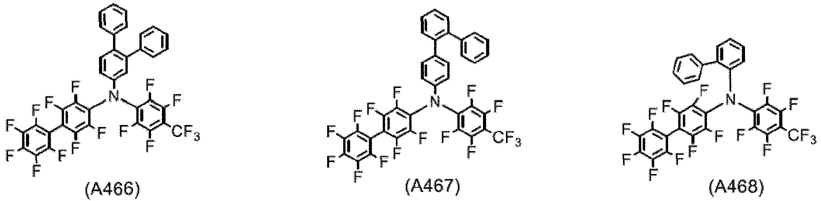

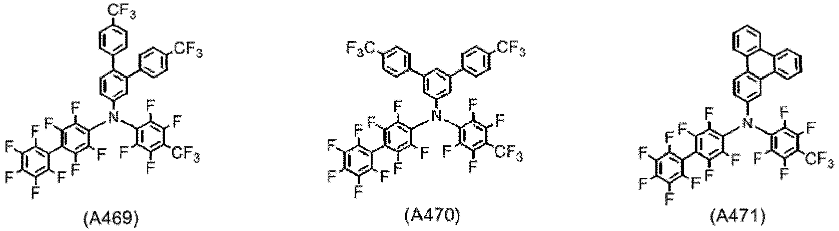

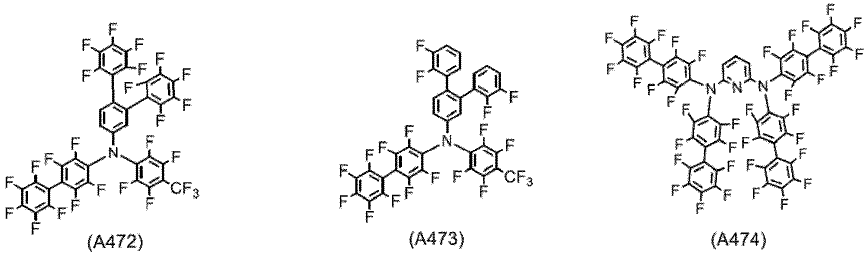

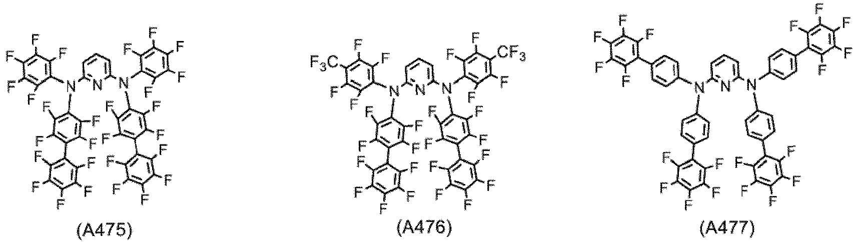

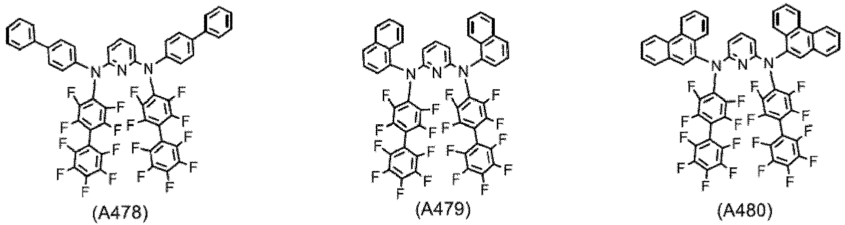

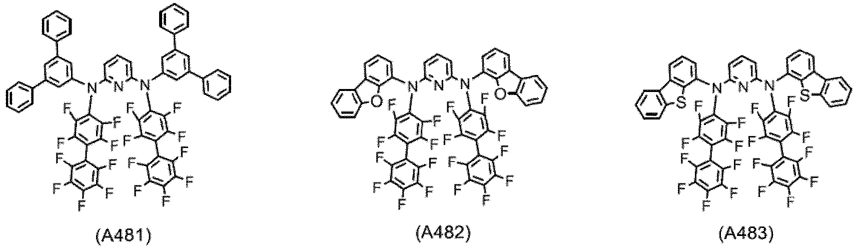

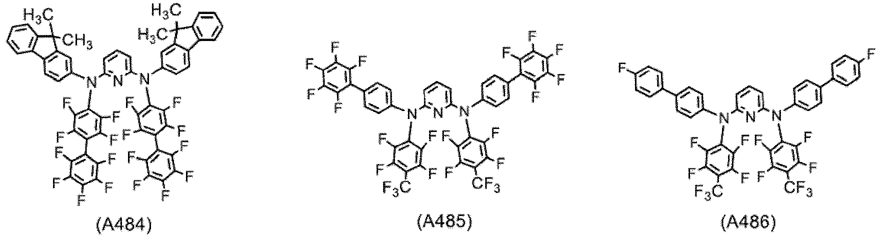

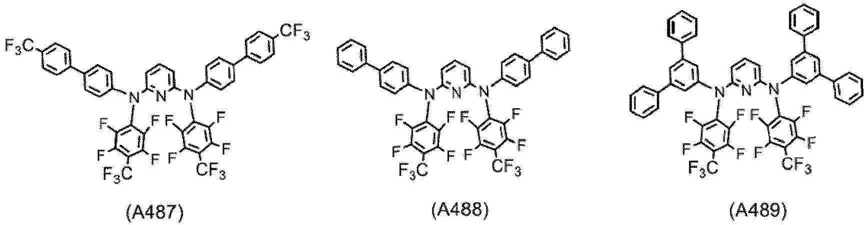

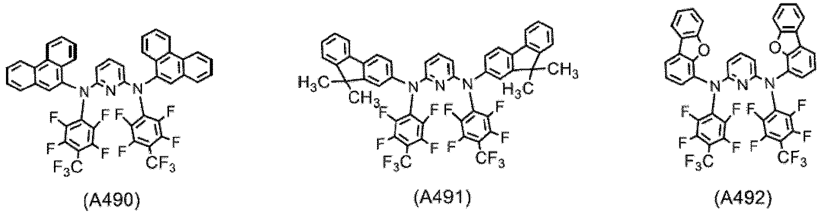

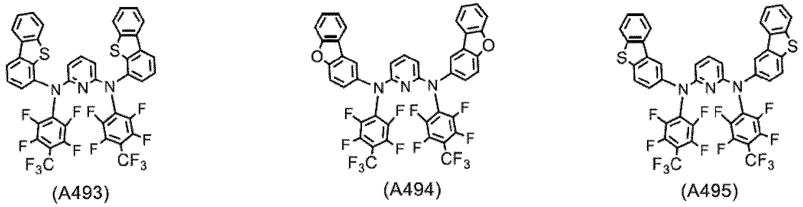

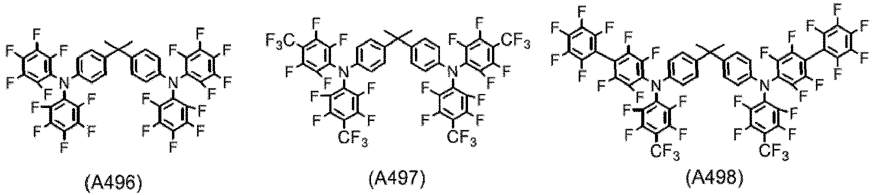

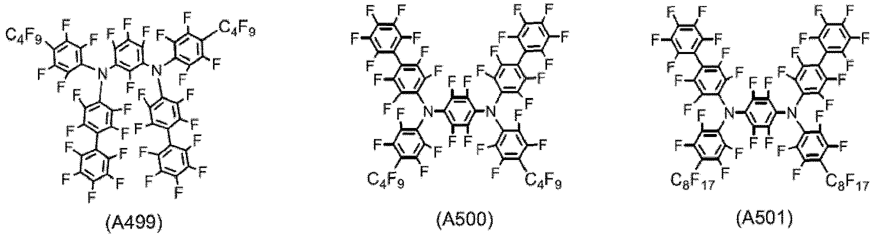

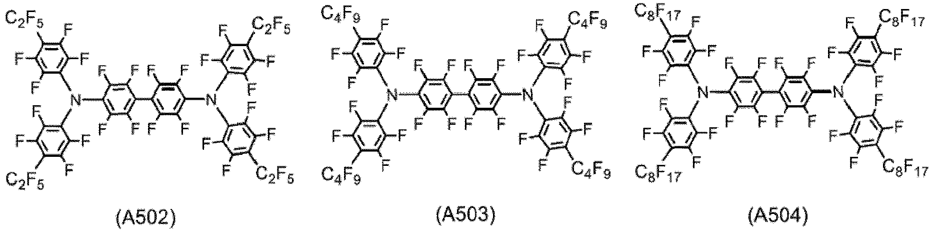

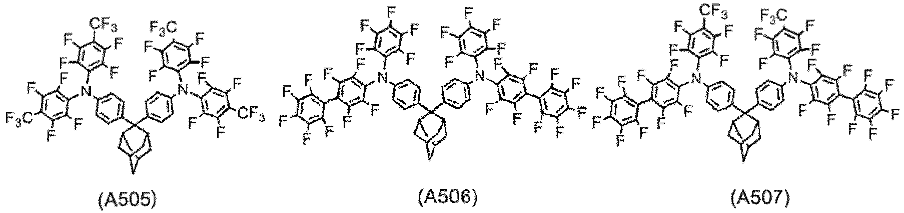

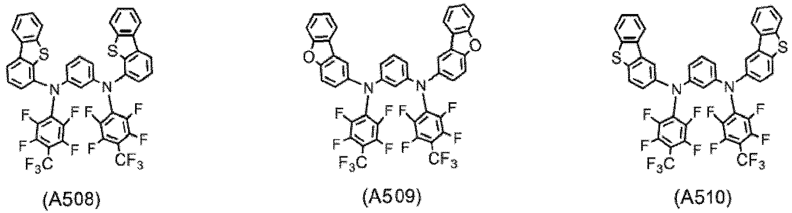

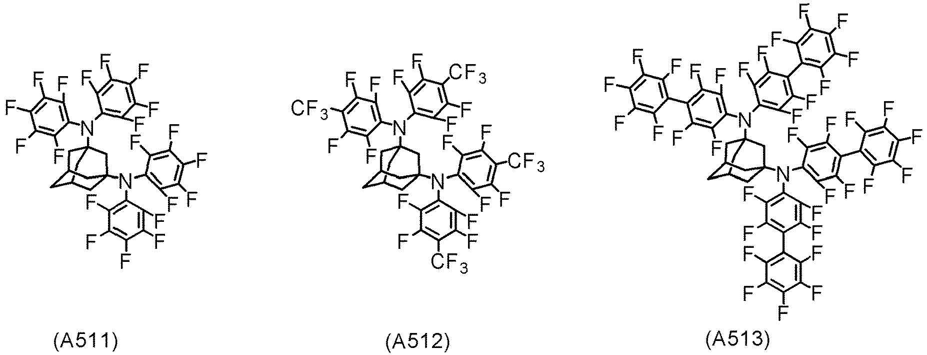

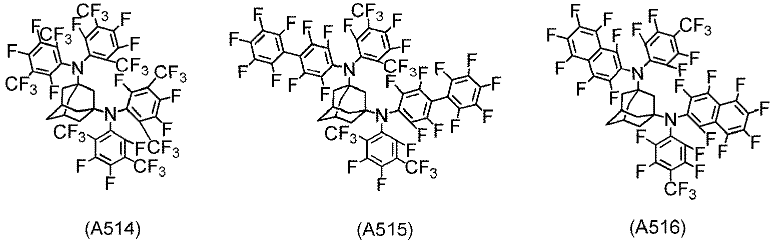

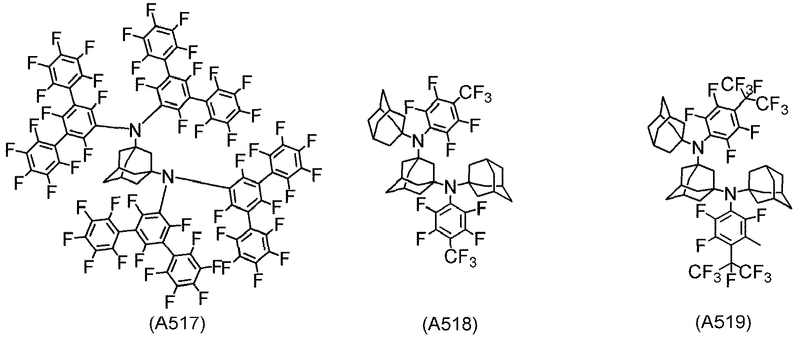

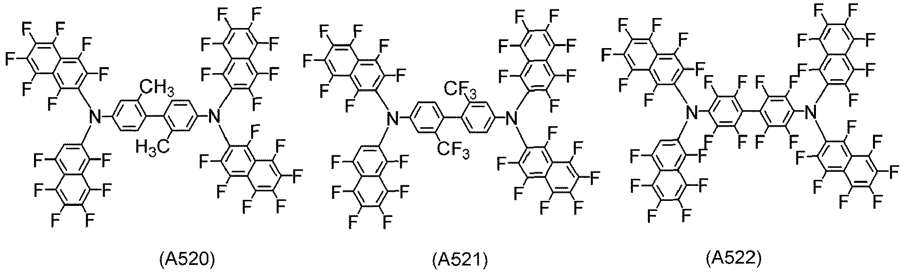

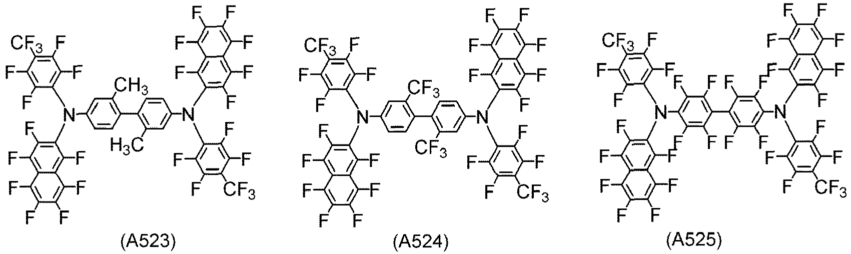

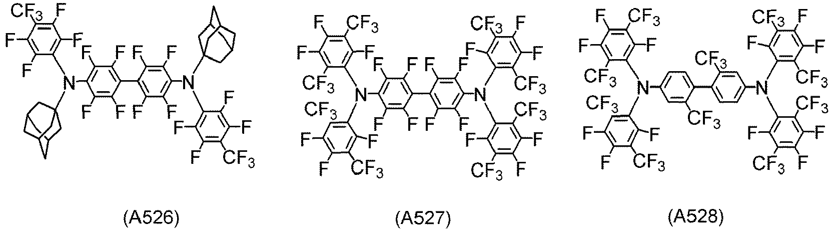

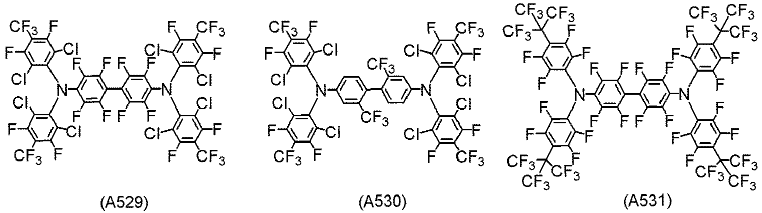

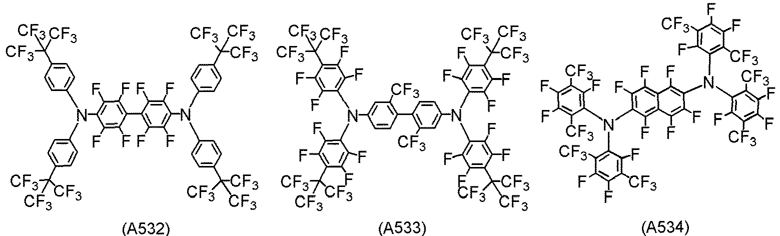

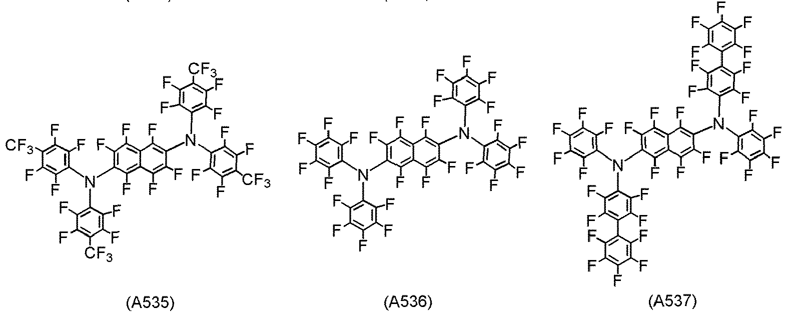

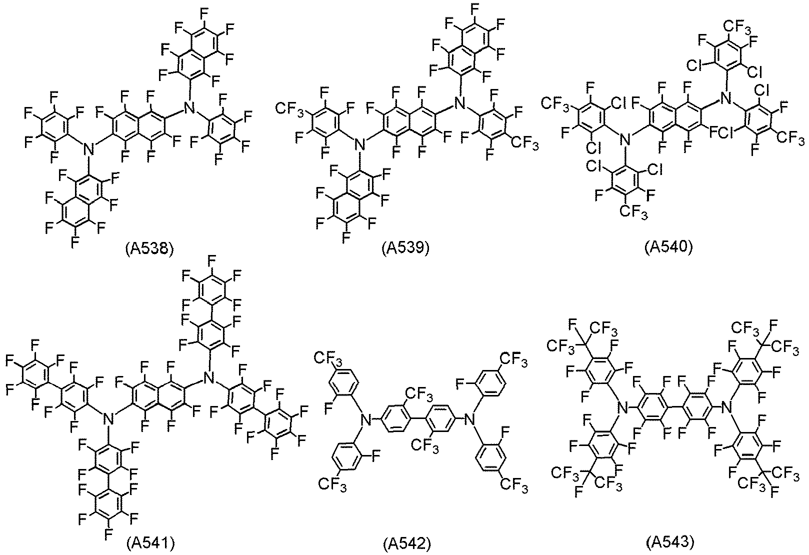

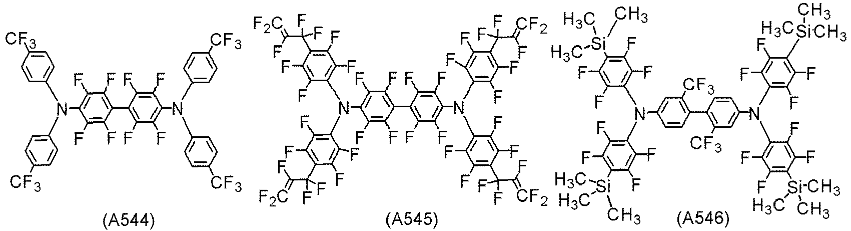

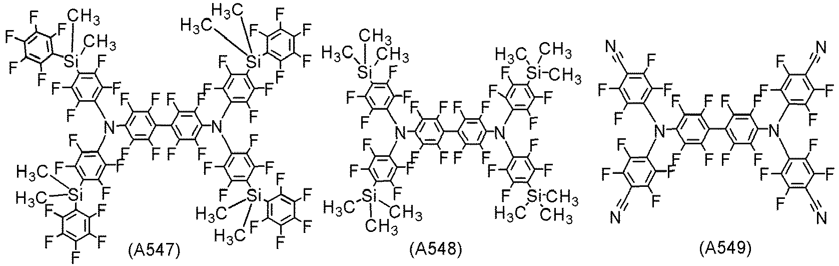

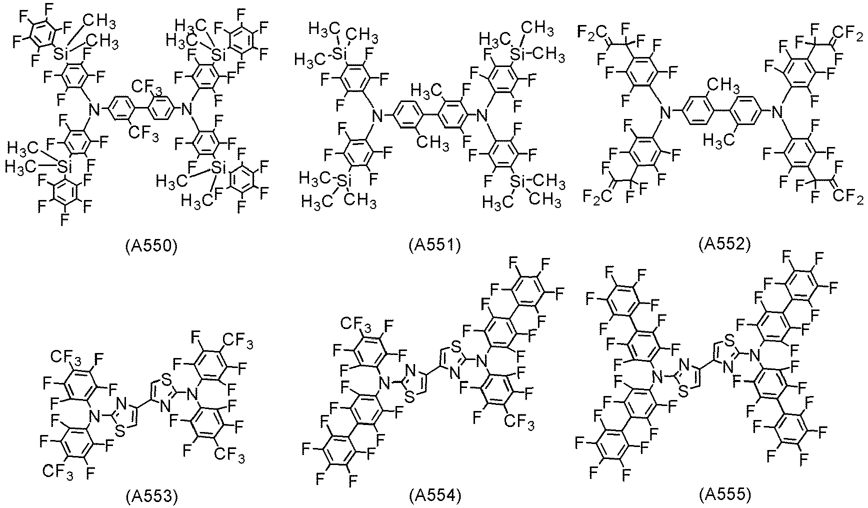

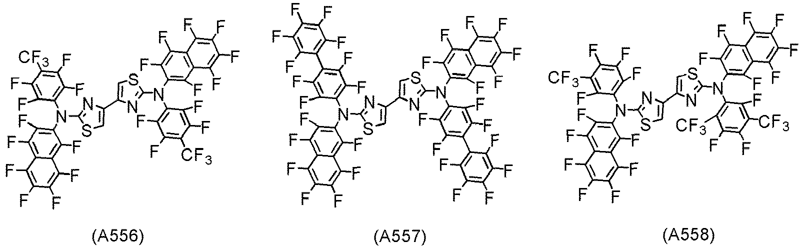

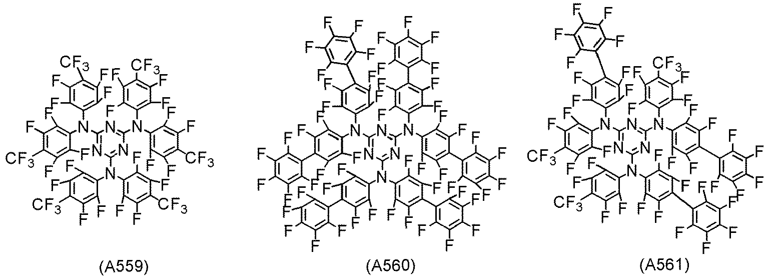

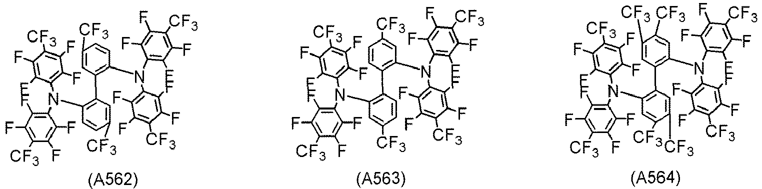

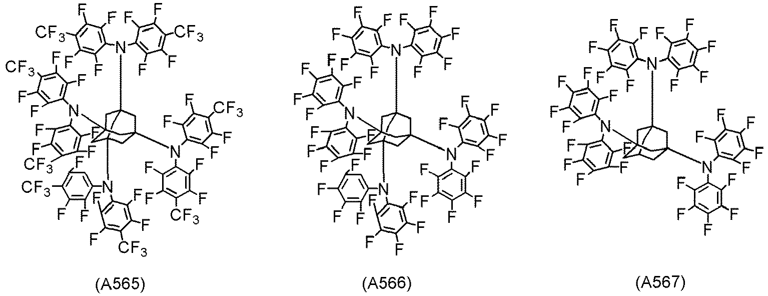

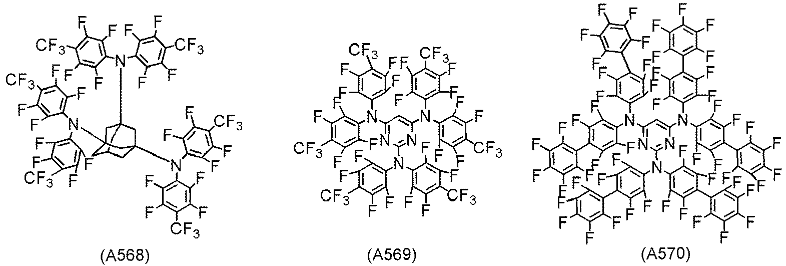

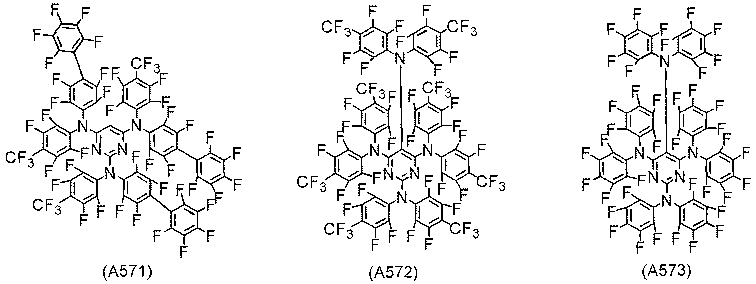

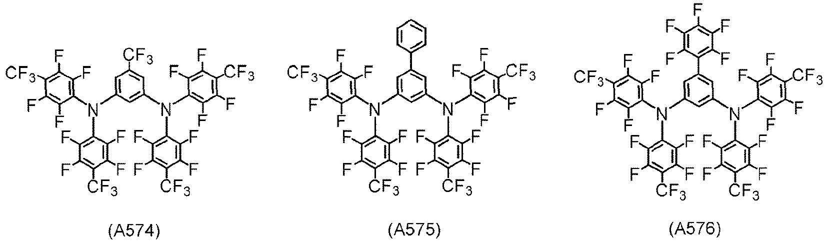

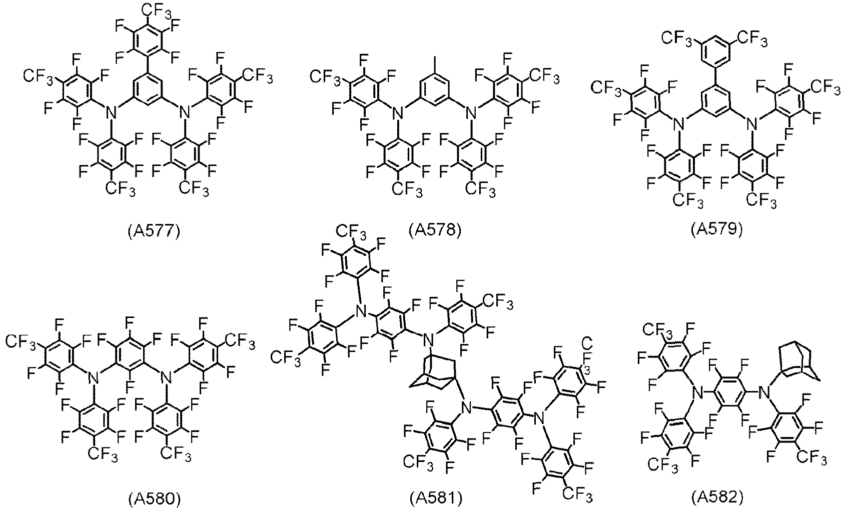

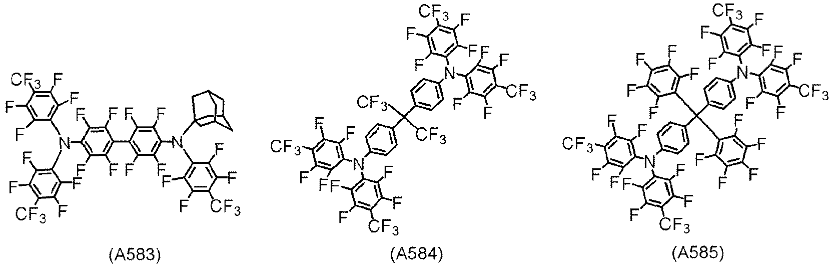

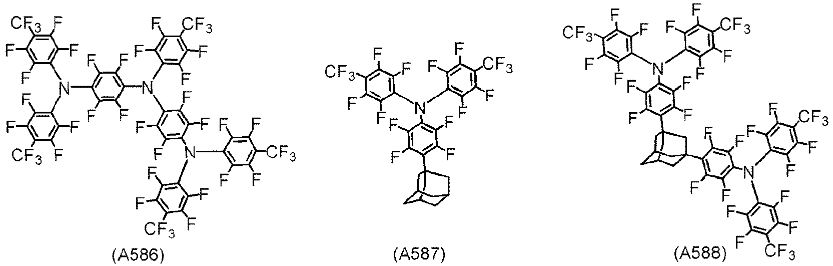

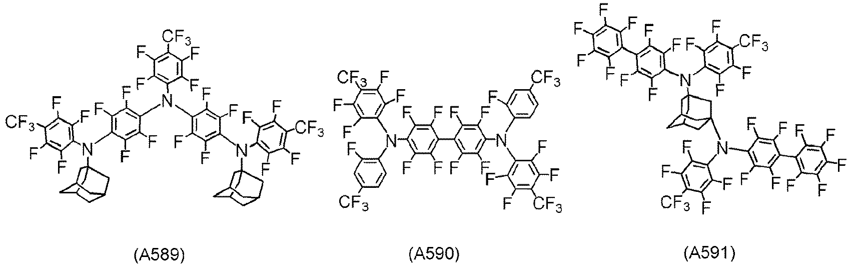

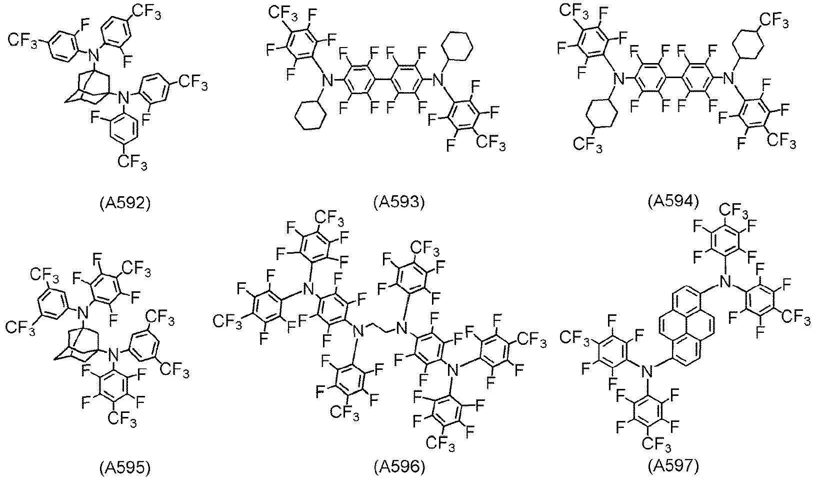

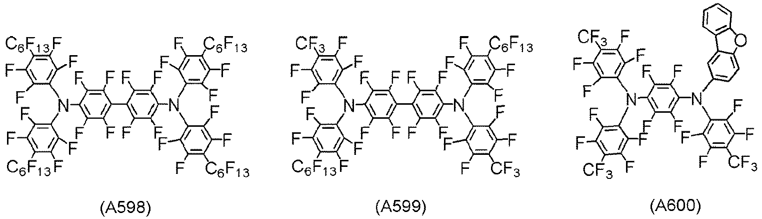

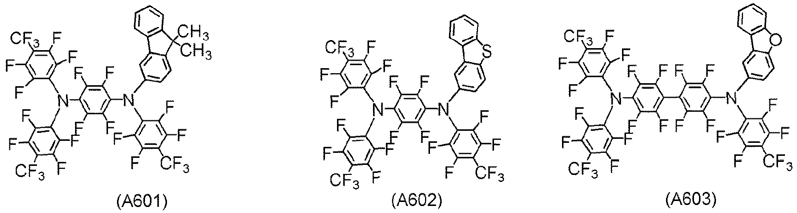

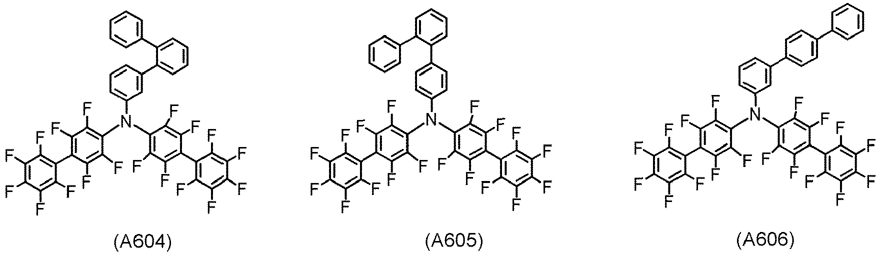

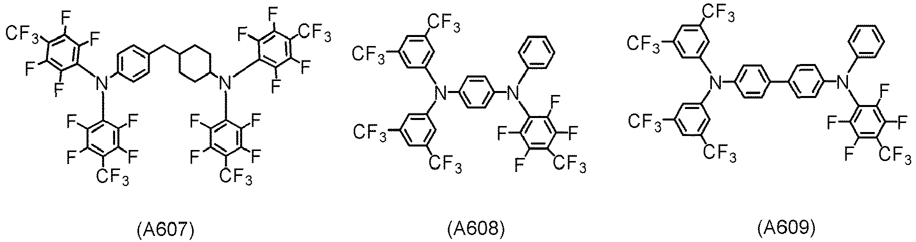

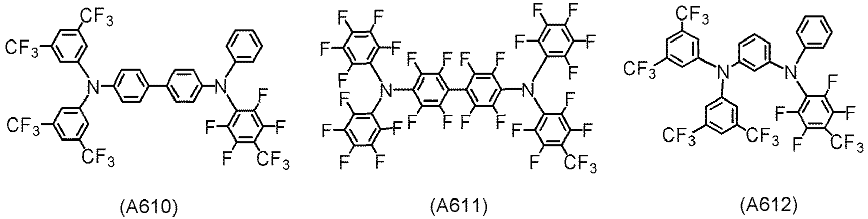

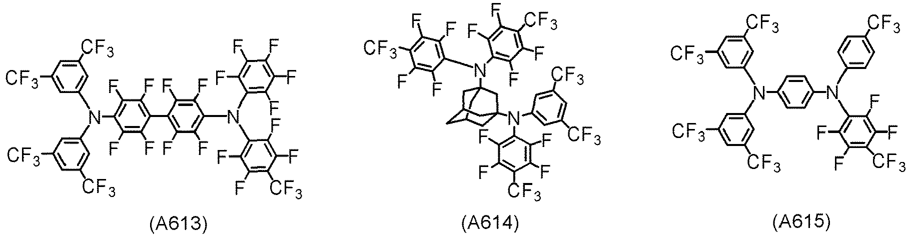

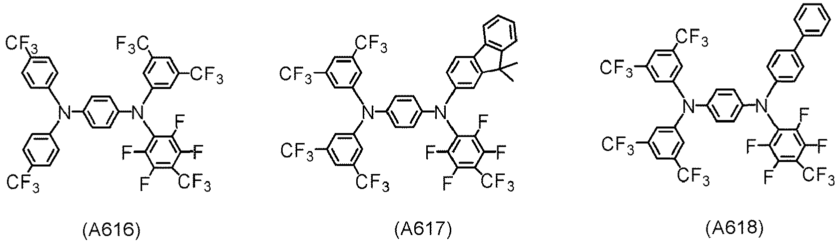

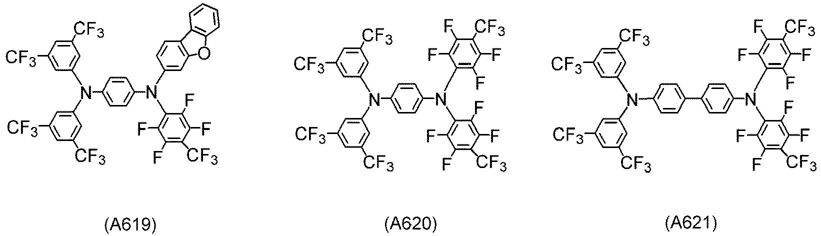

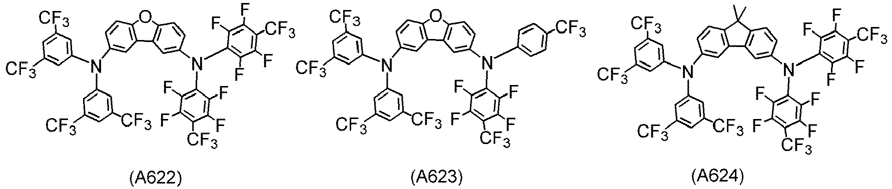

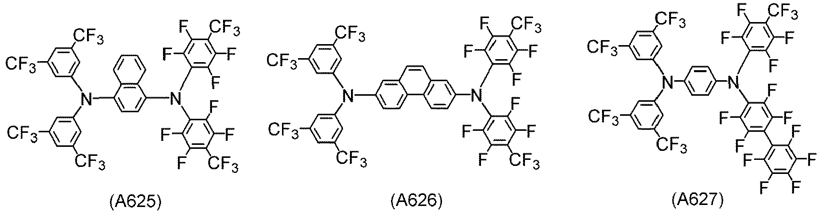

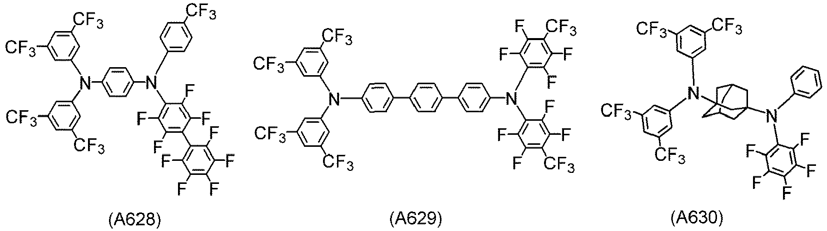

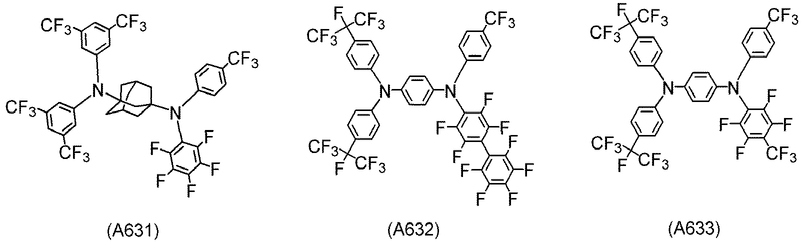

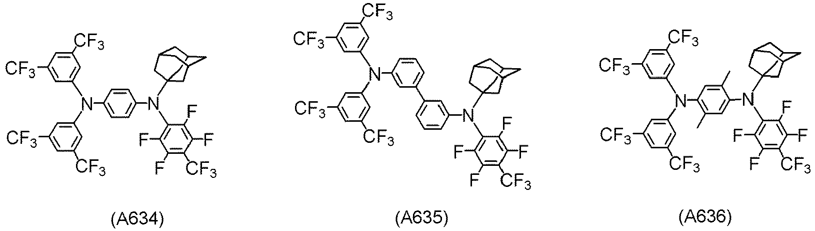

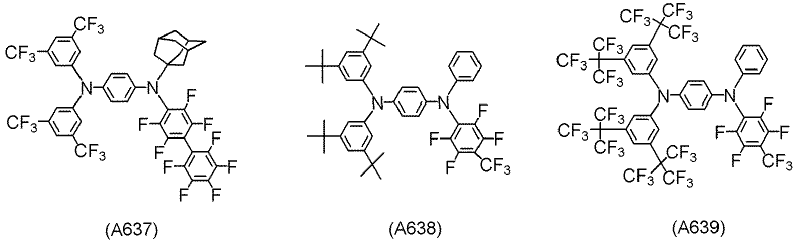

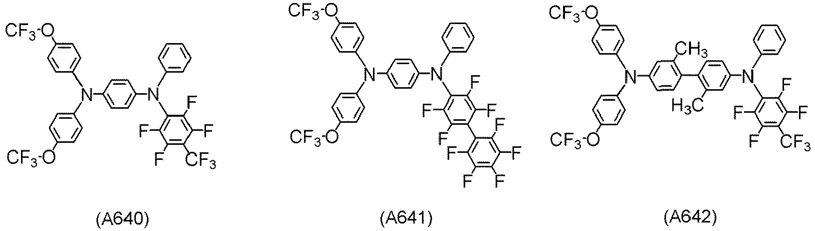

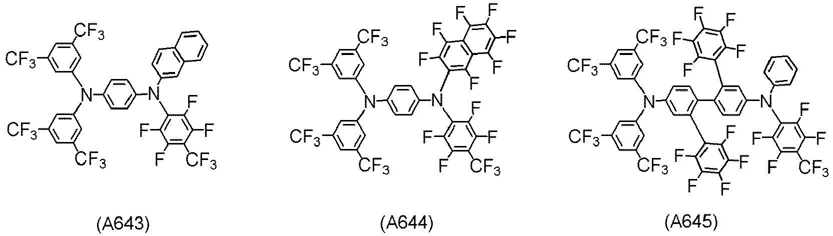

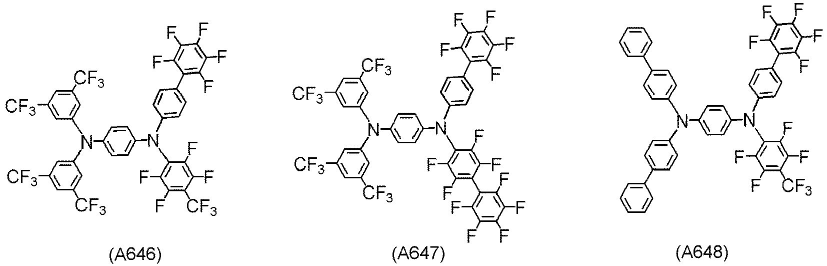

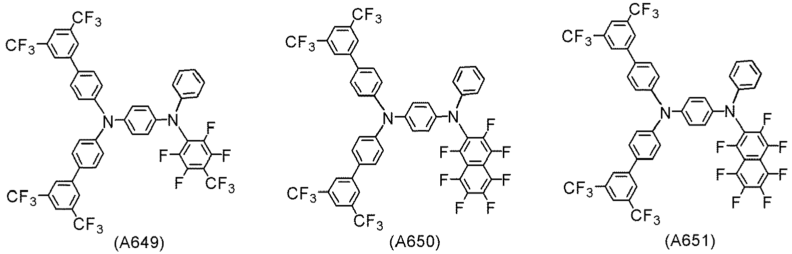

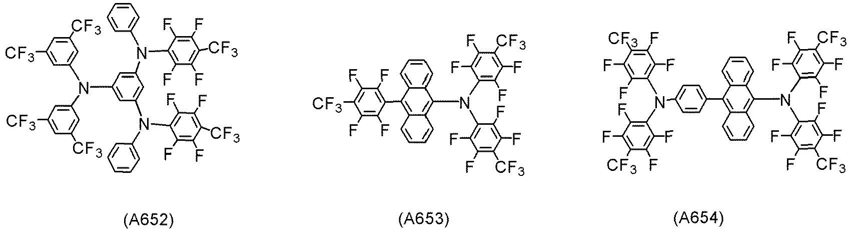

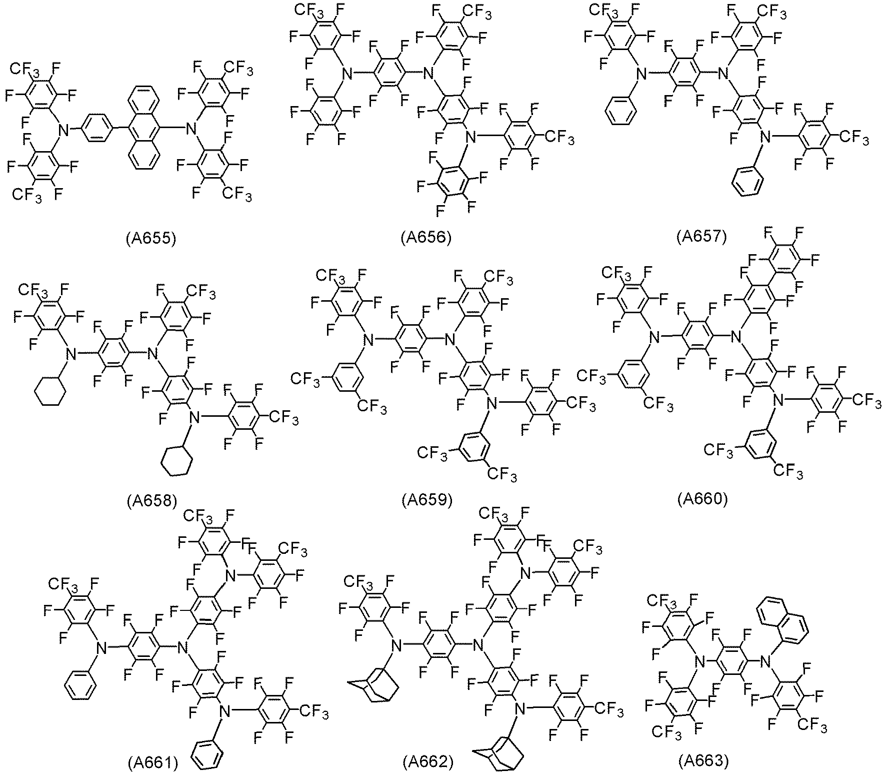

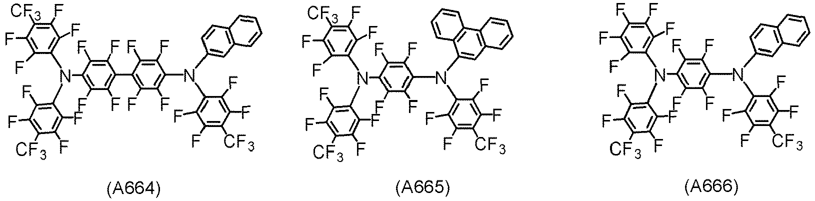

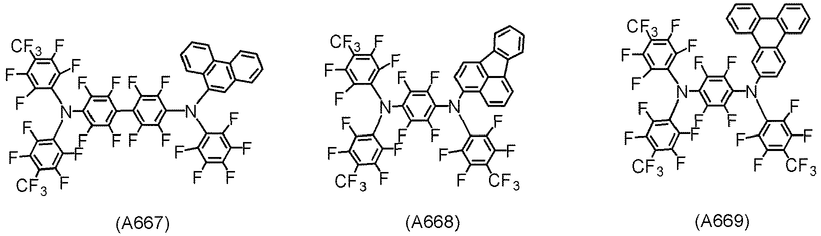

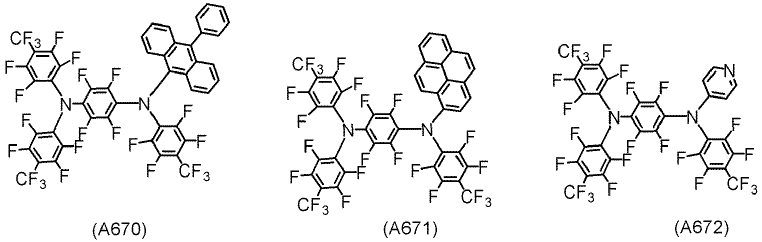

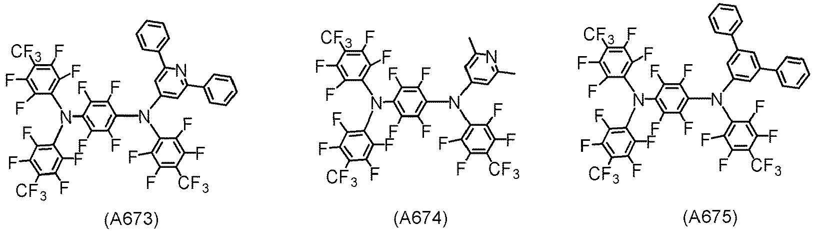

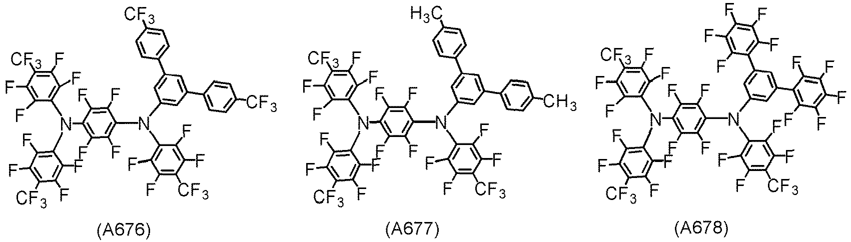

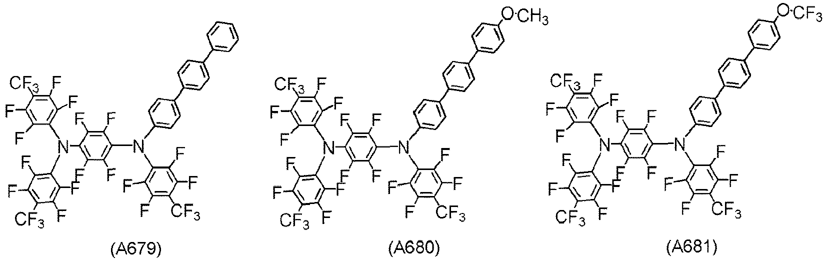

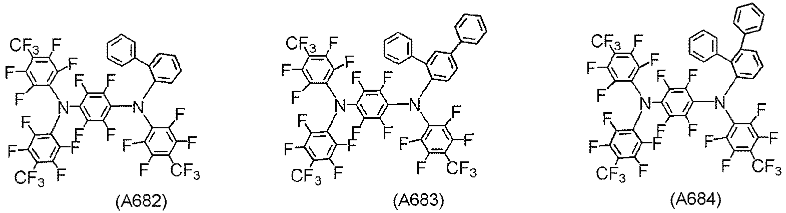

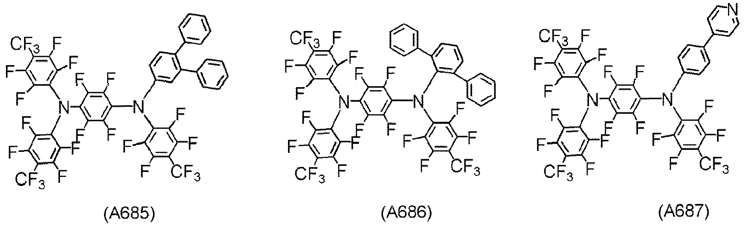

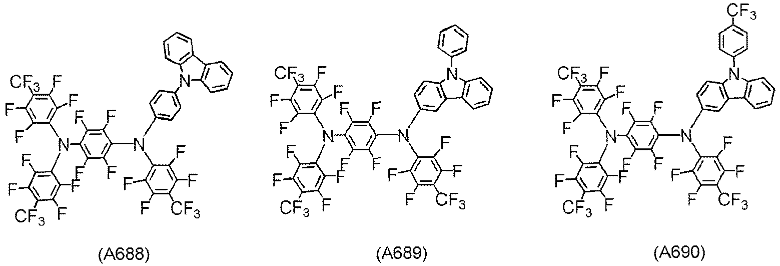

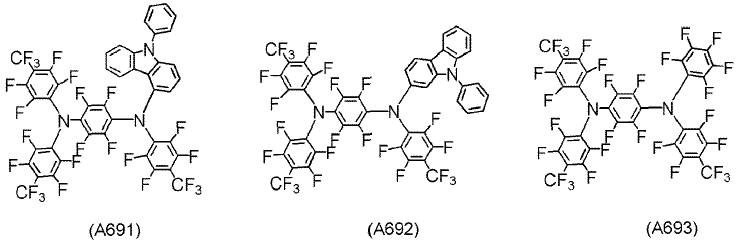

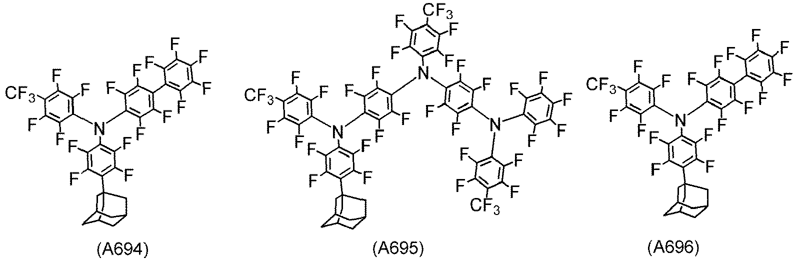

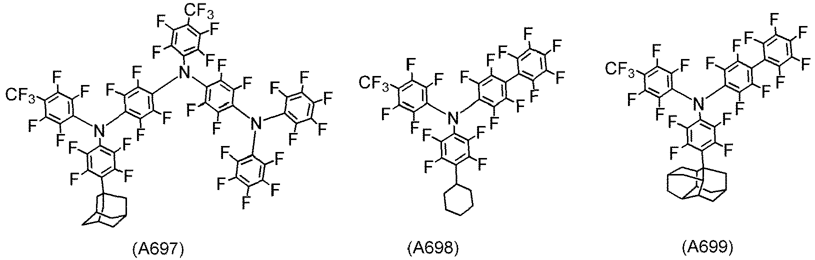

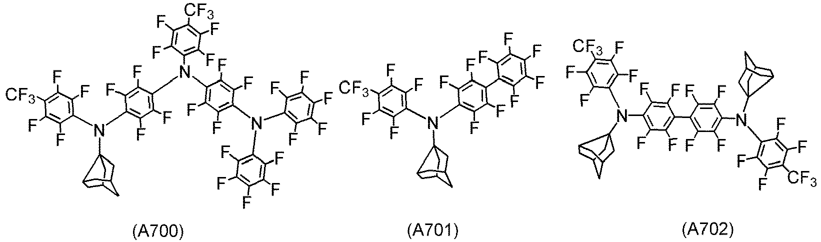

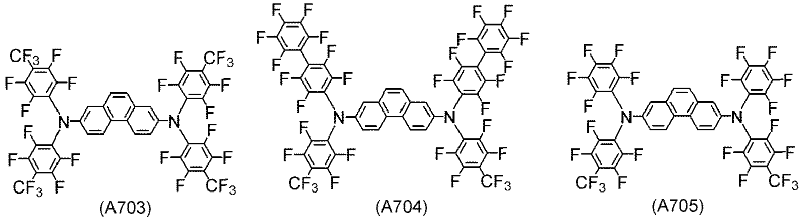

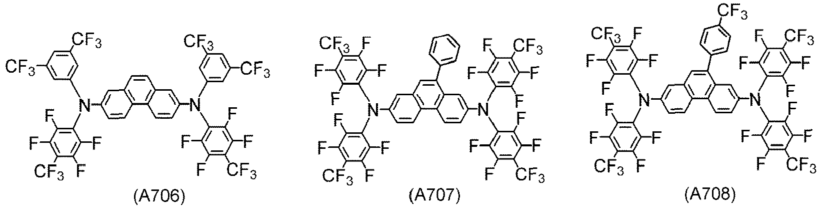

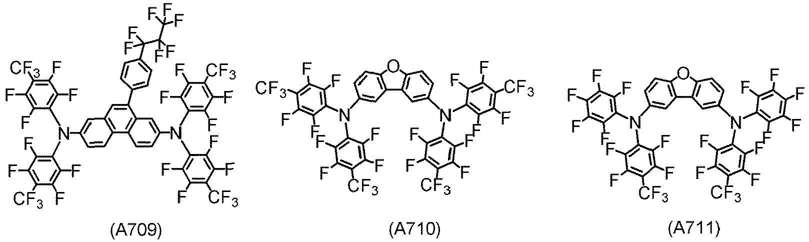

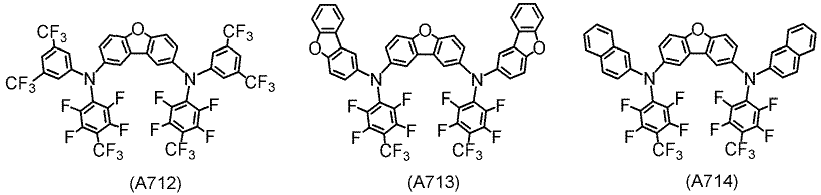

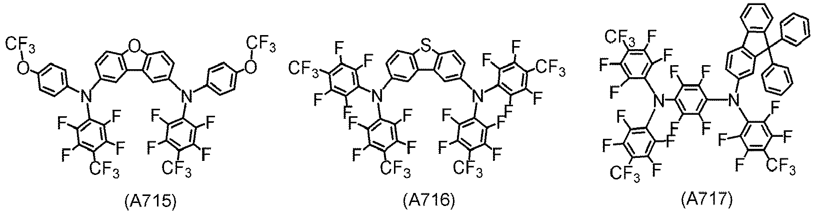

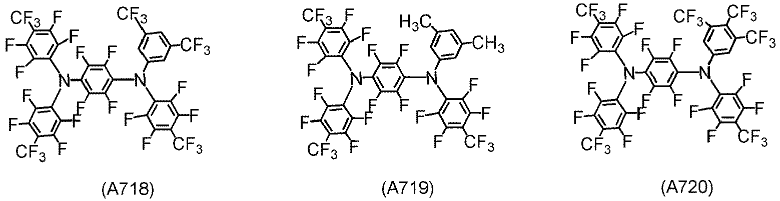

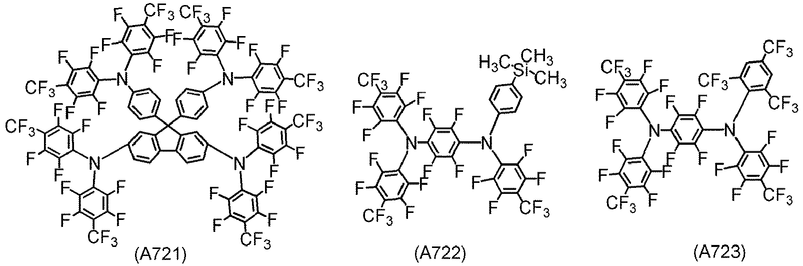

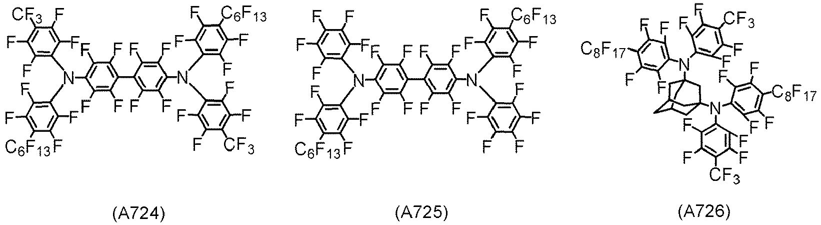

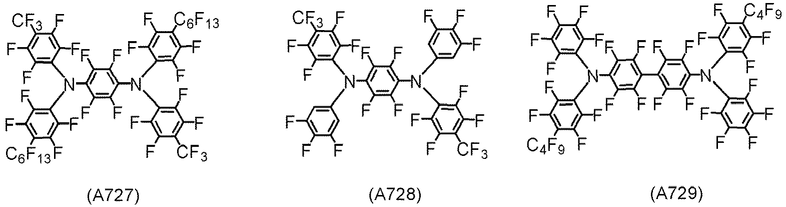

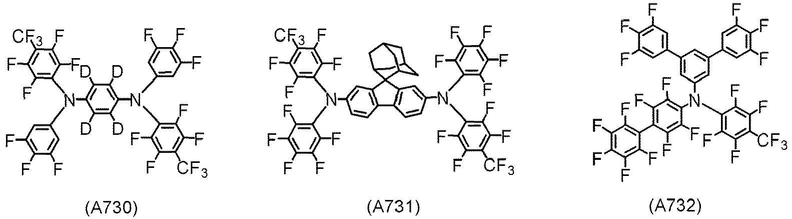

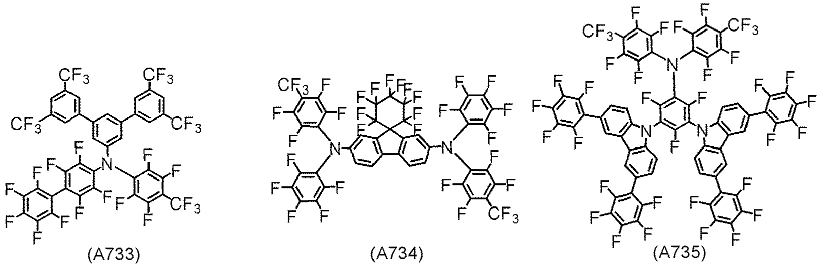

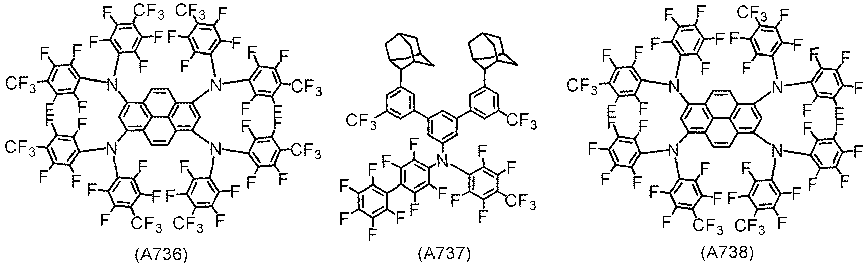

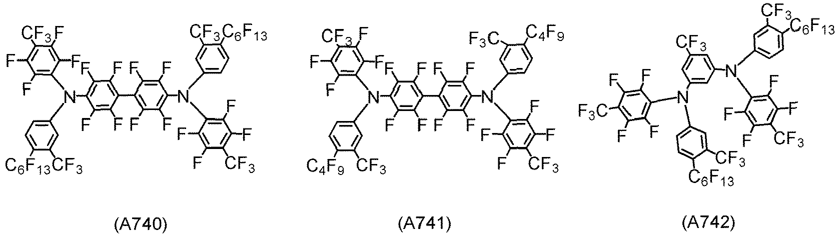

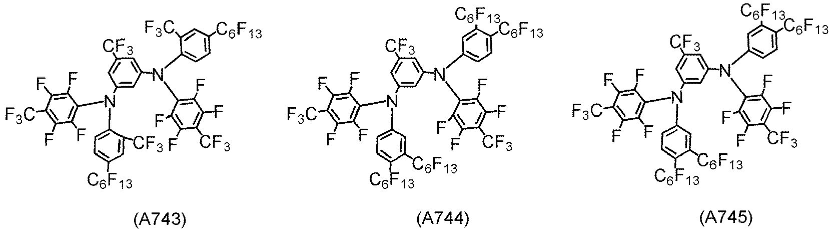

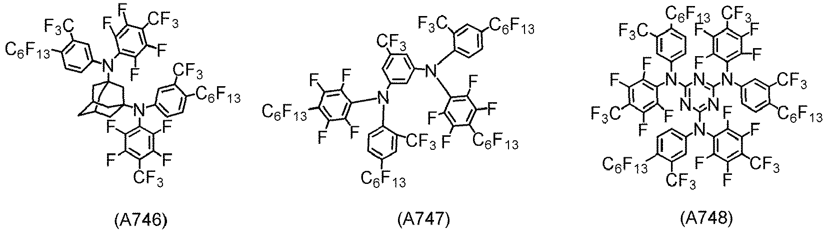

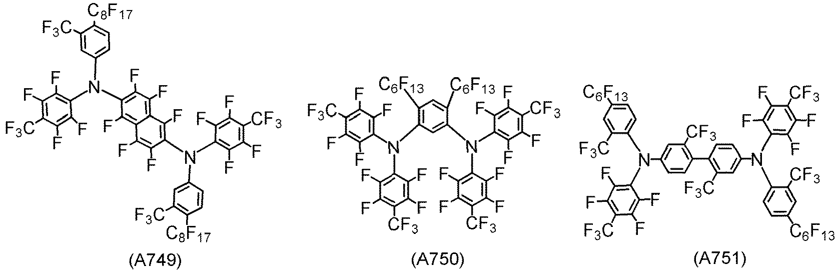

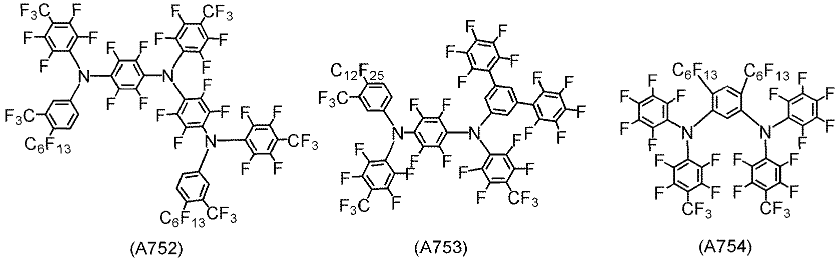

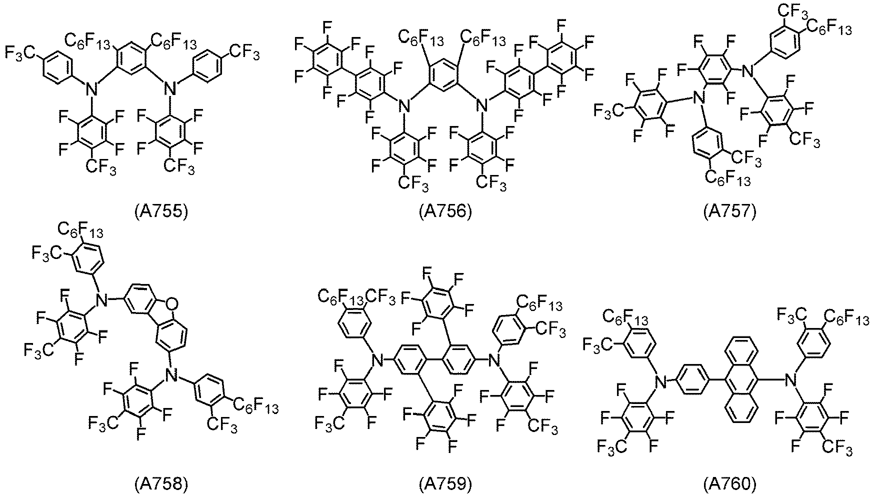

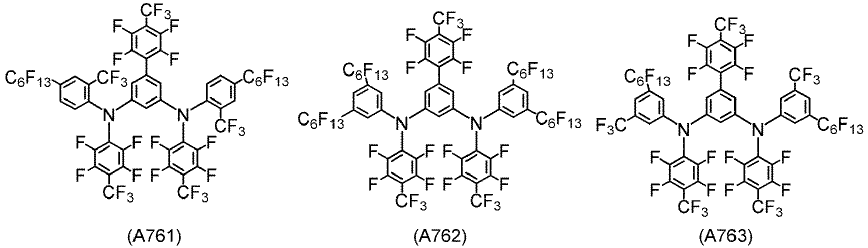

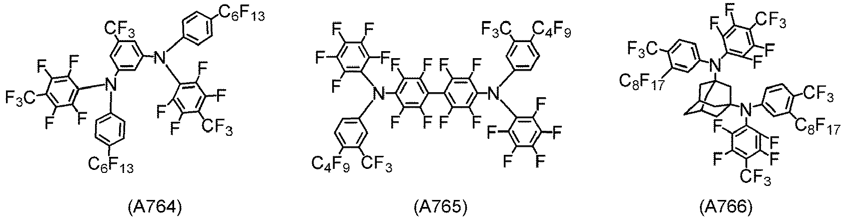

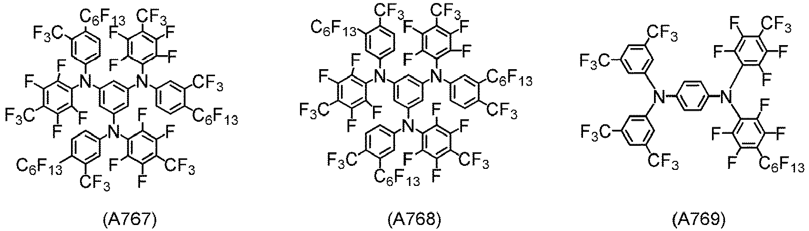

### [Second Embodiment: Metal patterning material without amine substituents]

A metal patterning material according to an embodiment of the present disclosure contains, in the molecule, a compound having an aromatic ring and/or a heteroaromatic ring, and a fluorine atom, in which
the proportion of the number of carbon atoms directly bonded to a fluorine atom among the number of carbon atoms forming the aromatic ring and the number of carbon atoms forming the heteroaromatic ring is 10% or more,
the proportion of the number of fluorine atoms relative to the number of carbon atoms in the molecule is 50% or more,
the molecular weight is 500 to 3000, and
has a glass transition temperature of 60°C or higher. The metal patterning material does not have nitrogen atoms forming amines in the molecule. In other words, the above-mentioned compound can be used as a metal patterning material.

### (Aromatic Ring)

The aromatic ring is preferably a monocyclic aromatic rings, a linked aromatic ring, or a condensed aromatic ring having 6 to 25 carbon atom. The metal patterning material has an aromatic group derived from the aromatic ring. As the aromatic group derived from the aromatic ring, for example, a phenyl group, a biphenylyl group, a terfenylyl group, a naphthyl group, a fluorenyl group, a spirobifluorenyl group, a benzofluorenyl group, a dibenzofluorenyl group, a phenanthryl group, a fluoranthenyl group, a triphenylenyl group, a pyrenyl group, an an anthryl group, a tetracenyl group, a crisenyl group, a perylenyl group, and a pentacenyl group, as well as a group in which one or more groups selected from the group consisting of a benzene, a naphthalene, and phenanthrenes are condensed in these groups can be exemplified.

### (Heteroaromatic Ring)

The heteroaromatic ring is preferably a monocyclic heteroaromatic ring, a linked heteroaromatic ring, or a condensed heteroaromatic ring having 3 to 25 carbon atoms. The metal patterning material has a heteroaromatic group derived from the heteroaromatic ring. As the heteroaromatic group derived from the heteroaromatic ring, for example, a pyrrolyl group, a thienyl group, a furyl group, an imidazolyl group, a thiazolyl group, an isothiazolyl group, an oxazolyl group, an isoxazolyl group, a pyridyl group, a pyrazyl group, a pyrazyl group, an indolyl group, a benzothienyl group, a benzofuranyl group, a benzoimidazolyl group, an indazolyl group, a benzothiazolyl group, a benzoisothiazolyl group, a 2,1,3-benzothiadiazolyl group, a benzoxazolyl group, a benzoisoxazolyl group, a 2,1,3-benzoxadiazolyl group, a quinolyl group, an isoquinolyl group, a carbazolyl group, a dibenzothienyl group, a dibenzofuranyl group, a phenoxazinyl group, a phenothiazinyl group, a phenazinyl group, and a thianthrenyl group, as well as a group in which one or more selected from group consisting of benzene, naphthalene and phenanthrene are condensed in these groups can be exemplified.

### (Content of Fluorine Atom (Part 1))

Due to being able to suppress the formation of the metal film on the film surface, the metal patterning material has a proportion of the number of carbon atoms directly bonded to the fluorine atom to the number of carbon atoms forming the aromatic ring and the number of carbon atoms forming the heteroaromatic ring of 10% or more. The proportion is more preferable in the order of 15% or more, 20% or more, 25% or more, 30% or more, 35% or more, or 40% or more.

### (Content of Fluorine Atom (Part 2))

Due to being able to suppress the formation of the metal film on the film surface, the metal patterning material has a proportion of the number of fluorine atoms to the number of carbon atoms in the molecule of 50% or more. The proportion is more preferable in the order of 55% or more, 60% or more, 65% or more, 70% or more, 75% or more, 80% or more.

### (Glass Transition Temperature)

Due to being able to maintain a stable thin film even under high temperature conditions, the metal patterning material has a glass transition temperature of 60°C or higher. The glass transition temperature is more preferably 65°C or higher, 70°C or higher, 75°C or higher, 80°C or higher, 85°C or higher, 90°C or higher, 95°C or higher, or 100°C or higher.

### (Molecular Weight)

The metal patterning material has a molecular weight of 3000 or less, due to being able to lower the heating temperature in vapor deposition and being able to suppress thermal decomposition of the material in the vapor deposition process for forming the thin film. The molecular weight is more preferably 2800 or less, 2500 or less, 2300 or less, 2100 or less, 2000 or less, 1900 or less, 1800 or less, or 1700 or less. The metal patterning material has a molecular weight of 500 or more. The molecular weight is preferably 500 to 2000.

The metal patterning material is preferably a compound represented by Formula (11).

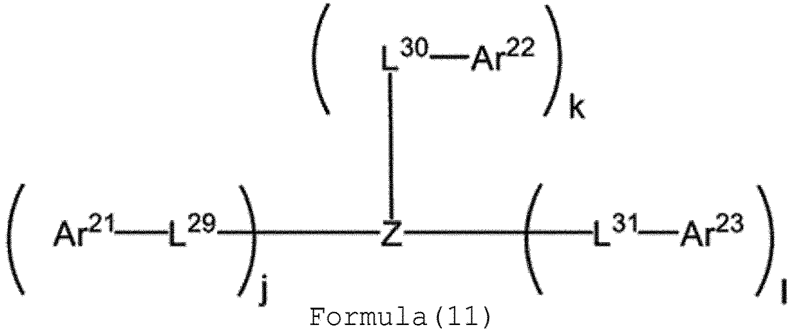

In Formula (11),
Ar²¹, Ar²², and Ar²³ each independently represent
an optionally substituted monocyclic, linked or condensed aromatic hydrocarbon group having 6 to 25 carbon atoms, or an optionally substituted monocyclic, linked, or condensed heteroaromatic groups of 3 to 25 carbon atoms;
L²⁹, L³⁰, and L³¹ each independently represent
an optionally substituted monocyclic, linked, or condensed monocyclic divalent aromatic hydrocarbon group having 6 to 25 carbon atoms,
an optionally substituted monocyclic, linked or condensed divalent heteroaromatic group having 3 to 25 carbon atoms,
an optionally substituted linear, branched or cyclic divalent alkyl group having 1 to 10 carbon atoms, or
a single bond;
j, k and 1 each independently represent an integer of 0 to 6; (j+k+l) is 2 or more and 6 or less;
Z represents
an optionally substituted monocyclic, linked, or condensed aromatic hydrocarbon group having 6 to 25 carbon atoms,
an optionally substituted monocyclic, linked or condensed dito hexavalent heteroaromatic group having 3 to 25 carbon atoms,
an optionally substituted linear, branched or cyclic di- to hexavalent alkyl groups having 1 to 10 carbon atoms, and a di- to tetravalent silicon atom.

When Ar²¹ to Ar²³ are substituted aromatic hydrocarbon groups or substituted heteroaromatic groups, each of these groups is independently substituted with one or more groups selected from the group consisting of:
a linear, branched, or cyclic alkyl group having 1 to 18 carbon atoms which may be substituted with a fluorine atom, a linear, branched, or cyclic alkoxy group having 1 to 18 carbon atoms which may be substituted with a fluorine atom, an aromatic hydrocarbon group having 6 to 20 carbon atoms which may be substituted with a fluorine atom,
a heteroaromatic group having 3 to 20 carbon atoms which may be substituted with a fluorine atom,
a cyano group,
a fluorine atom, or
a deuterium atom;
when L²⁹ to L³¹ are substituted divalent aromatic hydrocarbon groups or substituted divalent heteroaromatic groups, each of these groups preferably is independently substituted with one or more groups selected from the group consisting of:
   a linear, branched, or cyclic alkyl group having 1 to 18 carbon atoms which may be substituted with a fluorine atom;
   a linear, branched, or cyclic alkoxy group having 1 to 18 carbon atoms which may be substituted with a fluorine atom;
   an aromatic hydrocarbon group having 6 to 20 carbon atoms which may be substituted with a fluorine atom;
   a heteroaromatic group having 3 to 20 carbon atoms which may be substituted with a fluorine atom;
   a cyano group,
   a fluorine atom, or
   a deuterium atom;
   when Z is a substituted di- to hexavalent aromatic hydrocarbon group, a substituted di- to hexavalent heteroaromatic group, a substituted di- to hexavalent alkyl group, or a di- to tetravalent silicon atom, each of these groups preferably is independently substituted with one or more groups selected from the group consisting of:
      a linear, branched, or cyclic alkyl group having 1 to 18 carbon atoms which may be substituted with a fluorine atom;
      a linear, branched, or cyclic alkoxy group having 1 to 18 carbon atoms which may be substituted with a fluorine atom;
      an aromatic hydrocarbon group having 6 to 20 carbon atoms which may be substituted with a fluorine atom;
      a heteroaromatic group having 3 to 20 carbon atoms which may be substituted with a fluorine atom;
      a cyano group,
      a fluorine atom, or
      a deuterium atom.

As the monocyclic, linked, or condensed pentavalent aromatic hydrocarbon group having 6 to 25 carbon atoms, for example, a phenylpentayl group, a biphenylpentayl group, a terphenylpentayl group, a naphthylpentayl group, a fluorenepentayl group, a spirobifluorenepentayl group, a benzofluorenepile group, a dibenzofluorenepentayl group, a phenanthrenepentayl group, a fluoranthenepentayl group, a triphenylenepentayl group, a pyrenepentayl group, an anthracene pentayl group, a tetracene pentayl group, a chrysene pentayl group, a perylene pentayl group, and a pentacene pentayl group, as well as a group in which one or more rings selected from the group consisting of benzene, naphthalene, and phenanthrene are condensed in these groups can be exemplified.

As the monocyclic, linked, or condensed pentavalent heteroaromatic group having 3 to 25 carbon atoms, for example, a pyridine pentayl group, an indole pentayl group, a benzothiophene pentayl group, a benzofuran pentayl group, a benzimidazole pentayl group, an indazole pentayl group, a benzothiazole pentayl group, a benzoisothiazole pentayl group, a benzoxazole pentayl group, a benzoisoxazole pentayl group, a benzoisoxazole pentayl group, a quinoline pentayl group, an isoquinoline pentayl group, a carbazole pentayl group, a dibenzothiophene pentayl group, a dibenzofuran pentayl group, a phenoxazine pentayl group, a phenothiazine pentayl group, a phenazine pentayl group, and a thianthrene pentayl group, as well as a group in which one or more selected from the group consisting of benzene, naphthalene, and phenanthrene are condensed in these groups can be exemplified.

As the linear, branched, or cyclic pentavalent alkyl group having 1 to 10 carbon atoms, for example, an ethyl pentayl group, a propane pentayl group, a butane pentayl group, a pentane pentayl group, a hexane pentayl group, a cyclohexane pentayl group, and an adamantane pentayl group can be exemplified.

As the monocyclic, linked, or condensed hexavalent aromatic hydrocarbon group having 6 to 25 carbon atoms, for example, a phenylhexayl group, a biphenylhexayl group, a terphenylhexayl group, a naphthylhexayl group, a fluorenehexayl group, a spirobifluorenehexayl group, a benzofluorenehexayl group, a dibenzofluorenehexayl group, a phenanthrenehexayl group, a fluoranthenehexayl group, a triphenylenehexayl group, a pyrenehexayl group, an anthracenehexayl group, a tetracenehexayl group, a chrysenehexayl group, a perylenehexayl group, a pentacenehexayl group, and a group in which one or more selected from the group consisting of benzene, naphthalene, and phenanthrene are condensed in these groups can be exemplified.

As the monocyclic, linked, or condensed heterocyclic heteroaromatic group having 3 to 25 carbon atoms, for example, a pyridinehexayl group, an indolehexayl group, a benzothiophenehexayl group, a benzofuranhexayl group, a benzoimidazolehexayl group, an indazolehexayl group, a benzoisothiazolehexayl group, a benzooxazolehexayl group, a benzoxazolehexayl group, a benzoisoxazolehexayl group, a quinolinehexayl group, an isoquinolinehexayl group, a carbazolehexayl group, a dibenzothiophenehexayl group, a dibenzofuranhexayl group, a phenoxazinehexayl group, a phenothiazinehexayl group, a phenazinehexayl group, and a thianthrenehexayl group, as well as a group in which one or more groups selected from the group consisting of benzene, naphthalene, and phenanthrene are condensed in these groups can be exemplified.

As the linear, branched or cyclic hexavalent alkyl group having 1 to 10 carbon atoms, for example, an ethylhexayl group, a propanehexayl group, a butanehexayl group, a pentanehexayl group, a hexanehexayl group, a cyclohexanehexayl group, and an adamantanehexayl group can be exemplified.

It should be noted that, as described above, the monocyclic, linked or condensed penta- to hexavalent aromatic hydrocarbon group having 6 to 25 carbon atoms; the monocyclic, linked, or condensed penta- to hexavalent heteroaromatic group having 3 to 25 carbon atoms; and the linear, branched, or cyclic penta- to hexavalent alkyl group having 1 to 10 carbon atoms may have a substituent. When these have a substituent, each is preferably independently substituted by one or more groups selected from the group consisting of: a linear, branched, or cyclic alkyl group having 1 to 18 carbon atoms which may be substituted with a fluorine atom, a linear, branched, or cyclic alkoxy group having 1 to 18 carbon atoms which may be substituted with a fluorine atom, an aromatic hydrocarbon group having 6 to 20 carbon atoms which may be substituted with a fluorine atom, a heteroaromatic group having 3 to 20 carbon atoms which may be substituted with a fluorine atom, a cyano group, a fluorine atom, or a deuterium atom. In this case, the number of substituents is not particularly limited.

In Formula (11), the monocyclic, linked, or condensed aromatic hydrocarbon group having 6 to 25 carbon atoms; monocyclic, linked, or condensed heteroaromatic group having 3 to 25 carbon atoms; monocyclic, linked, or condensed aromatic hydrocarbon group having 6 to 25 carbon atoms; a monocyclic, linked or condensed divalent heteroaromatic group having 3 to 25 carbon atoms; linear, branched or cyclic divalent alkyl group having 1 to 10 carbon atoms; monocyclic, linked, or condensed trivalent aromatic hydrocarbon group having 6 to 25 carbon atoms; monocyclic, linked or condensed trivalent heteroaromatic group having 3 to 25 carbon atoms; linear, branched or cyclic trivalent alkyl group having 1 to 10 carbon atoms; monocyclic, linked, or condensed tetravalent aromatic hydrocarbon group having 6 to 25 carbon atoms; monocyclic, linked or condensed tetravalent heteroaromatic group having 3 to 25 carbon atoms; linear, branched or cyclic tetravalent alkyl groups having 1 to 10 carbon atoms; linear, branched, or cyclic alkyl group having 1 to 18 carbon atoms which may be substituted with a fluorine atom; linear, branched, or cyclic alkoxy group having 1 to 18 carbon atoms which may be substituted with a fluorine atom; aromatic hydrocarbon group having 6 to 20 carbon atoms which may be substituted with a fluorine atom; heteroaromatic group having 3 to 20 carbon atoms which may be substituted with a fluorine atom; aromatic hydrocarbon group having 6 to 25 carbon atoms; monocyclic, linked, or condensed heteroaromatic group having 3 to 25 carbon atoms; monocyclic, linked, or condensed aromatic hydrocarbon group having 6 to 25 carbon atoms; monocyclic, linked or condensed divalent heteroaromatic group having 3 to 25 carbon atoms; linear, branched or cyclic divalent alkyl group having 1 to 10 carbon atoms; monocyclic, linked, or condensed trivalent aromatic hydrocarbon group having 6 to 25 carbon atoms; monocyclic, linked or condensed trivalent heteroaromatic group having 3 to 25 carbon atoms; linear, branched or cyclic trivalent alkyl group having 1 to 10 carbon atoms; monocyclic, linked, or condensed tetravalent aromatic hydrocarbon group having 6 to 25 carbon atoms; monocyclic, linked or condensed tetravalent heteroaromatic group having 3 to 25 carbon atoms; linear, branched or cyclic tetravalent alkyl groups having 1 to 10 carbon atoms; linear, branched, or cyclic alkyl group having 1 to 18 carbon atoms which may be substituted with a fluorine atom; linear, branched, or cyclic alkoxy group having 1 to 18 carbon atoms which may be substituted with a fluorine atom; aromatic hydrocarbon group having 6 to 20 carbon atoms which may be substituted with a fluorine atom; heteroaromatic group having 3 to 20 carbon atoms which may be substituted with a fluorine atom are synonymous. It should be noted that, due to being able to suppress the formation of the metal film on the surface of the film, the number of carbon atoms of the condensed aromatic hydrocarbon group in Formula (11) is preferably 6 or more and 15 or less. Specifically, it is preferably from 6 to 15, more preferably from 6 to 14, and still more preferably from 6 to 13.

In Formula (11), due to having a high glass transition temperature and being able to suppress the formation of a metal film on the film surface, Ar²¹ to Ar²³ preferably each independently represent:
(xxxi) a phenyl group, a biphenylyl group, a terphenylyl group, a naphthyl group, a fluorenyl group, a spirobifluorenyl group, a benzofluorenyl group, a phenanthryl group, a fluoranthenyl group, a triphenylenyl group, an anthryl group, a pyrenyl group, a pyridyl group, a carbazolyl group, a benzofuranyl group, a benzothienyl group, a dibenzofuranyl group, or a dibenzothienyl group; or
(xxxii) a group in which the group represented by (xxxi) is substituted with one or more groups selected from the group consisting of: a methyl group, an ethyl group, a methoxy group, an ethoxy group, a trifluoromethyl group, a trifluoromethoxy group, an adamantyl group, a cyclohexyl group, a perfluoroalkyl group having 2 to 10 carbon atoms, a perfluoroalkoxy group having 2 to 10 carbon atoms, a cyano group, a deuterium atom, a phenyl group optionally substituted with a fluorine atom, a biphenyl group optionally substituted with a fluorine atom, a naphthyl group optionally substituted with a fluorine atom, a phenanthryl group which may be substituted with a fluorine atom, a pyridyl group which may be substituted with a fluorine atom, a carbazolyl group which may be substituted with a fluorine atom, a dibenzothienyl group which may be substituted with a fluorine atom, and a dibenzofuranyl group which may be substituted with a fluorine atom.

Due to having a high glass transition temperature and being able to suppress the formation of a metal film on the film surface, Ar²¹ to Ar²³ more preferably each independently represent:
(xxxi') a phenyl group, a biphenylyl group, a terphenylyl group, a naphthyl group, a fluorenyl group, a spirobifluorenyl group, a phenanthryl group, a fluoranthenyl group, a triphenylenyl group, an anthryl group, a pyrenyl group, a pyridyl group, a carbazolyl group, a dibenzofuranyl group, or a dibenzothienyl group; or
(xxxii') a group in which the group represented by (xxxi') is substituted with one or more groups selected from the group consisting of a methyl group, a trifluoromethyl group, a perfluoroalkyl group having 2 to 10 carbon atoms, a fluorine atom, and a phenyl group optionally substituted with a fluorine atom.

Due to having a high glass transition temperature and being able to suppress the formation of a metal film on the film surface, Ar²¹ to Ar²³ still more preferably each independently represent: a phenyl group, a phenyl group having at least one fluorine atom, a phenyl group having at least one trifluoromethyl group, a biphenylyl group, a biphenylyl group having at least one fluorine atom, a biphenylyl group having at least one trifluoromethyl group, a terphenylyl group, a terfenylyl group having at least one fluorine atom, a terphenylyl group having at least one trifluoromethyl group, a naphthyl group, a 9,9-dimethylfluorenyl group, a 9,9-diphenylfluorenyl group, a spirobiflurenyl group, a phenanthryl group, a carbazol-9-yl group, a 9-phenylcarbazolyl group, a dibenzofuranyl group, or a dibenzothienyl group.

In Formula (11), due to having a high glass transition temperature and being able to suppress the formation of a metal film on the film surface, L²⁹ to L³¹ preferably each independently represent:
(xxxiii) a phenylene group, a biphenylylene group, a terphenylene group, a naphthylene group, a pyridylene group, or a fluorenylene group;
(xxxix) a group in which the group represented by (xxxiii) is substituted with one or more groups selected from the group consisting of: a methyl group, an ethyl group, a methoxy group, an ethoxy group, a trifluoromethyl group, a trifluoromethoxy group, a perfluoroalkyl group having 2 to 10 carbon atoms, a perfluoroalkoxy group having 2 to 10 carbon atoms, a cyano group, a deuterium atom, a fluorine atom, a phenyl group optionally substituted with a fluorine atom, a biphenylyl group optionally substituted with a fluorine atom, a naphthyl group optionally substituted with a fluorine atom, a phenanthryl group optionally substituted with a fluorine atom, a pyridyl group optionally substituted with a fluorine atom, a carbazolyl group optionally substituted with a fluorine atom, a dibenzothienyl group optionally substituted with a fluorine atom, and a dibenzofuranyl group optionally substituted with a fluorine atom; or
(xxxv) a single bond.

In Formula (11), due to having a high glass transition temperature and being able to suppress the formation of a metal film on the film surface, L²⁹ to L³¹ more preferably each independently represent:
(xxxiii') a phenylene group, a biphenylylene group, a terphenylylene group, a naphthylene group, a pyridylene group, or a fluorenylene group;
(xxxiv') a group in which the group represented by the above (xxxiii') is substituted with one or more groups selected from the group consisting of a methyl group, a trifluoromethyl group, a fluorine atom and a phenyl group optionally substituted with a fluorine atom; or
(xxxv') a single bond.

In Formula (11), due to having a high glass transition temperature and being able to suppress the formation of a metal film on the film surface, L²⁹ to L³¹ still more preferably each independently represent: a phenylene group, a phenylene group having at least one fluorine atom, a phenylene group having at least one trifluoromethyl group, a biphenylylene group, a biphenylylene group having at least one fluorine atom, a biphenylylene group having at least one trifluoromethyl group, a terphenylylene group, a terphenylylene group having at least one fluorine atom, a terphenylylene group having at least one trifluoromethyl group, a naphthylene group, or a single bond.

In Formula (11), due to having a high glass transition temperature and being able to suppress the formation of a metal film on the film surface, Z preferably represents:
(xxxvi) a monovalent phenyl group, a biphenylyl group, a terphenylyl group, naphthyl group, a fluorenyl group, a spirobifluorenyl group, a benzofluorenyl group, a phenanthryl group, a fluoranthenyl group, a triphenylenyl group, a anthryl group, a pyrenyl group, a pyridyl group, a pyridyl group, a carbazolyl group, a benzofuranyl group, a benzothienyl group, a dibenzofuranyl group, a dibenzothienyl group, a cyclohexyl group, an adamantyl group, a methyl group, or a silyl group;
(xxxvii) a divalent phenylene group, a biphenylene group, a terphenylene group, a naphthylene group, a fluorenylene group, a spirobifluorenylene group, a benzofluorenylene group, a phenanetriene group, a fluoranthenylene group, a triphenylenylene group, an anthrylene group, a pyrenylene group, a pyridylene group, a carbazolylene group, a benzofuranylene group, a benzothienylene group, a dibenzofuranylene group, a dibenzothienylene group, a cyclohexylene group, an adamantylene group, a methylene group, or a silanediyl group;
(xxxviii) a trivalent phenyltriyl group, a biphenyltriyl group, a terphenyltriyl group, a naphthylyl group, a fluorenetriyl group, a spirobiflurenetriyl group, a benzofluorenetriyl group, a phenanthrenetriyl group, a fluoranthenetriyl group, a triphenylenetriyl group, an anthracenetriyl group, a pyrenetriyl group, a pyridinetriyl group, a carbazoletriyl group, a benzofurantriyl group, a benzothiophenetriyl group, a dibenzofurantriyl group, a dibenzothiophenetriyl group, a cyclohexanetriyl group, an adamantanetriyl group, a methanetriyl group, or a silanetriyl group;
(xxxiv) a tetravalent phenyl tetrayl group, a biphenyl tetrayl group, a terphenyl tetrayl group, a naphthyl tetrayl group, a fluorene tetrayl group, a spirobifluorene tetrayl group, a benzofluorene tetrayl group, a phenanthrene tetrayl group, a fluoranthene tetrayl group, a triphenylene tetrayl group, an anthracene tetrayl group, a pyrene tetrayl group, a pyridine tetrayl group, a carbazole tetrayl group, a benzofuran tetrayl group, a benzothiophene tetrayl group, a dibenzofuran tetrayl group, a dibenzothiophene tetrayl group, a cyclohexane tetrayl group, an adamantane tetrayl group, a methane tetrayl group, or a silane tetrayl group; or
(xxxx) a pentavalent phenylpentayl group, a biphenyl pentayl group, a terphenyl pentayl group, a naphthyl pentayl group, a fluorene pentayl group, a spirobifluorene pentayl group, a benzofluorene pentayl group, a phenanthrene pentayl group, a fluoranthene pentayl group, a triphenylene pentayl group, an anthracene pentayl group, a pyrene pentayl group, a pyridine pentayl group, a carbazole pentayl group, a benzofuran pentayl group, a benzothiophenepentayl group, a dibenzofuranepentayl group, a dibenzothiophenepentayl group, a cyclohexanepentayl group, or an adamantanepentayl group; or
(xxxxi) a hexavalent phenylhexayl group, a biphenylhexayl group, a terphenylhexayl group, a naphthylhexayl group, a fluorenehexayl group, a spirobifluorenehexayl group, a benzofluorenehexayl group, a phenanthrenehexayl group, a fluoranthenehexayl group, a triphenylenehexayl group, an anthracenehexayl group, a pyrenehexayl group, a pyridinehexayl group, a carbazolehexayl group, a benzofuranhexayl group, a benzothiophenehexayl group, a dibenzofuranhexayl group, a dibenzothiophenehexayl group, a cyclohexanehexayl group, or an adamantanehexayl group; or
(xxxxii) a group in which the group represented by (xxxvi) to (xxxxi) is substituted with one or more groups selected from the group consisting of: a methyl group, an ethyl group, a methoxy group, an ethoxy group, a trifluoromethyl group, a trifluoromethoxy group, an adamantyl group, a cyclohexyl group, a perfluoroalkyl group having 2 to 10 carbon atoms, a perfluoroalkoxy group having 2 to 10 carbon atoms, a cyano group, a deuterium atom, a fluorine atom, a phenyl group optionally substituted with a fluorine atom, a biphenyl group optionally substituted with a fluorine atom, a naphthyl group which may be substituted with a fluorine atom, a phenanthryl group which may be substituted with a fluorine atom, a pyridyl group which may be substituted with a fluorine atom, a carbazolyl group which may be substituted with a fluorine atom, a dibenzothienyl group which may be substituted with a fluorine atom, and a dibenzofuranyl group which may be substituted with a fluorine atom.

It more preferably represents: (xxxvi') a monovalent phenyl group, a biphenylyl group, a terphenylyl group, a naphthyl group, a fluorenyl group, a pyridyl group, a cyclohexyl group, an adamantyl group, a methyl group, or a silyl group;
(xxxvii') a divalent phenylene group, a biphenylene group, a terphenylene group, a naphthylene group, a fluorenylene group, a pyridylene group, a cyclohexylene group, an adamantylene group, a methylene group, or a silanediyl group;
(xxxviiii') a trivalent phenyltriyl group, a biphenyltriyl group, a terphenyltriyl group, a naphthyltriyl group, a fluorenetriyl group, a pyridinetriyl group, a cyclohexanetriyl group, an adamantanetriyl group, a methanetriyl group, or a silanetriyl group;
(xxxix') a tetravalent phenyl tetrayl group, a biphenyl tetrayl group, a terphenyl tetrayl group, a naphthyl tetrayl group, a fluorene tetrayl group, a pyridine tetrayl group, a cyclohexane tetrayl group, an adamantane tetrayl group, a methane tetrayl group, or a silane tetrayl group; or
(xxxx') a pentavalent phenylpentayl group, a biphenylpentayl group, a terphenylpentayl group, a naphthylpentayl group, a fluorenepentayl group, a pyridinepentayl group, a cyclohexanepentayl group, or a adamantanepentayl group; or (xxxxi') a hexavalent phenylhexayl group, a biphenylhexayl group, a terphenylhexayl group, a naphthylhexayl group, a fluorenehexayl group, a cyclohexanehexayl group, or an adamantanehexayl group; or
(xxxxii') a group in which the group represented by any one of (xxxvi') to (xxxxii') is substituted with one or more groups selected from the group consisting of a methyl group, a trifluoromethyl group, a perfluoroalkyl group having 2 to 10 carbon atoms, a fluorine atom, and a phenyl group optionally substituted with a fluorine atom.

In Formula (11), due to having a high glass transition temperature and being able to suppress the formation of a metal film on the film surface, Z still more preferably represents:
a phenylene group, a phenylene group having at least one fluorine atom, a phenylene group having at least one trifluoromethyl group, a phenyltriyl group, a phenyltriyl group having at least one fluorine atom, a phenyltriyl group having at least one trifluoromethyl group, a phenyl tetrayl group, a phenyl tetrayl group having at least one fluorine atom, a phenyl tetrayl group having at least one trifluoromethyl group, a phenyltopentayl group, a phenylpentayl group having at least one fluorine atom, a phenylpentayl group having at least one trifluoromethyl group, a phenylhexayl group, phenylhexayl group having at least one fluorine atom, a phenylhexayl group having at least one trifluoromethyl group, a biphenylylene group, a biphenylylene group having at least one fluorine atom, a biphenyltriyl group having at least one trifluoromethyl group, a biphenylylene group having at least one methyl group, a biphenyltriyl group, a biphenyltriyl group having at least one fluorine atom, a biphenyltriyl group having at least one trifluoromethyl group, a biphenyltriyl group having at least one methyl group, a biphenyl tetrayl group, a biphenyl tetrayl group having at least one fluorine atom, a biphenyl tetrayl group having at least one trifluoromethyl group, a biphenyl tetrayl group having at least one methyl group, a biphenyl pentayl group, a biphenyl pentayl group having at least one fluorine atom, a biphenyl pentayl group having at least one trifluoromethyl group, a biphenyl pentayl group having at least one methyl group, a biphenylhexayl group, a biphenylhexayl group having at least one fluorine atom, a biphenylhexayl group having at least one trifluoromethyl group, a biphenylhexayl group having at least one methyl group, a terphenylylene group, a terphenylylene group having at least one fluorine atom, a terphenylylene group having at least one trifluoromethyl group, a terphenyltriyl group, a terphenyltriyl group having at least one fluorine atom, and a terphenyltriyl group having at least one trifluoromethyl group, a terphenyl tetrayl group, a terphenyl tetrayl group having at least one fluorine atom, a terphenyl tetrayl group having at least one trifluoromethyl group, a terphenyl pentayl group, a terphenyl pentayl group having at least one fluorine atom, a terphenyl pentayl group having at least one trifluoromethyl group, a terphenylhexayl group, a terphenylhexayl group having at least one fluorine atom, a terphenylhexayl group having at least one trifluoromethyl group, a naphthylene group, a naphthylyl group, a naphthyltetrayl group, a naphthylpentayl group, a naphthylhexayl group, a pyridinediyl group, a pyridinediyl group having at least one fluorine atom, a pyridinediyl group having at least one trifluoromethyl group, a pyridinetriyl group, a pyridinetriyl group having at least one fluorine atom, a pyridinetriyl group having at least one trifluoromethyl group, a pyridine tetrayl group, a pyridine tetrayl group having at least one fluorine atom, a pyridine tetrayl group having at least one trifluoromethyl group, a pyridine pentayl group, a pyridine pentayl group having at least one fluorine atom, a pyridine pentayl group having at least one trifluoromethyl group, a pyridine pentayl group having at least one trifluoromethyl group, a pyridine hexayl group, a pyridinehexayl group having at least one fluorine atom, a pyridinehexayl group having at least one trifluoromethyl group, a fluorene-9,9-diyl group, a cyclohexane -1,1-diyl group, an adamantyl group, an adamantane -2,2-diyl group, an adamantane -1,3-diyl group, an adamantane -1,3,5-triyl group, or an adamantane -1,3,5,7-tetrayl group.

Due to being able to suppress the formation of a metal film on the surface of the film, the metal patterning material preferably has one or more groups selected from the group consisting of a perfluorophenyl group, a perfluorotolyl group, a perfluorodimethylphenyl group, and a perfluorobiphenylyl group in the molecule.

Due to being able to suppress the formation of a metal film on the surface of the film, the metal patterning material preferably has a proportion of the number of fluorine atoms to carbon atoms in the molecular structure of the metal patterning material larger than 1:4, more preferably larger than 1:3, and still more preferably larger than 1:2. From the viewpoint of the glass transition temperature and the sublimation temperature, the proportion is more preferably in the order of from 1:2 to 2 :1, from 1:2 to 1.9:1, from 1:2 to 1.8:1, from 1:2 to 1.7:1, from 1:2 to 1.6:1, and from 1:2 to 1.5:1.

### [Specific Examples of Metal Patterning Materials]

The metal patterning material according to one embodiment of the present disclosure is exemplified by compounds (B1) to (B114) below; however, the present disclosure is not to be limited to these compounds.

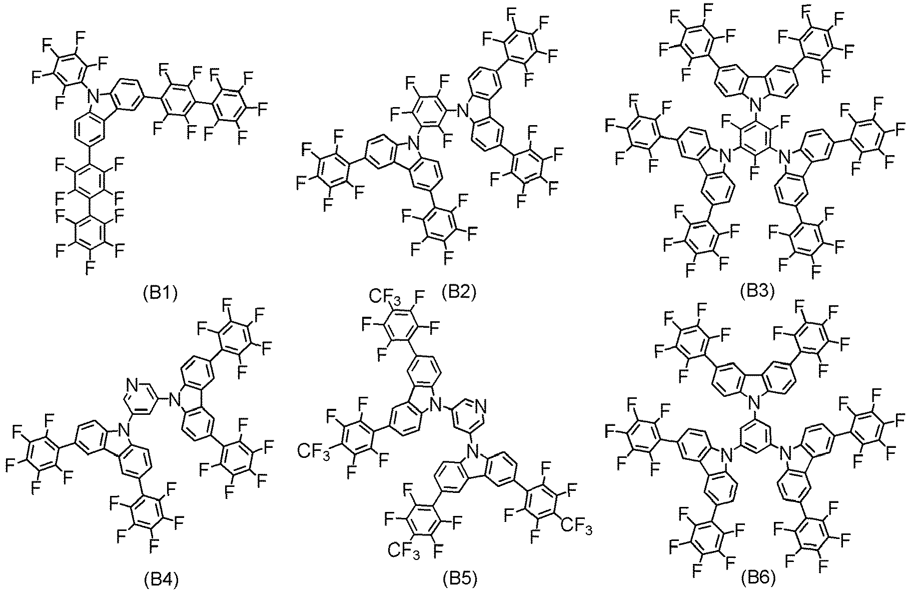

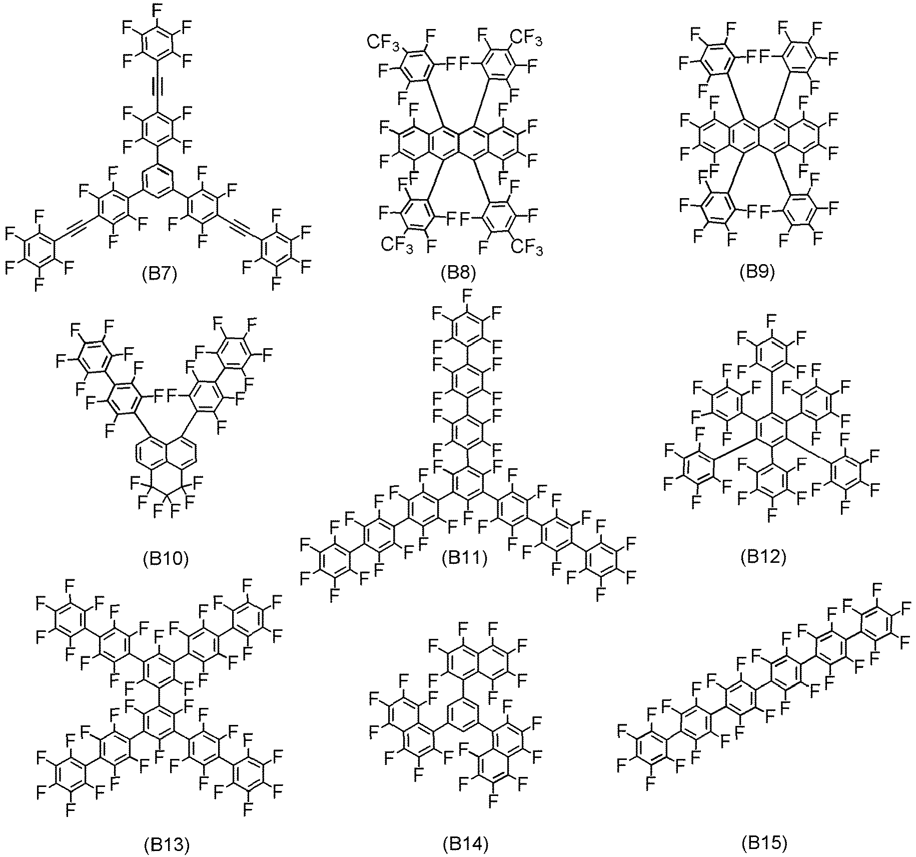

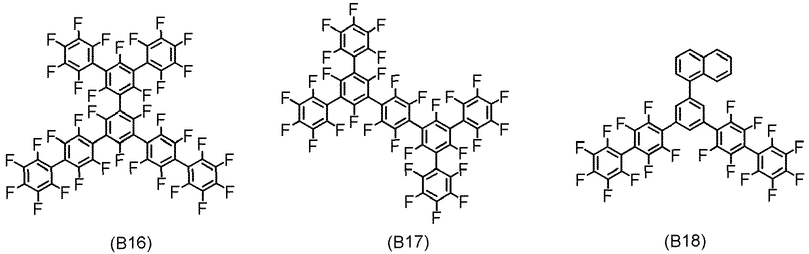

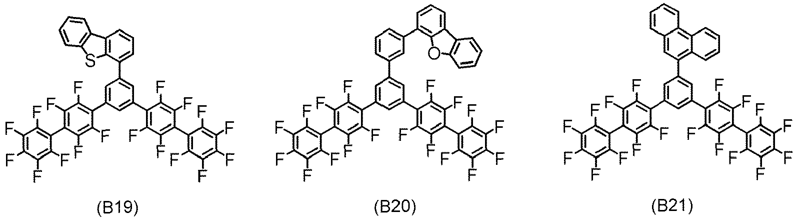

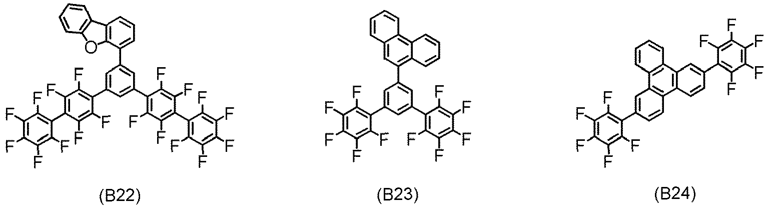

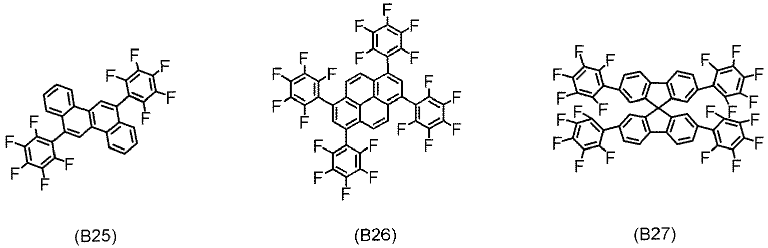

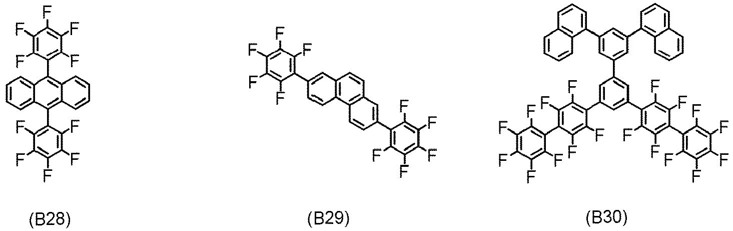

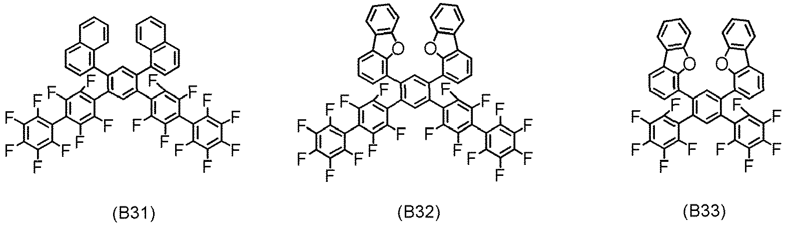

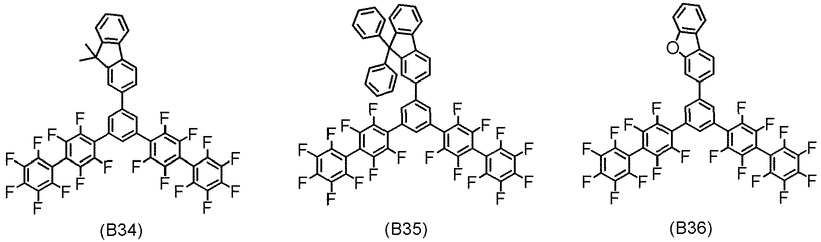

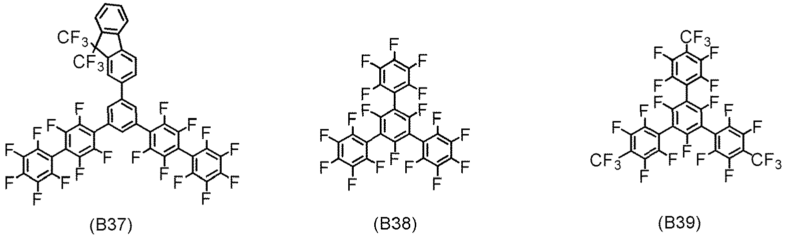

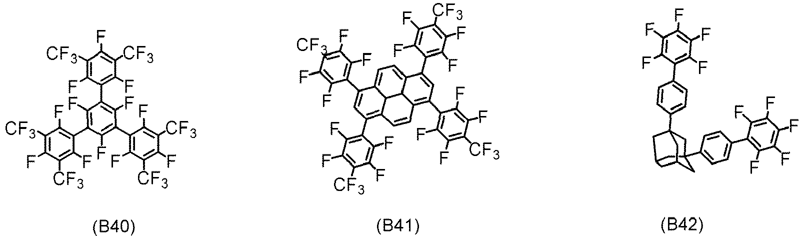

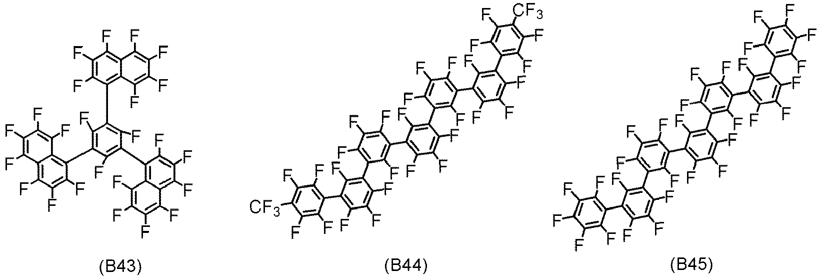

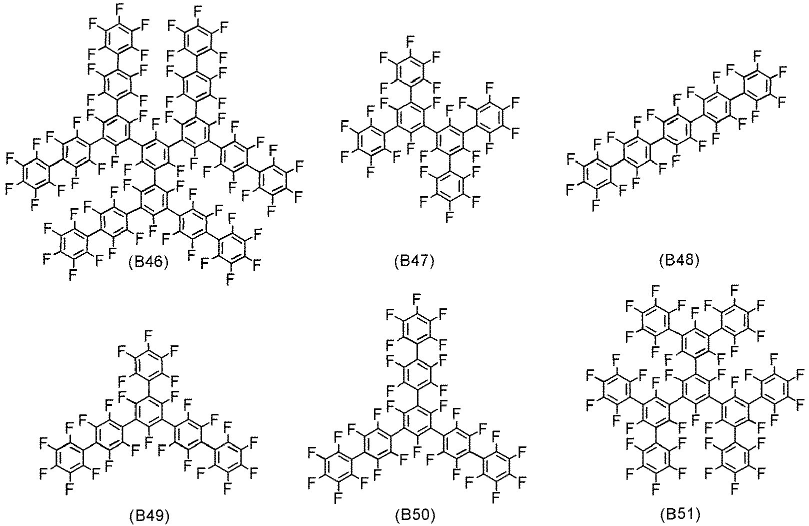

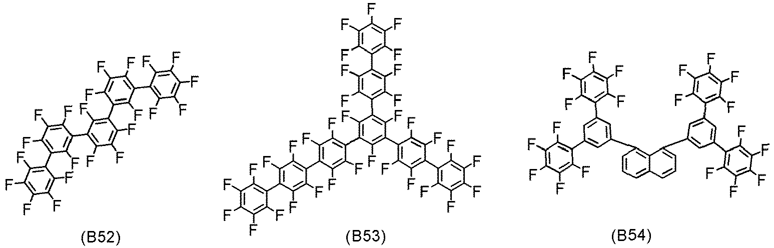

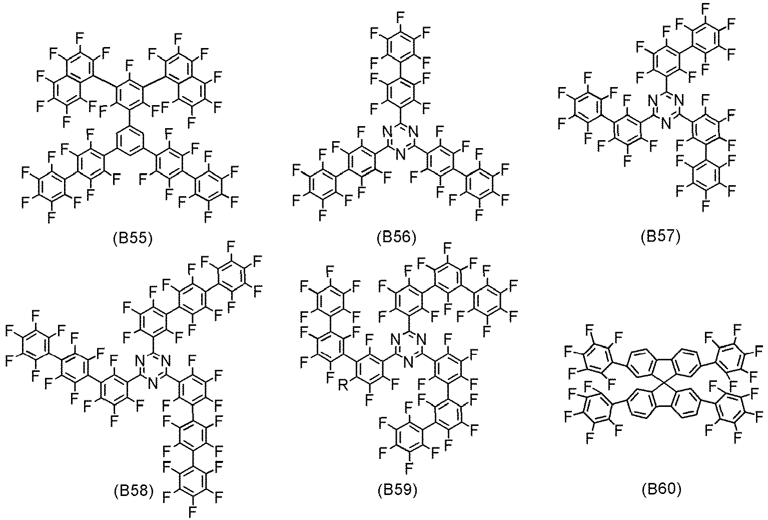

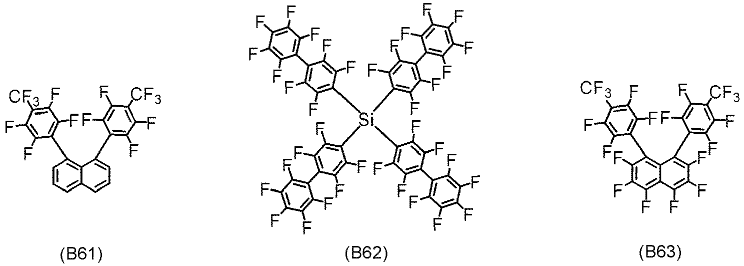

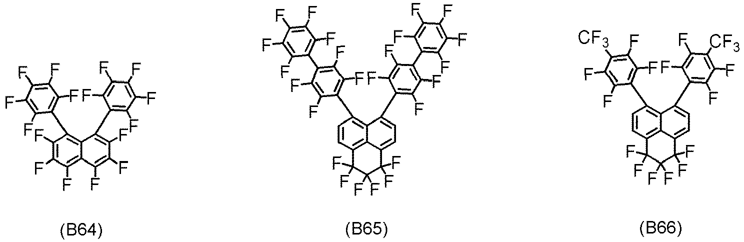

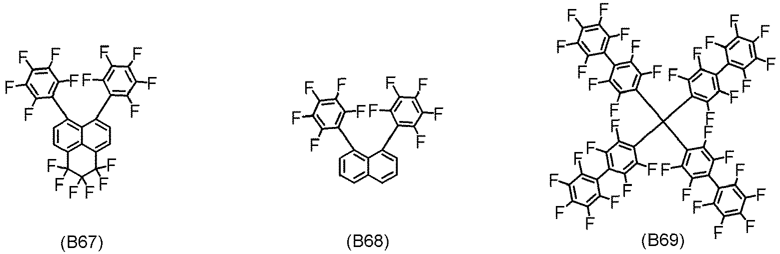

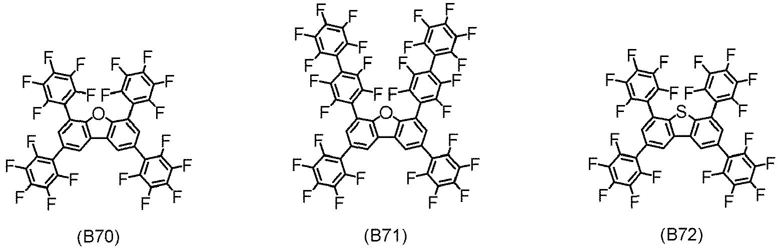

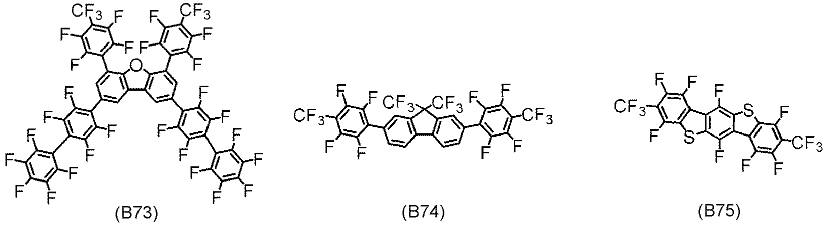

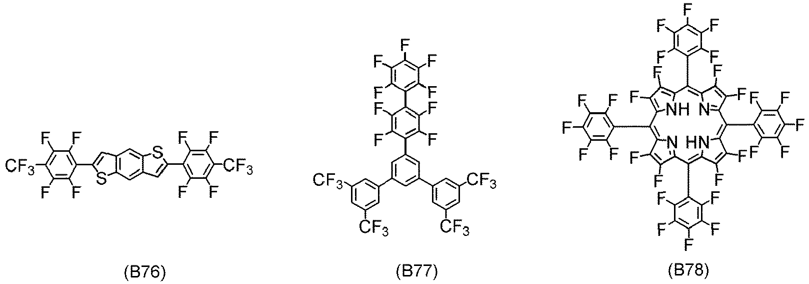

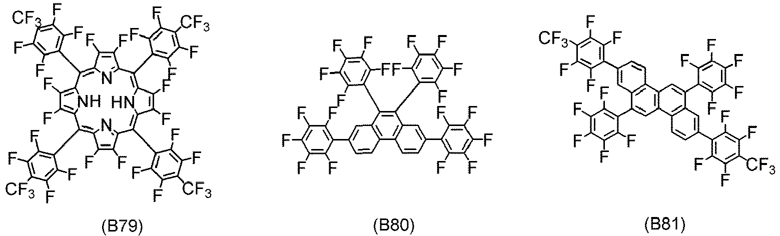

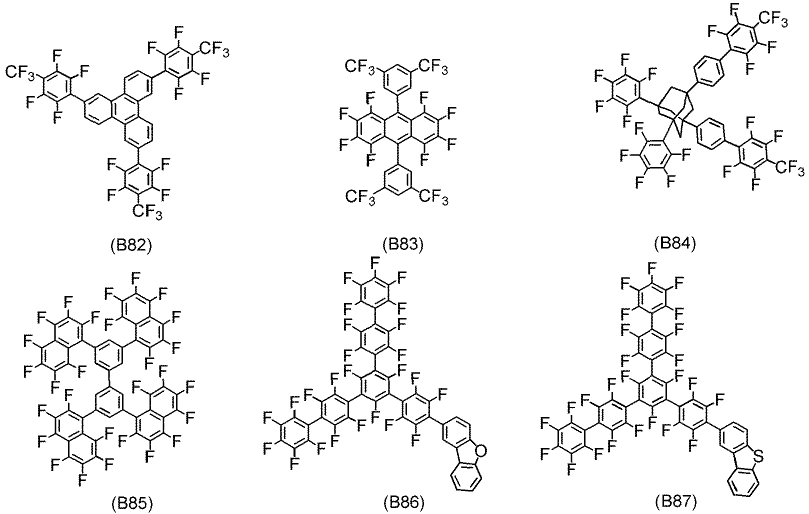

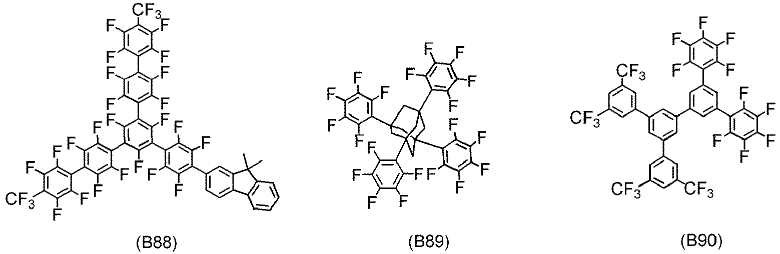

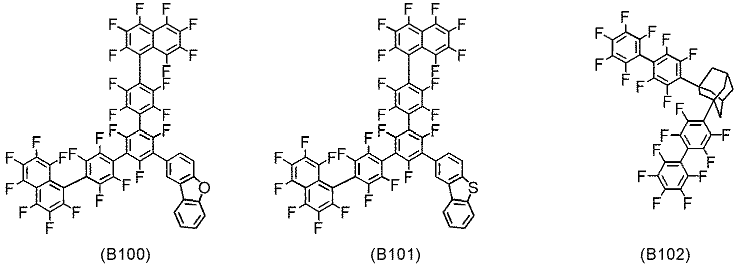

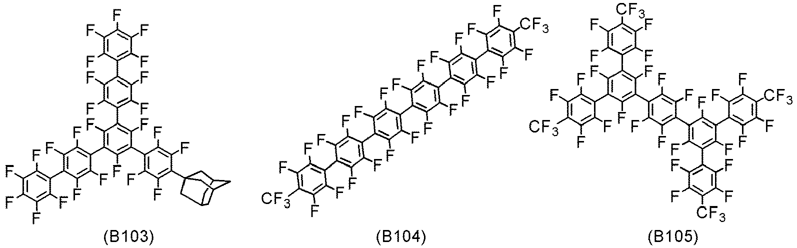

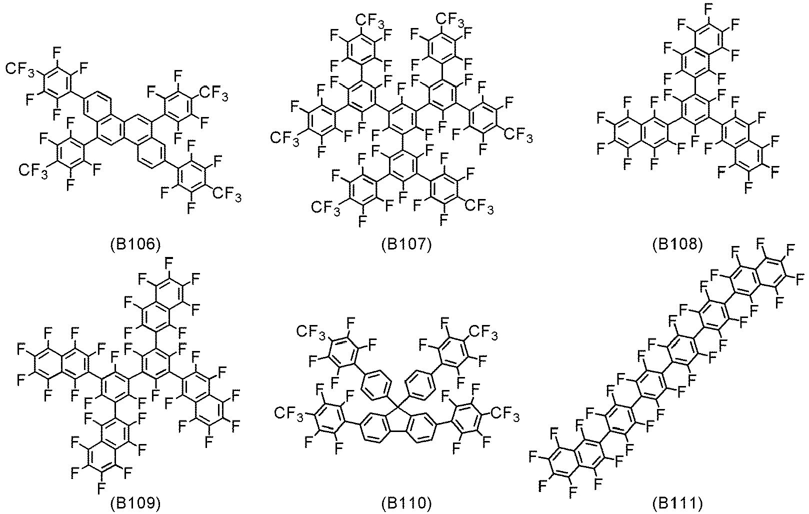

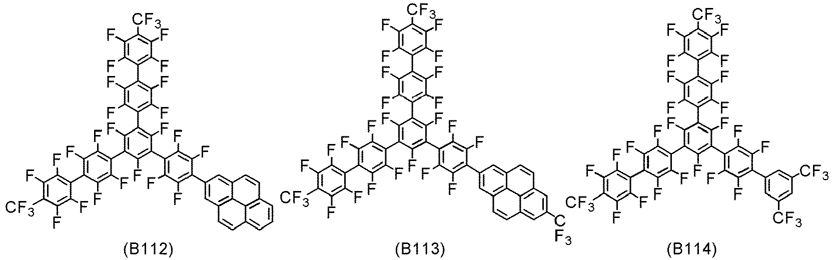

### [Metal Pattern Forming Method]

A method of forming a metal pattern according to an embodiment of the present disclosure includes:
forming the above-mentioned metal patterning material or an organic material pattern containing the above-mentioned amine compound; and
applying a metal material to a formation region of the organic material pattern and a non-formation region of the organic material pattern to form a metal pattern in the non-formation region.

The metal patterning material (amine compound) is used by forming a film at a location where it is desired to suppress adhesion of a metal. Here, a location where it is desired to suppress adhesion of the metal material corresponds to a formation region of the organic material pattern. Locations other than the location where it is desired to suppress adhesion of the metal material corresponds to a non-formation region of the organic material pattern. The non-formation region of the organic material pattern is a region that promotes adhesion of the metal material, and is a location where it is desired to form the metal pattern.

The method of forming the organic material pattern (film forming method) is not particularly limited, and known methods can be adopted such as a vacuum vapor deposition method, a spin coating method, a casting method, a dip coating method, a die coating method, a bar code method, an offset method, a spray coating method, an ink jet method, a screen method, an offset method, a flexo method, a gravure method, and a micro contact method. Further, after the film formation, the film may be annealed under a temperature environment higher than room temperature. The film thickness of the organic material pattern is not particularly limited.

In addition, another organic molecular material, a polymer, or the like may be optionally added to the metal patterning material as long as the formation of a metal film on the surface of the film can be suppressed.

The base on which forming the organic material pattern may be metal or nonmetal, and examples thereof include an organic film, a metal film, an oxide film, and an inorganic film, without particular limitation. The material of the substrate is not particularly limited, and glass, plastic, metal, ceramic, and any other materials can be used.

As the type of the metal material for forming the metal pattern using the metal patterning material is not particularly limited; however, an alkali metal, alkaline earth metal, transition metal, group 13 metal of the periodic table, or the like is preferable, and can be exemplified by lithium, sodium, potassium, rubidium, cesium, beryllium, magnesium, calcium, strontium, barium, aluminum, scandium, vanadium, zinc, yttrium, indium, cerium, samarium, europium, terbium, ytterbium, gold, silver, platinum, copper, iron, palladium, molybdenum, manganese, titanium, cobalt, nickel, tungsten, tin, and chromium, as well as alloys containing one or more of these metals. As the alloy, for example, magnesium-silver alloys, magnesium-indium alloys, magnesium-aluminum alloys, indium-silver alloys, lithium-aluminum alloys, lithiummagnesium alloys, lithium-indium alloys, and calcium-aluminum alloys can be exemplified.

The method of forming the metal pattern is not particularly limited, and a dry process such as a vacuum vapor deposition method or a sputtering method; an ink jet method using metal nano ink and the like can be exemplified. The thickness of the metal pattern is not particularly limited.

By applying the metal material to the formation region of the organic material pattern and the non-formation region of the organic material pattern, a film containing the metal material is momentarily formed in both regions; however, since adhesion of the metal is suppressed in the formation region of the organic material pattern, the metal pattern is naturally formed only in the non-formation region of the organic material pattern.

As a more specific method of the method for forming the metal pattern, for example, the following method indicated by 1) to 2) can be exemplified.
1) The above-mentioned metal patterning material is vapor deposited to form a film in a desired pattern using a metal mask or the like.
2) Subsequently, by vapor depositing the metal, an electrode is formed only on a portion where the metal patterning material is not formed as a film. In other words, a negativetype metal electrode is formed on the film forming pattern of the metal patterning material. It should be noted that the area and line width of the patterned metal electrode can be adjusted arbitrarily in the patterning shape of the metal patterning material.

According to the metal patterning material, amine compound and method for forming a metal pattern according to an aspect of the present disclosure, it is possible to form patterning of a metal electrode of a solar cell, optical sensor, image sensor, organic electro-luminescence (EL) element, organic solar cell, organic sensor, organic transistor or the like, and metal wiring on a circuit board.

The metal patterning material according to an aspect of the present disclosure can form a film having high glass transition temperature and superior in heat resistance, and thus is applicable also to deposition processes. On the other hand, the FTS according to Non-Patent Document 1 is a material for a coating process, and has limitations in the applicable processes such as not being able to form a film by a vacuum deposition process. In addition, the metal patterning material according to an embodiment of the present disclosure has at least a certain fluorine atom in the molecule, and thus formation of a metal thin film on the film surface is suppressed. Furthermore, the metal patterning material according to an aspect of the present disclosure can suppress adhesion of metal to a high degree, without heating the film containing the metal patterning material upon patterning a metal thin film. On the other hand, in the case of the method using 1,2- diarylethene derivatives according to Patent Document 1, there have also been cases where the film is heated to the glass transition temperature or higher in order to suppress adhesion of metals. Therefore, according to the present disclosure, it is possible to provide a metal patterning material superior in heat resistance, and capable of patterning a metal electrode by a simple process.

### (Electronic Device)

The electronic device according to an embodiment of the present disclosures includes the above-mentioned metal patterning material, or the above-mentioned amine compound. As mentioned above, upon forming a metal pattern, since an organic material pattern containing the metal patterning material or amine compound is formed, an organic material pattern is formed together with the metal pattern. Therefore, the electronic device according to an aspect of the present disclosure includes the organic material pattern containing the metal patterning material or amine compound, along with the metal pattern. As the electronic device, for example, a solar cell, optical sensor, image sensor, organic EL element, organic solar cell, organic sensor, organic transistor and the like can be exemplified. These electronic devices include patterning of a metal electrode, or metal wiring on a circuit board. In other words, so long as being an electronic device including patterning of a metal electrode, or metal wiring on a circuit board, it is possible to obtain the electrode device according to the present aspect, using a formation method of the aforementioned metal pattern. This electronic device has a high precision metal pattern.

### EXAMPLES

Hereinafter, the present invention will be explained in further detail based on examples; however, the present invention is not to be interpreted as limited in any way to these examples. It should be noted that the analytical equipment used in the present examples are listed below.

### (NMR Measurement)

### Measurement device: JNM-ECZ400S manufactured by JEOL

### (Measurement of glass transition temperature)

Measurement device: DSC7020 manufactured by Hitachi High-tech Science Corp. Measurement method: 5 mg of sample placed on sample pan made of aluminum, and measured at heating condition of 10°C/min

### (Transmittance measurement)

Measurement device: V-750 manufactured by Jasco Corp.
Measurement range: 550 to 800 nm

### Example 1: synthesis of compound (A177)

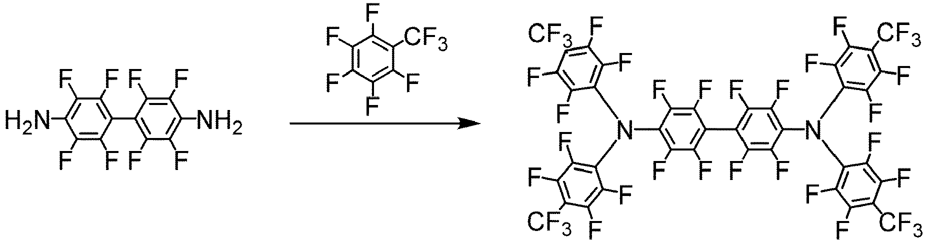

Under a nitrogen atmosphere, 4,4 '-diaminooctafluorobiphenyl (1.64 g, 5.0 mmol), perfluorotoluene (14 ml, 100 mmol), and potassium carbonate (3.46 g, 25 mmol) were suspended in dimethylsulfoxide (50 mL), and stirred at 100°C for 12 hours
After cooling to room temperature, water (100 ml), toluene (20 ml) and methanol (30 ml) were added to the reaction solution, and was stirred for 30 minutes at room temperature. The precipitated solid was collected by filtration to obtain compound (A177), which is the target substance of a white solid (yield: 5.12 g, 86%). The glass transition temperature of compound (A177) was 102°C.
19F- NMR (376.4 MHz, CDCl3)δ (ppm):-57.3 (t, J=17.3 Hz, 12F), -136.6 (s,4F),-139.5 (s,8F), -148.1 (d, J=17.3 Hz, 8F), -148.5 (s,4F)

Example 1' (Metal adhesion evaluation of compound (A177)) Boiling washing was performed with isopropyl alcohol on the glass substrate, and after further washing by UV/ozone, it was placed in the vapor deposition equipment, and evacuated to no more than 1.0×10⁻⁴ Pa by a vacuum pump. The compound (A177) was deposited to 100 nm at 0.2 nm/sec on a glass substrate on which a metal mask having a 20 mm × 10 mm opening was arranged. Subsequently, the metal mask was removed, and silver was vacuum deposited to 20 nm at a deposition rate of 0.2 nm/sec. Silver did not form a film on a portion where compound (A177) is deposited, and a 20 mm × 10 mm transparent area was formed.

### Example 2: Synthesis of Compound (A206)

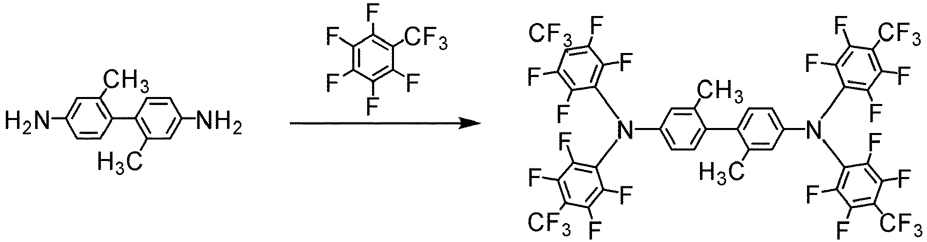

Under a nitrogen atmosphere, 4,4 '-diamino -2,2 '-dimethylbiphenyl (1.06 g, 5.0 mmol), perfluorotoluene (14 ml, 100 mmol), and potassium carbonate (3.46 g, 25 mmol) were suspended in dimethylsulfoxide (50 mL), and stirred at 100°C for 12 hours. After cooling to room temperature, water (100 ml), toluene (20 ml) and methanol (30 ml) were added to the reaction solution, and was stirred for 30 minutes at room temperature. The precipitated solid was collected by filtration to obtain compound (A206), which is the target substance of a white solid (yield: 4.16 g, 77%).
1H-NMR (400MHz, CDCl₃); 2.02 (s,6H), 6.80-6.83 (m, 4H), 7.09 (d,J=8.0,2H)
19F-NMR(376.4 MHz, CDCl₃)δ(ppm) : -57.2 (t,J=22.6Hz, 12F), - 140.7~-140.7(m, 8F), -145.9~-146.0 (m, 8F)

Example 2' (Metal adhesion evaluation of compound (A206)) Boiling washing was performed with isopropyl alcohol on the glass substrate, and after further washing by UV/ozone, it was placed in the vapor deposition equipment, and evacuated to no more than 1.0×10⁻⁴ Pa by a vacuum pump. The compound (A206) was deposited to 100 nm at 0.2 nm/sec on a glass substrate on which a metal mask having a 20 mm × 10 mm opening was arranged. Subsequently, the metal mask was removed, and magnesium was vacuum deposited to 30 nm at a deposition rate of 0.2 nm/sec. Magnesium did not form a film on a portion where compound (A206) is deposited, and a 20 mm × 10 mm transparent area was formed.

### Example 3: Synthesis of compound (A236)

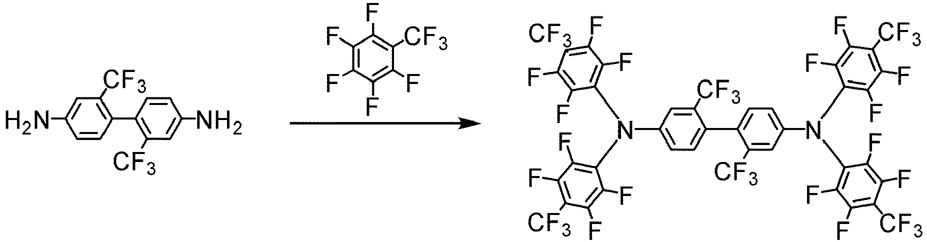

Under a nitrogen atmosphere, 4,4'-diamino-2,2'-bis (trifluoromethyl)biphenyl (0.96 g, 3.0 mmol), perfluorotoluene (14.2 g, 60 mmol), and potassium carbonate (3.18 g, 15 mmol) were suspended in dimethylsulfoxide (30 mL), and stirred at 100°C for 24 hours. After cooling to room temperature, liquid was separated using pure water and chloroform, and the organic layer was further washed with a saturated aqueous sodium chloride solution. After the organic layer was dried with anhydrous magnesium sulfate, column chromatography was performed with a small amount of silica gel to remove the highly polar component. Next, the solvent was removed under reduced pressure. Methanol (40 ml) and hexane (10 ml) were added to the obtained solid, and then stirred for 30 minutes at room temperature. The precipitated solid was collected by filtration to obtain compound (A236), which is the target substance of a white solid (yield: 3.11 g, 89%).
¹H-NMR (400 MHz, THF-d8); 7.32 (d, J=4.0, 2H), 7.62 (dd, J=4.0, 2.0, 2H), 7.92 (d, J=2.0, 2H)
¹⁹F- NMR (376.4 MHz, THF-d8) δ (ppm): -57.2 (t, J=22.6Hz, 12F), -64.0 (s,6F), -140.4--140.5 (m, 8F), -147.2~-147.3 (m, 8F)

Example 3' (Metal adhesion evaluation of compound (A236)) Boiling washing was performed with isopropyl alcohol on the glass substrate, and after further washing by UV/ozone, it was placed in the vapor deposition equipment, and evacuated to no more than 1.0×10⁻⁴ Pa by a vacuum pump. The compound (A236) was deposited to 100 nm at 0.2 nm/sec on a glass substrate on which a metal mask having a 20 mm × 10 mm opening was arranged. Subsequently, the metal mask was removed, and silver was vacuum deposited to 10 nm at a deposition rate of 0.2 nm/sec. Silver did not form a film on a portion where compound (A236) is deposited, and a 20 mm × 10 mm transparent area was formed.

### Example 4: Synthesis of compound (A1)

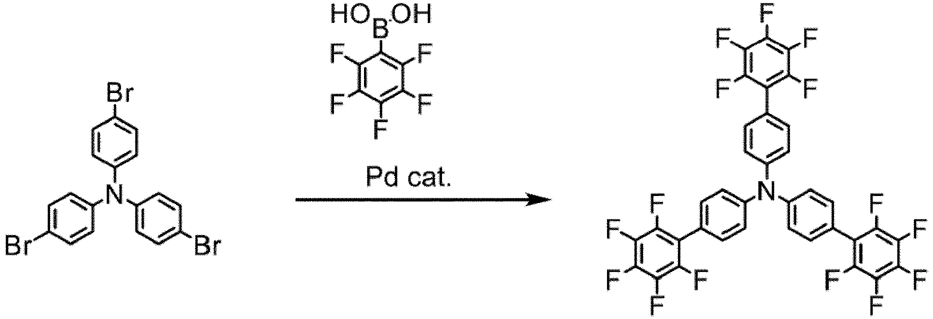

Under a nitrogen atmosphere, tris (4-bromophenyl)amine (1.69 g, 3.5 mmol), pentafluorophenyl boronic acid (2.67, 12.6 mmol), cesium fluoride (3.19 g, 21.0 mmol), silver oxide (I) (2.92 g, 12.6 mmol), and N,N-dimethylformamide (35 ml) were added, and the mixture was stirred for 60 minutes. Tris (dibenzylideneacetone)palladium (0) (160.2 mg, 0.18 mmol) and tri (tert-butyl)phosphine (85 mg, 0.42 mmol) were added to the resulting slurry mixture, followed by stirring at 100°C for 6 hours. After cooling to room temperature, silver oxide (I) was removed by filtration, and the filtrate was concentrated. Hexane was added to the residue, and was purified by silica gel column chromatography (hexane), thereby obtaining compound (A1), which is the target substance as a white solid (yield: 1.21 g, 47 %). FDMS:743
¹H-NMR (400 MHz, CDCl₃); 7.39-7.37 (m, 6H), 7.31-7.28 (m, 6H) ¹⁹F- NMR (376.4 MHz, CDCl₃) δ (ppm) :-144.62 (dd, J=30.1, 7.5, 6F), -157.0 (t, J=22.6, 3F), -163.4-163.5 (m, 6F)

Example 4' (Metal adhesion evaluation of compound (A1)) Boiling washing was performed with isopropyl alcohol on the glass substrate, and after further washing by UV/ozone, it was placed in the vapor deposition equipment, and evacuated to no more than 1.0×10⁻⁴ Pa by a vacuum pump. The compound (A1) was deposited to 100 nm at 0.2 nm/sec on a glass substrate on which a metal mask having a 20 mm × 10 mm opening was arranged. Subsequently, the metal mask was removed, and magnesium was vacuum deposited to 10 nm at a deposition rate of 0.2 nm/sec. Magnesium did not form a film on a portion where compound (A1) is deposited, and a 20 mm × 10 mm transparent area was formed.

### Example 5: Synthesis of compound (A512)

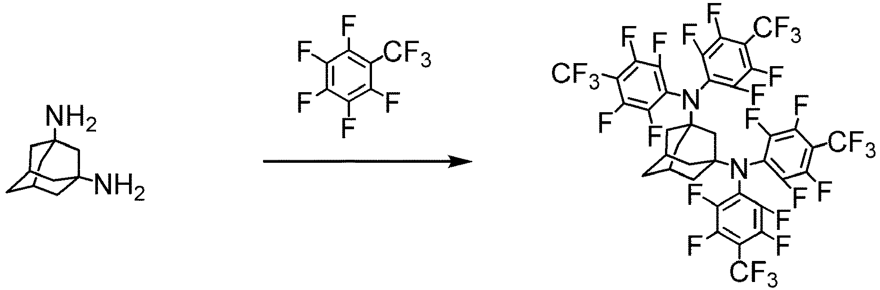

Under a nitrogen atmosphere, 1,3-diaminoadamantane (1.30 g, 7.8 mmol), perfluorotoluene (14.8 g, 62 .5 mmol), 18 crown 6 ether (103 mg, 0.40 mmol) and tripotassium phosphate (16.6 g, 78 .2 mmol) were suspended in dimethylsulfoxide (40 ml) and stirred at 100°C for 24 hours. After cooling to room temperature, water (50 ml) and methanol (50 ml) were added to the reaction solution, and were stirred at room temperature for 30 minutes. The precipitated solid was collected by filtration to obtain compound (A512), which is the target substance as a white solid (yield: 6.90 g, 86%). The glass transition temperature of the compound (A512) was 70°C.
¹H-NMR (400 MHz, CDCl₃); 2.34 (s, 2H), 2.01 (s, 2H), 1.91 (d, J=12.0, 4H), 1.72 (d, J=12.0, 4H), 1.58 (s, 2H)
¹⁹F- NMR (376.4 MHz, CDCl₃) δ (ppm): -57.5--57.7 (m, 12F), - 138.1 (s, 8F), -141.3 (d, J=23.7 Hz, 8F)

Example 5' (Metal adhesion evaluation of compound (A512)) Boiling washing was performed with isopropyl alcohol on the glass substrate, and after further washing by UV/ozone, it was placed in the vapor deposition equipment, and evacuated to no more than 1.0×10⁻⁴ Pa by a vacuum pump. The compound (A512) was deposited to 20 nm at 0.1 nm/sec on a glass substrate on which a metal mask having a 20 mm × 10 mm opening was arranged. Subsequently, the metal mask was removed, and gold was vacuum deposited to 12 nm at a deposition rate of 0.1 nm/sec. Gold did not form a film on a portion where compound (A512) is deposited, and a 20 mm × 10 mm transparent area was formed.

### Example 6: Synthesis of compound (A553)

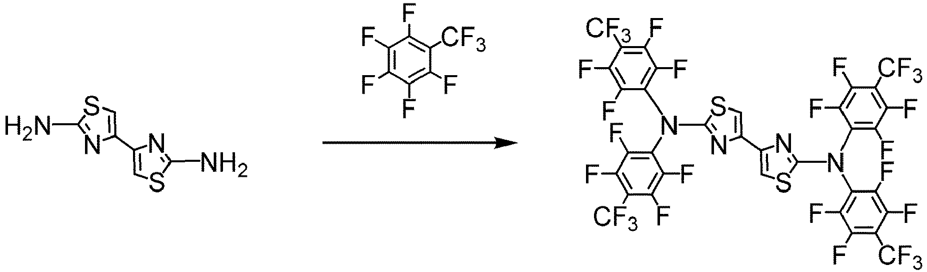

Under a nitrogen atmosphere, 2,2 '-diamino -4,4 '-bithiazole (0.59 g, 3.0 mmol), perfluorotoluene (14 g, 60 mmol) and tripotassium phosphate (3.2 g, 15 mmol) were suspended in dimethylsulfoxide (30 mL) and stirred at 100°C for 24 hours. After cooling to room temperature, water (30 ml) and methanol (30 ml) were added to the reaction solution, and were stirred at room temperature for 30 minutes. The precipitated solid was collected by filtration to obtain compound (A553), which is a target substance as a white solid (yield: 2.26 g, 71%).
¹H-NMR (400 MHz, CDCl₃); 7.22 (s, 2H),
1⁹F- NMR (376.4 MHz, CDCl₃) δ(ppm) : -57.4 (t, J=22.6 Hz, 12F), -139.5~-139.7 (m, 8F), -143.1~-143.2 (m, 8F)

Example 6' (Metal adhesion evaluation of compound (A553)) Boiling washing was performed with isopropyl alcohol on the glass substrate, and after further washing by UV/ozone, it was placed in the vapor deposition equipment, and evacuated to no more than 1.0×10⁻⁴ Pa by a vacuum pump. The compound (A553) was deposited to 50 nm at 0.2 nm/sec on a glass substrate on which a metal mask having a 20 mm × 10 mm opening was arranged. Subsequently, the metal mask was removed, and magnesium was vacuum deposited to 12 nm at a deposition rate of 0.1 nm/sec. Magnesium did not form a film on a portion where compound (A553) is deposited, and a 20 mm × 10 mm transparent area was formed.

### Example 7: Synthesis of compound (A559)

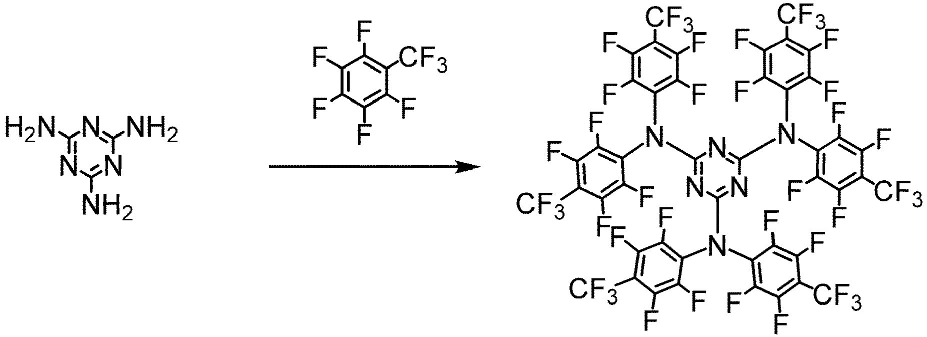

Under a nitrogen atmosphere, 2,4,6-triamino -1,3,5-triazine (0.25 g, 2.0 mmol), perfluorotoluene (14 g, 59 mmol), 18 crown 6 ether (26 mg, 0.1 mmol) and tripotassium phosphate (6.3 g, 30 mmol) were suspended in dimethylsulfoxide (30 mL), and stirred at 100°C for 24 hours. After cooling to room temperature, water (30 ml) and methanol (30 ml) were added to the reaction solution, and were stirred at room temperature for 30 minutes. The precipitated solid was collected by filtration to obtain compound (A559), which is a target substance as a white solid (yield: 2.26 g, 80%).
¹⁹F- NMR (376.4 MHz, THF-d8) δ (ppm): -55.9 (t, J=22.6 Hz, 18F), -139.8--140.0 (m, 24F)

Example 7' (Metal adhesion evaluation of compound (A559)) Boiling washing was performed with isopropyl alcohol on the glass substrate, and after further washing by UV/ozone, it was placed in the vapor deposition equipment, and evacuated to no more than 1.0×10⁻⁴ Pa by a vacuum pump. The compound (A59) was deposited to 20 nm at 0.2 nm/sec on a glass substrate on which a metal mask having a 20 mm × 10 mm opening was arranged. Subsequently, the metal mask was removed, and silver and magnesium (9/1) were vacuum deposited to 20 nm at a deposition rate of 0.1 nm/sec. An alloy of silver and magnesium did not form a film on a portion where compound (A559) is deposited, and a 20 mm × 10 mm transparent area was formed.

### Example 8: Synthesis of compound (A173)

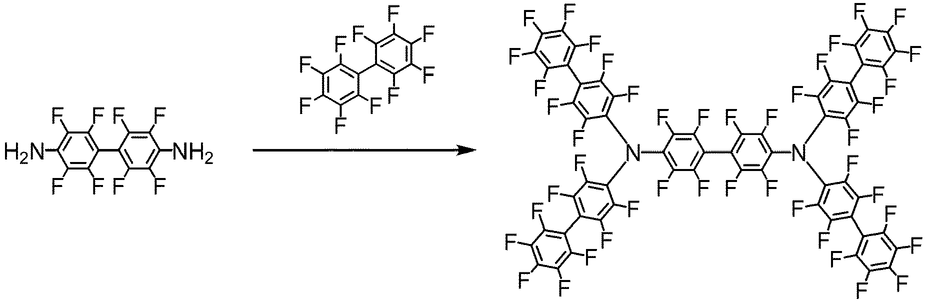

Under a nitrogen atmosphere, 4,4 '-diaminooctafluorobiphenyl (0.66 g, 2.0 mmol), perfluorobiphenyl (6.72 g, 20.1 mmol) and tripotassium phosphate (2.13 g, 10.1 mmol) were suspended in dimethylsulfoxide (15 mL) and cyclopentylmethyl ether (15 mL), and stirred at 100°C for 24 hours. After cooling to room temperature, the liquid was separated using pure water and chloroform, and the organic layer was further washed with saturated aqueous sodium chloride solution. The organic layer was dried over anhydrous magnesium sulfate, and column chromatography was performed on a small amount of silica gel to remove highly polar components. Then, the solvent was distilled off under reduced pressure. Methanol (40 ml) and hexane (10 ml) were added to the obtained solid, and were stirred at room temperature for 30 minutes. The precipitated solid was collected by filtration to obtain compound (A173), which is that target substance as a white solid (yield: 2.41 g, 77%).
¹⁹F-NMR (376.4 MHz, THF-d8) δ (ppm): -137.0 (m, 4F), -137.3 (m, 8F), -138.0 (m, 8F), -147.4 (m, 12F), -150.5 (m, 4F), -161.1 (m, 8F)

Example 8' (Metal adhesion evaluation of compound (A173)) Boiling washing was performed with isopropyl alcohol on the glass substrate, and after further washing by UV/ozone, it was placed in the vapor deposition equipment, and evacuated to no more than 1.0×10⁻⁴ Pa by a vacuum pump. The compound (A173) was deposited to 30 nm at 0.2 nm/sec on a glass substrate on which a metal mask having a 20 mm × 10 mm opening was arranged. Subsequently, the metal mask was removed, and aluminum was vacuum deposited to 10 nm at a deposition rate of 0.1 nm/sec. Aluminum did not form a film on a portion where compound (A173) is deposited, and a 20 mm × 10 mm transparent area was formed.

### Example 9: Synthesis of compound (A433)

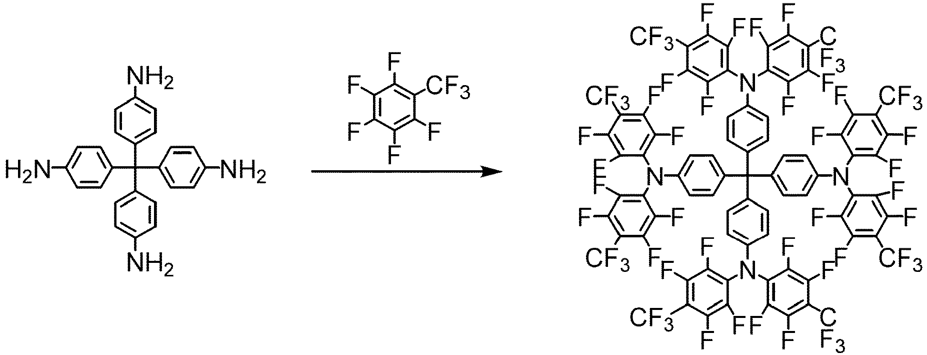

Under a nitrogen atmosphere, tetrakis (4-aminophenyl)methane (1.90 g, 4.99 mmol), perfluorotoluene (47.1 g, 200 mmol) and tripotassium phosphate (10.6 g, 49 .9 mmol) were suspended in dimethylsulfoxide (50 mL), and stirred at 100°C for 24 hours. After cooling to room temperature, water (60 ml) and methanol (60 ml) were added to the reaction solution, and were stirred at room temperature for 30 minutes. The precipitated solid was collected by filtration to obtain compound (A433), which was a target substance as a white solid (yield: 9.76 g, 93%).
¹H-NMR (400 MHz, THF-d8); 7.03-7.10 (m, 16H)
¹⁹F- NMR (376.4 MHz, THF-d8) δ (ppm): -55.5 (t, J=22.6 Hz, 24F), -140.3-140.4 (m, 16F), -144.4- -144.5 (m, 16F)

Example 9' (Metal adhesion evaluation of compound (A433)) Boiling washing was performed with isopropyl alcohol on the glass substrate, and after further washing by UV/ozone, it was placed in the vapor deposition equipment, and evacuated to no more than 1.0×10⁻⁴ Pa by a vacuum pump. The compound (A433) was deposited to 50 nm at 0.2 nm/sec on a glass substrate on which a metal mask having a 20 mm × 10 mm opening was arranged. Subsequently, the metal mask was removed, and magnesium was vacuum deposited to 12 nm at a deposition rate of 0.2 nm/sec. Magnesium did not form a film on a portion where compound (A433) is deposited, and a 20 mm × 10 mm transparent area was formed.

### Example 10: Synthesis of compound (A569)

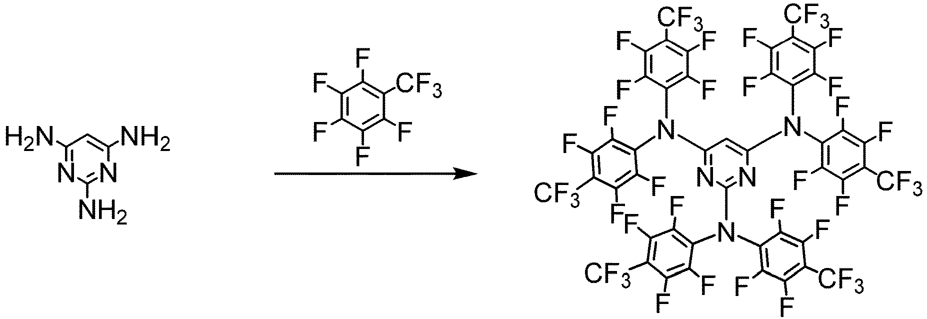

Under a nitrogen atmosphere, 2,4,6-triaminopyrimidine (0.50 mg, 4.00 mmol), perfluorotoluene (28.3 g, 120 mmol), 18 crown 6 ether (52.9 mg, 0.20 mmol), tripotassium phosphate (12.7 g, 60.0 mmol) were suspended in dimethylsulfoxide (40 mL), and stirred at 100°C for 24 hours. After cooling to room temperature, water (60 ml) and methanol (60 ml) were added to the reaction solution, and were stirred at room temperature for 30 minutes. The precipitated solid was collected by filtration to obtain compound (A569), which is the target substance as a white solid (yield: 5.69 g, 82%).
¹H-NMR (400 MHz, THF-d8); 5.96 (s, 1H)
¹⁹F-NMR (376.4 MHz, THF-d8) δ (ppm): -55.7~ -55.9 (m, 18F), - 140.3~ -141.1 (m, 24F)

### Example 10' (Metal adhesion evaluation of compound (A569))

Boiling washing was performed with isopropyl alcohol on the glass substrate, and after further washing by UV/ozone, it was placed in the vapor deposition equipment, and evacuated to no more than 1.0×10⁻⁴ Pa by a vacuum pump. The compound (A569) was deposited to 100 nm at 0.2 nm/sec on a glass substrate on which a metal mask having a 20 mm × 10 mm opening was arranged. Subsequently, the metal mask was removed, and silver was vacuum deposited to 20 nm at a deposition rate of 0.1 nm/sec. Silver did not form a film on a portion where compound (A569) is deposited, and a 20 mm × 10 mm transparent area was formed.

### Example 11: Synthesis of compound (A305)

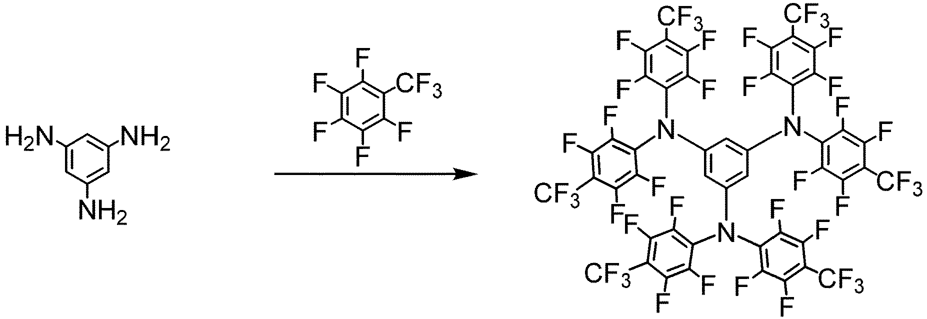

Under a nitrogen atmosphere, 1,3,5-triaminobenzene (1.73 g, 14 mmol), perfluorotoluene (40 g, 170 mmol), tripotassium phosphate (74 g, 340 mol), 18 crown 6 ether (103 mg, 0.40 mmol) were suspended in dimethylsulfoxide (200 mL), and stirred at 100°C for 24 hours. After cooling to room temperature, the reaction solution was added to a mixed solution of water (200 ml) and methanol (200 ml), and were stirred at room temperature for 30 minutes. The precipitated solid was collected by filtration to obtain compound (A305), which is the target substance of a white solid (yield: 2.54 g, 13%) .
¹⁹F-NMR (376.4 MHz, THF-d8) δ (ppm): -55.6 (t, J=22.6 Hz, 12F), -139.9- -140.0 (m, 12F), -144.0~ -144.1 (m, 12F)

### Example 11' (Metal adhesion evaluation of compound (A305))

Boiling washing was performed with isopropyl alcohol on the glass substrate, and after further washing by UV/ozone, it was placed in the vapor deposition equipment, and evacuated to no more than 1.0×10⁻⁴ Pa by a vacuum pump. The compound (A305) was deposited to 20 nm at 0.2 nm/sec on a glass substrate on which a metal mask having a 20 mm × 10 mm opening was arranged. Subsequently, the metal mask was removed, and ytterbium was vacuum deposited to 1 nm at a deposition rate of 0.01 nm/sec, and subsequently, silver was vacuum deposited to 12 nm at a deposition rate of 0.1 nm/sec. Silver did not form a film on a portion where compound (A305) is deposited, and a 20 mm × 10 mm transparent area was formed.

### Example 12: Synthesis of intermediate (AA1)

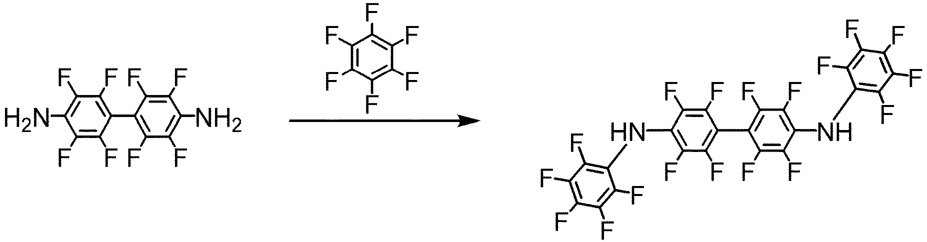

Under nitrogen atmosphere, 4,4'-diaminooctafluorobiphenyl (3.28 g, 10.0 mmol), perfluorobenzene (18.6 g, 100.0 mmol) and were suspended in tetrahydrofuran (100 mL), stirred at 100°C, and sodium tert-butoxide (9.61 g, 100.0 mmol) was added in 1 g increments each hour. After completion of the addition, the mixture was stirred at 100°C for 6 hours. After cooling to room temperature, the liquid was separated using pure water and chloroform, and the organic layer was further washed with saturated aqueous sodium chloride solution. The organic layer was dried over anhydrous magnesium sulfate and purified by silica gel column chromatography, thereby obtaining an intermediate (AA1), which is the target substance as a white solid (yield: 4.56 g, 69%). Compound identification was performed by FDMS measurement. FDMS: 660

### Example 13: Synthesis of compound (A176)

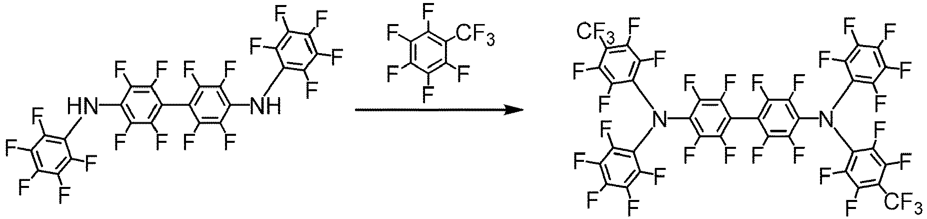

Under a nitrogen atmosphere, AAl (2.64 g, 4.0 mmol), perfluorotoluene (9.44 g, 40.0 mmol), tripotassium phosphate (2.12 g, 10.0 mmol) were suspended in dimethylsulfoxide (40 mL), and stirred at 100°C for 24 hours. After cooling to room temperature, water (40 ml) and methanol (40 ml) were added to the reaction solution, and were stirred at room temperature for 30 minutes. The precipitated solid was collected by filtration to obtain compound (A176), which is the target substance as a white solid (yield: 3.57 g, 82%). The glass transition temperature of the compound (A176) was 87°C.
¹⁹F-NMR (376.4 MHz, THF-d8) δ (ppm): -55.6 (t, J=22.6 Hz, 6F), -136.9- -137.0 (m, 4F), -139.7- -139.9 (m, 4F), -147.6- -147.7 (m, 12F), -154.6- -154.7 (m, 2F), -161.5- -161.6 (m, 4F)

### Example 13' (Metal adhesion evaluation of compound (A176))

Boiling washing was performed with isopropyl alcohol on the glass substrate, and after further washing by UV/ozone, it was placed in the vapor deposition equipment, and evacuated to no more than 1.0×10⁻⁴ Pa by a vacuum pump. The compound (A176) was deposited to 20 nm at 0.1 nm/sec on a glass substrate on which a metal mask having a 20 mm × 10 mm opening was arranged. Subsequently, the metal mask was removed, and silver was vacuum deposited to 30 nm at a deposition rate of 0.1 nm/sec. Silver did not form a film on a portion where compound (A176) is deposited, and a 20 mm × 10 mm transparent area was formed.

### Example 14: Synthesis of compound (A574)

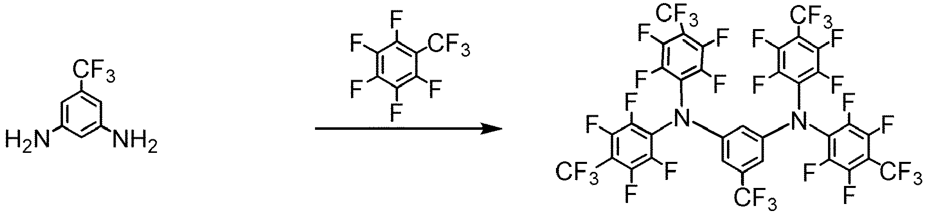

Under a nitrogen atmosphere, 5-trifluoromethyl-1,3-phenylenediamine (1.0 g, 5.7 mmol), perfluorotoluene (10.7 g, 45 mmol), tripotassium phosphate (12.3 g, 58 mmol), 18 crown 6 ether (103 mg, 0.40 mmol) were suspended in dimethylsulfoxide (60 mL), and stirred at 100°C for 24 hours. After cooling to room temperature, water (100 ml) and methanol (50 ml) were added to the reaction solution, and were stirred at room temperature for 30 minutes. The precipitated solid was collected by filtration to obtain compound (A574), which is the target substance as a white solid (yield: 3.21 g, 54%). ¹H-NMR (400 MHz, THF-d8); 7.26 (d, J=4.00, 2H), 7.02 (s, 1H) ¹⁹F-NMR (376.4 MHz, CDCl₃) δ (ppm): -55.6 (t, J=22.6 Hz, 12F), -62.2 (s, 3F), -139.7- -139.9 (m, 8F), -143.7- -143.8 (m, 8F), -142.2- -142.4 (m, 4F)

### Example 14' (Metal adhesion evaluation of compound (A574))

Boiling washing was performed with isopropyl alcohol on the glass substrate, and after further washing by UV/ozone, it was placed in the vapor deposition equipment, and evacuated to no more than 1.0×10⁻⁴ Pa by a vacuum pump. The compound (A574) was deposited to 50 nm at 0.1 nm/sec on a glass substrate on which a metal mask having a 20 mm × 10 mm opening was arranged. Subsequently, the metal mask was removed, and indium was vacuum deposited to 15 nm at a deposition rate of 0.1 nm/sec. Indium did not form a film on a portion where compound (A574) is deposited, and a 20 mm × 10 mm transparent area was formed.

### Example 15: Synthesis of compound (A580)

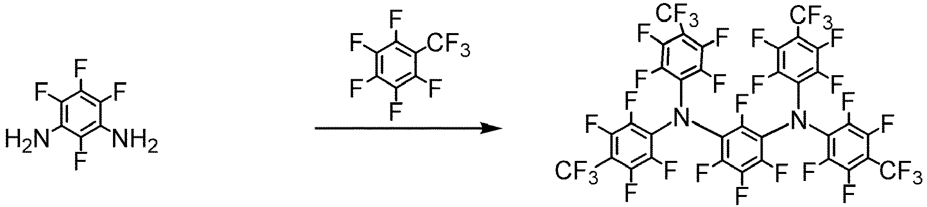

Under a nitrogen atmosphere, 1,3-diamino -2,4,5,6-tetrafluorobenzene (1.80 g, 10.0 mmol), perfluorotoluene (19.2 g, 81 mmol), and tripotassium phosphate (21.2 g, 100.0 mmol) were suspended in dimethylsulfoxide (50 mL), and stirred at 100°C for 24 hours. After cooling to room temperature, water (60 ml) and methanol (60 ml) were added to the reaction solution, and were stirred at room temperature for 30 minutes. The precipitated solid was collected by filtration to obtain compound (A580), which is the target substance as a white solid (yield: 3.94 g, 380). The glass transition temperature of compound (A580) was 66°C.
¹⁹F-NMR (376.4 MHz, THF-d8) δ (ppm): -55.6 (t, J=22.6 Hz, 6F), -132.6 (s, 1F), -138.7- -138.8 (m, 2F), -139.4- -139.6 (m, 8F), -147.6- -147.7 (m, 8F), -159.7- -159.8 (m, 1F)

### Example 15' (Metal adhesion evaluation of compound (A580))

Boiling washing was performed with isopropyl alcohol on the glass substrate, and after further washing by UV/ozone, it was placed in the vapor deposition equipment, and evacuated to no more than 1.0×10⁻⁴ Pa by a vacuum pump. The compound (A580) was deposited to 30 nm at 0.1 nm/sec on a glass substrate on which a metal mask having a 20 mm × 10 mm opening was arranged. Subsequently, the metal mask was removed, and ytterbium was vacuum deposited to 2 nm at a deposition rate of 0.01 nm/sec, and subsequently, silver and magnesium (9/1) were vacuum deposited to 15 nm at a deposition rate of 0.1 nm/sec. Silver did not form a film on a portion where compound (A580) is deposited, and a 20 mm × 10 mm transparent area was formed.

### Example 16: Synthesis of intermediate (AA2)

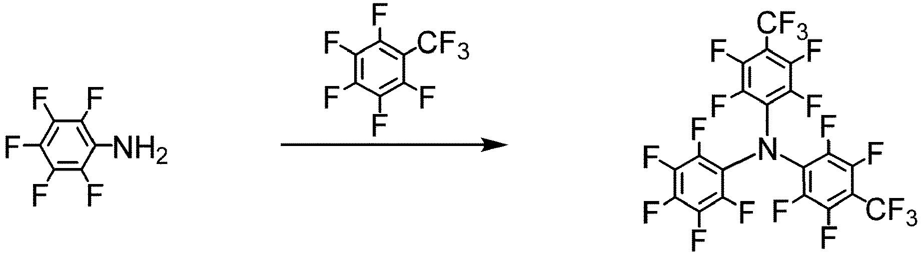

Under a nitrogen atmosphere, pentafluoroaniline (5.10 g, 27.8 mmol), perfluorotoluene (26.3 ml, 111.3 mmol), and tripotassium phosphate (29.77 g, 140 mmol) were suspended in dimethylsulfoxide (80 mL), and stirred at 100°C for 24 hours. After cooling to room temperature, the mixture was separated using pure water and chloroform, and the organic layer was further washed with saturated aqueous sodium chloride solution. The organic layer was dried over anhydrous magnesium sulfate and purified by silica gel column chromatography to obtain intermediate (AA2), which is the target substance as a white solid (yield: 14.82 g, 87%).
¹⁹F-NMR (376.4 MHz, CDCl₃) δ (ppm): -57.3 (t, J=22.6 Hz, 6F), - 139.8- -139.9 (m, 4F), -148.6~ -148.7 (m, 6F), -153.6~ -153.8 (m, 1F), -160.9- -161.0 (m, 2F)

### Example 17: Synthesis of intermediate (AA3)

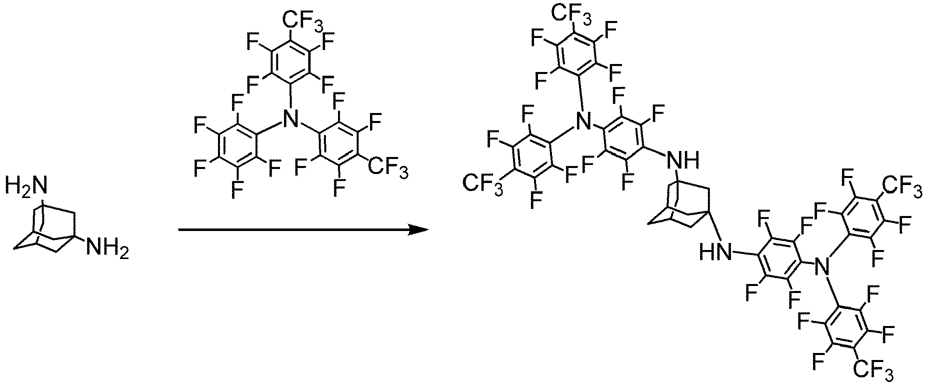

Under a nitrogen atmosphere, 1,3-diaminoadamantane (0.66 g, 4.0 mmol), AA2 (5.01 g, 8.1 mmol), and tripotassium phosphate (2.11 g, 10.0 mmol) were suspended in dimethylsulfoxide (40 mL), and stirred at 100°C for 24 hours. After cooling to room temperature, the mixture was separated using pure water and chloroform, and the organic layer was further washed with saturated aqueous sodium chloride solution. The organic layer was dried over anhydrous magnesium sulfate and purified by silica gel column chromatography to obtain intermediate (AA3), which is the target substance as a white solid (yield: 4.21 g, 78%).
¹H-NMR (400 MHz, CDCl₃); 3.48 (s, 2H), 2.34 (s, 2H), 1.90 (s, 2H), 1.78 (s, 8H), 1.59 (s, 2H)
¹⁹F-NMR (376.4 MHz, CDCl₃) δ (ppm): -57.3 (t, J=22.6 Hz, 12F), -140.5- -140.6 (m, 8F), -149.0~ -149.1 (m, 8F), -150.8~ -150.9 (m, 4F), -151.2~ -151.3 (m, 4F)

### Example 18: Synthesis of compound (A581)

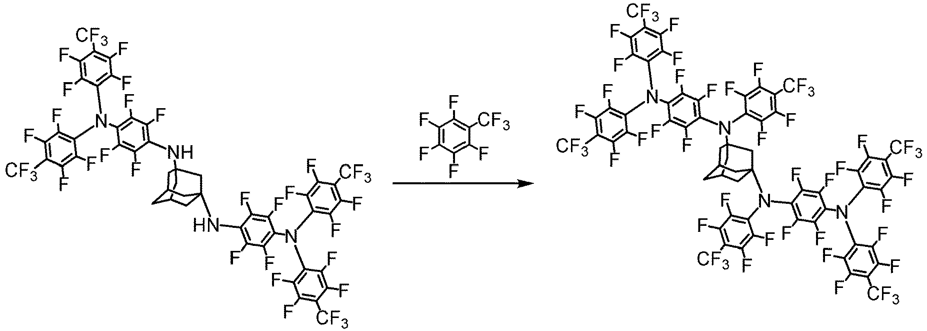

Under a nitrogen atmosphere, AA3 (4.07 g, 3.0 mmol), perfluorobenzene (2.12 g, 9.0 mmol), 18 crown 6 ether (39 .6 mg, 0.15 mmol) and tripotassium phosphate (3.18 g, 15.0 mmol) were suspended in dimethylsulfoxide (40 mL), and stirred at 100°C for 24 hours. After cooling to room temperature, water (40 ml) and methanol (40 ml) were added to the reaction solution, and were stirred at room temperature for 30 minutes. The precipitated solid was collected by filtration to obtain compound (A581), which is the target substance as a white solid (yield: 3.85 g, 72%). The glass transition temperature of the compound (A581) was 127°C.
¹H-NMR (400 MHz, THF-d8); 2.21 (s, 2H), 1.93 (s, 2H), 1.81-1.68 (m, 8H), 1.45 (s, 2H)
¹⁹F-NMR (376.4 MHz, THF-d8) δ (ppm): -55.6- -55.9 (m, 18F), - 136.8- -137.0 (m, 4F), -137.4~ -137.6 (m, 4F), -139.3~ -139.6 (m, 8F), -141.2~ -141.4 (m, 4F), -146.9~ -147.0 (m, 8F), - 148.8- -149.0 (m, 4F)

### Example 18' (Metal adhesion evaluation of compound (A581))

Boiling washing was performed with isopropyl alcohol on the glass substrate, and after further washing by UV/ozone, it was placed in the vapor deposition equipment, and evacuated to no more than 1.0×10⁻⁴ Pa by a vacuum pump. The compound (A581) was deposited to 10 nm at 0.2 nm/sec on a glass substrate on which a metal mask having a 20 mm × 10 mm opening was arranged. Subsequently, the metal mask was removed, and silver was vacuum deposited to 20 nm at a deposition rate of 0.2 nm/sec. Silver did not form a film on a portion where compound (A581) is deposited, and a 20 mm × 10 mm transparent area was formed.

### Example 19: Synthesis of intermediate (AA4)

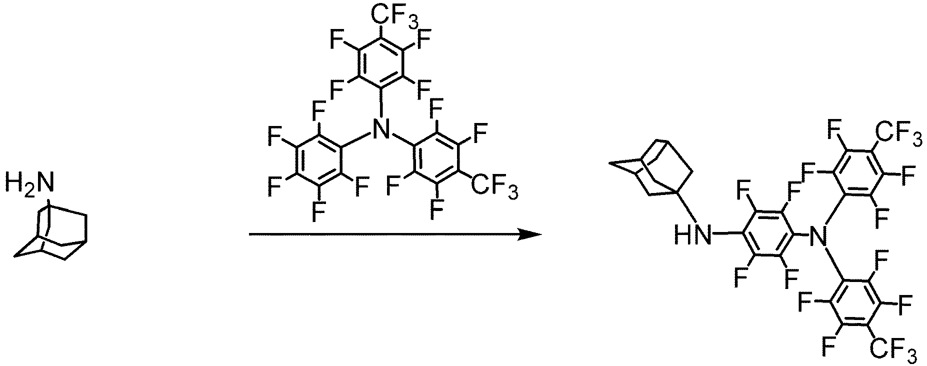

Under a nitrogen atmosphere, 1-aminoadamantane (1.51 g, 10.0 mmol), AA2 (6.15 g, 10.0 mmol) and tripotassium phosphate (2.23 g, 10.5 mmol) were suspended in dimethylsulfoxide (50 mL), and stirred at 100°C for 24 hours. After cooling to room temperature, the mixture was separated using pure water and chloroform, and the organic layer was further washed with saturated aqueous sodium chloride solution. The organic layer was dried over anhydrous magnesium sulfate and purified by silica gel column chromatography (hexane) to obtain intermediate (AA4), which is the target substance as a white solid (yield: 5.86 g, 79%). Compound identification was performed by FDMS measurement. FDMS: 746

### Example 20: Synthesis of compound (A582)

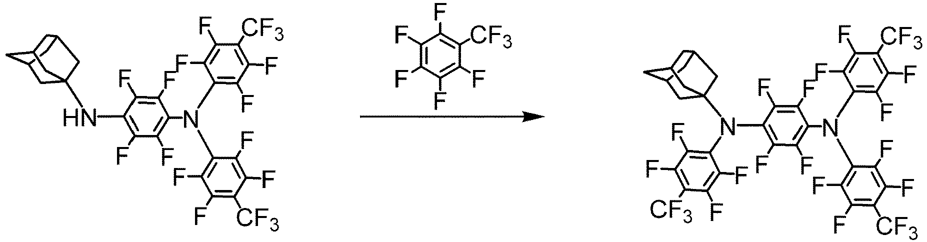

Under a nitrogen atmosphere, AA4 (2.99 g, 4.0 mmol), perfluorotoluene (2.83 g, 12.0 mmol), 18 crown 6 ether (52 .9 mg, 0.2 mmol) and tripotassium phosphate (4.25 g, 20.0 mmol) were suspended in dimethylsulfoxide (40 mL), and stirred at 100°C for 24 hours. After cooling to room temperature, water (40 ml) and methanol (40 ml) were added to the reaction solution, and the mixture was stirred at room temperature for 30 minutes. The precipitated solid was collected by filtration to obtain compound (A582), which is the target substance as a white solid (yield: 2.51 g, 65%).
¹H-NMR (400 MHz, CDCl₃); 2.16 (s, 3H), 1.88 (s, 6H), 1.70- 1.61 (m, 6H)
¹⁹F-NMR (376.4 MHz, CDCl₃) δ (ppm): -57.3- -57.5 (m, 9F), - 138.0- -138.2 (m, 4F) ,-139.7- -140.0 (m, 4F), -142.1- -142.3 (m, 2F), -148.2~ -148.3 (m, 4F), -150.3- -150.4 (m, 2F)

### Example 20' (metal adhesion evaluation of compound (A582))

Boiling washing was performed with isopropyl alcohol on the glass substrate, and after further washing by UV/ozone, it was placed in the vapor deposition equipment, and evacuated to no more than 1.0×10⁻⁴ Pa by a vacuum pump. The compound (A582) was deposited to 50 nm at 0.1 nm/sec on a glass substrate on which a metal mask having a 20 mm × 10 mm opening was arranged. Subsequently, the metal mask was removed, and ytterbium was vacuum deposited to 5 nm at a deposition rate of 0.01 nm/sec, and subsequently, silver was vacuum deposited to 12 nm at a deposition rate of 0.1 nm/sec. Ytterbium and silver did not form a film on a portion where compound (A582) is deposited, and a 20 mm × 10 mm transparent area was formed.

### Example 21: Synthesis of compound (A584)

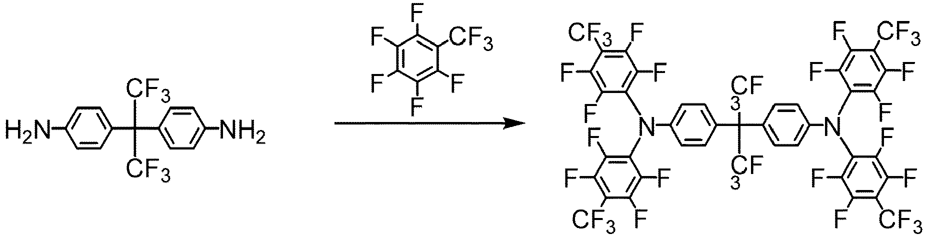

Under a nitrogen atmosphere, 2,2-bis (4-aminophenyl)hexafluoropropane (2.00 g, 6.0 mmol), perfluorotoluene (11.4 g, 48.0 mmol), tripotassium phosphate (12.8 g, 60.0 mmol), and 18 crown 6 ether (103 mg, 0.40 mmol) were suspended in dimethylsulfoxide (60 mL), and stirred at 100°C for 24 hours. After cooling to room temperature, water (120 ml) and methanol (50 ml) were added to the reaction solution, and were stirred at room temperature for 30 minutes. The precipitated solid was collected by filtration to obtain compound (A584), which is the target substance as a white solid (yield: 4.06 g, 57%). The glass transition temperature of compound (A584) was 88°C.
¹H-NMR (400 MHz, CDCl₃); 7.36(d, J=4.00, 4H), 6.92 (d, J=4.00, 4H)
¹⁹F- NMR (376.4 MHz, CDCl₃) δ(ppm) : -57.3 (t, J=22.6 Hz, 12F), -65.2 (s, 6F), -139.9- -140.1 (m, 8F), -144.9- -145.0 (m,8F)

### Example 21' (metal adhesion evaluation of compound (A584))

Boiling washing was performed with isopropyl alcohol on the glass substrate, and after further washing by UV/ozone, it was placed in the vapor deposition equipment, and evacuated to no more than 1.0×10⁻⁴ Pa by a vacuum pump. The compound (A584) was deposited to 100 nm at 0.2 nm/sec on a glass substrate on which a metal mask having a 20 mm × 10 mm opening was arranged. Subsequently, the metal mask was removed, and silver was vacuum deposited to 30 nm at a deposition rate of 0.2 nm/sec. Silver did not form a film on a portion where compound (A584) is deposited, and a 20 mm × 10 mm transparent area was formed.

### Example 22: Synthesis of intermediate (AA5)

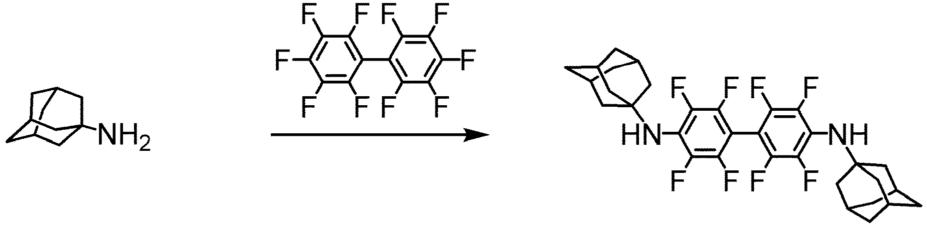

Under a nitrogen atmosphere, perfluorobiphenyl (3.34 g, 10.0 mmol), 1-aminoadamantane (3.03 g, 20.0 mmol) and tripotassium phosphate (5.31 g, 25.0 mmol) were suspended in dimethylsulfoxide (40 mL) and cyclopentylmethyl ether (10 mL), and stirred at 100°C for 24 hours. After cooling to room temperature, water (60 ml) and methanol (40 ml) were added to the reaction solution, and were stirred at room temperature for 30 minutes. The precipitated solid was collected by filtration to obtain intermediate (AA5), which is the target substance as a white solid (yield: 5.08 g, yield: 85%). ¹H-NMR (400 MHz, CDCl₃) ; 3.48 (s, 2H), 2.14 (s, 6H), 1.87 (s, 12H), 1.72- 1.64 (m, 12H)
¹⁹F- NMR (376.4 MHz, CDCl₃) δ (ppm): -142.0~ -142.1 (m, 4F), - 151.8~ -151.9 (m, 4F)

### Example 23: Synthesis of compound (A526)

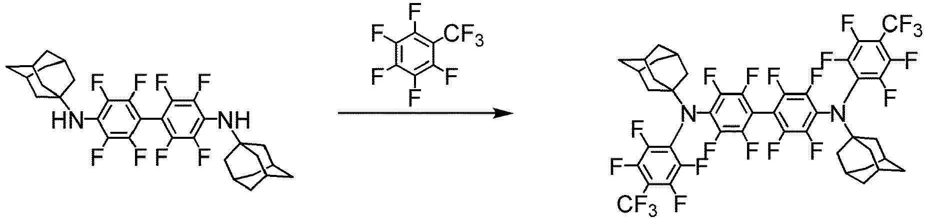

Under a nitrogen atmosphere, AA5 (2.98 g, 5.0 mmol), perfluorotoluene (5.90 g, 25.0 mmol), 18 crown 6 ether (66.1 mg, 0.25 mmol) and tripotassium phosphate (5.31 g, 25.0 mmol) were suspended in dimethylsulfoxide (50 mL), and stirred at 100°C for 24 hours. After cooling to room temperature, water (60 ml) and methanol (18 ml) were added to the reaction solution, and were stirred at room temperature for 30 minutes. The precipitated solid was collected by filtration to obtain compound (A526), which is the target substance as a white solid (yield: 4.08 g, 79%). The glass transition temperature of the compound (A526) was 130°C.
¹H-NMR (400 MHz, CDCl₃); 2.18 (s, 6H), 1.94 (s, 12H), 1.71-1.63 (m, 12H)
¹⁹F- NMR (376.4 MHz, CDCl₃) δ (ppm): -57.4 (t, J=22.6 Hz, 6F), -137.8- -137.93 (m, 4F), -138.9~ -139.0 (m, 4F), -139.5~ - 139.5 (m, 4F), -142.2- -142.4 (m, 4F)

### Example 23' (Metal adhesion evaluation of compound (A526))

Boiling washing was performed with isopropyl alcohol on the glass substrate, and after further washing by UV/ozone, it was placed in the vapor deposition equipment, and evacuated to no more than 1.0×10⁻⁴ Pa by a vacuum pump. The compound (A526) was deposited to 50 nm at 0.2 nm/sec on a glass substrate on which a metal mask having a 20 mm × 10 mm opening was arranged. Subsequently, the metal mask was removed, and bismuth was vacuum deposited to 20 nm at a deposition rate of 0.1 nm/sec. Bismuth did not form a film on a portion where compound (A526) is deposited, and a 20 mm × 10 mm transparent area was formed.

### Example 24: Synthesis of intermediate (AA6)

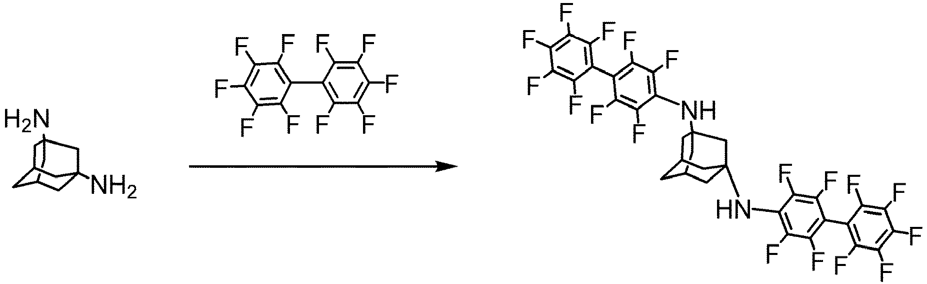

Under a nitrogen atmosphere, 1,3-diaminoadamantane (665 mg, 4.0 mmol), perfluorobiphenyl (2.74 g, 8.2 mmol) and tripotassium phosphate (4.25 g, 20.0 mmol) were suspended in dimethylsulfoxide (40 mL) and cyclopentylmethyl ether (10 mL), and stirred at 100°C for 24 hours. After cooling to room temperature, the mixture was separated using pure water and chloroform, and the organic layer was further washed with saturated aqueous sodium chloride solution. The organic layer was dried over anhydrous magnesium sulfate and purified by silica gel column chromatography (hexanes) to obtain intermediate (AA6), which is the target substance as a white solid (yield: 2.36 g, 74%). Compound identification was performed by FDMS measurement. FDMS: 794

### Example 25: Synthesis of compound (A591)

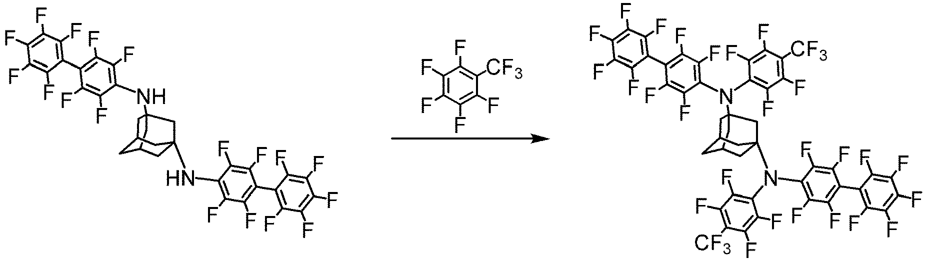

Under a nitrogen atmosphere, AA6 (2.38 g, 3.0 mmol) perfluorotoluene (2.12 g, 9.0 mmol), 18 crown 6 ether (793 mg, 3.0 mmol) and tripotassium phosphate (3.18 g, 15.0 mmol) were suspended in dimethylsulfoxide (30 mL), and stirred at 100°C for 24 hours. After cooling to room temperature, water (30 ml) and methanol (30 ml) were added to the reaction solution, and were stirred at room temperature for 30 minutes. The precipitated solid was collected by filtration to obtain compound (A591), which is the target substance as a white solid (yield: 3.02 g, 82%). The glass transition temperature of the compound (A591) was 93°C.
¹H-NMR (400 MHz, THF-d8); 2.36 (s, 2H), 2.18 (s, 2H), 2.01-1.86 (m, 8H), 1.62 (s, 2H)
¹⁹F- NMR (376.4 MHz, THF-d8) δ (ppm): -57.8 (t, J=22.6 Hz, 6F), -136.6- -136.7 (m, 4F), -137.3~ -137.5 (m, 4F), -138.0~ - 138.1 (m, 4F), -138.6- -138.7 (m, 4F), -141.3- -141.5 (m, 4F), -150.8- -150.9 (m, 2F), -161.2- -161.3 (m, 4F)

### Example 25' (Metal adhesion evaluation of compound (A591))

Boiling washing was performed with isopropyl alcohol on the glass substrate, and after further washing by UV/ozone, it was placed in the vapor deposition equipment, and evacuated to no more than 1.0×10⁻⁴ Pa by a vacuum pump. The compound (A591) was deposited to 50 nm at 0.1 nm/sec on a glass substrate on which a metal mask having a 20 mm × 10 mm opening was arranged. Subsequently, the metal mask was removed, and lead was vacuum deposited to 20 nm at a deposition rate of 0.2 nm/sec. Lead did not form a film on a portion where compound (A591) is deposited, and a 20 mm × 10 mm transparent area was formed.

### Example 26: Synthesis of intermediate (AA7)

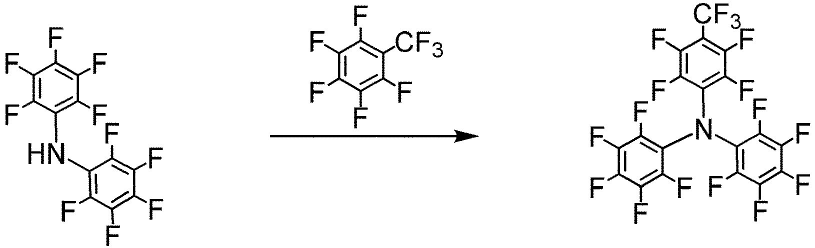

Under a nitrogen atmosphere, bis (pentafluorophenyl)amine (6.98 g, 20.0 mmol), perfluorotoluene (11.8 g, 50.0 mmol), 18 crown 6 ether (5.29 g, 20.0 mmol) and tripotassium phosphate (8.49 g, 40.0 mmol) were suspended in dimethylsulfoxide (80 mL), and stirred at 100°C for 24 hours. After cooling to room temperature, the mixture was separated using pure water and chloroform, and the organic layer was further washed with saturated aqueous sodium chloride solution. The organic layer was dried over anhydrous magnesium sulfate and purified by silica gel column chromatography to obtain compound (AA7), which is the target substance as a white solid (yield 8.65 g, 70%). Compound identification was performed by FDMS measurement. FDMS: 565

### Example 27: Synthesis of intermediate (AA8)

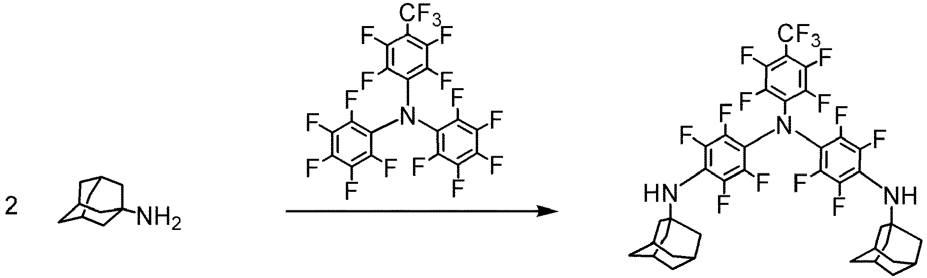

Under a nitrogen atmosphere, 1-aminoadamantane (1.24 g, 8.2 mmol), AA7 (2.26 g, 4.0 mmol), and tripotassium phosphate (2.55 g, 12.0 mmol) were suspended in dimethylsulfoxide (40 mL), and stirred at 100°C for 24 hours. After cooling to room temperature, the mixture was separated using pure water and chloroform, and the organic layer was further washed with saturated aqueous sodium chloride solution. The organic layer was dried over anhydrous magnesium sulfate and purified by silica gel column chromatography to obtain intermediate (AA8), which is the target substance as a white solid (yield: 2.89 g, 87%). Compound identification was performed by FDMS measurement. FDMS: 827

### Example 28: Synthesis of compound (A589)

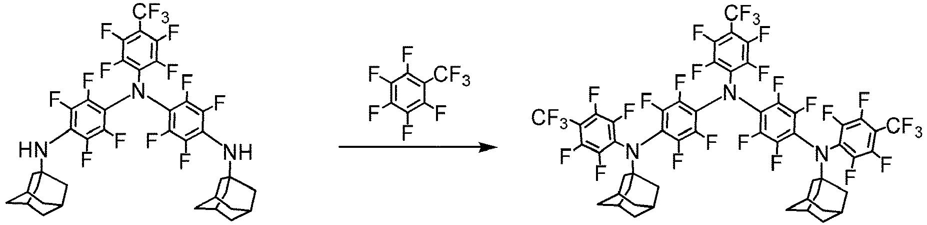

Under a nitrogen atmosphere, AA8 (2.07 g, 2.5 mmol), perfluorotoluene (1.77 g, 7.5 mmol), 18 crown 6 ether (33.0 mg, 0.13 mmol) and tripotassium phosphate (2.65 g, 12.5 mmol) were suspended in dimethylsulfoxide (50 mL), and stirred at 100°C for 24 hours. After cooling to room temperature, water (50 ml) and methanol (50 ml) were added to the reaction solution, and were stirred at room temperature for 30 minutes. The precipitated solid was collected by filtration to obtain compound (A589), which is the target substance as a white solid (yield: 2.46 g, 78%). The glass transition temperature of compound (A526) was 138°C.
¹H-NMR (400 MHz, CDCl₃); 2.15 (s, 6H), 1.87 (s, 12H), 1.69-1.60 (m, 12H)
¹⁹F- NMR (376.4 MHz, CDCl₃) δ (ppm): -57.2- -57.5 (m, 9F), - 138.0~ -138.2 (m, 4F), -138.7- -138.8 (m, 4F), -140.3- -140.4 (m, 2F), -142.1- -142.4 (m, 4F), -148.5- -148.6 (m, 2F), - 150.6~ -150.7 (m, 4F)

### Example 28' (Metal adhesion evaluation of compound (A589))

Boiling washing was performed with isopropyl alcohol on the glass substrate, and after further washing by UV/ozone, it was placed in the vapor deposition equipment, and evacuated to no more than 1.0×10⁻⁴ Pa by a vacuum pump. The compound (A589) was deposited to 50 nm at 0.2 nm/sec on a glass substrate on which a metal mask having a 20 mm × 10 mm opening was arranged. Subsequently, the metal mask was removed, and ytterbium was vacuum deposited to 20 nm at a deposition rate of 0.1 nm/sec. Silver did not form a film on a portion where compound (A589) is deposited, and a 20 mm × 10 mm transparent area was formed.

### Example 29' (Metal adhesion evaluation of compound (B51))

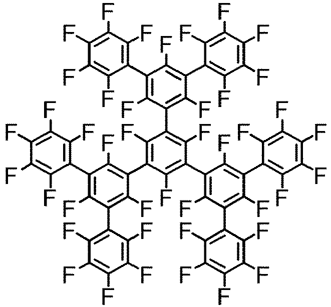

Boiling washing was performed with isopropyl alcohol on the glass substrate, and after further washing by UV/ozone, it was placed in the vapor deposition equipment, and evacuated to no more than 1.0×10⁻⁴ Pa by a vacuum pump. The compound (B51) was deposited to 20 nm at 0.1 nm/sec on a glass substrate on which a metal mask having a 20 mm × 10 mm opening was arranged. Subsequently, the metal mask was removed, and silver was vacuum deposited to 20 nm at a deposition rate of 0.1 nm/sec. Silver did not form a film on a portion where compound (B51) is deposited, and a 20 mm × 10 mm transparent area was formed.

### Example 30: Synthesis of compound (A565)

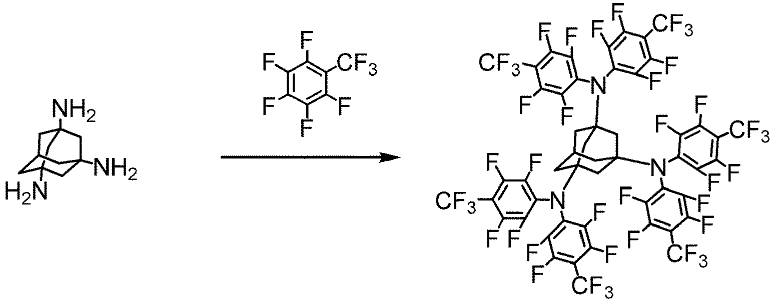

Under a nitrogen atmosphere, 1,3,5-triaminoadamantane (0.91 g, 5.0 mmol), perfluorotoluene (14.2 g, 60 mmol), 18 crown 6 ether (66.1 mg, 0.25 mmol) and tripotassium phosphate (15.9 g, 75 mmol) were suspended in dimethylsulfoxide (50 mL), and stirred at 100°C for 24 hours. After cooling to room temperature, water (50 ml) and methanol (50 ml) were added to the reaction solution, and were stirred at room temperature for 30 minutes. The precipitated solid was collected by filtration to obtain compound (A565), which is the target substance as a white solid (yield: 5.68 g, 77%). Compound identification was performed by FDMS measurement. FDMS: 1477

### Example 30' (Metal adhesion evaluation of compound (A565))

Boiling washing was performed with isopropyl alcohol on the glass substrate, and after further washing by UV/ozone, it was placed in the vapor deposition equipment, and evacuated to no more than 1.0×10⁻⁴ Pa by a vacuum pump. The compound (A565) was deposited to 30 nm at 0.2 nm/sec on a glass substrate on which a metal mask having a 20 mm × 10 mm opening was arranged. Subsequently, the metal mask was removed, and copper was vacuum deposited to 30 nm at a deposition rate of 0.1 nm/sec. Silver did not form a film on a portion where compound (A565) is deposited, and a 20 mm × 10 mm transparent area was formed.

### Example 31: Synthesis of compound (A568)

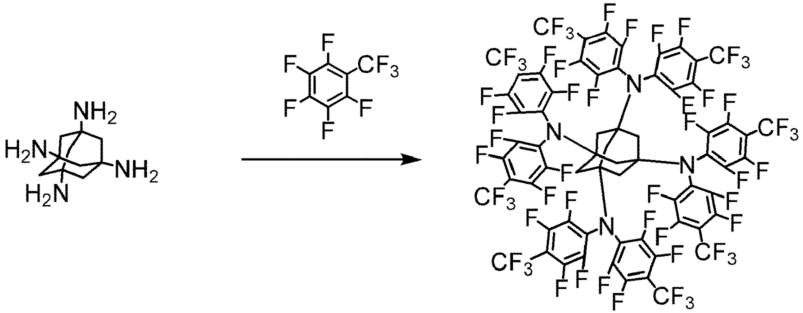

Under a nitrogen atmosphere, 1,3,5,7-tetraaminoadamantane (0.98 g, 5.0 mmol), perfluorotoluene (18.9 g, 80 mmol), 18 crown 6 ether (66.1 mg, 0.25 mmol), and tripotassium phosphate (21.2 g, 100 mmol) were suspended in dimethylsulfoxide (50 mL), and stirred at 100°C for 24 hours. After cooling to room temperature, water (50 ml) and methanol (50 ml) were added to the reaction solution, and were stirred at room temperature for 30 minutes. The precipitated solid was collected by filtration to obtain compound (A568), which is the target substance as a white solid (yield: 6.58 g, 68%). Compound identification was performed by FDMS measurement. FDMS: 1924

### Example 31' (Metal adhesion evaluation of compound (A568))

Boiling washing was performed with isopropyl alcohol on the glass substrate, and after further washing by UV/ozone, it was placed in the vapor deposition equipment, and evacuated to no more than 1.0×10⁻⁴ Pa by a vacuum pump. The compound (A568) was deposited to 30 nm at 0.2 nm/sec on a glass substrate on which a metal mask having a 20 mm × 10 mm opening was arranged. Subsequently, the metal mask was removed, and silver was vacuum deposited to 30 nm at a deposition rate of 0.1 nm/sec. Silver did not form a film on a portion where compound (A568) is deposited, and a 20 mm × 10 mm transparent area was formed.

### Example 32: Synthesis of compound (A390)

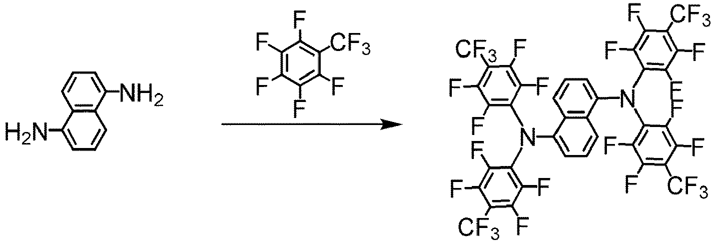

Under a nitrogen atmosphere, 1,5-diaminonaphthalene (0.791 g, 5.0 mmol), perfluorotoluene (9.44 g, 40 mmol) and tripotassium phosphate (10 .6 g, 50 mmol) were suspended in dimethylsulfoxide (50 mL), and stirred at 100°C for 24 hours. After cooling to room temperature, water (50 ml) and methanol (50 ml) were added to the reaction solution, and were stirred at room temperature for 30 minutes. The precipitated solid was collected by filtration to obtain compound (A390), which is the target substance as a white solid (yield 4.09 g, 80%). Compound identification was performed by FDMS measurement. FDMS: 1022

### Example 32' (Metal adhesion evaluation of compound (A390))

Boiling washing was performed with isopropyl alcohol on the glass substrate, and after further washing by UV/ozone, it was placed in the vapor deposition equipment, and evacuated to no more than 1.0×10⁻⁴ Pa by a vacuum pump. The compound (A390) was deposited to 50 nm at 0.2 nm/sec on a glass substrate on which a metal mask having a 20 mm × 10 mm opening was arranged. Subsequently, the metal mask was removed, and magnesium was vacuum deposited to 30 nm at a deposition rate of 0.1 nm/sec. Magnesium did not form a film on a portion where compound (A390) is deposited, and a 20 mm × 10 mm transparent area was formed.

### Example 33: Synthesis of intermediate (AA9)

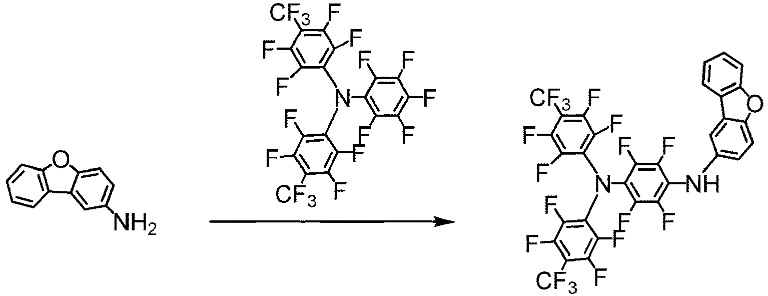

Under a nitrogen atmosphere, 2-aminodibenzofuran (1.47 g, 10.0 mmol), AA2 (6.15 g, 10.0 mmol) and tripotassium phosphate (2.23 g, 10.5 mmol) were suspended in dimethylsulfoxide (50 mL), and stirred at 100°C for 24 hours. After cooling to room temperature, the mixture was separated using pure water and chloroform, and the organic layer was further washed with saturated aqueous sodium chloride solution. The organic layer was dried over anhydrous magnesium sulfate and purified by silica gel column chromatography (hexane) to obtain intermediate (AA9), which is the target substance as a white solid (yield: 5.16 g, 66%). Compound identification was performed by FDMS measurement. FDMS: 778

### Example 34: Synthesis of compound (A600)

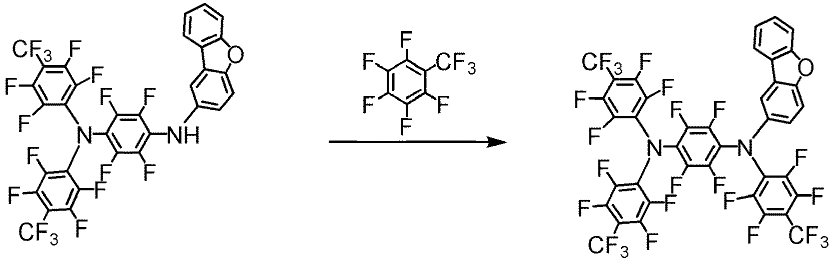

Under a nitrogen atmosphere, AA9 (3.11 g, 4.0 mmol), perfluorotoluene (2.83 g, 12 mmol) and tripotassium phosphate (4.25 g, 20 mmol) were suspended in dimethylsulfoxide (50 mL), and stirred at 100°C for 24 hours. After cooling to room temperature, water (50 ml) and methanol (50 ml) were added to the reaction solution, and were stirred at room temperature for 30 minutes. The precipitated solid was collected by filtration to obtain compound (A600), which is the target substance as a white solid (yield: 2.57 g, 65%). Compound identification was performed by FDMS measurement. FDMS: 994

### Example 34' (Metal adhesion evaluation of compound (A600))

Boiling washing was performed with isopropyl alcohol on the glass substrate, and after further washing by UV/ozone, it was placed in the vapor deposition equipment, and evacuated to no more than 1.0×10⁻⁴ Pa by a vacuum pump. The compound (A600) was deposited to 50 nm at 0.2 nm/sec on a glass substrate on which a metal mask having a 20 mm × 10 mm opening was arranged. Subsequently, the metal mask was removed, ytterbium was vacuum deposited to 2 nm at a deposition rate of 0.01 nm/sec, and subsequently, magnesium was vacuum deposited to 12 nm at a deposition rate of 0.1 nm/sec. Ytterbium and magnesium did not form a film on a portion where compound (A600) is deposited, and a 20 mm × 10 mm transparent area was formed.

### Example 35: Synthesis of intermediate (AA10)

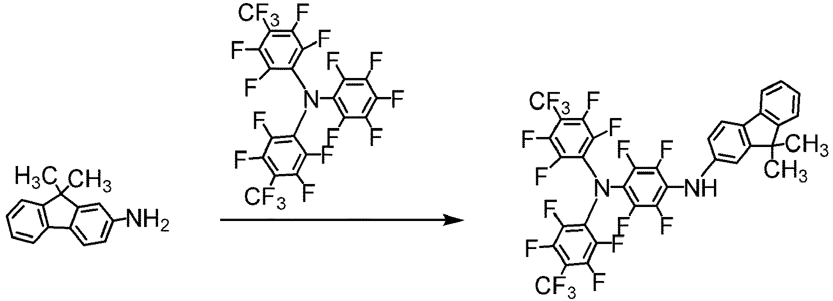

Under a nitrogen atmosphere, 2-amino-9,9-dimethylfluorene (2.09 g, 10.0 mmol), AA2 (6.15 g, 10.0 mmol) and tripotassium phosphate (2.23 g, 10.5 mmol) were suspended in dimethylsulfoxide (50 mL), and stirred at 100°C for 24 hours. After cooling to room temperature, the mixture was separated using pure water and chloroform, and the organic layer was further washed with saturated aqueous sodium chloride solution. The organic layer was dried over anhydrous magnesium sulfate and purified by silica gel column chromatography (hexane) to obtain intermediate (AA10), which is the target substance as a white solid (yield: 6.21 g, 77%). Compound identification was performed by FDMS measurement. FDMS: 804

### Example 36: Synthesis of compound (A601)

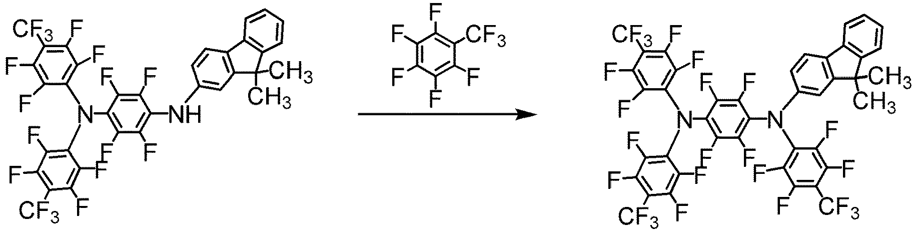

Under a nitrogen atmosphere, AA10 (3.22 g, 4.0 mmol), perfluorotoluene (2.83 g, 12 mmol), and tripotassium phosphate (4.25 g, 20 mmol) were suspended in dimethylsulfoxide (50 mL), and stirred at 100°C for 24 hours. After cooling to room temperature, water (50 ml) and methanol (50 ml) were added to the reaction solution, and were stirred at room temperature for 30 minutes. The precipitated solid was collected by filtration to obtain compound (A601), which is the target substance as a white solid (yield: 2.89 g, 71%). Compound identification was performed by FDMS measurement. FDMS: 1020

### Example 36' (Metal adhesion evaluation of compound (A601))

Boiling washing was performed with isopropyl alcohol on the glass substrate, and after further washing by UV/ozone, it was placed in the vapor deposition equipment, and evacuated to no more than 10×10⁻⁴ Pa by a vacuum pump. The compound (A601) was deposited to 50 nm at 0.2 nm/sec on a glass substrate on which a metal mask having a 20 mm × 10 mm opening was arranged. Subsequently, the metal mask was removed, and magnesium was vacuum deposited to 20 nm at a deposition rate of 0.1 nm/sec. Magnesium did not form a film on a portion where compound (A601) is deposited, and a 20 mm × 10 mm transparent area was formed.

### Example 37: Synthesis of intermediate (AA11)

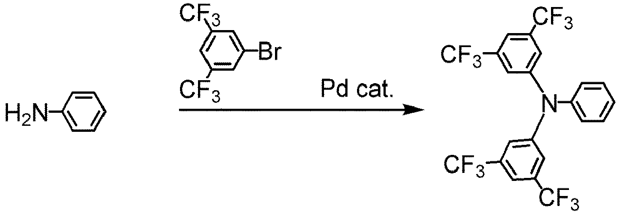

Under a nitrogen stream, a 300 mL three-necked flask was charged with 3,5-bis (trifluoromethyl)bromobenzene (14.7 g, 50.0 mmol), aniline (1.86 g, 20.0 mmol), sodium-tert-butoxide (5.77 g, 60.0 mmol), o-xylene (100 mL), palladium acetate 225 mg (1.0 mmol), and a 25 wt% toluene solution (1.62 g, 2.0 mmol) of tri (tert-butyl)phosphine, and were stirred at 140°C for 3 hours. After cooling to room temperature, the mixture was separated using pure water and toluene, and the organic layer was further washed with a saturated aqueous sodium chloride solution. The organic layer was dried over anhydrous magnesium sulfate and purified by silica gel column chromatography to obtain intermediate (AA11), which is the target substance as a white solid (yield: 7.66 g, 74%). Compound identification was performed by FDMS measurement. FDMS: 517

### Example 38: Synthesis of intermediate (AA12)

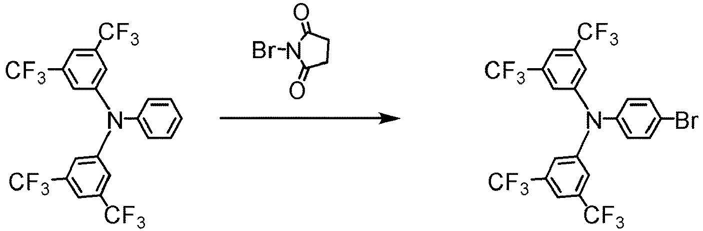

A 300 mL three-neck flask was charged with AA11 (7.55 g, 14.6 mmol), and tetrahydrofuran (75 mL), and stirred at 60°C for 10 min. Further, N-bromosuccinimide was added every 10 minutes for four times (total addition amount: 5.72 g, 32.3 mmol). After cooling to room temperature, the mixture was separated using an aqueous sodium thiosulfate solution and toluene, and the organic layer was further washed with a saturated aqueous sodium chloride solution. The organic layer was dried over anhydrous magnesium sulfate and purified by silica gel column chromatography to obtain an intermediate (AA12), which is the target substance as a white solid (yield: 6.23 g, 72%). Compound identification was performed by FDMS measurement. FDMS: 595

### Example 39: Synthesis of intermediate (AA13)

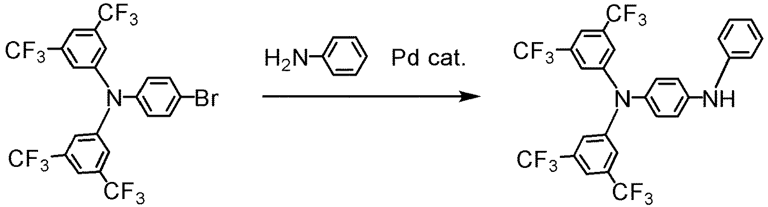

Under a nitrogen stream, AA12 (2.98 g, 5.0 mmol), aniline (0.61 g, 6.5 mmol), sodium-tert-butoxide (0.72 g, 7.5 mmol), o-xylene (25 mL), palladium acetate (0.112 mg, 0.05 mmol), and 4,5-bis (diphenylphosphino)-9,9-dimethylxanthene (86.8 mg, 0.15 mmol) were added to a 100 mL three-neck flask, and the mixture was stirred at 140°C for 5 hours. After cooling to room temperature, the mixture was separated using pure water and toluene, and the organic layer was further washed with a saturated aqueous sodium chloride solution. The organic layer was dried over anhydrous magnesium sulfate and purified by silica gel column chromatography to obtain intermediate (AA13), which is the target substance as a white solid (yield: 2.42 g, 80%). Compound identification was performed by FDMS measurement. FDMS: 608

### Example 40: Synthesis of compound (A608)

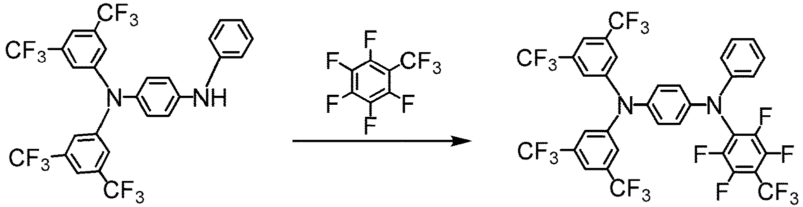

Under a nitrogen atmosphere, AA13 (2.13 g, 3.5 mmol), perfluorotoluene (3.30 g, 14 mmol), and tripotassium phosphate (3.71 g, 18 mmol) were suspended in dimethylsulfoxide (50 mL), and stirred at 100°C for 24 hours. After cooling to room temperature, the mixture was separated using pure water and chloroform, and the organic layer was further washed with saturated aqueous sodium chloride solution. The organic layer was dried over anhydrous magnesium sulfate and purified by silica gel column chromatography to obtain compound (A608), which is the target substance as a white solid (yield: 2.20 g, 85%). Compound identification was performed by FDMS measurement. FDMS: 824

### Example 40' (Metal adhesion evaluation of compound (A608))

Boiling washing was performed with isopropyl alcohol on the glass substrate, and after further washing by UV/ozone, it was placed in the vapor deposition equipment, and evacuated to no more than 10×10⁻⁴ Pa by a vacuum pump. The compound (A608) was deposited to 50 nm at 0.2 nm/sec on a glass substrate on which a metal mask having a 20 mm × 10 mm opening was arranged. Subsequently, the metal mask was removed, and silver was vacuum deposited to 10 nm at a deposition rate of 0.1 nm/sec. Silver did not form a film on a portion where compound (A608) is deposited, and a 20 mm × 10 mm transparent area was formed.

### Example 41' (Metal adhesion evaluation of compound (B11))

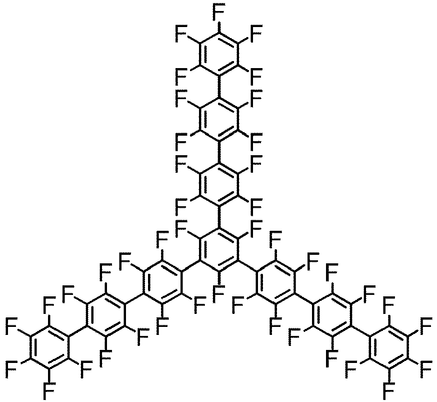

Boiling washing was performed with isopropyl alcohol on the glass substrate, and after further washing by UV/ozone, it was placed in the vapor deposition equipment, and evacuated to no more than 10×10⁻⁴ Pa by a vacuum pump. The compound (B11) was deposited to 20 nm at 0.1 nm/sec on a glass substrate on which a metal mask having a 20 mm × 10 mm opening was arranged. Subsequently, the metal mask was removed, and silver was vacuum deposited to 10 nm at a deposition rate of 0.1 nm/sec. Silver did not form a film on a portion where compound (B11) is deposited, and a 20 mm × 10 mm transparent area was formed.

### Example 42' (Metal adhesion evaluation of compound (B78))

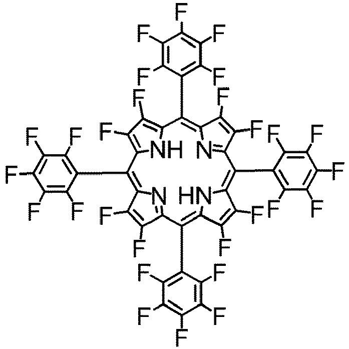

Boiling washing was performed with isopropyl alcohol on the glass substrate, and after further washing by UV/ozone, it was placed in the vapor deposition equipment, and evacuated to no more than 10×10⁻⁴ Pa by a vacuum pump. The compound (B78) was deposited to 20 nm at 0.1 nm/sec on a glass substrate on which a metal mask having a 20 mm × 10 mm opening was arranged. Subsequently, the metal mask was removed, and magnesium was vacuum deposited to 10 nm at a deposition rate of 0.1 nm/sec. Magnesium did not form a film on a portion where compound (B78) is deposited, and a 20 mm × 10 mm transparent area was formed.

### Example 43' (Metal adhesion evaluation of compound (B70))

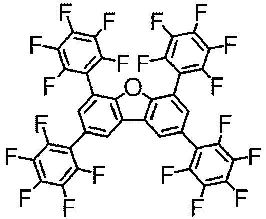

Boiling washing was performed with isopropyl alcohol on the glass substrate, and after further washing by UV/ozone, it was placed in the vapor deposition equipment, and evacuated to no more than 1.0×10⁻⁴ Pa by a vacuum pump. The compound (B70) was deposited to 20 nm at 0.1 nm/sec on a glass substrate on which a metal mask having a 20 mm × 10 mm opening was arranged. Subsequently, the metal mask was removed, and silver was vacuum deposited to 10 nm at a deposition rate of 0.1 nm/sec. Silver did not form a film on a portion where compound (B70) is deposited, and a 20 mm × 10 mm transparent area was formed.

### Example 44' (Metal adhesion evaluation of compound (B26))

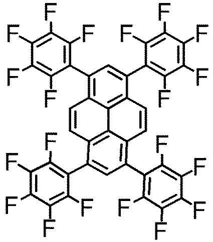

Boiling washing was performed with isopropyl alcohol on the glass substrate, and after further washing by UV/ozone, it was placed in the vapor deposition equipment, and evacuated to no more than 1.0×10⁻⁴ Pa by a vacuum pump. The compound (B26) was deposited to 20 nm at 0.1 nm/sec on a glass substrate on which a metal mask having a 20 mm × 10 mm opening was arranged. Subsequently, the metal mask was removed, and silver was vacuum deposited to 10 nm at a deposition rate of 0.1 nm/sec. Silver did not form a film on a portion where compound (B26) is deposited, and a 20 mm × 10 mm transparent area was formed.

### Example 45' (Metal adhesion evaluation of compound (B104))

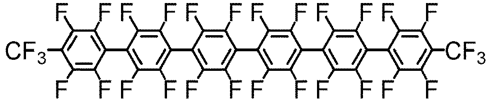

Boiling washing was performed with isopropyl alcohol on the glass substrate, and after further washing by UV/ozone, it was placed in the vapor deposition equipment, and evacuated to no more than 1.0×10⁻⁴ Pa by a vacuum pump. The compound (B104) was deposited to 20 nm at 0.1 nm/sec on a glass substrate on which a metal mask having a 20 mm × 10 mm opening was arranged. Subsequently, the metal mask was removed, and silver was vacuum deposited to 10 nm at a deposition rate of 0.1 nm/sec. Silver did not form a film on a portion where compound (B104) is deposited, and a 20 mm × 10 mm transparent area was formed.

### Example 46' (Metal adhesion evaluation of compound (Bill))

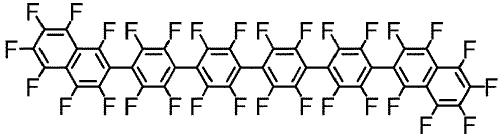

Boiling washing was performed with isopropyl alcohol on the glass substrate, and after further washing by UV/ozone, it was placed in the vapor deposition equipment, and evacuated to no more than 1.0×10⁻⁴ Pa by a vacuum pump. The compound (B111) was deposited to 20 nm at 0.1 nm/sec on a glass substrate on which a metal mask having a 20 mm × 10 mm opening was arranged. Subsequently, the metal mask was removed, and silver was vacuum deposited to 10 nm at a deposition rate of 0.1 nm/sec. Silver did not form a film on a portion where compound (Bill) is deposited, and a 20 mm × 10 mm transparent area was formed.

### Example 47: Synthesis of compound (A597)

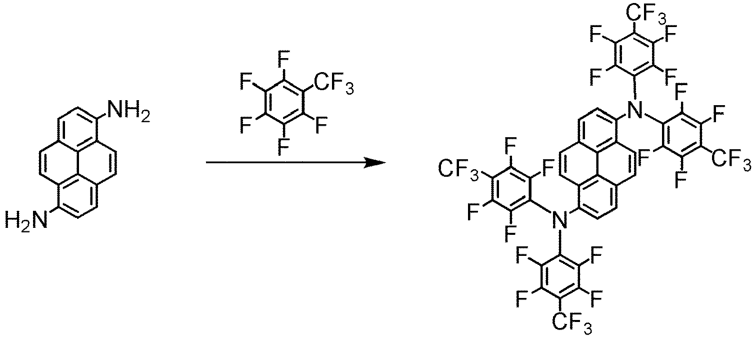

Under a nitrogen atmosphere, 1,6-diaminopyrene (1.16 g, 5.0 mmol), perfluorotoluene (9.44 g, 40 mmol) and tripotassium phosphate (10 .6 g, 50 mmol) were suspended in dimethylsulfoxide (50 mL), and stirred at 100°C for 24 hours. After cooling to room temperature, water (50 ml) and methanol (50 ml) were added to the reaction solution, and were stirred at room temperature for 30 minutes. The precipitated solid was collected by filtration to obtain compound (A597), which is the target substance as a white solid (yield: 4.39 g, 80%). Compound identification was performed by FDMS measurement. FDMS: 1096

### Example 47' (Metal adhesion evaluation of compound (A597))

Boiling washing was performed with isopropyl alcohol on the glass substrate, and after further washing by UV/ozone, it was placed in the vapor deposition equipment, and evacuated to no more than 1.0×10⁻⁴ Pa by a vacuum pump. The compound (A597) was deposited to 50 nm at 0.1 nm/sec on a glass substrate on which a metal mask having a 20 mm × 10 mm opening was arranged. Subsequently, the metal mask was removed, and silver was vacuum deposited to 5 nm at a deposition rate of 0.1 nm/sec. Silver did not form a film on a portion where compound (A597) is deposited, and a 20 mm × 10 mm transparent area was formed.

### Example 48: Synthesis of intermediate (AA14)

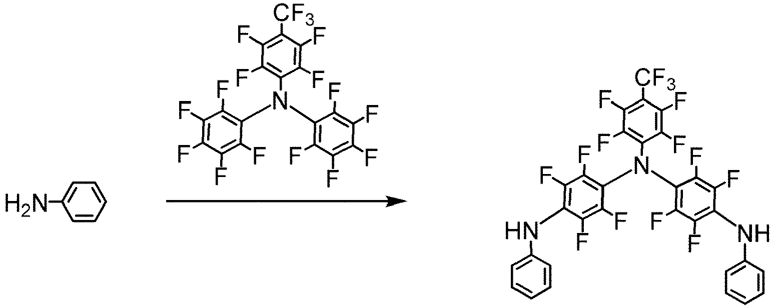

Under a nitrogen atmosphere, aniline (0.76 g, 8.2 mmol), AA7 (2.26 g, 4.0 mmol) and tripotassium phosphate (2.55 g, 12.0 mmol) were suspended in dimethylsulfoxide (40 mL), and stirred at 100°C for 24 hours. After cooling to room temperature, the mixture was separated using pure water and chloroform, and the organic layer was further washed with saturated aqueous sodium chloride solution. The organic layer was dried over anhydrous magnesium sulfate and purified by silica gel column chromatography to obtain intermediate (AA14), which is the target substance as a white solid (yield: 2.28 g, 80%). Compound identification was performed by FDMS measurement. FDMS: 711

### Example 49: Synthesis of compound (A657)

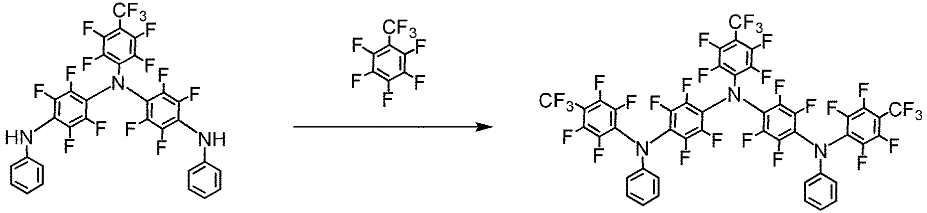

Under a nitrogen atmosphere, AA14 (1.78 g, 2.5 mmol), perfluorotoluene (1.77 g, 7.5 mmol), 18 crown 6 ether (33.0 mg, 0.13 mmol) and tripotassium phosphate (2.65 g, 12.5 mmol) were suspended in dimethylsulfoxide (50 mL), and stirred at 100°C for 24 hours. After cooling to room temperature, water (50 ml) and methanol (50 ml) were added to the reaction solution, and were stirred at room temperature for 30 minutes. The precipitated solid was collected by filtration to obtain compound (A657), which is the target substance as a white solid (yield: 2.29 g, 80%). Compound identification was performed by FDMS measurement. FDMS: 1143

### Example 49' (Metal adhesion evaluation of compound (A657))

Boiling washing was performed with isopropyl alcohol on the glass substrate, and after further washing by UV/ozone, it was placed in the vapor deposition equipment, and evacuated to no more than 1.0×10⁻⁴ Pa by a vacuum pump. The compound (A657) was deposited to 50 nm at 0.2 nm/sec on a glass substrate on which a metal mask having a 20 mm × 10 mm opening was arranged. Subsequently, the metal mask was removed, and silver was vacuum deposited to 10 nm at a deposition rate of 0.1 nm/sec. Silver did not form a film on a portion where compound (A657) is deposited, and a 20 mm × 10 mm transparent area was formed.

### Example 50: Synthesis of intermediate (AA15)

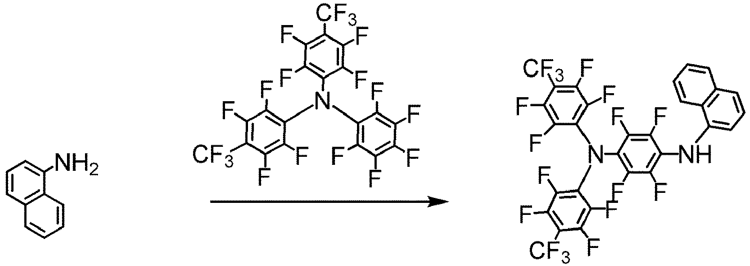

Under a nitrogen atmosphere, 1-naphthylamine (1.43 g, 10.0 mmol), AA2 (6.15 g, 10.0 mmol) and tripotassium phosphate (2.23 g, 10.5 mmol) were suspended in dimethylsulfoxide (50 mL), and stirred at 100°C for 24 hours. After cooling to room temperature, the mixture was separated using pure water and chloroform, and the organic layer was further washed with saturated aqueous sodium chloride solution. The organic layer was dried over anhydrous magnesium sulfate and purified by silica gel column chromatography to obtain intermediate (AA15), which is the target substance as a white solid (yield: 5.71 g, 73%). Compound identification was performed by FDMS measurement. FDMS: 738

### Example 51: Synthesis of compound (A663)

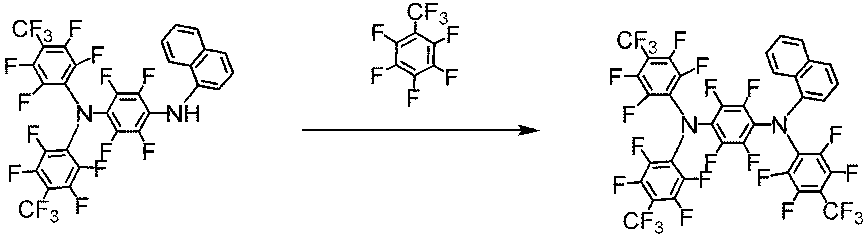

Under a nitrogen atmosphere, AA15 (2.95 g, 4.0 mmol), perfluorotoluene (2.83 g, 12 mmol), and tripotassium phosphate (4.25 g, 20 mmol) were suspended in dimethylsulfoxide (50 mL), and stirred at 100°C for 24 hours. After cooling to room temperature, the mixture was separated using pure water and chloroform, and the organic layer was further washed with saturated aqueous sodium chloride solution. The organic layer was dried over anhydrous magnesium sulfate and purified by silica gel column chromatography to obtain compound (A663), which is the target substance as a white solid (yield: 3.25 g, 85%). Compound identification was performed by FDMS measurement. FDMS: 954

### Example 51' (Metal adhesion evaluation of compound (A663))

Boiling washing was performed with isopropyl alcohol on the glass substrate, and after further washing by UV/ozone, it was placed in the vapor deposition equipment, and evacuated to no more than 1.0×10⁻⁴ Pa by a vacuum pump. The compound (A663) was deposited to 50 nm at 0.2 nm/sec on a glass substrate on which a metal mask having a 20 mm × 10 mm opening was arranged. Subsequently, the metal mask was removed, ytterbium was vacuum deposited to 1 nm at a deposition rate of 0.01 nm/sec, and subsequently, silver and magnesium (1/9) were vacuum deposited to 15 nm at a deposition rate of 0.1 nm/sec. Ytterbium, and an alloy of silver and magnesium did not form a film on a portion where compound (A663) is deposited, and a 20 mm × 10 mm transparent area was formed.

### Example 52: Synthesis of intermediate (AA16)

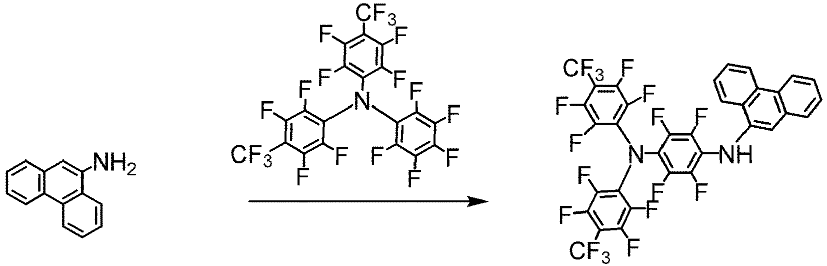

Under a nitrogen atmosphere, 9-aminophenanthrene (1.93 g, 10.0 mmol), AA2 (6.15 g, 10.0 mmol), and tripotassium phosphate (2.23 g, 10.5 mmol) were suspended in dimethylsulfoxide (50 mL), and stirred at 100°C for 24 hours. After cooling to room temperature, the mixture was separated using pure water and chloroform, and the organic layer was further washed with saturated aqueous sodium chloride solution. The organic layer was dried over anhydrous magnesium sulfate and purified by silica gel column chromatography to obtain intermediate (AA16), which is the target substance as a white solid (yield: 6.43 g, 82%). Compound identification was performed by FDMS measurement. FDMS: 788

### Example 53: Synthesis of compound (A665)

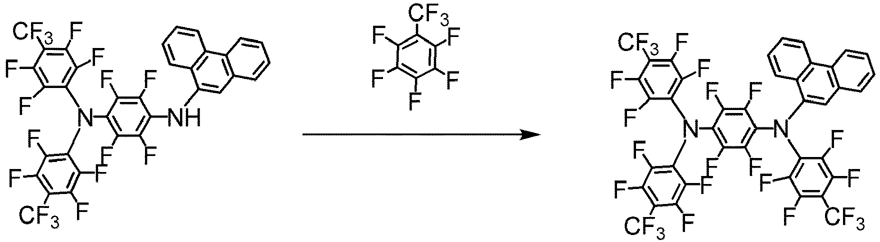

Under a nitrogen atmosphere, AA16 (3.15 g, 4.0 mmol), perfluorotoluene (2.83 g, 12 mmol), and tripotassium phosphate (4.25 g, 20 mmol) were suspended in dimethylsulfoxide (50 mL), and stirred at 100°C for 24 hours. After cooling to room temperature, the mixture was separated using pure water and chloroform, and the organic layer was further washed with saturated aqueous sodium chloride solution. The organic layer was dried over anhydrous magnesium sulfate and purified by silica gel column chromatography to obtain compound (A665), which is the target substance as a white solid (yield: 3.18 g, 79%). Compound identification was performed by FDMS measurement. FDMS: 1004

### Example 53' (Metal adhesion evaluation of compound (A665))

Boiling washing was performed with isopropyl alcohol on the glass substrate, and after further washing by UV/ozone, it was placed in the vapor deposition equipment, and evacuated to no more than 1.0×10⁻⁴ Pa by a vacuum pump. The compound (A665) was deposited to 50 nm at 0.2 nm/sec on a glass substrate on which a metal mask having a 20 mm × 10 mm opening was arranged. Subsequently, the metal mask was removed, ytterbium was vacuum deposited to 1 nm at a deposition rate of 0.01 nm/sec, and subsequently, bismuth was vacuum deposited to 10 nm at a deposition rate of 0.1 nm/sec. Ytterbium and bismuth did not form a film on a portion where compound (A665) is deposited, and a 20 mm × 10 mm transparent area was formed.

### Example 54: Synthesis of intermediate (AA17)

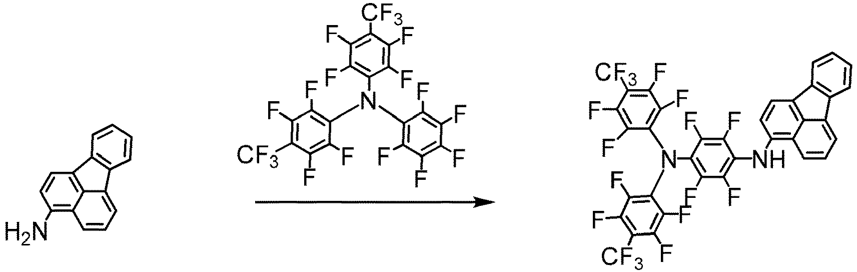

Under a nitrogen atmosphere, 3-aminofluoranthene (2.17 g, 10.0 mmol), AA2 (6.15 g, 10.0 mmol) and tripotassium phosphate (2.23 g, 10.5 mmol) were suspended in dimethylsulfoxide (50 mL), and stirred at 100°C for 24 hours. After cooling to room temperature, the mixture was separated using pure water and chloroform, and the organic layer was further washed with saturated aqueous sodium chloride solution. The organic layer was dried over anhydrous magnesium sulfate and purified by silica gel column chromatography to obtain intermediate (AA17), which is the target substance as a white solid (yield: 6.13 g, 76%). Compound identification was performed by FDMS measurement. FDMS: 812

### Example 55: Synthesis of compound (A668)

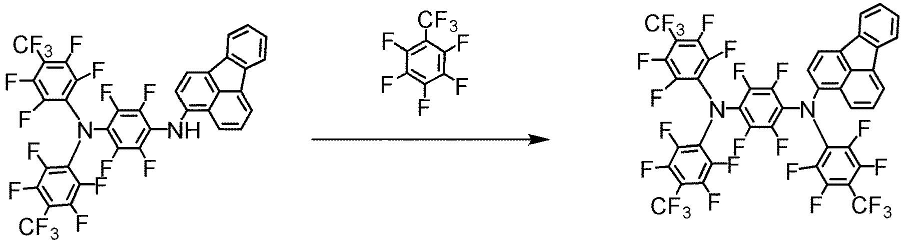

Under a nitrogen atmosphere, AA17 (3.25 g, 4.0 mmol), perfluorotoluene (2.83 g, 12 mmol), and tripotassium phosphate (4.25 g, 20 mmol) were suspended in dimethylsulfoxide (50 mL), and stirred at 100°C for 24 hours. After cooling to room temperature, the mixture was separated using pure water and chloroform, and the organic layer was further washed with saturated aqueous sodium chloride solution. The organic layer was dried over anhydrous magnesium sulfate and purified by silica gel column chromatography to obtain compound (A668), which is the target substance as a white solid (yield: 2.98 g, 73%). Compound identification was performed by FDMS measurement. FDMS: 1028

### Example 55' (Metal adhesion evaluation of compound (A668))

Boiling washing was performed with isopropyl alcohol on the glass substrate, and after further washing by UV/ozone, it was placed in the vapor deposition equipment, and evacuated to no more than 1.0×10⁻⁴ Pa by a vacuum pump. The compound (A668) was deposited to 50 nm at 0.2 nm/sec on a glass substrate on which a metal mask having a 20 mm × 10 mm opening was arranged. Subsequently, the metal mask was removed, and magnesium was vacuum deposited to 15 nm at a deposition rate of 0.1 nm/sec. Magnesium did not form a film on a portion where compound (A668) is deposited, and a 20 mm × 10 mm transparent area was formed.

### Example 56: Synthesis of intermediate (AA18)

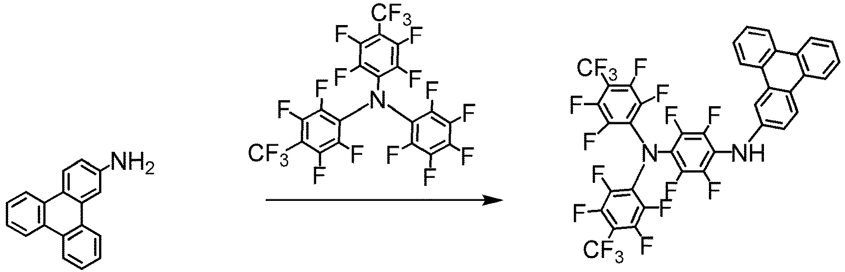

Under a nitrogen atmosphere, 2-aminotriphenylene (2.43 g, 10.0 mmol), AA2 (6.15 g, 10.0 mmol) and tripotassium phosphate (2.23 g, 10.5 mmol) were suspended in dimethylsulfoxide (50 mL), and stirred at 100°C for 24 hours. After cooling to room temperature, the mixture was separated using pure water and chloroform, and the organic layer was further washed with saturated aqueous sodium chloride solution. The organic layer was dried over anhydrous magnesium sulfate and purified by silica gel column chromatography to obtain intermediate (AA18), which is the target substance as a white solid (yield: 7.15 g, 85%). Compound identification was performed by FDMS measurement. FDMS: 838

### Example 57: Synthesis of compound (A669)

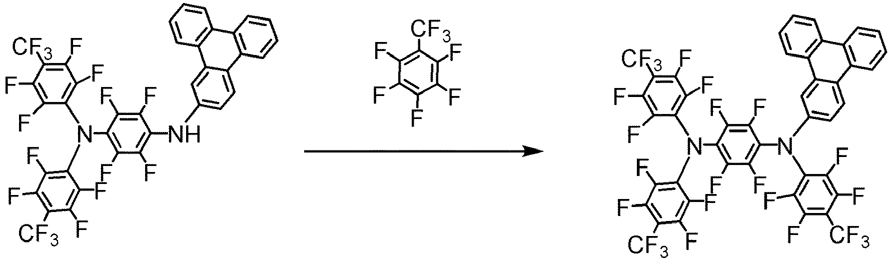

Under a nitrogen atmosphere, AA18 (3.35 g, 4.0 mmol), perfluorotoluene (2.83 g, 12 mmol), and tripotassium phosphate (4.25 g, 20 mmol) were suspended in dimethylsulfoxide (50 mL), and stirred at 100°C for 24 hours. After cooling to room temperature, the mixture was separated using pure water and chloroform, and the organic layer was further washed with saturated aqueous sodium chloride solution. The organic layer was dried over anhydrous magnesium sulfate and purified by silica gel column chromatography to obtain compound (A669), which is the target substance as a white solid (yield: 2.75 g, 65%). Compound identification was performed by FDMS measurement. FDMS: 1054

### Example 57' (Metal adhesion evaluation of compound (A669))

Boiling washing was performed with isopropyl alcohol on the glass substrate, and after further washing by UV/ozone, it was placed in the vapor deposition equipment, and evacuated to no more than 1.0×10⁻⁴ Pa by a vacuum pump. The compound (A669) was deposited to 50 nm at 0.2 nm/sec on a glass substrate on which a metal mask having a 20 mm × 10 mm opening was arranged. Subsequently, the metal mask was removed, and magnesium was vacuum deposited to 15 nm at a deposition rate of 0.1 nm/sec. Magnesium did not form a film on a portion where compound (A669) is deposited, and a 20 mm × 10 mm transparent area was formed.

### Example 58: Synthesis of intermediate (AA19)

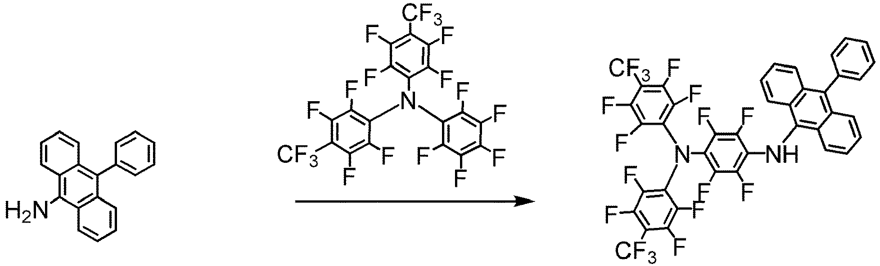

Under a nitrogen atmosphere, 9-amino-10 phenylanthracene (2.69 g, 10.0 mmol), AA2 (6.15 g, 10.0 mmol) and tripotassium phosphate (2.23 g, 10.5 mmol) were suspended in dimethylsulfoxide (50 mL), and stirred at 100°C for 24 hours. After cooling to room temperature, the mixture was separated using pure water and chloroform, and the organic layer was further washed with saturated aqueous sodium chloride solution. The organic layer was dried over anhydrous magnesium sulfate and purified by silica gel column chromatography to obtain intermediate (AA19), which is the target substance as a white solid (yield: 6.13 g, 71%). Compound identification was performed by FDMS measurement. FDMS: 864

### Example 59: Synthesis of compound (A670)

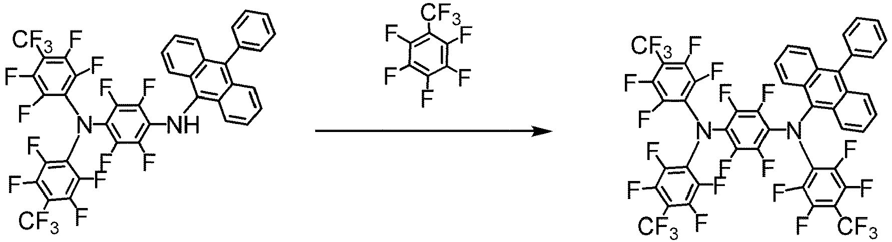

Under a nitrogen atmosphere, AA19 (3.46 g, 4.0 mmol), perfluorotoluene (2.83 g, 12 mmol), and tripotassium phosphate (4.25 g, 20 mmol) were suspended in dimethylsulfoxide (50 mL), and then stirred at 100°C for 24 hours. After cooling to room temperature, the mixture was separated using pure water and chloroform, and the organic layer was further washed with a saturated aqueous sodium chloride solution. The organic layer was dried over anhydrous magnesium sulfate and purified by silica gel column chromatography to obtain compound (A670), which is the target substance as a white solid (yield: 2.64 g, 610). Compound identification was performed by FDMS measurement. FDMS: 1080

### Example 59' (Metal adhesion evaluation of compound (A670))

Boiling washing was performed with isopropyl alcohol on the glass substrate, and after further washing by UV/ozone, it was placed in the vapor deposition equipment, and evacuated to no more than 1.0×10⁻⁴ Pa by a vacuum pump. The compound (A670) was deposited to 50 nm at 0.2 nm/sec on a glass substrate on which a metal mask having a 20 mm × 10 mm opening was arranged. Subsequently, the metal mask was removed, and ytterbium was vacuum deposited to 15 nm at a deposition rate of 0.1 nm/sec. Ytterbium did not form a film on a portion where compound (A670) is deposited, and a 20 mm × 10 mm transparent area was formed.

### Example 60: Synthesis of intermediate (AA20)

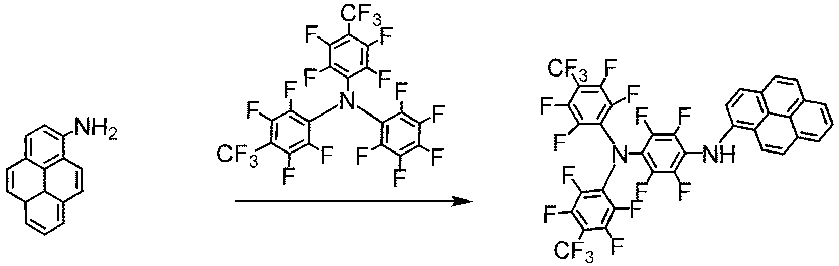

Under a nitrogen atmosphere, 1-aminopyrene (2.19 g, 10.0 mmol), AA2 (6.15 g, 10.0 mmol) and tripotassium phosphate (2.23 g, 10.5 mmol) were suspended in dimethylsulfoxide (50 mL), and then stirred at 100°C for 24 hours. After cooling to room temperature, the mixture was separated using pure water and chloroform, and the organic layer was further washed with a saturated aqueous sodium chloride solution. The organic layer was dried over anhydrous magnesium sulfate and purified by silica gel column chromatography to obtain intermediate (AA20), which is the target substance as a white solid (yield: 5.92 g, 73%). Compound identification was performed by FDMS measurement. FDMS: 812

### Example 61: Synthesis of compound (A671)

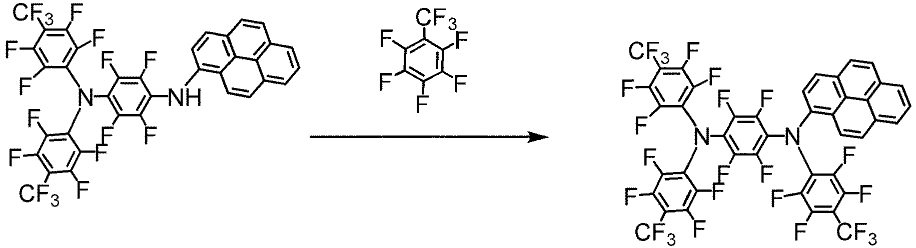

Under a nitrogen atmosphere, AA20 (3.25 g, 4.0 mmol), perfluorotoluene (2.83 g, 12 mmol), and tripotassium phosphate (4.25 g, 20 mmol) were suspended in dimethylsulfoxide (50 mL), and then stirred at 100°C for 24 hours. After cooling to room temperature, the mixture was separated using pure water and chloroform, and the organic layer was further washed with saturated aqueous sodium chloride solution. The organic layer was dried over anhydrous magnesium sulfate and purified by silica gel column chromatography to obtain compound (A671), which is the target substance as a white solid (yield: 3.12 g, 76%). Compound identification was performed by FDMS measurement. FDMS: 1028

### Example 61' (Metal adhesion evaluation of compound (A671))

Boiling washing was performed with isopropyl alcohol on the glass substrate, and after further washing by UV/ozone, it was placed in the vapor deposition equipment, and evacuated to no more than 1.0×10⁻⁴ Pa by a vacuum pump. The compound (A671) was deposited to 50 nm at 0.2 nm/sec on a glass substrate on which a metal mask having a 20 mm × 10 mm opening was arranged. Subsequently, the metal mask was removed, and magnesium was vacuum deposited to 15 nm at a deposition rate of 0.1 nm/sec. Magnesium did not form a film on a portion where compound (A671) is deposited, and a 20 mm × 10 mm transparent area was formed.

### Example 62: Synthesis of intermediate (AA21)

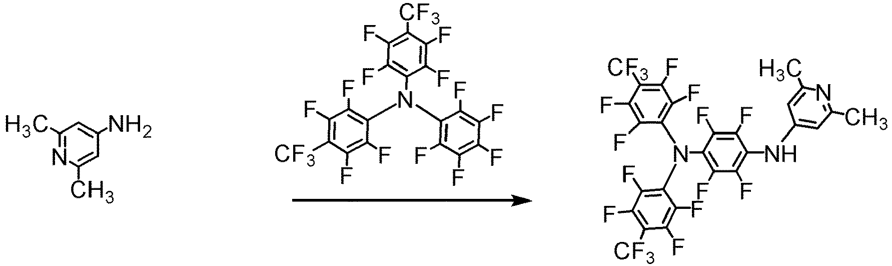

Under a nitrogen atmosphere, 4-amino -2,6-dimethylpyridine (1.22 g, 10.0 mmol), AA2 (6.15 g, 10.0 mmol) and tripotassium phosphate (2.23 g, 10.5 mmol) were suspended in dimethylsulfoxide (50 mL), and the stirred at 100°C for 24 hours. After cooling to room temperature, the mixture was separated using pure water and chloroform, and the organic layer was further washed with saturated aqueous sodium chloride solution. The organic layer was dried over anhydrous magnesium sulfate and purified by silica gel column chromatography to obtain intermediate (AA21), which is the target substance as a white solid (yield: 3.29 g, 46%). Compound identification was performed by FDMS measurement. FDMS: 717

### Example 63: Synthesis of compound (A674)

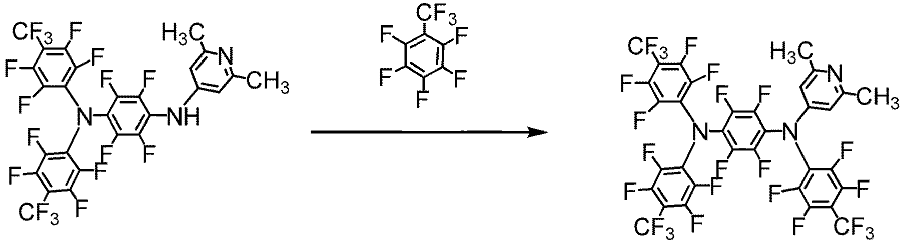

Under a nitrogen atmosphere, AA21 (2.87 g, 4.0 mmol), perfluorotoluene (2.83 g, 12 mmol), and tripotassium phosphate (4.25 g, 20 mmol) were suspended in dimethylsulfoxide (50 mL), and then stirred at 100°C for 24 hours. After cooling to room temperature, the mixture was separated using pure water and chloroform, and the organic layer was further washed with saturated aqueous sodium chloride solution. The organic layer was dried over anhydrous magnesium sulfate and purified by silica gel column chromatography to obtain compound (A674), which is the target substance as a white solid (yield: 2.17 g, 58%). Compound identification was performed by FDMS measurement. FDMS: 933

### Example 63' (Metal adhesion evaluation of compound (A674))

Boiling washing was performed with isopropyl alcohol on the glass substrate, and after further washing by UV/ozone, it was placed in the vapor deposition equipment, and evacuated to no more than 1.0×10⁻⁴ Pa by a vacuum pump. The compound (A674) was deposited to 50 nm at 0.2 nm/sec on a glass substrate on which a metal mask having a 20 mm × 10 mm opening was arranged. Subsequently, the metal mask was removed, and silver was vacuum deposited to 8 nm at a deposition rate of 0.1 nm/sec. Silver did not form a film on a portion where compound (A674) is deposited, and a 20 mm × 10 mm transparent area was formed.

### Example 64: Synthesis of intermediate (AA22)

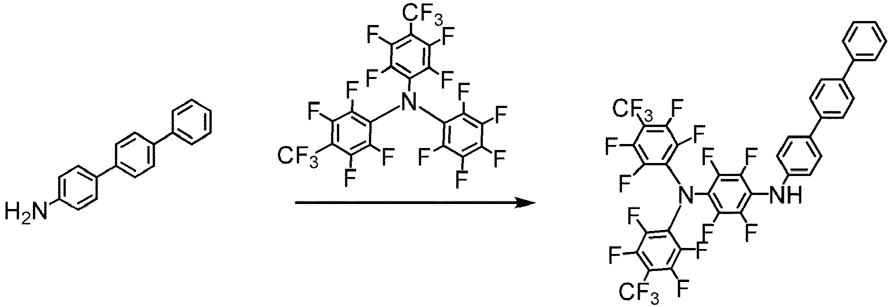

Under a nitrogen atmosphere, 4-amino-p-terphenyl (2.54 g, 10.0 mmol), AA2 (6.15 g, 10.0 mmol), and otassium phosphate (2.23 g, 10.5 mmol) were suspended in dimethylsulfoxide (50 mL), and then stirred at 100°C for 24 hours. After cooling to room temperature, the mixture was separated using pure water and chloroform, and the organic layer was further washed with saturated aqueous sodium chloride solution. The organic layer was dried over anhydrous magnesium sulfate and purified by silica gel column chromatography to obtain intermediate (AA22), which is the target substance as a white solid (yield: 6.60 g, 79%). Compound identification was performed by FDMS measurement. FDMS: 840

### Example 65: Synthesis of compound (A679)

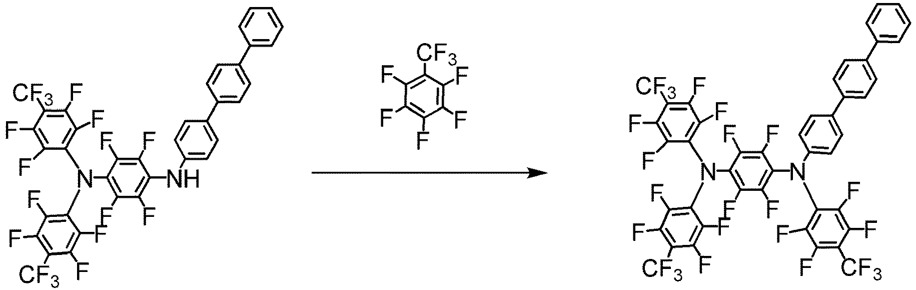

Under a nitrogen atmosphere, AA22 (3.36 g, 4.0 mmol), perfluorotoluene (2.83 g, 12 mmol), and tripotassium phosphate (4.25 g, 20 mmol) were suspended in dimethylsulfoxide (50 mL), and then stirred at 100°C for 24 hours. After cooling to room temperature, the mixture was separated using pure water and chloroform, and the organic layer was further washed with saturated aqueous sodium chloride solution. The organic layer was dried over anhydrous magnesium sulfate and purified by silica gel column chromatography to obtain compound (A679), which is the target substance as a white solid (yield: 3.09 g, 73%). Compound identification was performed by FDMS measurement. FDMS: 1056

### Example 65' (Metal adhesion evaluation of compound (A679))

Boiling washing was performed with isopropyl alcohol on the glass substrate, and after further washing by UV/ozone, it was placed in the vapor deposition equipment, and evacuated to no more than 1.0×10⁻⁴ Pa by a vacuum pump. The compound (A679) was deposited to 50 nm at 0.2 nm/sec on a glass substrate on which a metal mask having a 20 mm × 10 mm opening was arranged. Subsequently, the metal mask was removed, and magnesium was vacuum deposited to 15 nm at a deposition rate of 0.1 nm/sec. Magnesium did not form a film on a portion where compound (A679) is deposited, and a 20 mm × 10 mm transparent area was formed.

### Example 66: Synthesis of intermediate (AA23)

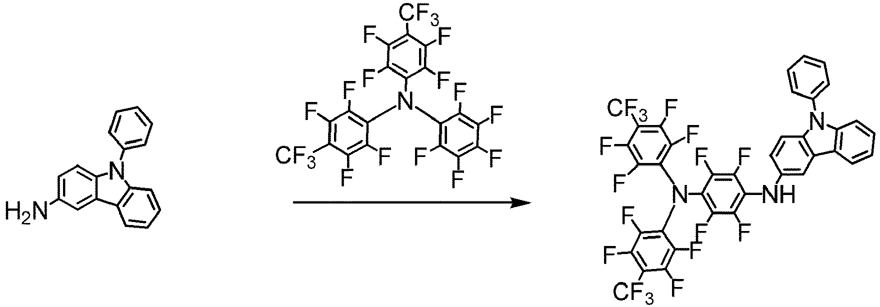

Under a nitrogen atmosphere, 3-amino-9-phenylcarbazole (2.58 g, 10.0 mmol), AA2 (6.15 g, 10.0 mmol) and tripotassium phosphate (2.23 g, 10.5 mmol) were suspended in dimethylsulfoxide (50 mL), and then stirred at 100°C for 24 hours. After cooling to room temperature, the mixture was separated using pure water and chloroform, and the organic layer was further washed with saturated aqueous sodium chloride solution. The organic layer was dried over anhydrous magnesium sulfate and purified by silica gel column chromatography to obtain intermediate (AA23), which is the target substance as a white solid (yield: 6.34 g, 74%). Compound identification was performed by FDMS measurement. FDMS: 853

### Example 67: Synthesis of compound (A689)

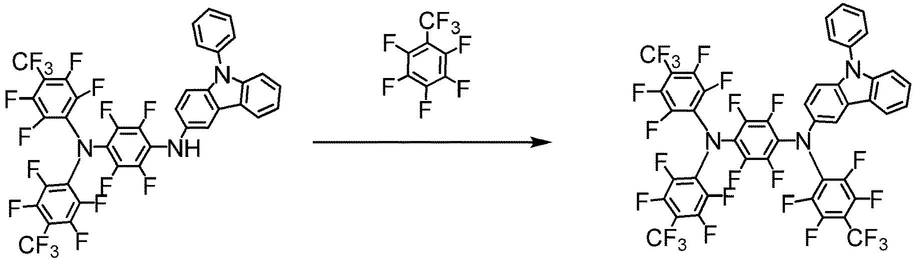

Under a nitrogen atmosphere, AA23 (3.41 g, 4.0 mmol), perfluorotoluene (2.83 g, 12 mmol) and tripotassium phosphate (4.25 g, 20 mmol) were suspended in dimethylsulfoxide (50 mL), and then stirred at 100°C for 24 hours. After cooling to room temperature, the mixture was separated using pure water and chloroform, and the organic layer was further washed with saturated aqueous sodium chloride solution. The organic layer was dried over anhydrous magnesium sulfate and purified by silica gel column chromatography to obtain compound (A689), which is the target substance as a white solid (yield: 3.27 g, 77%). Compound identification was performed by FDMS measurement. FDMS: 1069

### Example 67' (Metal adhesion evaluation of compound (A689))

Boiling washing was performed with isopropyl alcohol on the glass substrate, and after further washing by UV/ozone, it was placed in the vapor deposition equipment, and evacuated to no more than 1.0×10⁻⁴ Pa by a vacuum pump. The compound (A689) was deposited to 50 nm at 0.2 nm/sec on a glass substrate on which a metal mask having a 20 mm × 10 mm opening was arranged. Subsequently, the metal mask was removed, ytterbium was vacuum deposited to 1 nm at a deposition rate of 0.01 nm/sec, and subsequently, magnesium was vacuum deposited to 10 nm at a deposition rate of 0.1 nm/sec. Ytterbium and magnesium did not form a film on a portion where compound (A665) is deposited, and a 20 mm × 10 mm transparent area was formed.

### Example 68: Synthesis of intermediate (AA24)

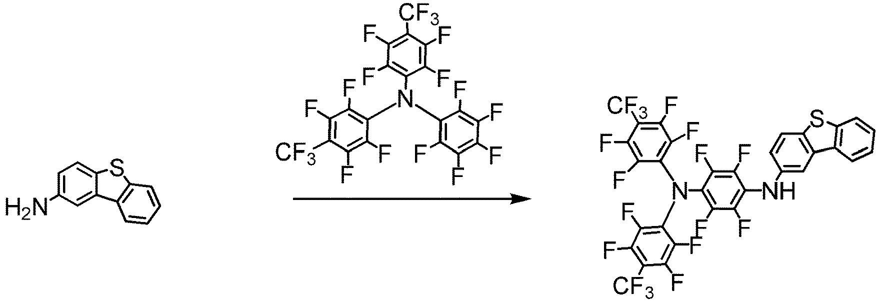

Under a nitrogen atmosphere, 2-aminobenzothiophene (1.99 g, 10.0 mmol), AA2 (6.15 g, 10.0 mmol) and tripotassium phosphate (2.23 g, 10.5 mmol) were suspended in dimethylsulfoxide (50 mL), and then stirred at 100°C for 24 hours. After cooling to room temperature, the mixture was separated using pure water and chloroform, and the organic layer was further washed with saturated aqueous sodium chloride solution. The organic layer was dried over anhydrous magnesium sulfate and purified by silica gel column chromatography to obtain intermediate (AA24), which is the target substance as a white solid (yield: 5.44 g, 69%). Compound identification was performed by FDMS measurement. FDMS: 794

### Example 69: Synthesis of compound (A602)

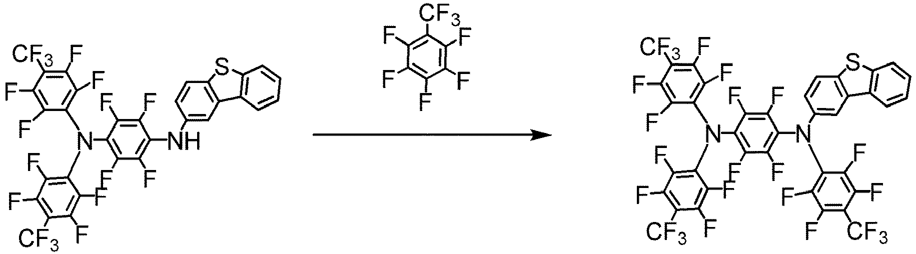

Under a nitrogen atmosphere, AA24 (3.18 g, 4.0 mmol), perfluorotoluene (2.83 g, 12 mmol), and tripotassium phosphate (4.25 g, 20 mmol) were suspended in dimethylsulfoxide (50 mL), and then stirred at 100°C for 24 hours. After cooling to room temperature, the mixture was separated using pure water and chloroform, and the organic layer was further washed with saturated aqueous sodium chloride solution. The organic layer was dried over anhydrous magnesium sulfate and purified by silica gel column chromatography to obtain compound (A602), which is the target substance as a white solid (yield: 2.69 g, 67%). Compound identification was performed by FDMS measurement. FDMS: 1010

### Example 69' (Metal adhesion evaluation of compound (A602))

Boiling washing was performed with isopropyl alcohol on the glass substrate, and after further washing by UV/ozone, it was placed in the vapor deposition equipment, and evacuated to no more than 1.0×10⁻⁴ Pa by a vacuum pump. The compound (A602) was deposited to 50 nm at 0.2 nm/sec on a glass substrate on which a metal mask having a 20 mm × 10 mm opening was arranged. Subsequently, the metal mask was removed, and magnesium was vacuum deposited to 15 nm at a deposition rate of 0.1 nm/sec. Magnesium did not form a film on a portion where compound (A602) is deposited, and a 20 mm × 10 mm transparent area was formed.

### Example 70: Synthesis of intermediate (AA25)

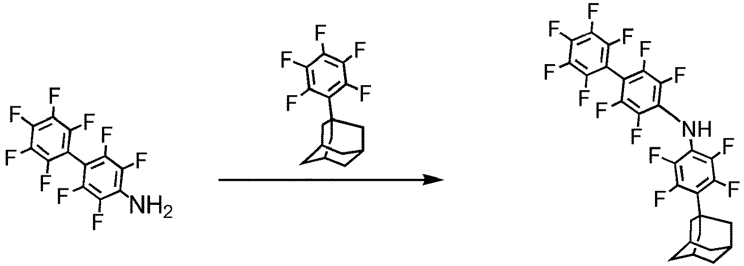

Under a nitrogen atmosphere, 4-aminononafluorobiphenyl (3.31 g, 10.0 mmol), 1-pentafluorophenyladamantane (3.02 g, 10.0 mmol) and tripotassium phosphate (2.33 g, 11.0 mmol) were suspended in dimethylsulfoxide (50 mL), and then stirred at 100°C for 24 hours. After cooling to room temperature, the mixture was separated using pure water and chloroform, and the organic layer was further washed with saturated aqueous sodium chloride solution. The organic layer was dried over anhydrous magnesium sulfate and purified by silica gel column chromatography to obtain intermediate (AA25), which is the target substance as a white solid (yield: 4.45 g, 73%). Compound identification was performed by FDMS measurement. FDMS: 613

### Example 71: Synthesis of compound (A699)

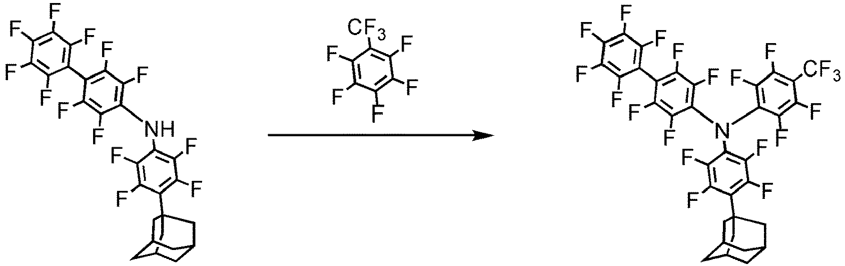

Under a nitrogen atmosphere, AA25 (2.45 g, 4.0 mmol), perfluorotoluene (2.83 g, 12 mmol), and tripotassium phosphate (4.25 g, 20 mmol) were suspended in dimethylsulfoxide (50 mL), and then stirred at 100°C for 24 hours. After cooling to room temperature, the mixture was separated using pure water and chloroform, and the organic layer was further washed with saturated aqueous sodium chloride solution. The organic layer was dried over anhydrous magnesium sulfate and purified by silica gel column chromatography to obtain compound (A699), which is the target substance as a white solid (yield: 2.51 g, 76%). Compound identification was performed by FDMS measurement. FDMS: 829

### Example 71' (Metal adhesion evaluation of compound (A699))

Boiling washing was performed with isopropyl alcohol on the glass substrate, and after further washing by UV/ozone, it was placed in the vapor deposition equipment, and evacuated to no more than 1.0×10⁻⁴ Pa by a vacuum pump. The compound (A699) was deposited to 50 nm at 0.2 nm/sec on a glass substrate on which a metal mask having a 20 mm × 10 mm opening was arranged. Subsequently, the metal mask was removed, and silver was vacuum deposited to 15 nm at a deposition rate of 0.1 nm/sec. Silver did not form a film on a portion where compound (A699) is deposited, and a 20 mm × 10 mm transparent area was formed.

### Example 72: Synthesis of compound (A420)

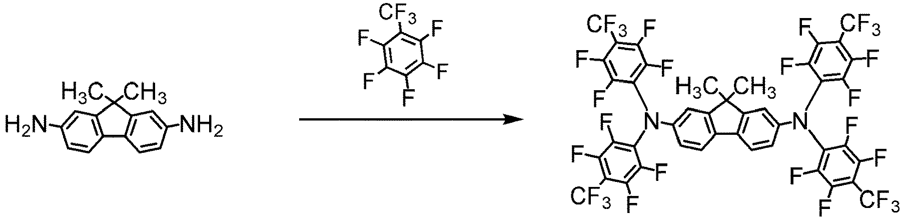

Under a nitrogen atmosphere, 2,7-diamino-9,9-dimethylfluorene (0.977 g, 5.0 mmol), perfluorotoluene (9.44 g, 40 mmol) and tripotassium phosphate (10 .6 g, 50 mmol) were suspended in dimethylsulfoxide (50 mL), and then stirred at 100°C for 24 hours. After cooling to room temperature, water (50 ml) and methanol (50 ml) were added to the reaction solution, and the mixture was stirred at room temperature for 30 minutes. The precipitated solid was collected by filtration to obtain compound (A420), which is the target substance as a white solid (yield: 3.95 g, 73%). Compound identification was performed by FDMS measurement. FDMS: 1088

### Example 72' (Metal adhesion evaluation of compound (A420))

Boiling washing was performed with isopropyl alcohol on the glass substrate, and after further washing by UV/ozone, it was placed in the vapor deposition equipment, and evacuated to no more than 1.0×10⁻⁴ Pa by a vacuum pump. The compound (A420) was deposited to 50 nm at 0.2 nm/sec on a glass substrate on which a metal mask having a 20 mm × 10 mm opening was arranged. Subsequently, the metal mask was removed, and silver and magnesium (1/9) were vacuum deposited to 15 nm at a deposition rate of 0.1 nm/sec. An alloy of silver and magnesium did not form a film on a portion where compound (A420) is deposited, and a 20 mm × 10 mm transparent area was formed.

### Example 73: Synthesis of compound (A721)

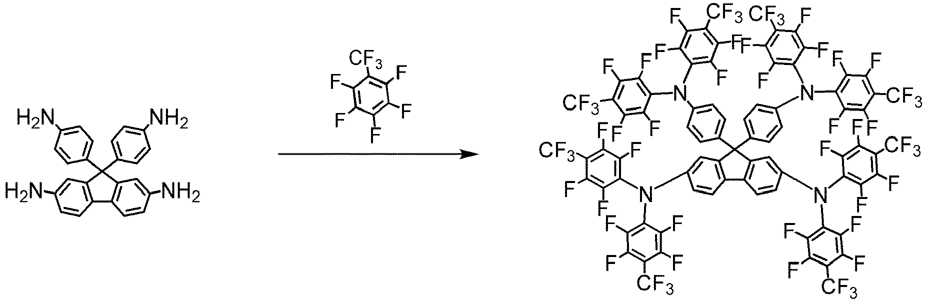

Under a nitrogen atmosphere, 9,9-bis (4-aminophenyl)-9H-fluorene -2,7-diamine (1.89 g, 5.0 mmol), perfluorotoluene (18 .8 g, 80 mmol), and tripotassium phosphate (12 .7 g, 60 mmol) were suspended in dimethylsulfoxide (50 mL), and then stirred at 100°C for 24 hours. After cooling to room temperature, water (50 ml) and methanol (50 ml) were added to the reaction solution, and the mixture was stirred at room temperature for 30 minutes. The precipitated solid was collected by filtration to obtain compound (A721), which is the target substance as a white solid (yield: 6.73 g, 63%). Compound identification was performed by FDMS measurement. FDMS: 2138

### Example 73' (Metal adhesion evaluation of compound (A721))

Boiling washing was performed with isopropyl alcohol on the glass substrate, and after further washing by UV/ozone, it was placed in the vapor deposition equipment, and evacuated to no more than 1.0×10⁻⁴ Pa by a vacuum pump. The compound (A721) was deposited to 50 nm at 0.2 nm/sec on a glass substrate on which a metal mask having a 20 mm × 10 mm opening was arranged. Subsequently, the metal mask was removed, ytterbium was vacuum deposited to 1 nm at a deposition rate of 0.01 nm/sec, and subsequently, silver and magnesium (1/1) were vacuum deposited to 15 nm at a deposition rate of 0.1 nm/sec. Ytterbium, and an alloy of silver and magnesium did not form a film on a portion where compound (A721) is deposited, and a 20 mm × 10 mm transparent area was formed.

### Example 74: Synthesis of compound (A703)

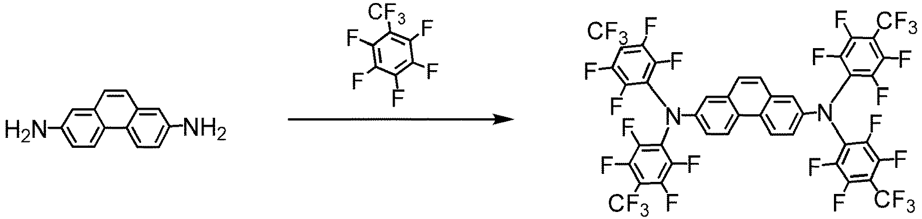

Under a nitrogen atmosphere, 2,7-diaminophenanthrene (1.04 g, 5.0 mmol), perfluorotoluene (9.44 g, 40 mmol) and tripotassium phosphate (10 .6 g, 50 mmol) were suspended in dimethylsulfoxide (50 mL), and then stirred at 100°C for 24 hours. After cooling to room temperature, water (50 ml) and methanol (50 ml) were added to the reaction solution, and the mixture was stirred at room temperature for 30 minutes. The precipitated solid was collected by filtration to obtain compound (A703), which is the target substance as a white solid (yield: 3.97 g, 74%). Compound identification was performed by FDMS measurement. FDMS: 1072

### Example 74' (Metal adhesion evaluation of compound (A703))

Boiling washing was performed with isopropyl alcohol on the glass substrate, and after further washing by UV/ozone, it was placed in the vapor deposition equipment, and evacuated to no more than 1.0×10⁻⁴ Pa by a vacuum pump. The compound (A703) was deposited to 50 nm at 0.2 nm/sec on a glass substrate on which a metal mask having a 20 mm × 10 mm opening was arranged. Subsequently, the metal mask was removed, and magnesium was vacuum deposited to 15 nm at a deposition rate of 0.1 nm/sec. Magnesium did not form a film on a portion where compound (A703) is deposited, and a 20 mm × 10 mm transparent area was formed.

### Example 75: Synthesis of compound (A710)

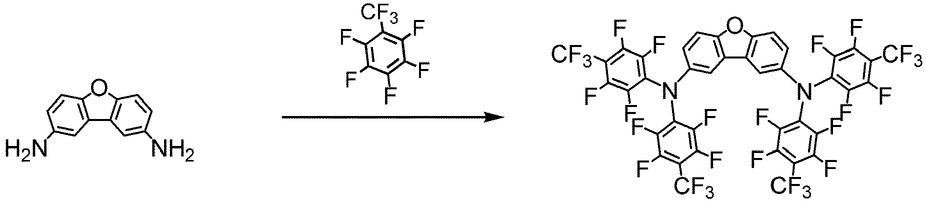

Under a nitrogen atmosphere, 2,8-diaminodibenzofuran (0.991 g, 5.0 mmol), perfluorotoluene (9.44 g, 40 mmol) and tripotassium phosphate (10.6 g, 50 mmol) were suspended in dimethylsulfoxide (50 mL), and then stirred at 100°C for 24 hours. After cooling to room temperature, water (50 ml) and methanol (50 ml) were added to the reaction solution, and the mixture was stirred at room temperature for 30 minutes. The precipitated solid was collected by filtration to obtain compound (A710), which is the target substance as a white solid (yield: 4.15 g, 78%). Compound identification was performed by FDMS measurement. FDMS: 1062

### Example 75' (Metal adhesion evaluation of compound (A710))

Boiling washing was performed with isopropyl alcohol on the glass substrate, and after further washing by UV/ozone, it was placed in the vapor deposition equipment, and evacuated to no more than 1.0×10⁻⁴ Pa by a vacuum pump. The compound (A710) was deposited to 50 nm at 0.2 nm/sec on a glass substrate on which a metal mask having a 20 mm × 10 mm opening was arranged. Subsequently, the metal mask was removed, and silver and magnesium (1/9) were vacuum deposited to 12 nm at a deposition rate of 0.1 nm/sec. An alloy of silver and magnesium did not form a film on a portion where compound (A710) is deposited, and a 20 mm × 10 mm transparent area was formed.

### Example 76: Synthesis of compound (A716)

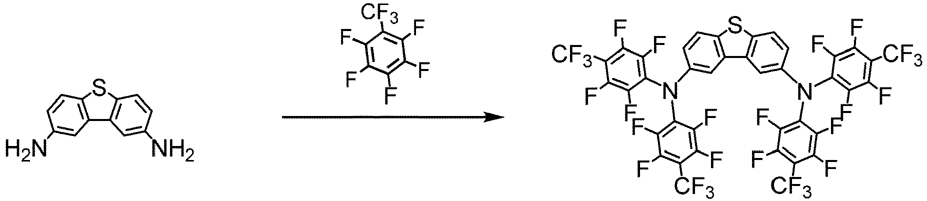

Under a nitrogen atmosphere, 2,8-diaminodibenzothiophene (1.07 g, 5.0 mmol), perfluorotoluene (9.44 g, 40 mmol) and tripotassium phosphate (10 .6 g, 50 mmol) were suspended in dimethylsulfoxide (50 mL), and then stirred at 100°C for 24 hours. After cooling to room temperature, water (50 ml) and methanol (50 ml) were added to the reaction solution, and the mixture was stirred at room temperature for 30 minutes. The precipitated solid was collected by filtration to obtain compound (A716), which is the target substance as a white solid (yield: 4.06 g, 75%). Compound identification was performed by FDMS measurement. FDMS: 1078

### Example 76' (Metal adhesion evaluation of compound (A716))

Boiling washing was performed with isopropyl alcohol on the glass substrate, and after further washing by UV/ozone, it was placed in the vapor deposition equipment, and evacuated to no more than 1.0×10⁻⁴ Pa by a vacuum pump. The compound (A716) was deposited to 50 nm at 0.2 nm/sec on a glass substrate on which a metal mask having a 20 mm × 10 mm opening was arranged. Subsequently, the metal mask was removed, and silver and magnesium (1/9) were vacuum deposited to 12 nm at a deposition rate of 0.1 nm/sec. An alloy of silver and magnesium did not form a film on a portion where compound (A716) is deposited, and a 20 mm × 10 mm transparent area was formed.

### Example 77: Synthesis of compound (A426)

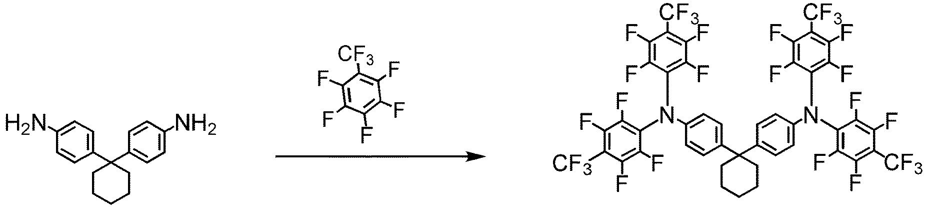

Under a nitrogen atmosphere, 1,1-bis (4-aminophenyl)cyclohexane (1.33 g, 5.0 mmol), perfluorotoluene (9.44 g, 40 mmol) and tripotassium phosphate (10 .6 g, 50 mmol) were suspended in dimethylsulfoxide (50 mL), and then stirred at 100°C for 24 hours. After cooling to room temperature, the mixture was separated using pure water and toluene, and the organic layer was further washed with a saturated aqueous sodium chloride solution. The organic layer was dried over anhydrous magnesium sulfate and purified by silica gel column chromatography to obtain compound (A426), which is the target substance as a white solid (yield: 3.70 g, 65%). Compound identification was performed by FDMS measurement. FDMS: 1078

### Example 77' (Metal adhesion evaluation of compound (A426))

Boiling washing was performed with isopropyl alcohol on the glass substrate, and after further washing by UV/ozone, it was placed in the vapor deposition equipment, and evacuated to no more than 1.0×10⁻⁴ Pa by a vacuum pump. The compound (A426) was deposited to 50 nm at 0.2 nm/sec on a glass substrate on which a metal mask having a 20 mm × 10 mm opening was arranged. Subsequently, the metal mask was removed, and magnesium was vacuum deposited to 15 nm at a deposition rate of 0.1 nm/sec. Magnesium did not form a film on a portion where compound (A426) is deposited, and a 20 mm × 10 mm transparent area was formed.

### Example 78: Synthesis of intermediate (BB1)

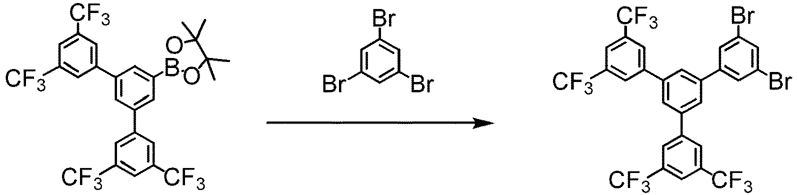

2- [3,5-Di (3 ',5 '-ditrifluoromethylbenzene -2-yl)] - 4,4,5,5-tetramethyl -1,3,2-dioxaborolan (6.28 g, 10.0 mmol), 1,3,5 tribromobenzene (9.44 g, 30.0 mmol), palladium acetate (22 .5 mg, 0.1 mmol), tri-o-trilphosphine (121.7 mg, 0.3 mmol), toluene (100 mL), and a 2M aqueous potassium carbonate solution were added, and then stirred at 100° C for 24 hours. After cooling to room temperature, the mixture was separated using pure water and toluene, and the organic layer was further washed with a saturated aqueous sodium chloride solution. The organic layer was dried over anhydrous magnesium sulfate and purified by silica gel column chromatography to obtain compound (BB1), which is the target substance as a white solid (yield: 5.23 g, 71%). Compound identification was performed by FDMS measurement. FDMS: 734

### Example 79: Synthesis of compound (B90)

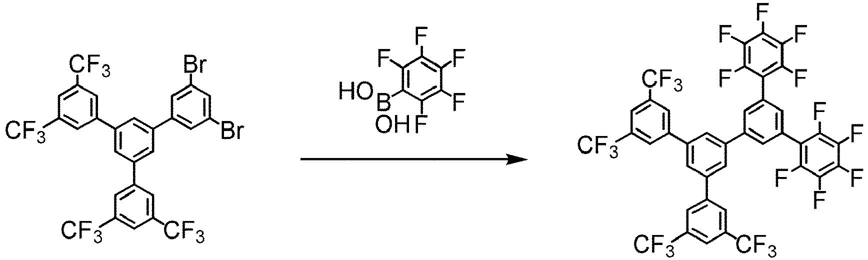

Under a nitrogen atmosphere, BB1 (2.58 g, 3.5 mmol), pentafluorophenyl boronic acid (1.78, 8.4 mmol), cesium fluoride (2.13 g, 14.0 mmol), silver oxide (I) (1.95 g, 8.4 mmol), and N,N-dimethylformamide (35 ml) were added, and then stirred for 60 minutes. Tris (dibenzylideneacetone)palladium (0) (160.2 mg, 0.18 mmol) and tri (tert-butyl)phosphine (85 mg, 0.42 mmol) were added to the resulting slurry mixture, followed by stirring at 100°C for 6 hours. After cooling to room temperature, silver oxide (I) was removed by a filter, and the filtrate was concentrated. Hexane was added to the residue and the residue was purified by silica gel column chromatography to obtain compound (B90), which is the target substance as a white solid (yield: 1.37 g, 43%). Compound identification was performed by FDMS measurement. FDMS: 910

Example 79' (Metal adhesion evaluation of compound (B90)) Boiling washing was performed with isopropyl alcohol on the glass substrate, and after further washing by UV/ozone, it was placed in the vapor deposition equipment, and evacuated to no more than 1.0×10⁻⁴ Pa by a vacuum pump. The compound (B90) was deposited to 50 nm at 0.2 nm/sec on a glass substrate on which a metal mask having a 20 mm × 10 mm opening was arranged. Subsequently, the metal mask was removed, and silver was vacuum deposited to 10 nm at a deposition rate of 0.1 nm/sec. Silver did not form a film on a portion where compound (B90) is deposited, and a 20 mm × 10 mm transparent area was formed.

### Example 80: Synthesis of compound (A504)

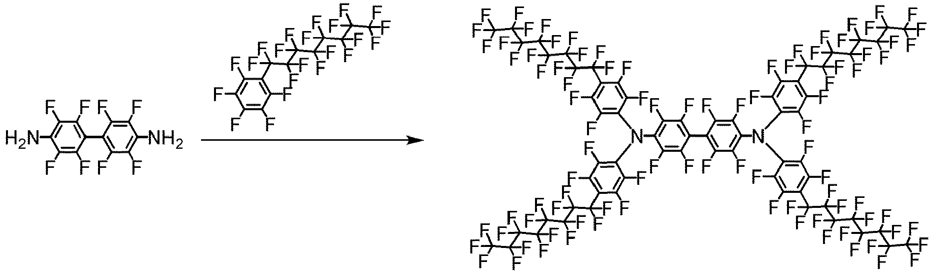

Under a nitrogen atmosphere, 4,4 '-diamino -2,2 '-bis (trifluoromethyl)biphenyl (0.96 g, 3.0 mmol), perfluorooctylbenzene (14 .1 g, 24 mmol), 18 crown 6 ether (103 mg, 0.40 mmol) and tripotassium phosphate (10 .2 g, 48 mmol) were suspended in dimethylsulfoxide (60 ml), and then stirred at 100°C for 24 hours. After cooling to room temperature, water (50 ml) and methanol (50 ml) were added to the reaction solution, and the mixture was stirred at room temperature for 30 minutes. The precipitated solid was collected by filtration to obtain compound (A504), which is the target substance as a white solid (yield: 4.2 g, 54%). Compound identification was performed by FDMS measurement. FDMS: 2592

### Example 80' (Metal adhesion evaluation of compound (A504))

Boiling washing was performed with isopropyl alcohol on the glass substrate, and after further washing by UV/ozone, it was placed in the vapor deposition equipment, and evacuated to no more than 1.0×10⁻⁴ Pa by a vacuum pump. The compound (A504) was deposited to 20 nm at 0.1 nm/sec on a glass substrate on which a metal mask having a 20 mm × 10 mm opening was arranged. Subsequently, the metal mask was removed, and silver was vacuum deposited to 30 nm at a deposition rate of 0.1 nm/sec. Silver did not form a film on a portion where compound (A504) is deposited, and a 20 mm × 10 mm transparent area was formed.

### Comparative Example 1' (Metal adhesion evaluation of compound (X1))

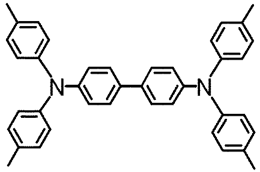

By a method similar to Example 1', the adhesion of metal to compound (X1) was evaluated. Silver formed a film on the film of compound (X1), and a transparent area was not formed.

### Comparative Example 2' (Metal adhesion evaluation of 4,4 '-bis [N- (1-naphthyl)-N-phenyl]bipheny (compound (X2))

By a method similar to Example 1', the adhesion of metal to compound (X2) was evaluated. Silver formed a film on the film of 4,4 '-bis [N- (1-naphthyl)-N-phenyl]bipheny (compound (X2), and a transparent area was not formed.

### Comparative Example 3: Synthesis of intermediate (XX1)

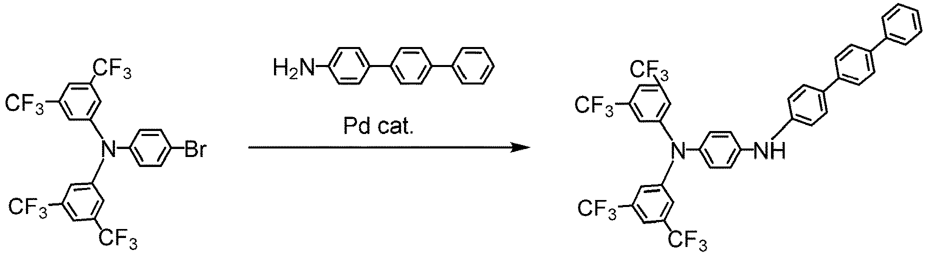

Under a nitrogen stream, AA12 (2.98 g, 5.0 mmol), 4-amino-p-terphenyl (1.60 g, 6.5 mmol), sodium-tert-butoxide (0.72 g, 7.5 mmol), o-xylene (25 mL), palladium acetate (11 .2 mg, 0.05 mmol), and 4,5-bis (diphenylphosphino)-9,9-dimethylxanthene (86.8 mg, 0.15 mmol) were added to a 100 mL three-neck flask, and then stirred at 140°C for 5 hours. After cooling to room temperature, the mixture was separated using pure water and toluene, and the organic layer was further washed with a saturated aqueous sodium chloride solution. The organic layer was dried over anhydrous magnesium sulfate and purified by silica gel column chromatography to obtain intermediate (XX1), which is the target substance as a white solid (yield: 3.35 g, 88%). Compound identification was performed by FDMS measurement. FDMS: 760

### Comparative Example 4: Synthesis of compound (X3)

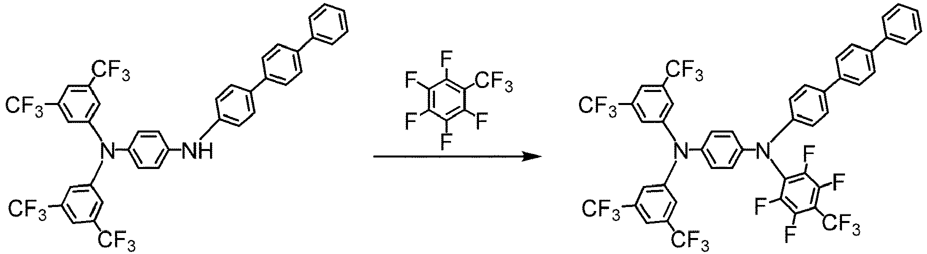

Under a nitrogen atmosphere, XX1 (2.66 g, 3.5 mmol), perfluorotoluene (3.30 g, 14 mmol) and tripotassium phosphate (3.71 g, 18 mmol) were suspended in dimethylsulfoxide (50 mL), and then stirred at 100°C for 24 hours. After cooling to room temperature, water (50 ml) and methanol (50 ml) were added to the reaction solution, and the mixture was stirred at room temperature for 30 minutes. The precipitated solid was collected by filtration to obtain compound (X3), which is the target substance as a white solid (yield: 2.85 g, 83%). Compound identification was performed by FDMS measurement. FDMS: 976

### Comparative Example 4' (Metal adhesion evaluation of compound (X3))

By a method similar to Example 1', the adhesion of metal to compound (X3) was evaluated. Silver formed a film on the film of compound (X3), and a transparent area was not formed.

### Comparative Example 5: Synthesis of compound (X4)

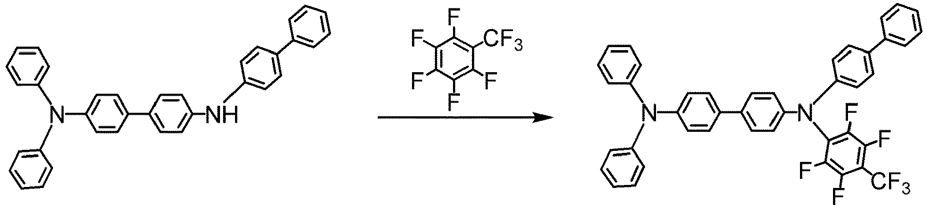

Under a nitrogen atmosphere, [1,1 '-biphenyl] -4,4 '-diamine, N4 '- [1,1 '-biphenyl] -4-yl-N4,N4-biphenyl- (2.20 g, 4.5 mmol), and perfluorotoluene (2.67 g, 11 mmol), tripotassium phosphate (2.40 g, 11 mmol) were suspended in dimethylsulfoxide (45 mL), and then stirred at 100°C for 24 hours. After cooling to room temperature, water (50 ml) and methanol (50 ml) were added to the reaction solution, and the mixture was stirred at room temperature for 30 minutes. The precipitated solid was collected by filtration to obtain compound (X4), which is the target substance as a white solid (yield: 2.43 g, 76.3%). Compound identification was performed by FDMS measurement. FDMS: 704

### Comparative Example 5' (Metal adhesion evaluation of compound (X4))

By a method similar to Example 1', the adhesion of metal to compound (X4) was evaluated. Silver formed a film on the film of Comparative Example (X4), and a transparent area was not formed.

### Comparative Example 6: Synthesis of compound (XX2)

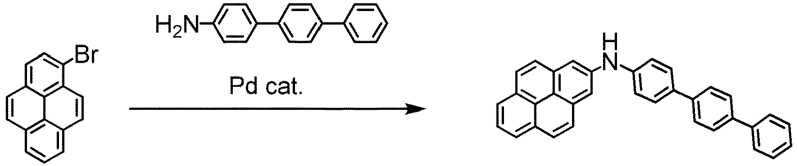

Under a nitrogen stream, 1-bromopyrene (2.82 g, 10 mmol), 4-amino-p-terphenyl (3.20 g, 13 mmol), sodium-tert-butoxide (1.44 g, 15 mmol), o-xylene (50 mL), palladium acetate (22.4 mg, 0.05 mmol), and 4,5-bis (diphenylphosphino)-9,9-dimethylxanthene (173 mg, 0.3 mmol) were added to a 100-mL three-neck flask, and then stirred at 140°C for 12 hours. After cooling to room temperature, the mixture was separated using pure water and toluene, and the organic layer was further washed with a saturated aqueous sodium chloride solution. The organic layer was dried over anhydrous magnesium sulfate and purified by silica gel column chromatography to obtain an intermediate (XX2), which is the target substance as a white solid (yield: 3.26 g, 73%). Compound identification was performed by FDMS measurement. FDMS: 445

### Comparative Example 7: Synthesis of compound (XX5)

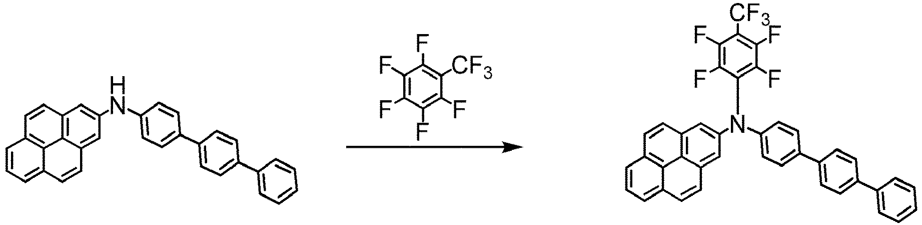

Under a nitrogen atmosphere, XX2 (2.22 g, 5.0 mmol), perfluorotoluene (4.7 g, 20 mmol) and tripotassium phosphate (5.15 g, 25 mmol) were suspended in dimethylsulfoxide (50 mL), and then stirred at 100°C for 24 hours. After cooling to room temperature, water (50 ml) and methanol (50 ml) were added to the reaction solution, and the mixture was stirred at room temperature for 30 minutes. The precipitated solid was collected by filtration to obtain compound (X5), which is the target substance as a white solid (yield: 2.74 g, 83%). Compound identification was performed by FDMS measurement. FDMS: 661

### Comparative Example 7' (Metal adhesion evaluation of compound (X5))

By a method similar to Example 1', the adhesion of metal to compound (X5) was evaluated. Silver formed a film on the film of compound (X5), and a transparent area was not formed.

### Comparative Example 8' (Metal adhesion evaluation of compound (X6))

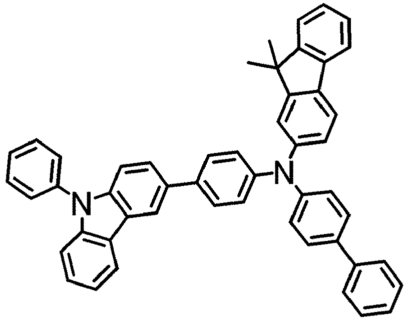

By a method similar to Example 1', the adhesion of metal to compound (X6) was evaluated. Silver formed a film on the film of compound (X6), and a transparent area was not formed.

### Comparative Example 9' (Metal adhesion evaluation of compound (X7))

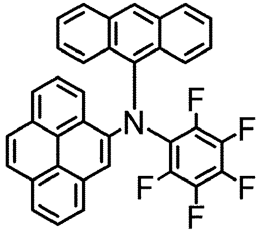

By a method similar to Example 1', the adhesion of metal to compound (X7) was evaluated. Silver formed a film on the film of compound (X7), and a transparent area was not formed.

### Comparative Example 10' (Metal adhesion evaluation of compound (Y1))

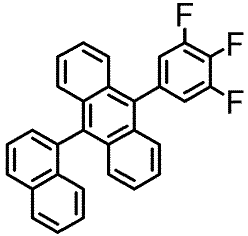

By a method similar to Example 29', the adhesion of metal to compound (Y1) was evaluated. Silver formed a film on the film of compound (Y1), and a transparent area was not formed.

### Comparative Example 11' (Metal adhesion evaluation of compound (Y2))

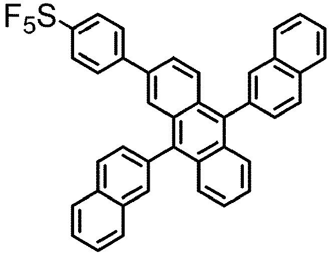

By a method similar to Example 29', the adhesion of metal to compound (Y2) was evaluated. Silver formed a film on the film of compound (Y2), and a transparent area was not formed.

### Comparative Example 12' (Metal adhesion evaluation of compound (Y3))

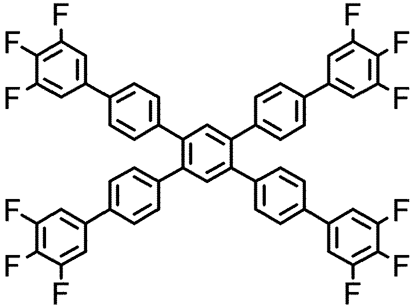

By a method similar to Example 29', the adhesion of metal to compound (Y3) was evaluated. Silver formed a film on the film of compound (Y3), and a transparent area was not formed.

### <<Transmittance Measurement>>

Evaluation of the metal adhesion is possible by transmittance measurement, and the transmittance declines when metal adheres. In addition, when the adhered amount increased, the transmittance decreases in correlation thereto.

### 1. Metal patterning material having amine substituent (example belonging to first embodiment)

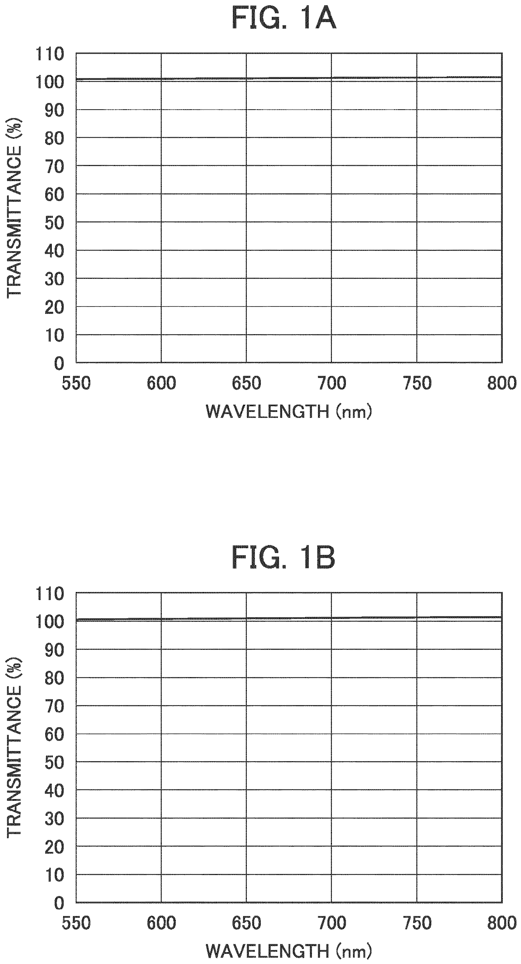

Example 1": Transmittance measurement of compound (A177) Boiling washing was performed with isopropyl alcohol on the glass substrate, and after further washing by UV/ozone, it was placed in the vapor deposition equipment, and evacuated to no more than 1.0×10⁻⁴ Pa by a vacuum pump. The compound (A177), which is the metal patterning material, was deposited to 20 nm at 0.1 nm/sec on a glass substrate. The results of transmittance measurement are shown in FIG. 1A. Silver was further deposited to 10 nm at a deposition rate of 0.1 nm/sec on a thin film of A177. The results of transmittance measurement are shown in FIG. 1B. In FIG. 1B, the transmittance of 550 to 800 nm after metal deposition was "= 80%", and the formation of a metal film was suppressed.

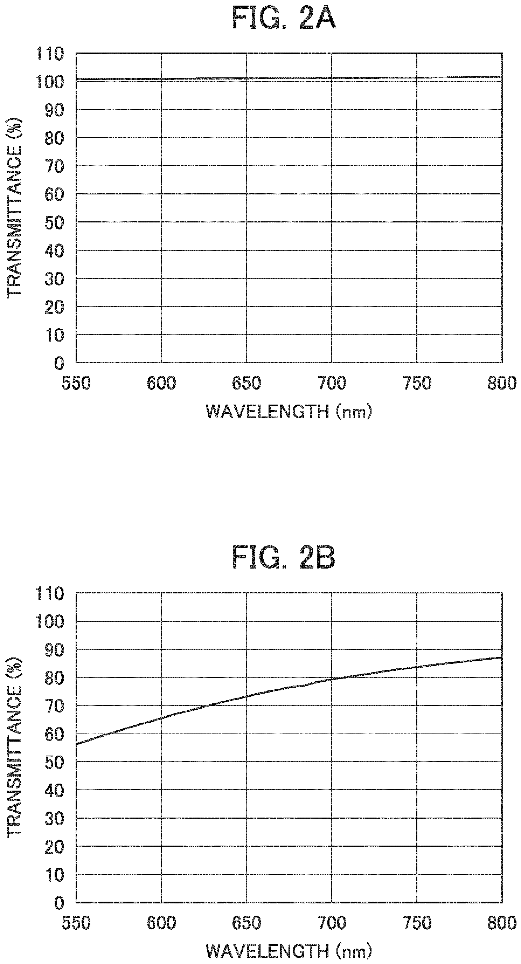

Example 9": Transmittance measurement of compound (A433) By a method similar to Example 1", the transmittance of compound (A433) was measured. The results of transmittance measurement before metal deposition and after metal deposition are respectively shown in FIGS. 2A and 2B. In FIG. 2B, the transmittance of 550 to 800 nm after metal deposition was "≧ 50%", and the formation of a metal film was suppressed.

### Comparative Example 1": Transmittance measurement of compound (X1)

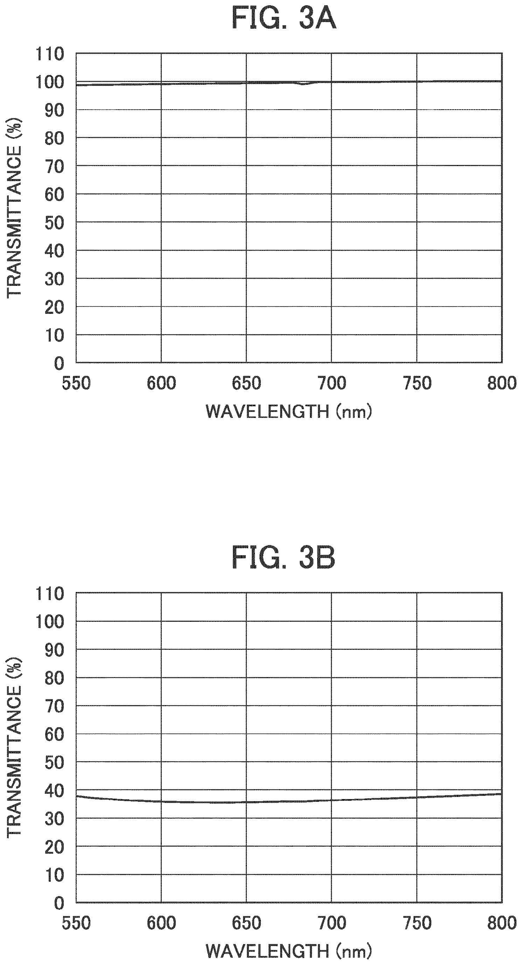

By a method similar to Example 1", the transmittance of compound (X1) was measured. The results of transmittance measurement before metal deposition and after metal deposition are respectively shown in FIGS. 3A and 3B. In FIG. 3B, the transmittance of 550 to 800 nm after metal deposition was "≧ 30%", and the formation of a metal film was suppressed.

### Comparative Example 5": Transmittance measurement of compound (X4)

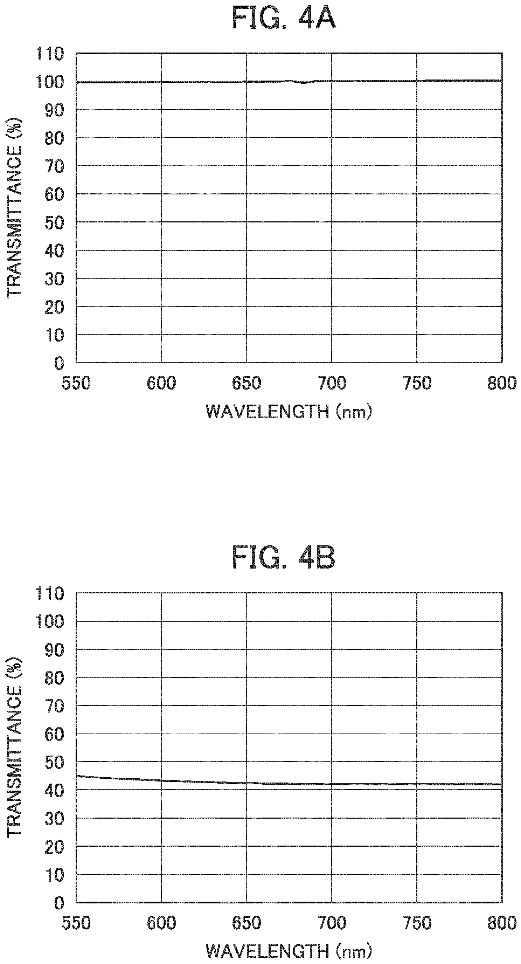

By a method similar to Example 1", the transmittance of compound (X4) was measured. The results of transmittance measurement before metal deposition and after metal deposition are respectively shown in FIGS. 4A and 4B. In FIG. 4B, the transmittance of 550 to 800 nm after metal deposition was "= 40%", and the formation of a metal film was suppressed.

By a method similar to Example 1", transmittance measurement was conducted on compounds A206, A236, A512, A559, A173, A569, A305, A176, A574, A580, A581, A582, A584, A526, A591, A390, A608, A663, A597, X3 and X5. The results thereof are shown in Table 1. Among the carbon atom number forming the aromatic ring, and the carbon atom number forming the heteroaromatic ring, the proportions of carbon atom number directly bonding with fluorine atom (abbreviated as C_{Ar-F}) are shown in Table 43.

**[Table 43]**

| | Compound number | C_{Ar}-F | Transmittance after metal vapor deposition |
|---|---|---|---|
| Example1" | A177 | 66.7% | ≥80 % |
| Example2" | A206 | 44.4% | ≥50% |
| Example3" | A236 | 44.4% | ≥60% |
| Example5" | A512 | 66.70 | ≥80% |
| Example7" | A559 | 61.5% | ≥80% |
| Example8" | A173 | 73.3% | ≥80% |
| Example9" | A433 | 44.4% | ≥50% |
| Example10" | A569 | 60.0% | ≥80% |
| Example11" | A305 | 57.1% | ≥80% |
| Example13" | A176 | 72.2% | ≥80% |
| Example14" | A574 | 53.3% | ≥80% |
| Example15" | A580 | 66.7% | ≥80% |
| Example18" | A581 | 66.7% | ≥80% |
| Example20" | A582 | 66.7% | ≥80% |
| Example21" | A584 | 44.4% | ≥80% |
| Example23" | A526 | 66.7% | ≥60% |
| Example25" | A591 | 72.2% | ≥80% |
| Example32" | A390 | 47.1% | ≥80% |
| Example40" | A608 | 13.3% | ≥50% |
| Example51" | A663 | 47.1% | ≥60% |
| Reference Example47" | A597 | 40.0% | ≥40% |
| Comparative Example1" | X1 | 0.0% | ≥30% |
| Comparative Example4" | X3 | 9.5% | ≥40% |
| Comparative Example5" | X4 | 9.5% | ≥40% |
| Comparative Example7" | X5 | 10.0% | ≥30% |

### 2. Metal patterning material without amine substituent (example belonging to second embodiment)

By a method similar to Example 1", transmittance measurement was conducted on compounds B51, B26, B104, B90, Y1, Y2 and Y3. The results thereof are shown in Table 44. Among the carbon atom number forming the aromatic ring, and the carbon atom number forming the heteroaromatic ring, the proportions of carbon atom number directly bonding with fluorine atom (abbreviated as C_{Ar-F}) are noted in Table 44. The proportions of fluorine atom (abbreviated as C_{A11-F}) relative to carbon atom number in a molecule are noted in Table 44.

**[Table 44]**

| | Compound number | C_{Ar}-F | C_{All}-F | Transmittance after metal vapor deposition |
|---|---|---|---|---|
| Example29" | B51 | 70.0% | 70.0% | ≥80% |
| Example44" | B26 | 50.0% | 50.0% | ≥80% |
| Example45" | B104 | 66.7% | 78.9% | ≥60% |
| Example79" | B90 | 27.8% | 55.0% | ≥50% |
| Comparative Example10" | Y1 | 10.0% | 10.0% | ≥30% |
| Comparative Example11" | Y2 | 0.0% | 13.0% | ≥30% |
| Comparative Example12" | Y3 | 22.2% | 22.2% | ≥40% |

Although the present invention has been explained in detail by referencing specific embodiments, it is clear to those skilled in the art that it is possible to apply various modifications and improvements without departing from the gist and scope of the present invention.

It should be noted that the disclosures, claims, drawings and entire contents of abstracts of Japanese Patent Application No. 2020-136094 filed on August 12, 2020, Japanese Patent Application No. 2021-021921 filed on February 15, 2021, and Japanese Patent Application No. 2021-112394 filed on July 6, 2021 are cited herein, and incorporated as disclosures of the specification of the present invention.

## Claims

1. A metal patterning material, comprising a compound having, in a molecule: an aromatic ring and/or heteroaromatic ring, a fluorine atom, and at least one tertiary amine,
wherein the aromatic ring is at least one selected from the group consisting of a monocyclic aromatic ring, a linked aromatic ring, and a condensed aromatic ring having from 6 to 15 carbon atoms,
and is free of condensed aromatic rings having 16 or more carbon atoms,
wherein a proportion of carbon atom number directly binding with the fluorine atom among a carbon atom number forming the aromatic ring and a carbon atom number forming the heteroaromatic ring is at least 10%,
wherein molecular weight is at least 500 and no more than 3,000, and
wherein a glass transition temperature is 60°C or higher.

2. The metal patterning material according to claim 1, wherein the tertiary amine is an aromatic tertiary amine, and
wherein a molecular weight is at least 500 and no more than 2,000.

3. The metal patterning material according to claim 1 or 2 represented by Formula (1), (2) or (3): 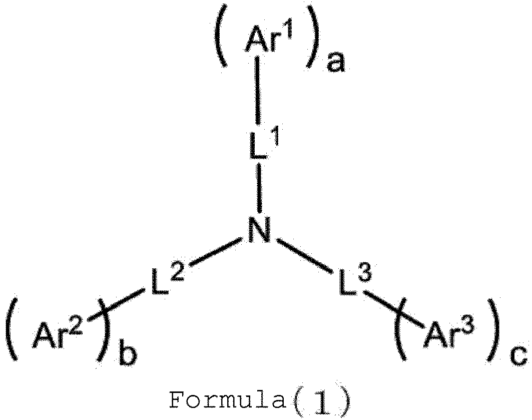 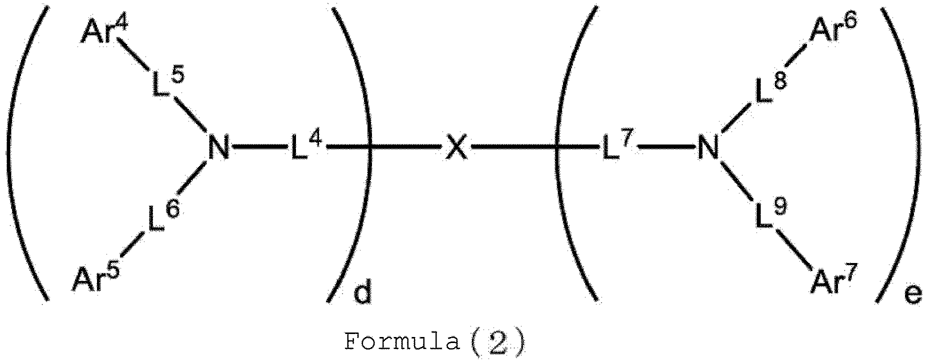 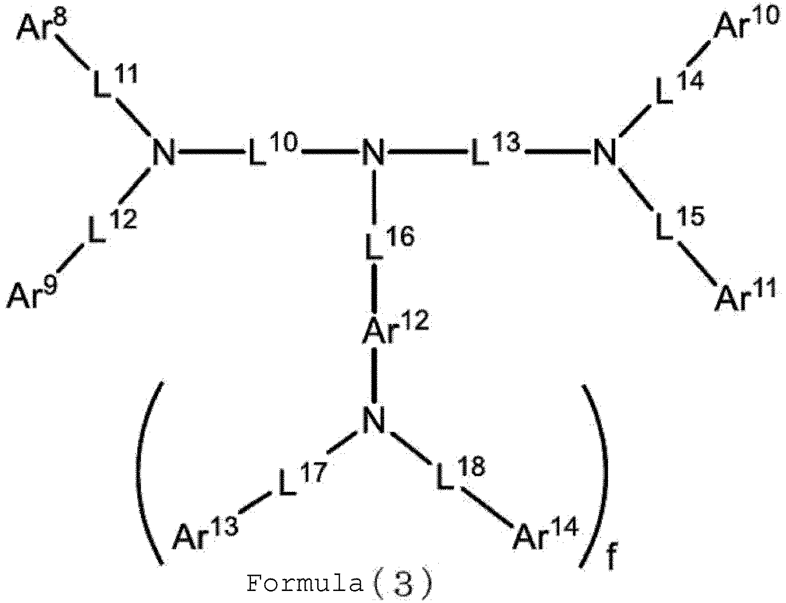 wherein
Ar¹ to Ar¹⁴ each independently represent
a monocyclic, linked or condensed aromatic hydrocarbon group having 6 to 25 carbon atoms which may be substituted, or
a monocyclic, linked or condensed heteroaromatic group having 3 to 25 carbon atoms which may be substituted;
L¹ to L¹⁸ each independently represent
a monocyclic, linked or condensed divalent aromatic hydrocarbon group having 6 to 25 carbon atoms which may be substituted,
a monocyclic, linked or condensed divalent heteroaromatic group having 3 to 25 carbon atoms which may be substituted, or
a single bond;
X represents
a monocyclic, linked or condensed divalent to tetravalent aromatic hydrocarbon group having 6 to 25 carbon atoms which may be substituted,
a monocyclic, linked or condensed divalent to tetravalent heteroaromatic group having 3 to 25 carbon atoms which may be substituted,
a linear, branched or cyclic divalent to tetravalent alkyl group having 1 to 10 carbon atoms which may be substituted, or
a divalent to tetravalent silicon atom which may be substituted;
a, b and c each independently represent an integer of 1 to 3;
d and e each independently represent an integer of 1 or 2; and
f represents an integer of 0 or 1.

4. The metal patterning material according to claim 3,
wherein, when Ar¹ to Ar¹⁴ are substituted aromatic hydrocarbon groups or substituted heteroaromatic groups, these groups are each independently substituted by at least one selected from the group consisting of:
a linear, branched or cyclic alkyl group having 1 to 18 carbon atoms which may be substituted by a fluorine atom,
a linear, branched or cyclic alkoxy group having 1 to 18 carbon atoms which may be substituted by a fluorine atom,
an aromatic hydrocarbon group having 6 to 20 carbon atoms which may be substituted by a fluorine atom,
a heteroaromatic group having 3 to 20 carbon atoms which may be substituted by a fluorine atom,
a cyano group,
a fluorine atom, and
a deuterium atom;
when L¹ to L¹⁸ are a substituted divalent aromatic hydrocarbon group or substituted divalent heteroaromatic group, these groups are each independently
a linear, branched or cyclic alkyl group having 1 to 18 carbon atoms which may be substituted by a fluorine atom,
a linear, branched or cyclic alkoxy group having 1 to 18 carbon atoms which may be substituted by a fluorine atom,
an aromatic hydrocarbon group having 6 to 20 carbon atoms which may be substituted by a fluorine atom,
a heteroaromatic group having 3 to 20 carbon atoms which may be substituted by a fluorine atom,
a cyano group,
a fluorine atom, and
a deuterium atom; and
when X is a substituted divalent to tetravalent aromatic hydrocarbon group, substituted divalent to tetravalent heteroaromatic group, substituted divalent to tetravalent alkyl group, or substituted divalent to tetravalent silicon atom, these groups are each independently represented by at least one selected from the group consisting of:
a linear, branched or cyclic alkyl group having 1 to 18 carbon atoms which may be substituted by a fluorine atom,
a linear, branched or cyclic alkoxy group having 1 to 18 carbon atoms which may be substituted by a fluorine atom,
an aromatic hydrocarbon group having 6 to 20 carbon atoms which may be substituted by a fluorine atom,
a heteroaromatic group having 3 to 20 carbon atoms which may be substituted by a fluorine atom,
a cyano group,
a fluorine atom, and
a deuterium atom.

5. The metal patterning material according to claim 1 or 2 represented by Formula (4), (5) or (6), 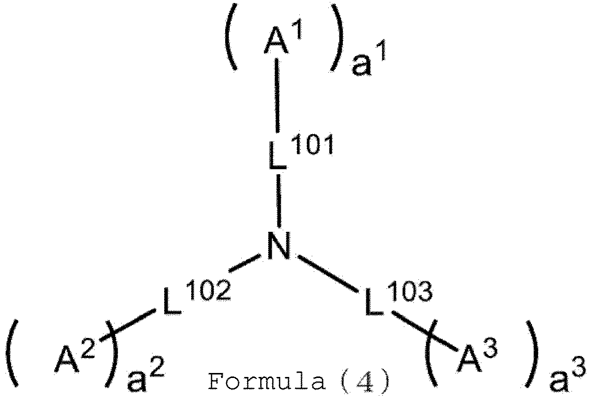 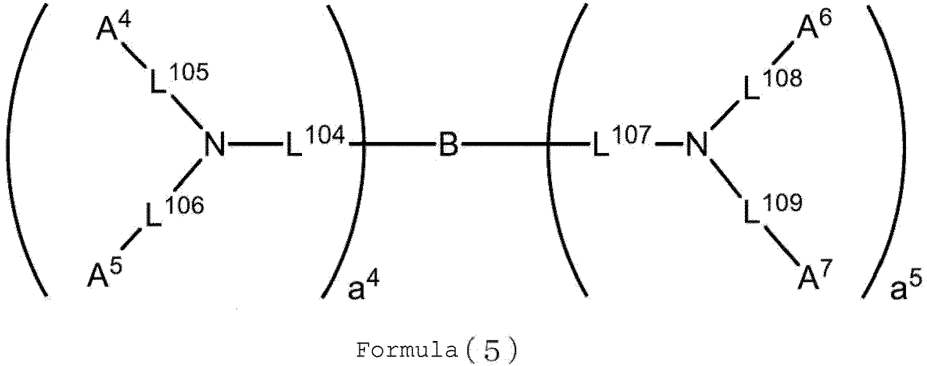 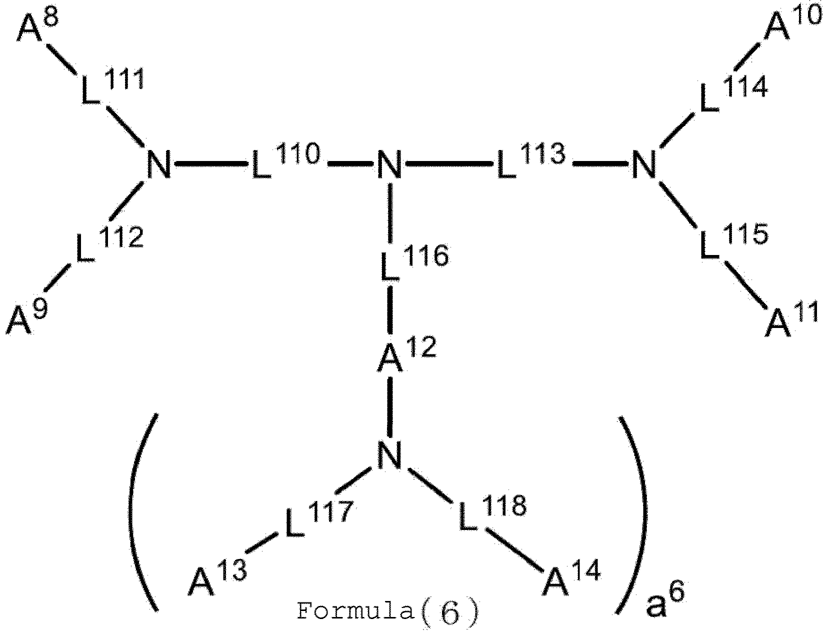 wherein
a¹, a² and a³ each independently represent an integer of 1 to 3;
a⁴ and a⁵ each independently represent an integer of 1 or 2;
a⁶ represents an integer of 0 or 1;
A¹ to A¹⁴ each independently represent
a monocyclic, linked or condensed aromatic hydrocarbon group having 6 to 25 carbon atoms which may be substituted,
a monocyclic, linked or condensed heteroaromatic group having 3 to 25 carbon atoms which may be substituted, or
a cyclic alkyl group having 3 to 25 carbon atoms which may be substituted;
provided that
at least one of A¹ to A³ is each independently a cyclic alkyl group having 3 to 25 carbon atoms which may be substituted,
at least one of A⁴ to A⁷ is each independently a cyclic alkyl group having 3 to 25 carbon atoms which may be substituted;
when a⁶ is 0, at least one of A⁸ to A¹¹ is each independently a cyclic alkyl group having 3 to 25 carbon atoms which may be substituted;
when a⁶ is 1, at least one of A⁸ to A¹⁴ is each independently a cyclic alkyl group having 3 to 25 carbon atoms which may be substituted;
L¹⁰¹ to L¹¹⁸ each independently represent
a monocyclic, linked or condensed divalent aromatic hydrocarbon group having 6 to 25 carbon atoms which may be substituted,
a monocyclic, linked or condensed divalent heteroaromatic group having 3 to 25 carbon atoms which may be substituted, or
a single bond; and
B represents
a monocyclic, linked or condensed divalent to tetravalent aromatic hydrocarbon group having 6 to 25 carbon atoms which may be substituted,
a monocyclic, linked or condensed divalent to tetravalent heteroaromatic group having 3 to 25 carbon atoms which may be substituted,
a linear, branched or cyclic divalent to tetravalent alkyl group having 1 to 10 carbon atoms which may be substituted, or
a divalent to tetravalent silicon atom which may be substituted.

6. An amine compound represented by Formula (7) or (8), 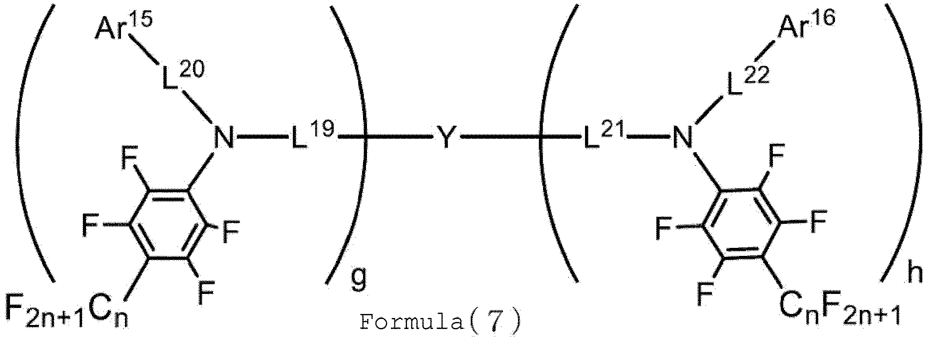 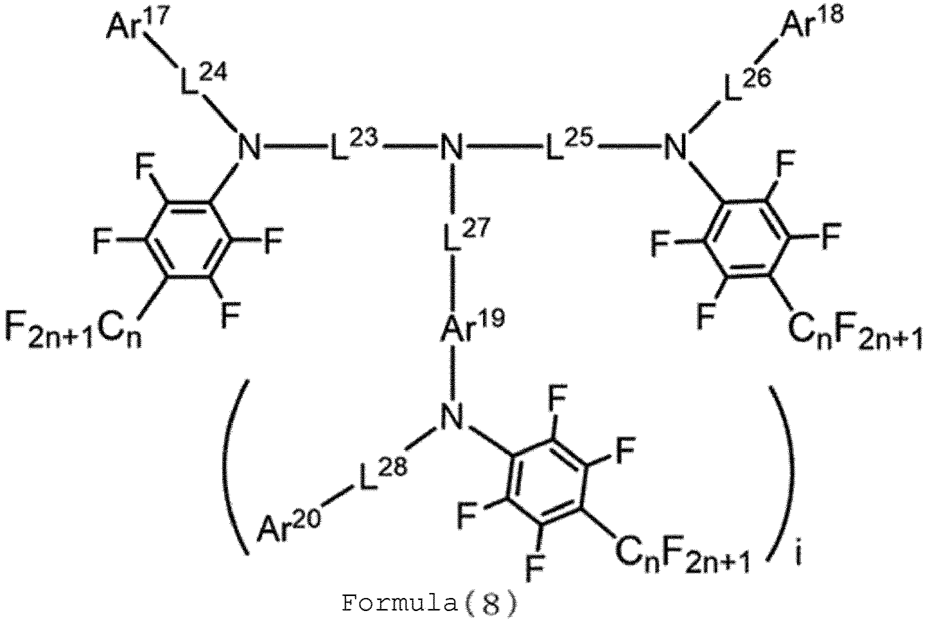 wherein
Ar¹⁵ to Ar²⁰ each independently represent
a monocyclic, linked or condensed aromatic hydrocarbon group having 6 to 25 carbon atoms which may be substituted, or
a monocyclic, linked or condensed heteroaromatic group having 3 to 25 carbon atoms which may be substituted;
L¹⁹ to L²⁸ each independently represent
a monocyclic, linked or condensed divalent aromatic hydrocarbon group having 6 to 25 carbon atoms which may be substituted,
a monocyclic, linked or condensed divalent heteroaromatic group having 3 to 25 carbon atoms which may be substituted, or
a single bond;
Y represents
a monocyclic, linked or condensed divalent to tetravalent aromatic hydrocarbon group having 6 to 25 carbon atoms which may be substituted,
a monocyclic, linked or condensed divalent to tetravalent heteroaromatic group having 3 to 25 carbon atoms which may be substituted,
a linear, branched or cyclic divalent to tetravalent alkyl group having 1 to 10 carbon atoms which may be substituted, or
a divalent to tetravalent silicon atom which may be substituted;
n represents an integer of 1 to 18;
g and h each independently represents an integer of 1 or 2; and
i represents an integer of 0 or 1.

7. The amine compound according to claim 6, wherein
when Ar¹⁵ to Ar²⁰ are substituted aromatic hydrocarbon groups, or substituted heteroaromatic groups, these groups are each independently substituted by at least one selected from the group consisting of:
a linear, branched or cyclic alkyl group having 1 to 18 carbon atoms which may be substituted by a fluorine atom,
a linear, branched or cyclic alkoxy group having 1 to 18 carbon atoms which may be substituted by a fluorine atom,
an aromatic hydrocarbon group having 6 to 20 carbon atoms which may be substituted by a fluorine atom,
a heteroaromatic group having 3 to 20 carbon atoms which may be substituted by a fluorine atom,
a cyano group,
a fluorine atom, and
a deuterium atom;
when L¹⁹ to L²⁸ are substituted divalent aromatic hydrocarbon groups, or substituted divalent heteroaromatic groups, these groups are each independently substituted by at least one selected from the group consisting of:
a linear, branched or cyclic alkyl group having 1 to 18 carbon atoms which may be substituted by a fluorine atom,
a linear, branched or cyclic alkoxy group having 1 to 18 carbon atoms which may be substituted by a fluorine atom,
an aromatic hydrocarbon group having 6 to 20 carbon atoms which may be substituted by a fluorine atom,
a heteroaromatic group having 3 to 20 carbon atoms which may be substituted by a fluorine atom,
a cyano group,
a fluorine atom, and
a deuterium atom;
when Y is a substituted divalent to tetravalent aromatic hydrocarbon group, substituted divalent to tetravalent heteroaromatic group, substituted divalent to tetravalent alkyl group, or substituted divalent to tetravalent silicon atom, these groups are each independently substituted by at least one selected from the group consisting of:
a linear, branched or cyclic alkyl group having 1 to 18 carbon atoms which may be substituted by a fluorine atom,
a linear, branched or cyclic alkoxy group having 1 to 18 carbon atoms which may be substituted by a fluorine atom,
an aromatic hydrocarbon group having 6 to 20 carbon atoms which may be substituted by a fluorine atom,
a heteroaromatic group having 3 to 20 carbon atoms which may be substituted by a fluorine atom,
a cyano group,
a fluorine atom, and
a deuterium atom.

8. The amine compound according to claim 6 or 7, wherein Y represents:
(xx) a phenylene group, biphenylene group, terphenylylene group, naphthylene group, fluorenylene group, spirobifluorenylene group, benzofluorenylene group, phenanthrene group, fluoranthenylene group, triphenylenylene group, anthracenediyl group, pyridylene group, pyrenediyl group, pyrimidinediyl group, triazinediyl group, carbazolediyl group, benzofuryl group, benzothiophenediyl group, dibenzofuryl group, dibenzothiophenediyl group, adamantanediyl group, methylene group, silanediyl group, cyclohexylene group,
phenyltriyl group, biphenyltriyl group, terphenyltriyl group, naphthylyl group, fluorenetriyl group, spirobifluorenetriyl group, benzofluorenetriyl group, phenanthrenetriyl group, fluoranthenetriyl group, triphenylenetriyl group, anthracenetriyl group, pyrenetriyl group, pyridinetriyl group, pyrimidinetriyl group, triazinetriyl group, carbazoletriyl group, benzofurantriyl group, benzothiophenetriyl group, dibenzofurantriyl group, dibenzothiophenetriyl group, adamantanetriyl group, methanetriyl group, silanetriyl group, cyclohexanetriyl group,
phenyl tetrayl group, biphenyl tetrayl group, terphenyl tetrayl group, naphthyl tetrayl group, fluorene tetrayl group, spirobiflurene tetrayl group, benzofluorene tetrayl group, phenanthrene tetrayl group, fluoranthene tetrayl group, triphenylene tetrayl group, anthracene tetrayl group, pyrene tetrayl group, pyridine tetrayl group, pyrimidine tetrayl group, carbazole tetrayl group, benzofuran tetrayl group, benzothiophene tetrayl group, dibenzofuran tetrayl group, dibenzothiophene tetrayl group, adamantane tetrayl group, methane tetrayl group, silane tetrayl group, or cyclohexane tetrayl group; or
(xxi) a group in which the group represented by (xx) is substituted with one or more groups selected from the group consisting of: a methyl group, an ethyl group, a methoxy group, an ethoxy group, a trifluoromethyl group, a trifluoromethoxy group, a perfluoroalkyl group having 2 to 10 carbon atoms, a perfluoroalkoxy group having 2 to 10 carbon atoms, a cyano group, a deuterium atom, a fluorine atom, a phenyl group optionally substituted with a fluorine atom, a biphenylyl group optionally substituted with a fluorine atom, a naphthyl group optionally substituted with a fluorine atom, a phenanthryl group optionally substituted with a fluorine atom, a pyridyl group optionally substituted with a fluorine atom, a carbazolyl group optionally substituted with a fluorine atom, a dibenzothienyl group optionally substituted with a fluorine atom, and a dibenzofuranyl group optionally substituted with a fluorine atom.

9. The amine compound according to any one of claims 6 to 8, wherein n is 1.

10. An amine compound represented by Formula (9) or (10), 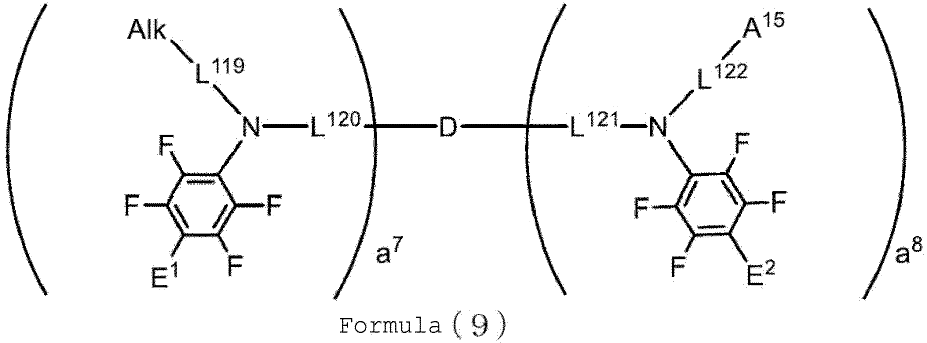 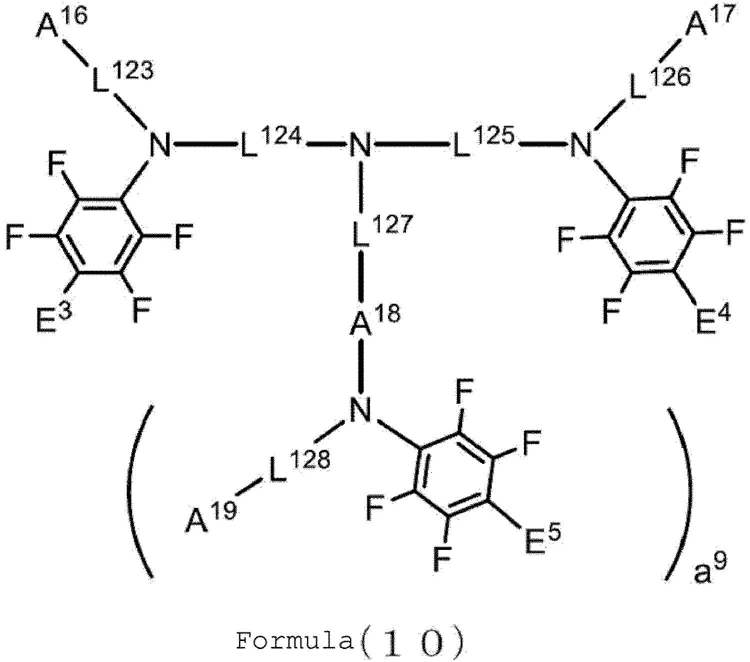 wherein
a⁷ represents an integer of 1 or 2;
a⁸ represents an integer of 0 to 2;
a⁹ represents an integer of 0 or 1;
Alk represents a cyclic alkyl group having 3 to 25 carbon atoms which may be substituted;
A¹⁵ to A¹⁹ each independently represent
a monocyclic, linked or condensed aromatic hydrocarbon group having 6 to 25 carbon atoms which may be substituted,
a monocyclic, linked or condensed heteroaromatic group having 3 to 25 carbon atoms which may be substituted, or
a cyclic alkyl group having 3 to 25 carbon atoms which may be substituted;
provided that
when a⁹ is 0, at least one of A¹⁶ to A¹⁸ are each independently a cyclic alkyl group having 3 to 25 carbon atoms which may be substituted, and
when a⁹ is 1, at least one of A¹⁶ to A¹⁹ are each independently a cyclic alkyl group having 3 to 25 carbon atoms which may be substituted
L¹¹⁹ to L¹²⁸ each independently represent:
a monocyclic, linked or condensed divalent aromatic hydrocarbon group having 6 to 25 carbon atoms which may be substituted,
a monocyclic, linked or condensed divalent heteroaromatic group having 3 to 25 carbon atoms which may be substituted, or
a single bond;
D represents
a monocyclic, linked or condensed monovalent to tetravalent aromatic hydrocarbon group having 6 to 25 carbon atoms which may be substituted,
a monocyclic, linked or condensed monovalent to tetravalent aromatic hydrocarbon group having 3 to 25 carbon atoms which may be substituted,
a monocyclic, linked or condensed monovalent to alkyl group having 1 to 10 carbon atoms which may be substituted, or
a monovalent to tetravalent silicon atom which may be substituted;
E¹ to E⁵ each independently represent
a fluorine atom, or
a linear, branched or cyclic alkyl group having 1 to 18 carbon atoms which may be substituted by a fluorine atom.

11. A metal patterning material comprising a compound having, in a molecule, an aromatic ring and/or heteroaromatic ring, and a fluorine atom,
wherein a proportion of carbon atom number directly binding with the fluorine atom among carbon atom number forming the aromatic ring and carbon atom number forming the heteroaromatic ring is at least 10%,
wherein a proportion of fluorine atoms relative to carbon atoms in the molecule is at least 50%,
wherein molecular weight is at least 500 and no more than 3000, and
wherein glass transition temperature is at least 60°C.

12. The metal patterning material according to claim 11 represented by Formula (11) 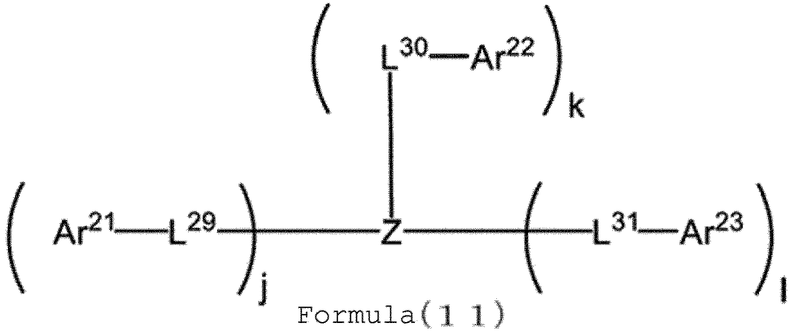 wherein in Formula (11),
Ar²¹, Ar²² and Ar²³ each independently represent
a monocyclic, linked or condensed aromatic hydrocarbon group having 6 to 25 carbon atoms which may be substituted, or
a monocyclic, linked or condensed heteroaromatic group having 3 to 25 carbon atoms which may be substituted;
L²⁹, L³⁰ and L³¹ each independently represent
a monocyclic, linked or condensed divalent aromatic hydrocarbon group having 6 to 25 carbon atoms which may be substituted,
a monocyclic, linked or condensed divalent heteroaromatic group having 3 to 25 carbon atoms which may be substituted, or
a single bond; and
j, k and 1 each independently represent an integer of 0 to 6;
(j+k+l) is at least 2 and no more than 6; and
Z represents
a monocyclic, linked or condensed divalent to hexavalent aromatic hydrocarbon group having 6 to 25 carbon atoms which may be substituted,
a monocyclic, linked or condensed divalent to hexavalent heteroaromatic group having 3 to 25 carbon atoms which may be substituted,
a monocyclic, linked or condensed divalent to hexavalent alkyl group having 1 to 10 carbon atoms which may be substituted, or
a divalent to tetravalent silicon atom.

13. The metal patterning material according to claim 12, wherein,
when Ar²¹ to Ar²³ are a substituted aromatic hydrocarbon groups or substituted heteroaromatic groups, these groups are each independently substituted by at least one selected from the group consisting of:
a linear, branched or cyclic alkyl group having 1 to 18 carbon atoms which may be substituted by a fluorine atom,
a linear, branched or cyclic alkoxy group having 1 to 18 carbon atoms which may be substituted by a fluorine atom,
an aromatic hydrocarbon group having 6 to 20 carbon atoms which may be substituted by a fluorine atom,
a heteroaromatic group having 3 to 20 carbon atoms which may be substituted by a fluorine atom,
a cyano group,
a fluorine atom, and
a deuterium atom;
when L²⁹ to L³¹ are substituted divalent aromatic hydrocarbon groups, or substituted divalent heteroaromatic groups, these groups are each independently represented by
a linear, branched or cyclic alkyl group having 1 to 18 carbon atoms which may be substituted by a fluorine atom,
a linear, branched or cyclic alkoxy group having 1 to 18 carbon atoms which may be substituted by a fluorine atom,
an aromatic hydrocarbon group having 6 to 20 carbon atoms which may be substituted by a fluorine atom,
a heteroaromatic group having 3 to 20 carbon atoms which may be substituted by a fluorine atom,
a cyano group,
a fluorine atom, and
a deuterium atom;
when Z is a substituted divalent to hexavalent aromatic hydrocarbon group, a substituted divalent to hexavalent heteroaromatic group, a substituted divalent to hexavalent alkyl group or a divalent to tetravalent silicon atom, these groups are each independently substituted by at least one selected from the group consisting of:
a linear, branched or cyclic alkyl group having 1 to 18 carbon atoms which may be substituted by a fluorine atom,
a linear, branched or cyclic alkoxy group having 1 to 18 carbon atoms which may be substituted by a fluorine atom,
an aromatic hydrocarbon group having 6 to 20 carbon atoms which may be substituted by a fluorine atom,
a heteroaromatic group having 3 to 20 carbon atoms which may be substituted by a fluorine atom,
a cyano group,
a fluorine atom, and
a deuterium atom.

14. The metal patterning material according to claim 12 or 13, wherein Ar²¹, Ar²² and Ar²³
(xxxi) a phenyl group, a biphenylyl group, a terphenylyl group, a naphthyl group, a fluorenyl group, a spirobifluorenyl group, a benzofluorenyl group, a phenanthryl group, a fluoranthenyl group, a triphenylenyl group, an anthryl group, a pyrenyl group, a pyridyl group, a carbazolyl group, a benzofuranyl group, a benzothienyl group, a dibenzofuranyl group, or a dibenzothienyl group; or
(xxxii) a group in which the group represented by (xxxi) is substituted with one or more groups selected from the group consisting of: a methyl group, an ethyl group, a methoxy group, an ethoxy group, a trifluoromethyl group, a trifluoromethoxy group, an adamantyl group, a cyclohexyl group, a perfluoroalkyl group having 2 to 10 carbon atoms, a perfluoroalkoxy group having 2 to 10 carbon atoms, a cyano group, a deuterium atom, a phenyl group optionally substituted with a fluorine atom, a biphenyl group optionally substituted with a fluorine atom, a naphthyl group optionally substituted with a fluorine atom, a phenanthryl group optionally substituted with a fluorine atom, a pyridyl group optionally substituted with a fluorine atom, a carbazolyl group optionally substituted with a fluorine atom, a dibenzothienyl group optionally substituted with a fluorine atom, and a dibenzofuranyl group optionally substituted with a fluorine atom.

15. The metal patterning material according to any one of claims 12 to 14, wherein L29, L30 and L31 are each independently
(xxxiii) a phenylene group, a biphenylylene group, a terphenylene group, a naphthylene group, a pyridylene group, or a fluorenylene group;
(xxxiv) a group in which the group represented by (xxxiii) is substituted with one or more groups selected from the group consisting of: a methyl group, an ethyl group, a methoxy group, an ethoxy group, a trifluoromethyl group, a trifluoromethoxy group, a perfluoroalkyl group having 2 to 10 carbon atoms, a perfluoroalkoxy group having 2 to 10 carbon atoms, a cyano group, a deuterium atom, a fluorine atom, a phenyl group optionally substituted with a fluorine atom, a biphenylyl group optionally substituted with a fluorine atom, a naphthyl group optionally substituted with a fluorine atom, a phenanthryl group optionally substituted with a fluorine atom, a pyridyl group optionally substituted with a fluorine atom, a carbazolyl group optionally substituted with a fluorine atom, a dibenzothienyl group optionally substituted with a fluorine atom, and a dibenzofuranyl group optionally substituted with a fluorine atom; or
(xxxv) a single bond.

16. The metal patterning material according to any one of claims 12 to 15, wherein Z is
(xxxvi) a monovalent phenyl group, a biphenylyl group, a terphenylyl group, a naphthyl group, a fluorenyl group, a spirobifluorenyl group, a benzofluorenyl group, a phenanthryl group, a fluoranthenyl group, a triphenylenyl group, an anthryl group, a pyrenyl group, a pyridyl group, a carbazolyl group, a benzofuranyl group, a benzothienyl group, a dibenzofuranyl group, a dibenzothienyl group, a cyclohexyl group, an adamantyl group, a methyl group, or a silyl group;
(xxxvii) a divalent phenylene group, a biphenylene group, a terphenylene group, a naphthylene group, a fluorenylene group, a spirobifluorenylene group, a benzofluorenylene group, a phenanetriene group, a fluoranthenylene group, a triphenylenylene group, an anthrylene group, a pyrenylene group, a pyridylene group, a carbazolylene group, a benzofuranylene group, a benzothienylene group, a dibenzofuranylene group, a dibenzothienylene group, a cyclohexylene group, an adamantylene group, a methylene group, or a silanediyl group,
(xxxviii) a trivalent phenyltriyl group, a biphenyltriyl group, a terphenyltriyl group, a naphthylyl group, a fluorenetriyl group, a spirobiflurenetriyl group, a benzofluorenetriyl group, a phenanthrenetriyl group, a fluoranthenetriyl group, a triphenylenetriyl group, an anthracenetriyl group, a pyrenetriyl group, a pyridinetriyl group, a carbazoletriyl group, a benzofurantriyl group, a benzothiophenetriyl group, a dibenzofurantriyl group, a dibenzothiophenetriyl group, a cyclohexanetriyl group, an adamantanetriyl group, a methanetriyl group, or a silanetriyl group;
(xxxix) a tetravalent phenyl tetrayl group, a biphenyl tetrayl group, a terphenyl tetrayl group, a naphthyl tetrayl group, a fluorene tetrayl group, a spirobifluorene tetrayl group, a benzofluorene tetrayl group, a phenanthrene tetrayl group, a fluoranthene tetrayl group, a triphenylene tetrayl group, anthracene tetrayl group, a pyrene tetrayl group, a pyridine tetrayl group, a carbazole tetrayl group, a benzofuran tetrayl group, a benzothiophene tetrayl group, a dibenzofuran tetrayl group, a dibenzothiophene tetrayl group, a cyclohexane tetrayl group, an adamantane tetrayl group, a methane tetrayl group, or a silane tetrayl group; or
(xxxx) a pentavalent phenylpentayl group, a biphenyl pentayl group, a terphenyl pentayl group, a naphthyl pentayl group, a fluorene pentayl group, spirobifluorene pentayl group, a benzofluorene pentayl group, a phenanthrene pentayl groups, a fluoranthene pentayl group, a triphenylene pentayl group, an anthracene pentayl group, a pyrene pentayl group, a pyridine pentayl group, a carbazole pentayl group, a benzofuran pentayl group, a benzothiophenepentayl group, a dibenzofuranepentayl group, a dibenzothiophenepentayl group, a cyclohexanepentayl group, or an adamantanepentayl group; or
(xxxxi) a hexavalent phenylhexayl group, a biphenylhexayl group, a terphenylhexayl group, a naphthylhexayl group, a fluorenehexayl group, a spirobifluorenehexayl group, a benzofluorenehexayl group, a phenanthrenehexayl group, a fluoranthenehexayl group, a triphenylenehexayl group, an anthracenehexayl group, a pyrenehexayl group, a pyridinehexayl group, a carbazolehexayl group, a benzofuranhexayl group, benzothiophenehexayl group, a dibenzofuranhexayl group, a dibenzothiophenehexayl group, a cyclohexanehexayl group, or an adamantanehexayl group; or
(xxxxii) a group in which the group represented by (xxxvi) to (xxxxi) is substituted with one or more groups selected from the group consisting of: a methyl group, an ethyl group, a methoxy group, an ethoxy group, a trifluoromethyl group, a trifluoromethoxy group, an adamantyl group, a cyclohexyl group, a perfluoroalkyl group having 2 to 10 carbon atoms, a perfluoroalkoxy group having 2 to 10 carbon atoms, a cyano group, a deuterium atom, a fluorine atom, a phenyl group optionally substituted with a fluorine atom, a biphenyl group optionally substituted with a fluorine atom, a naphthyl group optionally substituted with a fluorine atom, a phenanthryl group optionally substituted with a fluorine atom, a pyridyl group optionally substituted with a fluorine atom, a carbazolyl group optionally substituted with a fluorine atom, a dibenzothienyl group optionally substituted with a fluorine atom, and a dibenzofuranyl group optionally substituted with a fluorine atom.

17. The metal patterning material according to any one of claims 12 to 16, comprising in a molecule at least one perfluorophenyl group, perfluorotolyl group, perfluorodimethyl phenyl group or perfluorobiphenylyl group.

18. A method for forming a metal pattern, comprising:
forming an organic material pattern including the metal patterning material according to any one of claims 1 to 5 and 11 to 17, or the amine compound according to any one of claims 6 to 10; and
applying a metal material on a formation region of the organic material pattern and a non-formation region of the organic material pattern to form a metal pattern on the non-formation region.

19. An electronic device comprising the metal patterning material according to any one of claims 1 to 5 and 11 to 17, or the amine compound according to any one of claims 6 to 10.
